(19) Europäisches Patentamt
European Patent Office
Office européen des brevets

(11) Numéro de publication: **0 437 120 B1**

# (12) FASCICULE DE BREVET EUROPEEN

(45) Date de publication de fascicule du brevet: **02.08.95**

(51) Int. Cl.[6]: **C07D 211/76**, C07D 409/04, A61K 31/445

(21) Numéro de dépôt: **90402899.0**

(22) Date de dépôt: **16.10.90**

## (54) Nouveaux dérivés de la pyridone, leur procédé de préparation, les nouveaux intermédiaires obtenus, leur application à titre de médicaments et les compositions pharmaceutiques les renfermant.

(30) Priorité: **17.10.89 FR 8913545**

(43) Date de publication de la demande:
**17.07.91 Bulletin 91/29**

(45) Mention de la délivrance du brevet:
**02.08.95 Bulletin 95/31**

(84) Etats contractants désignés:
**AT BE CH DE DK ES FR GB GR IT LI LU NL SE**

(56) Documents cités:
**EP-A- 0 021 552**

(73) Titulaire: **ROUSSEL-UCLAF**
**35, Boulevard des Invalides**
**F-75007 Paris (FR)**

(72) Inventeur: **Clemence, François**
**2, rue Turgot**
**F-75009 Paris (FR)**
Inventeur: **Fortin, Michel**
**12, Passage Cottin**
**F-75018 Paris (FR)**
Inventeur: **Le Martret, Odile**
**42, Avenue de Versailles**
**F-75016 Paris (FR)**

(74) Mandataire: **Bourgouin, André et al**
**Département des Brevets**
**ROUSSEL UCLAF**
**111, route de Noisy**
**B.P. no 9**
**F-93230 Romainville (FR)**

EP 0 437 120 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de nouveaux dérivés de la pyridone, leur procédé de préparation, les nouveaux intermédiaires obtenus, leur application à titre de médicaments et les compositions pharmaceutiques les renfermant.

L'invention a pour objet les composés de formule (I) :

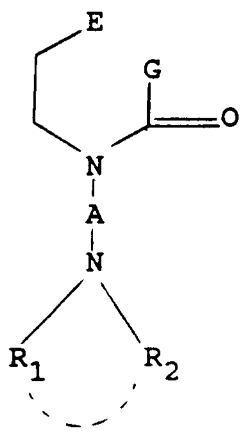

dans laquelle E et G sont tels que :

- soit E et G forment ensemble un groupement

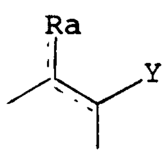

lequel représente l'un des groupements suivants :

a) 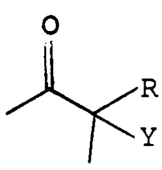 ou, le cas échéant, son tautomère 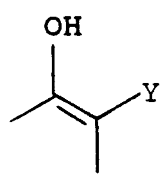 ,

dans lequel R représente un atome d'hydrogène, un radical alkyle linéaire ou ramifié renfermant de 1 à 6 atomes de carbone, un radical alkényle ou alkynyle linéaire ou ramifié renfermant de 2 à 6 atomes de carbone éventuellement substitué par un ou plusieurs substituants choisis soit parmi les radicaux aryle renfermant de 6 à 14 atomes de carbone, aryle substitué par un atome d'halogène, un radical hydroxy, trifluorométhyle, alkoxy renfermant de 1 à 6 atomes de carbone, nitro, amino et cyano, soit parmi les radicaux carboxy libre, estérifié ou salifié, acyle renfermant de 1 à 7 atomes de carbone, alcoxy renfermant de 1 à 6 atomes de carbone, hydroxy, amino, alkylamino, dialkylamino, acyloxy renfermant de 1 à 7 atomes de carbone, cyano, carbamoyle et les atomes d'halogènes ;

-- Y représente un radical aryle choisi parmi les radicaux aryles carbocycliques renfermant de 6 à 14 atomes de carbone, les radicaux aryles hétéromonocycliques renfermant 5 à 7 chaînons et les radicaux aryles hétérocycliques condensés, tous ces radicaux étant éventuellement substitués,

b) 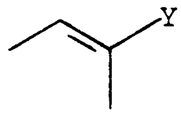 ou

c) 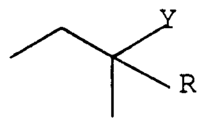

Y et R étant définis comme ci-dessus ;

- soit E représente un radical $-CO_2R_3$, dans lequel $R_3$ représente un atome d'hydrogène ou un radical alkyle linéaire ou ramifié (1 à 4 carbones) et G représente un groupement

$$-CH-Y$$
$$R$$

dans lequel Y et R sont définis comme ci-dessus ;

-- A représente :
  - soit un radical carbocyclique, éventuellement substitué,
  - soit le groupement

$$-CH-CH_2-$$
$$B$$

où B représente :
- un atome d'hydrogène,
- un radical aryle ou arylalkyle renfermant de 6 à 14 atomes de carbone, ces radicaux étant éventuellement substitués,
- un radical cycloalkyle renfermant de 3 à 7 atomes de carbone éventuellement substitué,
- un radical alkyle, alkényle, alkynyle ou alkyloxy, ces radicaux étant linéaires ou ramifiés, renfermant au plus 7 atomes de carbone et étant éventuellement substitués,

-- $R_1$ et $R_2$, identiques ou différents, représentent un atome d'hydrogène ou un radical alkyle, alkényle ou alkynyle, ces radicaux étant linéaires ou ramifiés, renfermant au plus 7 atomes de carbone et étant éventuellement substitués, ou bien $R_1$ et $R_2$ forment ensemble avec l'atome d'azote auquel ils sont liés un hétérocycle saturé ou insaturé à 5, 6 ou 7 chaînons comportant éventuellement un autre hétéroatome tel que l'oxygène, l'azote ou le soufre, cet hétérocycle étant éventuellement substitué,

lesdits produits de formule (I) étant sous toutes les formes isomères possibles racémiques, énantiomères et diastéréoisomères, ainsi que les sels d'addition avec les acides minéraux et organiques ou avec les bases desdits produits de formule (I).

Dans les produits de formule (I) et dans ce qui suit :

- le terme aryle, que peuvent représenter R, Y et B en particulier, désigne pour R un radical carbocyclique et pour Y et B un radical carbocyclique ou hétérocyclique, éventuellement substitué ; comme exemples de radical aryle, éventuellement substitué, on peut citer les radicaux phényle ; naphtyle, par exemple 1-naphtyle ; indényle ; un groupe hétérocyclique, saturé ou insaturé, à 5, 6 ou 7 chainons contenant au moins un hétéroatome choisi parmi le soufre, l'azote et l'oxygène, étant entendu que lorsque ce groupe hétérocyclique comporte plus d'un hétéroatome, les hétéroatomes de ce groupe hétérocyclique peuvent être identiques ou différents : comme exemples d'un tel radical aryle, on peut citer les radicaux thiényle tel que 2-thiényle et 3-thiényle, furyle tel que 2-furyle, pyridyle tel que 3-pyridyle, pyrimidyle, pyrrolyle, pyrrolyle N-substitué, par exemple N-méthyl pyrrolyle, thiazolyle, isothiazolyle, diazolyle, triazolyle, tétrazolyle, thiadiazolyle, thiatriazolyle, oxazolyle, oxadiazolyle, 3- ou 4-isoxazolyle, 3- ou 4-isoxazolyle substitué, par exemple, 3-aryl-5-méthyl isoxazol-4-yle, le groupe aryle étant par exemple, un groupe phényle ou halophényle ; des groupes

hétérocycliques condensés contenant au moins un hétéroatome choisi parmi le soufre, l'azote et l'oxygène, par exemple benzothiényle tel que 3-benzothiényle, benzofuryle, benzopyrrolyle, benzimidazolyle, benzoxazolyle, thionaphtyle, indolyle ou purinyle,

- le terme arylalkyle désigne un radical carbocyclique ou hétérocyclique, éventuellement substitué, dans lequel le radical alkyle inférieur peut contenir de 1 à 4 atomes de carbone, par exemple benzyle, phényléthyle, diphénylméthyle, triphénylméthyle, thiénylméthyle tel que 2-thiénylméthyle, furylméthyle tel que furfuryle, pyridylméthyle ou pyrrolylméthyle, les radicaux aryle et arylalkyle pouvant comporter un ou plusieurs substituants choisis, par exemple, dans le groupe formé par les halogènes, par exemple le chlore ou le brome, comme dans le groupe o-chloro-phényle ; hydroxy ; alkyle inférieur, par exemple méthyle, éthyle, ou également isopropyle ou tert-butyle ; alkényle ; alkynyle ; trihaloalkyle tel que, par exemple, trifluorométhyle ; trihaloalkylthio tel que, par exemple, trifluorométhylthio ; trihaloalkyloxy tel que, par exemple, trifluorométhoxy ; cyano ; nitro ; amino ; amino substitué tel que, par exemple, alkylamino, monoalkyl inférieur amino comme méthylamino ou éthylamino, dialkyl inférieur amino comme diméthylamino ; sulfamoyle ; alcanoyle inférieur, par exemple formyle, acétyle ; benzoyle ; alcanoyl inférieur amido ; alkyloxy inférieur, par exemple méthoxy, éthoxy, ou également isopropoxy ; alkyl inférieur thio, par exemple méthylthio, éthylthio ; carboxy libre ou estérifié, tel que par exemple méthoxycarbonyle ou éthoxycarbonyle ; carbamoyle ; carbamoyle substitué ; ou un radical aryle tel que, par exemple, phényle,
- le terme radical carbocyclique, que peut représenter A en particulier, désigne un radical cycloalkyle, cycloalkényle, cycloalcadiényle ou un groupe non aromatique, éventuellement substitué ;
  comme exemples de radical carbocyclique, éventuellement substitué, on peut citer les radicaux cyclohexyle, cyclopentyle, cyclobutyle, cyclopropyle, 1,3-cyclohexadiényle, 1,4-cyclohexadiényle, diméthyl-1,3 cyclohexényle.
- parmi les valeurs de A lorsque celui-ci représente un radical carbocyclique, on préfère les radicaux cycloalkyle de formule

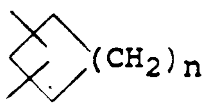

  dans laquelle n représente un entier de 0 à 4, de préférence les valeurs 2 et 3, éventuellement substitué,
- le terme radical cycloalkyle (3 à 7 carbones) que peut représenter B en particulier, désigne de préférence un radical cyclopropyle, cyclobutyle, cyclopentyle ou cyclohexyle,
- le terme radical alkyle linéaire ou ramifié, en particulier le terme alkyle inférieur (1 à 6 carbones), désigne de préférence les radicaux méthyle, éthyle, propyle ou isopropyle, mais peut également représenter un radical butyle, isobutyle, sec-butyle, tert- butyle ou pentyle,
- le terme radical alkényle linéaire ou ramifié, en particulier le terme alkényle inférieur (2 à 6 carbones), désigne de préférence un radical vinyle, allyle, 1-propényle, butényle ou pentényle,
- le terme radical alkynyle linéaire ou ramifié (2 à 6 carbones) désigne de préférence un radical éthynyle, propargyle, butynyle ou pentynyle,
- le terme radical alkyloxy linéaire ou ramifié, en particulier le terme alkyloxy inférieur (1 à 6 carbones), désigne de préférence un radical méthoxy ou éthoxy, mais peut aussi représenter un radical propoxy, isopropoxy, butoxy linéaire, secondaire ou tertiaire,
- le terme radical acyle (1 à 7 carbones) désigne de préférence un radical formyle, acétyle, propionyle ou benzoyle,
- dans le terme radical acyloxy (1 à 7 carbones), acyle désigne de préférence l'un des radicaux nommés ci-dessus.

Les radicaux carbocycliques comme par exemple cycloalkyle et les radicaux alkyle, alkényle, alkynyle, ou alkyloxy peuvent ne pas être substitués ou porter un ou plusieurs substituants choisis, par exemple, dans le groupe formé par les atomes d'halogène, tel que chloro ou bromo, comme dans, par exemple, le groupe 2-bromoéthyle ; les radicaux hydroxy ; alkyle ; alkényle ; alkynyle ; aryle, par exemple phényle ou naphtyle ; arylalkyle, par exemple benzyle ou phénéthyle ; cycloalkyle, par exemple cyclopropyle, cyclopentyle ou cyclohexyle ; alkyloxy par exemple méthoxy, éthoxy, propoxy ou isopropoxy comme dans par exemple les groupes méthoxyméthyle ou 1-éthoxyéthyle ; aryloxy, par exemple phénoxy ; aralkyloxy, par exemple benzyloxy ; mercapto ; alkylthio, par exemple méthylthio ou éthylthio ; arylthio ; (arylalkyl) thio

; amino comme dans, par exemple, le groupe 2-aminoéthyle ; amino substitué tel que, par exemple, monoalkylamino, comme méthylamino ou éthylamino, dialkylamino, comme diméthylamino ; nitro ; cyano ; azido ; carboxy ; carboxy estérifié, par exemple méthoxycarbonyle ou éthoxycarbonyle ; formyle ; acyle, par exemple acétyle, propionyle ou benzoyle ; acyloxy, par exemple acétoxy ou propionyloxy ; cyano ; phtalimido ; acylamino, par exemple acétamido ou benzamido ; alkyloxycarbonylamino, par exemple méthoxycarbonylamino ou éthoxycarbonylamino ; ou (arylalkyl) oxycarbonylamino, par exemple benzyloxy-carbonylamino.

Lorsque $R_1$ et $R_2$ forment ensemble avec l'atome d'azote auquel ils sont liés un hétérocycle, il s'agit, par exemple, d'un cycle pyrrolidino, pipéridino, morpholino, pipérazinyle.

Le second atome d'azote que peut comporter l'hétérocycle formé par $R_1$ et $R_2$ peut être substitué, par exemple, par un radical alkyle ou alkyloxy linéaire ou ramifié renfermant de 1 à 5 atomes de carbone, un radical phényle ou benzyle, ces radicaux pouvant eux-même être substitués par les substituants déjà mentionnés ci-dessus pour les radicaux aryle et arylalkyle : on peut citer, comme exemples, les radicaux méthylpipérazinyle, éthylpipérazinyle, propylpipérazinyle, phénylpipérazinyle ou benzylpipérazinyle.

Dans les produits de formule (I) et dans ce qui suit :

- le terme atome d'halogène désigne de préférence l'atome de chlore, mais peut aussi représenter un atome de fluor, de brome ou d'iode,
- le terme radical monoalkyl- ou dialkylamino désigne de préférence les radicaux monoalkyl- ou dialkylamino dans lesquels les radicaux alkyles linéaires ou ramifiés renferment de 1 à 5 atomes de carbone et en particulier des radicaux méthyle, éthyle ou isopropyle,
- le terme radical acyle ayant de 2 à 6 atomes de carbone désigne de préférence un radical acétyle, propionyle, butyryle ou benzoyle, mais également un radical valéryle, hexanoyle, acryloyle, crotonoyle ou carbamoyle,
- le terme carboxy estérifié désigne de préférence un groupe alkyloxy inférieur carbonyle tel que méthoxycarbonyle, éthoxycarbonyle ou encore un groupe benzyloxycarbonyle,
- le radical carbamoyle substitué désigne un groupe N-monoalkyl inférieur carbamoyle, par exemple N-méthylcarbamoyle, N-éthylcarbamoyle ; un groupe N,N-dialkyl inférieur carbamoyle, par exemple N,N-diméthylcarbamoyle, N,N-diéthylcarbamoyle ; un groupe N-(hydroxyalkyl inférieur) carbamoyle, par exemple N-(hydroxyméthyl) carbamoyle, N-(hydroxyéthyl) carbamoyle ; un groupe carbamoylalkyle inférieur, par exemple carbamoylméthyle, carbamoyléthyle.

Parmi les substituants que peut comporter le radical aryle, et plus particulièrement le radical phényle que peuvent représenter, par exemple, R, Y et B, peuvent être cités, par exemple, les atomes d'halogène, tels que chloro et bromo ; les radicaux hydroxy ; alkyle, par exemple méthyle, éthyle, isopropyle ou tert-butyle ; alkényle ; alkynyle ; alkyloxy, par exemple méthoxy, éthoxy ou isopropoxy ; radicaux alkyle, alkényle, alkynyle ou alkyloxy substitués par un ou plusieurs radicaux, identiques ou différents, choisis, par exemple, dans le groupe formé par les radicaux hydroxy, alkyle et alkyloxy linéaire ou ramifié, par exemple méthyle, éthyle, tert-butyle, méthoxy, éthoxy, isopropoxy ; amino substitué, tel que monoalkyl- et dialkylamino, par exemple méthylamino, éthylamino ou diméthylamino.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldi-sulfoniques tels que par exemple l'acide méthanedisulfonique, l'acide alpha, bêta-éthanedisulfonique, les acides arylmonosulfoniques tels que l'acide benzènesulfonique et les acides aryldisulfoniques tel que l'acide paratoluène sulfonique.

Les sels de bases peuvent être notamment les sels de sodium, de potassium, de lithium, de calcium, de magnésium ou d'ammonium, les sels de bases organiques, par exemple de méthylamine, propylamine, triméthylamine, diéthylamine, triéthylamine, N,N-diméthyléthanolamine, tris (hydroxyméthyl) amino méthane, d'éthanolamine, de pyridine, picoline, dicyclohexylamine, morpholine, benzylamine, procaïne, lysine, d'arginine, d'histidine ou de N-méthylglucamine.

Lorsque A représente le groupement

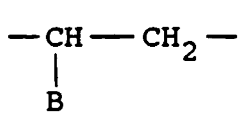

,

les produits de formule (I) peuvent se présenter sous l'une ou l'autre des deux formes suivantes :

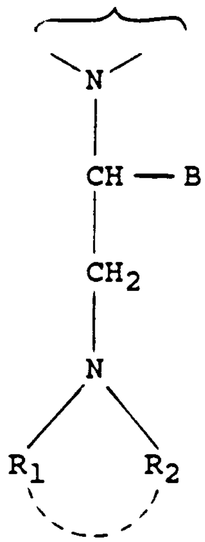

ou

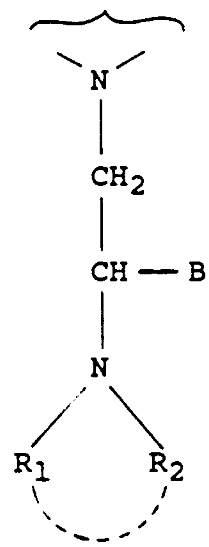

Lorsque A représente un radical cycloalkyle substitué, on préfère les produits dans lesquels les substituants sont en position trans.

Parmi les composés de formule (I), on peut citer notamment ceux répondant à la formule ($I_A$).

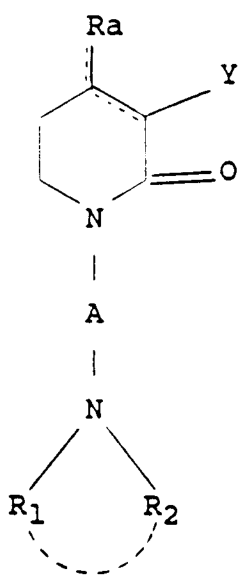

($I_A$)

dans laquelle le groupement :

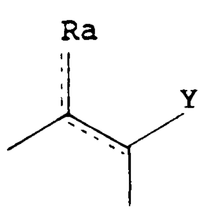

,

A, $R_1$ et $R_2$ sont définis comme précédemment et notamment ceux dans laquelle le groupement

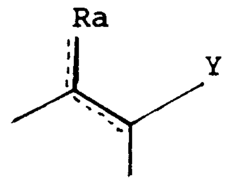

représente

a) 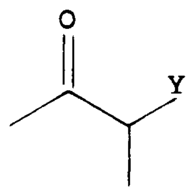 ou le cas échéant son tautomère 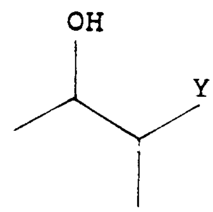

b) 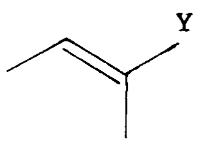

c) 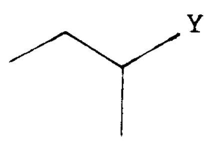

Y étant défini comme ci-dessus,
ainsi que ceux répondant à la formule ($I_B$) :

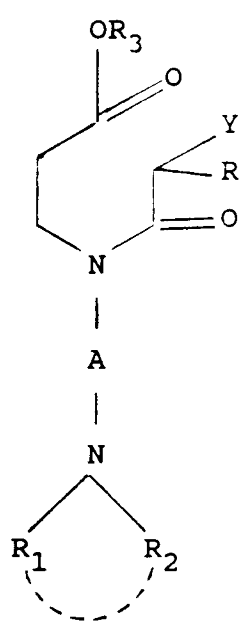

($I_B$)

dans laquelle $R_1$, $R_2$, $R_3$, A, R et Y sont définis comme précédemment.

L'invention a aussi, notamment pour objet les produits de formule (I) tels que définis ci-dessus, caractérisés en ce que le ou les substituants, identiques ou différents, que peuvent porter les radicaux

aryles, arylalkyles et hétérocycliques ou ceux que peuvent former $R_1$ et $R_2$ avec l'atome d'azote auquel ils sont liés, sont choisis dans le groupe formé par :

- les atomes d'halogène,
- les radicaux alkyle, alkényle, alkynyle ou alkyloxy, ces radicaux étant linéaires ou ramifiés renfermant au plus 7 atomes de carbone et étant éventuellement substitués,
- les radicaux hydroxy, trifluorométhyle, trifluorométhylthio, trlfluorométhoxy, cyano, nitro, sulfamoyle, amino, monoalkyl-et dialkylamino,
- les radicaux formyle, acyle renfermant de 2 à 6 atomes de carbone et benzoyle,
- les radicaux carboxy libres, estérifiés par un radical alkyle linéaire ou ramifié renfermant au plus 5 atomes de carbone et salifiés,
- le radical carbamoyle éventuellement substitué par un ou plusieurs radicaux, identiques ou différents, choisis dans le groupe formé par les radicaux alkyle, alkyloxy ou hydroxyalkyle et le radical carbamoylalkyle, ces radicaux alkyle et alkyloxy renfermant de 1 à 4 atomes de carbone,
- et le radical phényle ou benzyle lui-même substitué éventuellement par un ou plusieurs radicaux, identiques ou différents, choisis dans le groupe formé par les atomes d'halogène, les radicaux hydroxy, cyano, nitro, les radicaux alkyle, alkényle, alkynyle et alkyloxy linéaires ou ramifiés renfermant au plus 5 atomes de carbone,

lesdits produits de formule (I) étant sous toutes les formes isomères possibles racémiques, énantiomères et diastéréoisomères, ainsi que les sels d'addition avec les acides minéraux et organiques ou avec les bases desdits produits de formule (I).

L'invention a également pour objet les produits de formule (I) tels que définis ci-dessus, caractérisés en ce que le ou les substituants, identiques ou différents, que peuvent porter les radicaux alkyle, alkényle, alkynyle, cycloalkyle, carbocyclique et alkyloxy sont choisis dans le groupe formé par :

- les atomes d'halogène,
- les radicaux alkyle, alkényle, alkynyle ou alkyloxy renfermant au plus 5 atomes de carbone,
- les radicaux formyle, acyle renfermant de 2 à 6 atomes de carbone et benzoyle,
- les radicaux carboxy libres, estérifiés par un radical alkyle linéaire ou ramifié renfermant au plus 5 atomes de carbone et salifiés,
- les radicaux hydroxy, cyano, amino, monoalkyl- et dialkylamino,
- les radicaux aryle et arylalkyle renfermant de 6 à 14 atomes de carbone, ces radicaux étant éventuellement substitués par un ou plusieurs radicaux, identiques ou différents, choisis dans le groupe formé par les atomes d'halogène, les radicaux hydroxy, cyano, nitro, les radicaux alkyle, alkényle, alkynyle et alkyloxy linéaires ou ramifiés renfermant au plus 5 atomes de carbone,

lesdits produits de formule (I) étant sous toutes les formes isomères possibles racémiques, énantiomères et diastéréoisomères, ainsi que les sels d'addition avec les acides minéraux et organiques ou avec les bases desdits produits de formule (I).

L'invention a particulièrement pour objet les produits de formule (I) tels que définis ci-dessus, dans laquelle R, lorsqu'il représente un radical alkyle, alkényle ou alkynyle, substitué par aryle ou un aryle substitué, est un tel radical substitué par un phényle ou un phényl substitué.

L'invention a aussi particulièrement pour objet les produits de formule (I) tels que définis ci-dessus, caractérisés en ce que Y représente un radical phényle, naphtyle, thionaphtyle, benzofuryle, benzopyrrolyle ou indényle éventuellement substitué par un ou plusieurs radicaux choisis dans le groupe formé par :

- les atomes d'halogène,
- les radicaux alkyle, alkényle, alkynyle ou alkyloxy linéaires ou ramifiés renfermant au plus 7 atomes de carbone et étant éventuellement substitués par un ou plusieurs radicaux choisis dans le groupe formé par le radical hydroxy et les radicaux alkyle et alkyloxy linéaires ou ramifiés renfermant au plus 5 atomes de carbone,
- les radicaux acyle renfermant de 2 à 6 atomes de carbone,
- les radicaux hydroxy, trifluorométhyle, cyano, nitro, amino, les radicaux monoalkyl- et dialkylamino renfermant de 1 à 5 atomes de carbone,
- et le radical phényle lui-même substitué éventuellement par un ou plusieurs radicaux, identiques ou différents, choisis dans le groupe formé par les atomes d'halogène, les radicaux hydroxy, cyano, nitro, les radicaux alkyle, alkényle, alkynyle et alkyloxy linéaires ou ramifiés renfermant au plus 5 atomes de carbone,

lesdits produits de formule (I) étant sous toutes les formes isomères possibles racémiques, énantiomères et diastéréoisomères, ainsi que les sels d'addition avec les acides minéraux et organiques ou avec les bases desdits produits de formule (I) ainsi que les produits de formule (I) tels que définis ci-dessus, caractérisés en ce que A représente :

- soit un radical cycloalkyle de formule

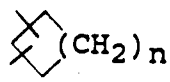

où n est un entier pouvant prendre les valeurs 0 à 4, éventuellement substitué par un radical alkyle ou alkyloxy linéaire ou ramifié renfermant au plus 5 atomes de carbone,

- soit le groupement

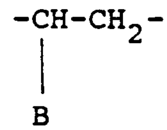

dans lequel B représente un radical phényle éventuellement substitué par un ou plusieurs radicaux, identiques ou différents, choisis dans le groupe formé par les atomes d'halogène, les radicaux hydroxy, cyano, nitro, les radicaux alkyle, alkényle, alkynyle et alkyloxy linéaires ou ramifiés renfermant au plus 5 atomes de carbone,

lesdits produits de formule (I) étant sous toutes les formes isomères possibles racémiques, énantiomères et diastéréoisomères, ainsi que les sels d'addition avec les acides minéraux et organiques ou avec les bases desdits produits de formule (I).

Parmi les produits de formule (I) dans laquelle A représente un radical cycloalkyle, on préfère les produits dans lesquels A représente un radical cyclohexyle ou cyclopentyle non substitué.

L'invention a plus particulièrement pour objet les produits de formule (I) tels que définis ci-dessus, caractérisés en ce que l'hétérocycle que peuvent former $R_1$ et $R_2$, ensemble avec l'atome d'azote auquel ils sont liés, comporte un second atome d'azote et que celui-ci est éventuellement substitué par un radical alkyle ou alkyloxy linéaire ou ramifié renfermant de 1 à 5 atomes de carbone, lesdits produits de formule (I) étant sous toutes les formes isomères possibles racémiques, énantiomères et diastéréoisomères, ainsi que les sels d'addition avec les acides minéraux et organiques ou avec les bases desdits produits de formule (I).

Le second atome d'azote que peut comporter l'hétérocycle formé par $R_1$ et $R_2$ peut être substitué, par exemple, par un radical méthyle, éthyle, isopropyle, méthoxy ou éthoxy.

Parmi les produits objet de l'invention, peuvent être cités tout particulièrement :

la (1S)-3-(3,4-dichlorophényl)-1-[1-phényl-2-(1-pyrrolidinyl) éthyl]-2-pipéridinone (isomère B)
la (1S)-3-(3,4-dichlorophényl)-1-(1-phényl-2-(1-pyrrolidinyl) éthyl)-2-pipéridinone (isomère A)
la [trans,(±)]-3-(3,4-dichlorophényl)-1-(2-(1-pyrrolidinyl) cyclohexyl)-2-pipéridinone (isomère A)
la (1S)-3-(3,4-dichlorophényl)-1-(2-méthyl-1-((1-pyrrolidinyl) méthyl) propyl)-2-pipéridinone (isomère A)
la (1S)-3-(benzo(b)thien-4-yl)-1-(1-phényl-2-(1-pyrrolidinyl) éthyl)-2-pipéridinone (isomère A)
la (1S)-3-(3,4-dichlorophényl)-3-méthyl-1-(1-phényl-2-(1-pyrrolidinyl) éthyl)-2-pipéridinone (isomère A)
la (1S)-3-(3,4-dichlorophényl)-3-éthyl-1-(1-phényl-2-(1-pyrrolidinyl) éthyl)-2-pipéridinone (isomère A)
la (1S)-3-(3,4-dichlorophényl)-1-(1-phényl-2-(1-pyrrolidinyl) éthyl)-3-(2-propényl)-2-pipéridinone (isomère A)
le (S)-3-(((3,4-dichlorophényl) acétyl) (1-phényl-2-(1-pyrrolidinyl) éthyl) amino) propanoate d'éthyle
le (S)-3-(((benzo(b)thien-4-yl) acétyl) (1-phényl-2-(1-pyrrolidinyl) éthyl) amino) propanoate d'éthyle
le (S)-3-(((4-trifluorométhyl) acétyl) (1-phényl-2-(1-pyrrolidinyl) éthyl) amino) propanoate d'éthyle
et le cas échéant leurs sels.

L'invention a également pour objet un procédé de préparation de produits de formule (I) telle que définie ci-dessus, caractérisé en ce que l'on fait réagir la diamine de formule (II) :

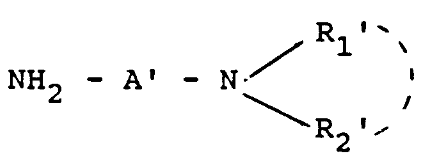

(II)

dans laquelle A' et

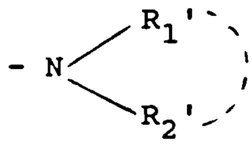

ont les significations indiquées ci-dessus, respectivement pour A et

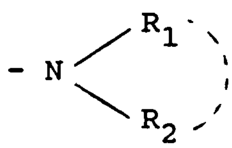

ou celles dans lesquelles les fonctions réactives que peuvent porter A et

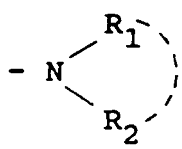

sont protégées, avec l'ester acrylique de formule (III) :

$CH_2$ = CH - $COOR_3$ (III)

dans laquelle $R_3$ représente un radical alkyle linéaire ou ramifié ayant au plus 6 atomes de carbone, pour obtenir le produit condensé de formule (IV) :

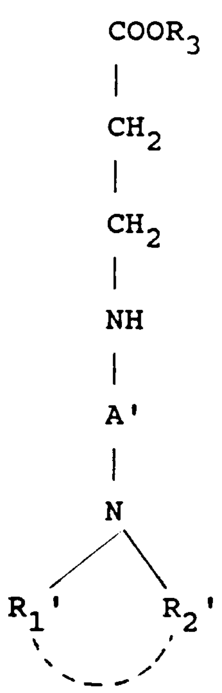

(IV)

dans laquelle A',

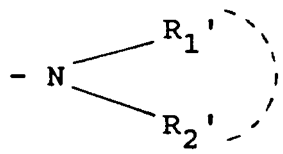

et $R_3$ ont les significations indiquées ci-dessus, que l'on fait réagir avec le composé de formule (V) :

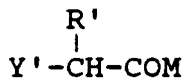

(V)

dans laquelle R' représente un atome d'hydrogène, les valeurs de R indiquées ci-dessus ou celles dans lesquelles les fonctions réactives sont protégées, M représente un radical hydroxyle ou un atome d'halogène et Y' représente les valeurs de Y indiquées ci-dessus, ou celles dans lesquelles les fonctions réactives sont protégées, pour obtenir le produit de formule (VI) :

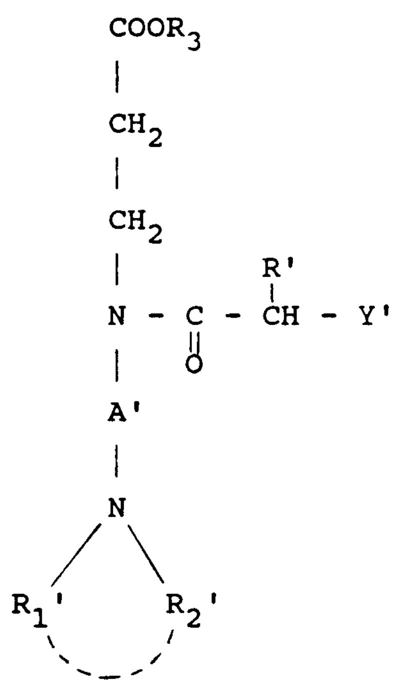

(VI)

dans laquelle A',

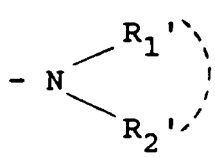,

R', $R_3$ et Y' ont les significations indiquées ci-dessus, correspondant dans le cas où R' représente un atome d'hydrogène ou les valeurs de R et A', $NR'_1R'_2$ et Y' ont respectivement les valeurs de A, $NR_1R_2$ et Y, à un produit de formule ($I_B$) telle que définie ci-dessus, produit de formule (VI) dans lequel le cas échéant, l'on élimine le ou les groupements protecteurs, pour obtenir un même produit de formule ($I_B$) ou que l'on soumet éventuellement à une réaction de cyclisation pour obtenir le produit de formule (VII) :

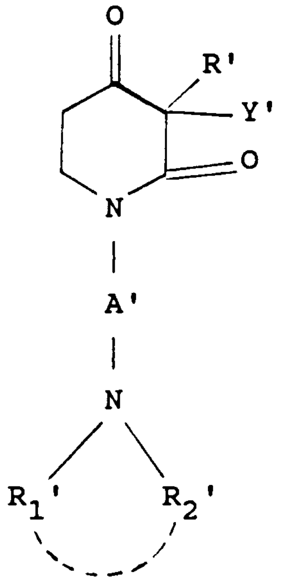

(VII)

dans laquelle A',

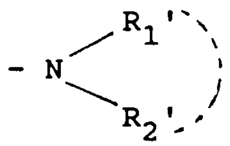 ,

R' et Y' ont les significations indiquées ci-dessus, correspondant dans le cas où R' représente un atome d'hydrogène ou les valeurs de R et A', $NR'_1R'_2$ et Y' ont respectivement les valeurs de A, $NR_1R_2$ et Y, à un produit de formule ($I_A$) telle que définie ci-dessus appelé ($I_{A1}$), produit de formule (VII) dans lequel le cas échéant, l'on élimine le ou les groupements protecteurs, pour obtenir un même produit de formule ($I_{A1}$)-,puis, le cas échéant :

- soit, quand R' ou R représente un atome d'hydrogène, soumet ledit produit de formule (VII) ou ($I_{A1}$) :
  -- soit à une réaction de réduction de la fonction oxo en position gamma de l'atome d'azote pour obtenir le produit de formule (VIII) :

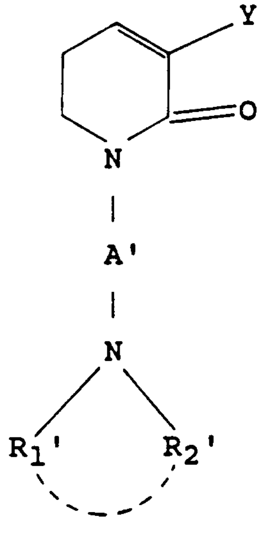

(VIII)

dans laquelle A',

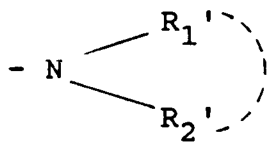

et Y′ ont les significations indiquées ci-dessus, correspondant dans le cas où Y′, A′ et $NR'_1R'_2$ ont respectivement les valeurs de Y, A et $NR_1R_2$, à un produit de formule ($I_A$) telle que définie ci-dessus, appelé ($I_{A2}$), produit de formule (VIII) dans lequel l'on élimine, le cas échéant, les groupements protecteurs, pour obtenir un même produit de formule ($I_{A2}$),

-- soit à une réaction de réduction par hydrogénation catalytique de la même fonction oxo pour obtenir le produit de formule (IX) :

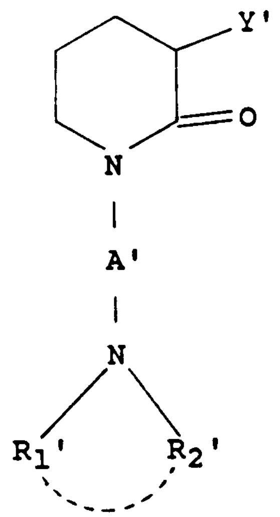

(IX)

dans laquelle A′,

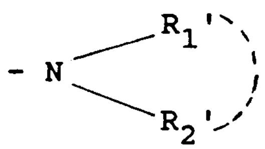

et Y′ ont les significations indiquées ci-dessus, correspondant dans le cas où Y′, A′ et $NR'_1R'_2$ ont respectivement les valeurs de Y, A et $NR_1R_2$, à un produit de formule ($I_A$) telle que définie ci-dessus, appelé ($I_{A3}$), produit de formule (IX) dans lequel l'on élimine, le cas échéant, les groupements protecteurs, pour obtenir un même produit de formule ($I_{A3}$),produit de formule (IX) ou ($I_{A3}$) que l'on traite éventuellement au moyen d'une base forte pour former l'anion correspondant, que l'on soumet à l'action d'un réactif capable de greffer un radical R′, R′ ayant les valeurs indiquées ci-dessus à l'exception d'un atome d'hydrogène,
pour obtenir un produit de formule (X) :

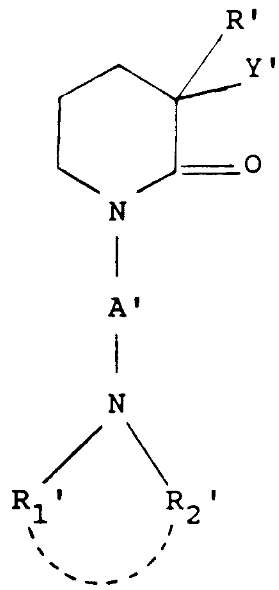

(X)

dans laquelle R′, Y′, A′ et

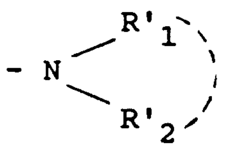

ont les valeurs indiquées ci-dessus, correspondant dans le cas où R′, Y′ A′ et $NR'_1R'_2$ ont respectivement les valeurs de R (à l'exception d'hydrogène), Y, A et $NR_1R_2$, à un produit de formule ($I_A$) telle que définie ci-dessus, appelé ($I_{A4}$), produit de formule (X) dans lequel l'on élimine, le cas échéant, les groupements protecteurs, pour obtenir un même produit de formule ($I_{A4}$) ;

- soit quand R′ ou R est différent d'un atome d'hydrogène, soumet ledit produit de formule (VII) ou ($I_{A1}$) à l'action d'un agent de réduction de la fonction oxo, pour obtenir le produit de formule (X) telle que définie précédemment qui ou bien correspond à un produit de formule ($I_{A4}$) ou bien est transformé en ce produit de formule ($I_{A4}$), et traite, si désiré, les produits de formule (I) par un acide minéral ou organique ou, le cas échéant par une base, pour obtenir le sel correspondant, lesdits produits de formule (I) étant sous toutes les formes isomères possibles racémiques, énantiomères et diastéréoisomères.

Dans des conditions préférentielles de mise en oeuvre de l'invention, le procédé ci-dessus est réalisé de la manière suivante :

- la réaction de condensation de la diamine de formule (II) avec un ester acrylique de formule (III), dans laquelle $R_3$ représente préférentiellement un radical alkyle inférieur tel que, par exemple, dans l'acrylate d'éthyle ou de méthyle mais qui peut également représenter l'acrylate de butyle, peut être effectuée dans un solvant inerte qui peut être, par exemple, un alcool tel que l'éthanol, le méthanol ou le butanol : la solution peut être d'abord refroidie à une température d'environ 10 °C puis laissée revenir à la température ambiante sous agitation pour que la réaction d'addition permettant d'obtenir le produit de formule (IV) se réalise.
- dans la réaction d'obtention de l'amide de formule (VI), l'acylation de l'amine secondaire de formule (IV) peut être réalisée par les méthodes habituelles par exemple par l'acide de formule (V) dans laquelle M représente un radical hydroxy en présence de N,N′carbonyldiimidazole ou encore de cyclohexylcarbodiimide ou encore par l'halogénure d'acide de formule (V) dans laquelle M représente un atome d'halogène tel que, par exemple, un atome de chlore : la réaction, selon les procédés couramment utilisés, se produit dans un solvant organique tel que, par exemple, le tétrahydrofuranne ou encore dans un solvant chloré tel que, par exemple, le chlorure de méthylène sous agitation à la température ambiante.
- la réaction de cyclisation du produit de formule (VI) en produit correspondant de formule (VII) peut être effectuée en milieu basique tel que, par exemple, en présence d'hydrure de sodium ou de

potassium ou encore d'un alcoolate tel que, par exemple, le terbutylate de potassium ou de sodium dans un solvant organique tel que, par exemple, le tétrahydrofuranne, le toluène ou l'éther. Le milieu réactionnel peut être d'abord légèrement refroidi à une température de 12°C à 15°C puis ramené à la température ambiante ou chauffé au reflux.

- la réaction de réduction du produit de formule (VII) ou ($I_{A1}$) en produit de formule (VIII) ou ($I_{A2}$) peut être effectuée, par exemple, par transformation de la fonction oxo du produit de formule (VII), en hydrazone par un réactif tel que, par exemple, une arylsulfonylhydrazine telle que, par exemple, la para-toluènesulfonylhydrazine dans un solvant organique tel que, par exemple, l'acide acétique, ou un alcool tel que, par exemple, le méthanol ou l'éthanol à la température ambiante.

  Le produit réduit de formule (VIII) peut être obtenu par décomposition de l'hydrazone intermédiaire, décomposition réalisée, par exemple, par un alcoolate de sodium ou de potassium, tel que l'éthylate de sodium ou encore le méthylate ou le butylate de sodium ou de potassium : la réaction est effectuée dans un solvant hydroxylé tel que, par exemple, l'éthylène glycol à chaud à une température d'environ 120°C à 160°C.
- la réaction de réduction du produit de formule (VIII) ou ($I_{A2}$) en produit de formule (IX) ou ($I_{A3}$) peut être effectuée, par exemple, par hydrogénation catalytique de la fonction éthylénique en présence d'un hydracide tel que, par exemple, l'acide chlorhydrique ou encore l'acide bromhydrique : la réaction est réalisée dans un alcool, tel que l'éthanol, dans l'acide acétique ou encore dans un ester d'acide tel que, par exemple, l'acétate d'éthyle, sur oxyde de platine à la température ambiante.

La réaction de réduction du produit de formule (VIII) en produit de formule (IX) peut également être effectuée par action d'un hydrure mixte, par exemple le borohydrure de sodium, au sein d'un solvant organique, par exemple un alcool inférieur, de préférence l'éthanol.

La base forte utilisée est de préférence un alcoolate alcalin, par exemple le terbutylate de potassium.

L'agent capable de greffer un radical R′ sur le produit de formule (IX) ou ($I_{A3}$) peut être un alkylsulfonate mais de préférence un halogénure.

L'alkylsulfonate peut être un mésylate mais de préférence un tosylate. L'halogénure peut être un chlorure mais de préférence un bromure ou un iodure.

La réduction de la fonction oxo du produit de formule (VII) dans laquelle R ou R′ est differnt d'un atome d'hydrogène est effectuée selon des conditions identiques à celles indiquées ci-dessus pour l'obtention du produit de formule (VIII).

Dans les produits de formule (V), (VI), (VII), (VIII), (IX) et (X), les diverses fonctions réactives qui peuvent, si nécessaire, être protégées par les groupements protecteurs appropriés, sont, par exemple, des radicaux hydroxyle, acyle, carboxy libres ou encore amino et monoalkylamino.

La liste suivante, non exhaustive, d'exemples de protection de fonctions réactives peut être citée :

- les groupements hydroxy peuvent être protégés par exemple par les radicaux triméthylsilyle, dihydropyranne, benzyle, méthoxy ou méthoxyméthyle,
- les groupements amino peuvent être protégés par exemple par les radicaux trityle, benzyle, ter butoxycarbonyle, phtalimido ou d'autres radicaux connus dans la chimie des peptides,
- les groupements acyles tel que le groupement formyle peuvent être protégés par exemple sous forme de cétals cycliques ou non cycliques tels que le diméthyl ou diéthylcétal ou l'éthylène dioxycétal,
- les groupements carboxy peuvent être protégés par exemple sous forme d'esters formés avec les esters facilement clivables tels que les esters benzyliques ou ter butyliques ou des esters connus dans la chimie des peptides.

L'élimination de ces groupements protecteurs est effectuée dans les conditions usuelles connues de l'homme de métier notamment l'hydrolyse acide effectuée avec un acide tel que l'acide chlorhydrique, benzène sulfonique ou para-toluène sulfonique, formique ou trifluoroacétique.

Le groupement phtalimido est éliminé par l'hydrazine. On trouvera une liste de différents groupements protecteurs utilisables par exemple dans le brevet BF 2 499 995.

L'invention a aussi pour objet un procédé de préparation des produits de formule (I) telle que définie ci-dessus caractérisé en ce que l'on met en oeuvre le procédé au départ des produits de formule (IV) tels que définis ci-dessus et effectue le procédé tel qu'indiqué ci-dessus.

Les formes optiquement actives des produits de formule (I) peuvent être préparés par dédoublement des racémiques selon les méthodes usuelles.

Les composés de formule (I) tels que définis ci-dessus ainsi que leurs sels d'addition avec les acides présentent d'intéressantes propriétés pharmacologiques. Ils présentent en particulier une forte affinité pour les récepteurs opiacés et notamment pour les récepteurs kappa et sont doués de propriétés analgésiques centrales.

Ils sont doués également de propriétés diurétiques, de propriétés anti-arythmiques, anti-ischémiques cérébrales et hypotensives.

Ces propriétés justifient leur application en thérapeutique et l'invention a également pour objet à titre de médicaments, les produits tels que définis par la formule (I) ci-dessus, lesdits produits de formule (I) étant sous toutes les formes isoméres possibles racémiques ou optiquement actives, ainsi que les sels d'addition avec les acides minéraux ou organiques pharmaceutiquement acceptables desdits produits de formule (I).

L'invention a plus particulièrement pour objet, à titre de médicament, les produits de formule (I) suivants :

la (1S)-3-(3,4-dichlorophényl)-1-[1-phényl-2-(1-pyrrolidinyl) éthyl]-2-pipéridinone (isomère B)
la (1S)-3-(3,4-dichlorophényl)-1-(1-phényl-2-(1-pyrrolidinyl) éthyl)-2-pipéridinone (isomère A)
la [trans,(±)]-3-(3,4-dichlorophényl)-1-(2-(1-pyrrolidinyl) cyclohexyl)-2-pipéridinone (isomère A)
la (1S)-3-(3,4-dichlorophényl)-1-(2-méthyl-1-((1-pyrrolidinyl) méthyl) propyl)-2-pipéridinone (isomère A)
la (1S)-3-(benzo(b)thien-4-yl)-1-(1-phényl-2-(1-pyrrolidinyl) éthyl)-2-pipéridinone (isomère A)
la (1S)-3-(3,4-dichlorophényl)-3-méthyl-1-(1-phényl-2-(1-pyrrolidinyl) éthyl)-2-pipéridinone (isomère A)
la (1S)-3-(3,4-dichlorophényl)-3-éthyl-1-(1-phényl-2-(1-pyrrolidinyl) éthyl)-2-pipéridinone (isomère A)
la (1S)-3-(3,4-dichlorophényl)-1-(1-phényl-2-(1-pyrrolidinyl) éthyl)-3-(2-propényl)-2-pipéridinone (isomère A)
ainsi que, le cas échéant, leurs sels pharmaceutiquement acceptables.

Les médicaments, objet de l'invention, permettent notamment de soulager une douleur quelle qu'en soit l'origine, par exemple une douleur de nature musculaire, articulaire ou nerveuse.

Ils peuvent être aussi utilisés dans le traitement des douleurs dentaires, des migraines, de zona, dans le traitement des douleurs intenses, en particulier rebelles aux antalgiques périphériques, par exemple au cours du processus néoplasique, dans le traitement des pancréatites, coliques néphrétiques ou biliaires, dans le traitement des douleurs post-opératoires et post-traumatiques.

Ils peuvent également être utilisés dans le traitement des insuffisances cérébrales d'origine ischémique dans les troubles de la mémoire et de l'attention.

La posologie varie notamment en fonction de la voie d'administration, de l'affection traitée et du sujet en cause.

Par exemple, chez l'adulte, elle peut varier entre 20 et 400 mg de principe actif par jour, par voie orale et entre 5 et 100 mg de principe actif par jour, par voie parentérale.

Les médicaments, objet de l'invention, trouvent aussi leur emploi dans le traitement des arythmies.

La dose usuelle, variable selon le dérivé utilisé, le sujet traité et l'affection en cause, peut être, par exemple, de 50 mg à 1 g par jour.

Par voie orale, le principe actif peut être administré à la dose quotidienne de 200 à 800 mg, par exemple, dans le traitement des arythmies ventriculaires, supraventriculaires et jonctionnelles, soit environ de 3 à 12 mg par kilogramme de poids corporel.

Les médicaments, objet de l'invention, peuvent aussi être utilisés dans le traitement des situations d'hyponétrémie et de rétention hydrique inappropriée, comme celles observées dans les syndromes oedémateux, l'insuffisance cardiaque, certaines obésités, les cyrrhoses, dans le traitement des oedèmes sévères et réfractaires, comme les oedèmes cérébraux et ceux de l'insuffisance cardiaque congestive. Ils peuvent également être utilisés dans le traitement au long cours de l'hypertension artérielle.

La dose quotidienne de principe actif est variable. Elle peut être par exemple de 10 à 100 mg par jour par voie orale.

L'invention s'étend aux compositions pharmaceutiques renfermant comme principe actif les médicaments définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous toutes les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Certains produits de départ sont connus : il s'agit, en particulier, des produits de formule (IIa) :

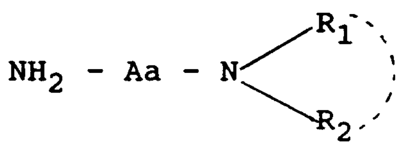

(IIa)

dans laquelle Aa représente un radical cyclohexyle et $R_1$ et $R_2$ ont la signification indiquée ci-dessus, produits de formule (IIa) dont il est fait mention, par exemple, dans les documents référencés comme suit :

- CAS 072 - 078 895
- CAS 52-P 5460
- brevet UPJOHN US-A-728/779 déposé le 13.5.68

D'autres produits de départ connus sont les produits de formule (IIb) :

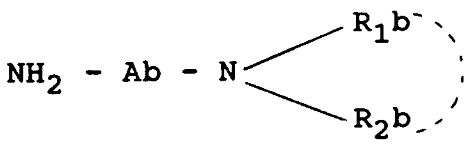

(IIb)

dans laquelle Ab représente le groupement

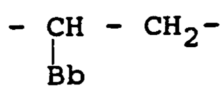

dans lequel Bb représente un radical phényle et le groupement

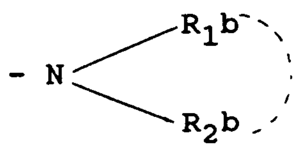

représente un radical pyrrolidinyle, produits de formule (IIb) dont il est fait mention, par exemple, dans les documents référencés comme suit :

- CAS 074 - 141 412
- CAS 102 - 149 185
- CAS 084 - 121 724

Certains produits de départ sont nouveaux et des exemples de préparation de ces produits de départ, exemples non exhaustifs, peuvent être indiqués, comme suit :

- les produits de formule (II) telle que définie ci-dessus, dans laquelle A' représente le groupement :

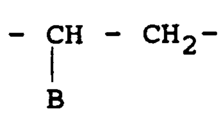

dans lequel B a la signification indiquée ci-dessus,
peuvent être préparés, par exemple, à partir d'un composé de formule (F) :

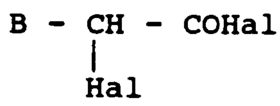

(F)

dans laquelle B a la signification indiquée ci-dessus et Hal représente un atome d'halogène, que l'on fait réagir avec de l'ammoniaque pour obtenir l'amide correspondant de formule (G) :

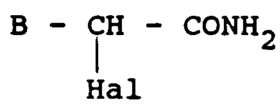

(G)

dans laquelle Hal et B ont les significations indiquées ci-dessus, que l'on fait réagir avec une amine de formule (H) :

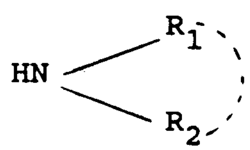

(H)

dans laquelle $R_1$ et $R_2$ ont les significations indiquées ci-dessus, pour obtenir l'amide correspondant de formule (K) :

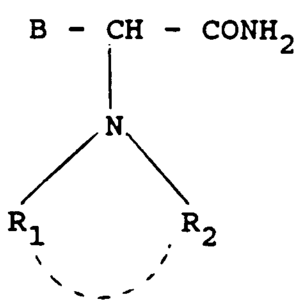

(K)

dans laquelle B, $R_1$ et $R_2$ ont les significations indiquées ci-dessus, dont on réduit la fonction amide par les procédés classiques pour obtenir le produit de formule (II) correspondant.

Les produits de formule (II) telle que définie ci-dessus, dans laquelle A représente un radical carbocyclique peuvent être préparés, par exemple, à partir de la benzylamine du commerce que l'on fait réagir, par exemple, avec un oxyde de cyclohexyle en milieu aqueux pour obtenir un composé de formule (L) :

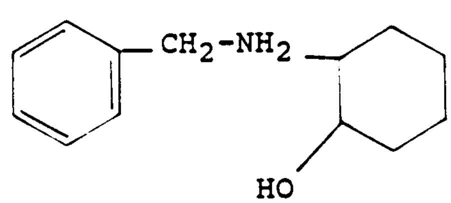

(L)

que l'on fait réagir, par exemple, avec de l'acide chlorosulfonique en présence de chlorure de méthylène, pour obtenir le dérivé sulfaté de formule (M) :

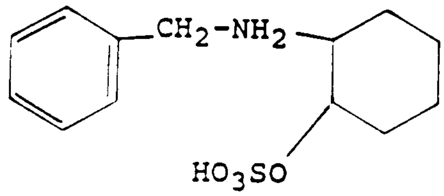

(M)

que l'on fait réagir, par exemple, avec de la soude en milieu aqueux pour obtenir l'aziridine correspondante de formule (N) :

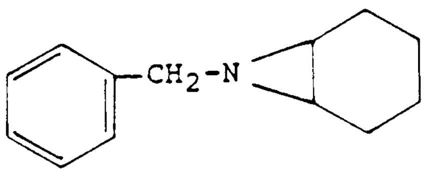

(N)

que l'on fait réagir, par exemple en présence de chlorure d'ammonium, avec l'amine de formule (H) :

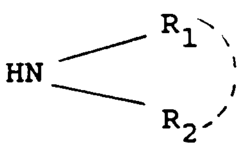

(H)

dans laquelle $R_1$ et $R_2$ ont les significations indiquées ci-dessus, pour obtenir le composé de formule (O) :

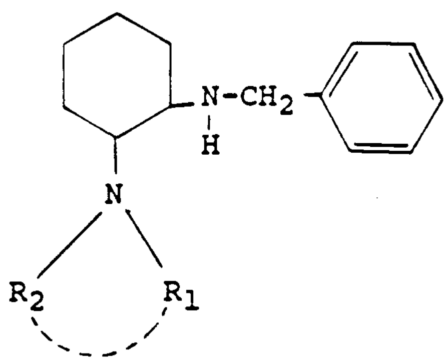

(O)

que l'on débenzyle par hydrogénation catalytique pour obtenir le produit de formule (II) correspondant.

Les autres produits de départ de formule (II) peuvent être préparés par des méthodes comparables connues, de l'homme de métier.

L'invention concerne aussi, à titre de produits industriels, les composés de formules (IV), (VI), (VII), (VIII), (IX) et (X).

Les exemples donnés ci-aprés illustrent l'invention sans toutefois la limiter.

**EXEMPLE 1 : [Trans,(±)]-3-[[2-(1-pyrrolidinyl) cyclohexyl] [(3,4-dichlorophényl) acétyl] amino] propanoate d'éthyle**

STADE A : [Trans,(±)]-3-[[2-(1-pyrrolidinyl) cyclohexyl] amino] propanoate d'éthyle.

A une solution de 8,4 g de 2-(1-pyrrolidinyl) cyclohexane amine dans 50 $cm^3$ d'éthanol, refroidie à + 10 °C, on ajoute en 30 minutes 5,4 $cm^3$ d'acrylate d'éthyle en solution dans 50 $cm^3$ d'éthanol. On agite 6

heures à température ambiante. On évapore à sec, on recueille 12,7 g de produit utilisé tel quel pour le stade suivant.

| | |
|---|---|
| Spectre IR ($CHCl_3$) | |
| -C-<br>‖<br>O | 1726 $cm^{-1}$ |
| -NH | 3260 $cm^{-1}$ |
| RMN ($CDCl_3$) | |
| 1,25 (t)<br>4,13 (q) | $COOC_2H_5$ |
| 2,52 | 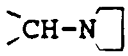 axial |
| 0,95 à 3,0 | les autres protons |

STADE B : [Trans,(±)]-3-[[2-(1-pyrrolidinyl) cyclohexyl] [(3,4-dichlorophényl) acétyl] amino] propanoate d'éthyle.

On agite 1 heure à température ambiante un mélange de 10,65 g d'acide 3,4-dichlorophényl acétique, 8,40 g de carbonyldiimidazole et 70 $cm^3$ de tétrahydrofuranne anhydre. On ajoute, en 10 minutes, une solution de 12,65 g de l'amine obtenue au stade A dans 70 $cm^3$ de tétrahydrofuranne anhydre. On agite 5 heures à température ambiante. On extrait à l'acétate d'éthyle, lave avec 120 $cm^3$ d'une solution saturée de bicarbonate de sodium puis avec 2 fois 70 $cm^3$ d'eau, on sèche et concentre à sec sous pression réduite. Le résidu 22 g, est chromatographié sur silice (éluant : acétate d'éthyle-triéthylamine (97-3)). On obtient 17,6 g de produit attendu utilisé tel quel pour le stade suivant :

| | |
|---|---|
| Spectre IR | |
| 1725 $cm^{-1}$ | région -C-<br>‖<br>O ester |
| 1730 $cm^{-1}$ | amide tertiaire |

**EXEMPLE 2 : [Trans,(±)]-3-(3,4-dichlorophényl) 1-[2-(1-pyrrolidinyl) cyclohexyl] 2,4-pipéridinedione et son chlorhydrate.**

a) Préparation de la base.

A une suspension agitée à 0°C de 2,4 g d'hydrure de sodium (à 50 % dans l'huile) et 100 $cm^3$ de tétrahydrofuranne anhydre, on ajoute en 15 minutes, à 0°/+5°C, une solution de 15,14 g du produit obtenu à l'exemple 1, dans 50 $cm^3$ de tétrahydrofuranne anhydre. On agite 1 heure 45 en laissant le milieu revenir à température ambiante, puis 2 heures supplémentaires à température ambiante (on observe un dégagement d'hydrogène) on concentre ensuite, à sec sous pression réduite et reprend le résidu avec 150 $cm^3$ d'eau et glace. On extrait la solution avec 2 fois 100 $cm^3$ d'éther sulfurique et acidifie la phase aqueuse avec 5 $cm^3$ d'acide acétique. On ajoute ensuite 2,5 g de carbonate de potassium, on agite à température ambiante jusqu'à obtention de cristaux que l'on essore, lave à l'eau avec 3 fois 20 $cm^3$, puis à l'éther avec

3 fois 20 $cm^3$, on sèche le produit sous pression réduite à 50 ° C. On obtient 11,75 g du produit attendu sous forme de base. F = 133 ° C.

Spectre IR (Nujol)
Absorption OH/NH

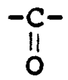 1734 $cm^{-1}$ (f)
1653 $cm^{-1}$

absorption ~ 1603 $cm^{-1}$ (F, complexe)

Aromatique 1545 $cm^{-1}$
1508 $cm^{-1}$

b) Préparation du chlorhydrate.

On dissout à chaud 1,756 g de la base obtenue ci-dessus, dans 10 $cm^3$ d'éthanol 95, filtre un léger insoluble, rince avec 3 fois 1 $cm^3$ d'éthanol 95 bouillant et ajoute 1 $cm^3$ d'une solution 5,75 N d'éthanol chlorhydrique. On refroidit à 20 ° C et agite 1 heure, essore, lave 2 fois 1 $cm^3$ d'éthanol 95, 2 fois 1 $cm^3$ d'éthanol 100 et 3 fois 5 $cm^3$ d'éther. Après séchage sous pression réduite à 70 ° C, on obtient 1,484 g de produit recherché F > 260 ° C.

| Analyse pour $C_{21}H_{26}Cl_2N_2O_2$ | | | | |
|---|---|---|---|---|
| | C % | H % | N % | Cl % |
| % calculés<br>% trouvés | 56,58<br>56,5 | 6,10<br>6,2 | 6,28<br>6,2 | 23,85<br>23,6 |

RMN DMSO (250 MHz)

4,38 (m) 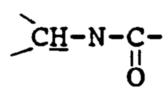 axial

3,66 (dt,j = 3,5 11 et 11) 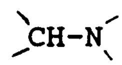 axial

2,64 (m) 1H
2,84 (m) 1H
3,2 à 3,6
} les autres H en alpha de N

7,27 (dd) 1H
7,47 2H
} aromatiques

1,2 à 2,2 les autres protons.

**EXEMPLE 3 : [Trans,(±)]-3-(3,4-dichlorophényl) 5,6-dihydro 1-[2-(1-pyrrolidinyl) cyclohexyl] 2(1H)-pyridinone et son fumarate.**

STADE A : [Trans,(±)]-2-[3-(3,4-dichlorophényl) 2-oxo 1-[2-(1-pyrrolidinyl) cyclohexyl] 1,2,5,6-tétrahydro 4-pyridyl] hydrazide de l'acide 4-méthyl benzène sulfonique.

On agite pendant 4 heures à température ambiante un mélange de 5 g de la base obtenue à l'exemple 2, 2,95 g de paratoluène sulfonyl hydrazide et 25 $cm^3$ d'acide acétique. On ajoute au milieu réactionnel 150

cm$^3$ d'eau saturée de chlorure de sodium puis 50 cm$^3$ d'éther sulfurique, on agite 2 heures à température ambiante. On essore, lave à l'eau saturée de chlorure de sodium 20 cm$^3$ et éther sulfurique 5 cm$^3$ puis avec une solution de carbonate de potassium à 15 %, à l'eau et à l'éther. On sèche sous pression réduite à 20 °C, on obtient 6,25 g de produit attendu. F ≃ 200 °C que l'on utilise tel quel pour le stade suivant.

| Spectre IR ($CHCl_3$) | | |
|---|---|---|
| $-SO_2-$ | | 1336-1165 $cm^{-1}$ |
| >C =O | | 1627 $cm^{-1}$ |
| Région | C = C<br>C = N | 1601, 1545, 1490, 1474 $cm^{-1}$ |
| = C-NH | | ≃ 3360 $cm^{-1}$ complexe |

STADE B : [Trans,(±)]-3-(3,4-dichlorophényl) 5,6-dihydro 1-[2-(1-pyrrolidinyl) cyclohexyl] 2(1H)-pyridinone et son fumarate.

a) Préparation de la base.

On introduit, en 30 minutes, de 20° à 35 °C, 0,65 g de sodium dans 28 cm$^3$ d'éthanol 100, on chauffe ensuite à 60 °C et ajoute 5,64 g du produit obtenu au stade A et 22 cm$^3$ d'éthylène glycol. On distille l'éthanol à pression ordinaire puis chauffe en 30 minutes jusqu'à 160 °C dans le milieu réactionnel jusqu'à fin de dégagement gazeux. On refroidit à 20 °C et extrait avec du chlorure de méthylène, on sèche, filtre et concentre à sec sous pression réduite, on recueille 3,78 g de produit sous forme de base. On chromatographie sur silice, 1,319 g de la base obtenue (éluant : acétate d'éthyle à 0,5 % de triéthylamine).

b) Préparation du fumarate :

On dissout les 0,63 g du produit obtenu après chromatographie dans 5 cm$^3$ d'éthanol, on ajoute 248 mg d'acide fumarique et porte au reflux. On essore à 20 °C, lave à l'éthanol avec 3 fois 0,5 cm$^3$ puis avec de l'éther 3 fois 5 cm$^3$. On obtient après sèchage 0,681 g de produit recherché. F = 202 °C.

| Analyse pour $C_{21}H_{26}Cl_2N_2O$ | | | | |
|---|---|---|---|---|
| | C % | H % | N % | Cl % |
| % calculés<br>% trouvés | 57,14<br>57,1 | 5,68<br>5,9 | 4,94<br>4,9 | 12,49<br>12,5 |

RMN $CDCl_3$ (250 MHz)

6,59 (t,j ~ 4,5) HC=C<

7,50 (d)
7,32 (d)
7,23 (dd)  } 3H aromatiques

4,47 (dt) >CH-N-C(=O) axial (isomère trans)

3,30 (m) $CH_2$-N-C(=O)

2,25 à 2,80 7H, $CH_2$ en alpha de N pyrrolidine, >CH-N et $CH_2$-C-C=C-$C_6H_5$

0,8 à 1,9 les autres protons.

**EXEMPLE 4 : [Trans,(±)]-3-(3,4-dichlorophényl) 1-[2-(1-pyrrolidinyl) cyclohexyl] 2-piperidinone (isomère B) et son chlorhydrate.**

**EXEMPLE 5 : [Trans,(±)]-3-(3,4-dichlorophényl) 1-[2-(1-pyrrolidinyl) cyclohexyl] 2-piperidinone (isomère A) et son fumarate.**

A une solution de 5,61 g du produit obtenu au stade B de l'exemple 3 dans 112 $cm^3$ d'éthanol 100 et 11,2 $cm^3$ d'acide chlorydrique 37 %, on ajoute 0,56 g d'oxyde de platine 80 % et agite sous atmosphère d'hydrogène (sous une pression de 1835 mbars) pendant 4 heures 30 (au total 473 $cm^3$ d'hydrogène sont absorbés). On filtre le catalyseur et évapore le filtrat à sec sous pression réduite. On reprend le résidu avec 10 $cm^3$ d'eau et 50 $cm^3$ d'acétate d'éthyle, on alcalinise par un excès de carbonate de sodium et décante, la phase aqueuse est réextraite avec 30 $cm^3$ d'acétate d'éthyle, lave les extraits organique à l'eau salée, sèche, filtre et amène à sec sous pression réduite. On chromatographie le résidu (5,45 g) sur silice (éluant : acétate d'éthyle à 0,5 % de triéthylamine (isolement de l'isomère A) puis acétate d'éthyle à 1 puis à 2 % de triéthylamine pour l'isomère B). On recueille ainsi isomère A 2,452 g, F = 109 °C, isomère B 2,359 g.

Produit de l'exemple 4 : chlorhydrate de l'isomère B.

2,359 g de l'isomère B obtenu sont dissous dans 2 $cm^3$ d'éthanol, on ajoute 4 $cm^3$ d'une solution 1,68 N d'éthanol chlorhydrique (pH 1). On essore, lave avec de l'éthanol à +5/+10 °C, puis à l'éther sulfurique. On obtient, après séchage 1,813 g de produit recherché. F > 260 °C. On recristallise 1,778 g du chlorhydrate obtenu ci-dessus dans 25 $cm^3$ d'isopropanol, on recueille 1,498 g de produit attendu F > 260 °C.

| Analyse pour $C_{21}H_{28}Cl_2N_2O$,HCl | | | | |
|---|---|---|---|---|
| | C % | H % | N % | Cl % |
| % calculés<br>% trouvés | 58,4<br>58,6 | 6,77<br>6,7 | 6,48<br>6,6 | 24,63<br>24,5 |

RMN $CDCl_3$ (250 MHz)

>C$\underline{H}$-C(=O)-N 3,63 (m)

7,27 (d)
7,35 (d) les aromatiques
7,05 (d)

>NH-C$\underline{H}$ ≃ 4,45

2,4 à 2,9 les $C\underline{H}_2N$ et $C\underline{H}N$

1,0 à 2,2 les autres protons.

Produit de l'exemple 5 : (isomère A). Fumarate de l'isomère A.

On dissout à chaud 2,452 g de l'isomère A dans 10 $cm^3$ d'éthanol, on ajoute 865 mg d'acide fumarique. On chauffe au reflux puis refroidit à 20 °C et essore et lave avec de l'éthanol puis avec de l'éther. On obtient 2,638 g de produit recherché. F = 153 °C.

| Analyse pour $C_{21}H_{28}Cl_2N_2O,C_4H_4O_4$ | | | | |
|---|---|---|---|---|
| | C % | H % | N % | Cl % |
| % calculés | 58,71 | 6,31 | 5,48 | 13,86 |
| % trouvés | 58,6 | 6,4 | 5,6 | 14,0 |

RMN $CDCl_3$ (250 MHz)

>C$\underline{H}$-C(=O)-N 3,63 (t,j = 7)

7,28 (d), 7,34 (d), 7,09 (dd) les aromatiques

>C$\underline{H}$-N-C 4,61 (dt j = 3, 11, 11)

>CH-N-C$\underline{H}_2$ 3,2 à 3,4

2,45 à 2,8 $C\underline{H}_2N$ et C$\underline{H}$-N
1,07 à 2,2 les autres protons.

**EXEMPLE 6 : [Trans,(±)]-3-[[(3,4-diméthoxy phényl) acétyl] [2-(1-pyrrolidinyl) cyclohexyl] amino] propanoate d'éthyle.**

On opère comme au stade B de l'exemple 1 à partir de 16,67 g de produit obtenu au stade A de l'exemple 1 et en utilisant 14,12 g d'acide 3,4-diméthoxy phénylacétique et 11,67 g de carbonyldiimidazole. On obtient après chromatographie (éluant : acétate d'éthyle à 1 % de triéthylamine) 23,69 g de produit recherché utilisé tel que pour le stade suivant.

Spectre IR ($CHCl_3$)

C = O ester 1724 $cm^{-1}$

C = O amide 1625 $cm^{-1}$

Aromatiques 1592 $cm^{-1}$, 1514 $cm^{-1}$ } du type 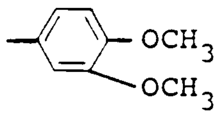

**EXEMPLE 7 : [Trans,(±)]-3-(3,4-diméthoxy phényl) 1-[2-(1-pyrrolidinyl) cyclohexyl] 2,4-piperidinedione.**

On opère comme à l'exemple 2 à partir de 5,2 g du produit obtenu à l'exemple 6. On isole le produit en reprenant, l'extrait sec obtenu après évaporation sous pression réduite, avec de l'eau saturée en chlorure de sodium, on essore, lave à l'hexane et sèche sous pression réduite.

On obtient 3,638 g de produit attendu F # 260 ° C. On empâte le produit avec du tétrahydrofuranne puis le dissout dans le mélange chlorure de méthylène-méthanol (7-3), filtre sur clarcel et évapore à sec sous pression réduite.

On obtient 2,935 g de produit attendu. F ≃ 260 ° C.

Spectre IR (Nujol)

Absorption région OH/NH

1640 $cm^{-1}$ faible absorption 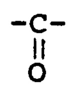

1583 $cm^{-1}$, 1534 $cm^{-1}$ (ep), 1516 $cm^{-1}$ (max) } région aromatique

produit utilisé tel quel pour le stade suivant.

**EXEMPLE 8 : [Trans-(±)]-5,6-dihydro 3-(3,4-diméthoxy phényl) 1-[2-(1-pyrrolidinyl) cyclohexyl] 2(1H)-pyridinone.**

STADE A : [Trans,(±)]-5,6-dihydro 3-(3,4-diméthoxy phényl) 4-[2-[(4-méthyl phényl) sulfonyl] hydrazinyl] 1-[2-(1-pyrrolidinyl) cyclohexyl] 2(1H)-pyridinone.

On opère comme au stade A de l'exemple 3 à partir de 11,61 g de produit obtenu comme à l'exemple 7 avec 7,02 g de paratoluènesulfonhydrazide, on obtient 16,27 g de produit attendu, F = 195 ° -200 ° C, utilisé tel quel pour le stade suivant.

Après recristallisation de 205 mg de produit brut dans l'éthanol, on obtient 92 mg de produit F = 238-240 ° C.

Spectre IR ($CHCl_3$)

| | | |
|---|---|---|
| =C-NH- | 3340 $cm^{-1}$ | (complexe) |
| 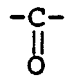 | 1628 $cm^{-1}$ | |
| Région syst. conjugué | 1601 $cm^{-1}$ | |
| + aromatique type 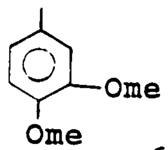 | 1516 $cm^{-1}$ | |
| | 1495 $cm^{-1}$ | |
| $-SO_2$ | 1337 $cm^{-1}$ | |
| | 1165 $cm^{-1}$ | |

STADE B : [Trans-(±)]-5,6-dihydro 3-(3,4-diméthoxy phényl) 1-[2-(1-pyrrolidinyl) cyclohexyl] 2(1H)-pyridinone.

On opère comme au stade B de l'exemple 3 à partir de 16,42 g de produit obtenu au stade A ci-dessus, en utilisant 1,99 g de sodium et 60 $cm^3$ d'éthylène glycol, on obtient 12,83 g de produit recherché utilisé tel quel pour l'exemple suivant.

**EXEMPLE 9 : [(1alpha, 2béta) (±)]-3-(3,4-diméthoxy phényl) 1-[2-(1-pyrrolidinyl) cyclohexyl] 2-piperidinone (isomère A et isomère B) et oxalate de l'isomère B.**

On opère comme à l'exemple 4 à partir de 12,36 g du produit obtenu à l'exemple 8, on obtient après chromatographie (éluant : d'abord chlorure de méthylène-méthanol (95-5) puis (90-10)), 7,84 g de l'isomère A et 2,8 g de l'isomère B. Les 2,8 g de l'isomère B sont empâtés dans 8 $cm^3$ d'éther sulfurique. On obtient 0,538 g de produit. F = 188-191 °C. On chromatographie à nouveau le filtrat et recueille ainsi 1,85 g de résidu que l'on joint au 0,538 g ci-dessus pour préparer le fumarate dans l'éthanol puis que l'on cristallise dans l'isopropanol. On revient à la base à partir de 1,419 g de fumarate obtenu ci-dessus, par déplacement avec du bicarbonate de sodium et du carbonate de sodium. L'extrait sec 1,050 g est dissous dans du tétrahydrofuranne à 50 °C et additionné de 423 mg d'acide oxalique dihydraté chauffé au reflux. On essore à 20 °C, lave au tétrahydrofuranne puis à l'éther éthylique. On obtient 987 mg de produit que l'on recristallise dans le tétrahydrofuranne à 5 % d'eau, après élimination azéotropique de l'eau, on essore et obtient 964 mg du produit attendu.
F = 192-196 °C.

| Analyse pour $C_{23}H_{34}N_2O_3,C_2H_2O_4$ | | | |
|---|---|---|---|
| | C % | H % | N % |
| % calculés | 63,01 | 7,61 | 5,88 |
| % trouvés | 62,7 | 7,7 | 5,8 |

**EXEMPLE 10 : [(1alpha, 2béta) (±)]-3-(3,4-diméthoxy phényl) 1-[2-(1-pyrrolidinyl) cyclohexyl] 2-piperidinone (isomère A) et son chlorhydrate.**

On empâte 3,556 g de l'isomère A obtenu à l'exemple 9 dans 10 $cm^3$ d'éther sulfurique au reflux. On recueille 3,31 g de produit attendu sous forme de base. F = 111-113 °C.

Obtention du chlorhydrate :

1,6 g du produit ci-dessus sont dissous dans 4 $cm^3$ d'éthanol 100, on ajoute 0,4 $cm^3$ d'une solution 6,6 N d'éthanol chlorhydrique. Essore, lave à l'éthanol et à l'éther, on obtient 1,326 g de produit recherché. F = 193 ° C.

| Analyse pour $C_{23}H_{34}N_2O_3$,HCl | | | | |
|---|---|---|---|---|
| | C % | H % | N % | Cl % |
| % calculés<br>% trouvés | 65,31<br>65,3 | 8,34<br>8,4 | 6,62<br>6,4 | 8,38<br>8,5 |

**EXEMPLE 11 : (S)-3-[[(3,4-dichlorophényl) acétyl] [1-phényl 2-(1-pyrrolidinyl) éthyl] amino] propanoate d'éthyle.**

STADE A : (S)-3-[[1-phényl 2-(1-pyrrolidinyl) éthyl] amino] propanoate d'éthyle et son dichlorhydrate.

On opère comme au stade A de l'exemple 1 à partir de 16,19 g de (S)-alpha-phényl 1-pyrrolidinéthanamine et 11 $cm^3$ d'acrylate d'éthyle. On agite 40 heures à 20/22 ° C. On évapore à sec et obtient 22,08 g de produit brut sous forme de base.

Obtention du chlorhydrate :

On dissout 22,08 g du produit brut dans 200 $cm^3$ d'éthanol 100 et ajoute à 20 ° C, 25 $cm^3$ d'une solution 6,6 N d'éthanol chlorhydrique. On essore, lave à l'éthanol puis à l'éther, on obtient après sèchage à 60 ° C sous pression réduite, 22,22 g de produit attendu. F = 245 ° -250 ° C.
$[alpha]_D$ -19 ° 5 ± 1 (c = 1 % $H_2o$).

STADE B : (S)-3-[[(3,4-dichlorophényl) acétyl] [1-phényl 2-(1-pyrrolidinyl) éthyl] amino] propanoate d'éthyle.

On opère comme au stade B de l'exemple 1 à partir de 22,22 g du chlorhydrate obtenu au stade A ci-dessus, avec 16,3 g d'acide (3,4-dichloro phényl) acétique et 12,89 g de carbonyldiimidazole. On obtient 30,48 g de produit attendu que l'on utilise tel quel pour l'exemple suivant.

**EXEMPLE 12 : (1S)-3-(3,4-dichlorophényl) 1-[1-phényl 2-(1-pyrrolidinyl) éthyl] 2,4-pipéridinedione.**

On opère comme à l'exemple 2 à partir de 28,6 g du produit obtenu à l'exemple 11, en utilisant 3,3 g d'hydrure de sodium. On obtient 24,74 g de produit recherché que l'on utilise tel quel pour l'exemple suivant.
F = 110-115 ° C cristallisé de l'éther diéthylique.

**EXEMPLE 13 : (S)-3-(3,4-dichlorophényl) 5,6-dihydro 1-[1-phényl 2-(1-pyrrolidinyl) éthyl] 2(1H)-pyridinone et son fumarate.**

STADE A : (S)-2-[3-(3,4-dichlorophényl) 2-oxo 1-[1-phényl 2-(1-pyrrolidinyl) éthyl] 1,2,5,6-tétrahydro 4-pyridyl] hydrazide de l'acide 4-méthyl benzènesulfonique.

On opère comme au stade A de l'exemple 3, à partir de 30 g du produit obtenu comme à l'exemple 12 et 14 g de paratoluènesulfonhydrazide. On agite pendant 5 heures et distille l'acide acétique sous pression réduite. On alcalinise à pH 9 avec 12,5 g de carbonate de sodium. On extrait avec du chlorure de méthylène, sèche, filtre et amène à sec sous pression réduite. On obtient 39 g de produit utilisé tel quel pour le stade suivant.
$[alpha]_D$ (sur produit purifié) + 64,5 ° ± 1 ° 5 (c = 1 % méthanol).

STADE B : (S)-3-(3,4-dichlorophényl) 5,6-dihydro 1-[1-phényl 2-(1-pyrrolidinyl) éthyl] 2(1H)-pyridinone et son fumarate.

On opère comme au stade B de l'exemple 3 à partir de 39 g de produit obtenu au stade A ci-dessus, en utilisant 6,05 g de sodium et 250 $cm^3$ d'éthylène glycol. On obtient 22,33 g de produit attendu sous forme de base.

Obtention du fumarate :

On dissout 22,17 g du produit ci-dessus dans 65 $cm^3$ d'éthanol 100 à chaud et ajoute 6,4 g d'acide fumarique. On chauffe à 60 °C jusqu'à dissolution puis refroidit et essore à 20 °C, lave à l'éthanol glacé puis à l'éther. On obtient 15,64 g de produit recherché. F = 162-164 °C.

Retour à la base :

6,9 g du produit obtenu ci-dessus sont dissous dans 100 $cm^3$ d'acétate d'éthyle et 40 $cm^3$ d'une solution aqueuse de carbonate de sodium à 10 %. On décante et réextrait à l'acétate d'éthyle, sèche, filtre et distille à sec sous pression réduite, on obtient 5,4 g de produit sous forme de base que l'on utilise tel quel pour l'exemple suivant, un échantillon analytique de fumarate a été préparé par recristallisation de 500 mg du fumarate ci-dessus dans 4 $cm^3$ d'éthanol 100. On obtient 267 mg de fumarate pur. F = 162-164 °C.
$[alpha]_D$ +118 ° ± 2 ° (c = 1 % méthanol).

| Analyse pour $C_{23}H_{24}ON_2Cl_2,C_4H_4O_4$ | | | | |
|---|---|---|---|---|
| | C % | H % | N % | Cl % |
| % calculés | 61,02 | 5,31 | 5,27 | 13,34 |
| % trouvés | 61,0 | 5,1 | 5,2 | 13,5 |

**EXEMPLE 14 : (1S)-3-(3,4-dichlorophényl) 1-[1-phényl 2-(1-pyrrolidinyl) éthyl] 2-piperidinone (isomère A et isomère B) et maléate de l'isomère A.**

1ère méthode :

On opère comme à l'exemple 4 à partir de 5,4 g du produit obtenu à l'exemple 13, en utilisant 0,6 g d'oxyde de platine à 80 % et en hydrogénant sous une pression de 1500 mbars, 400 $cm^3$ d'hydrogène sont absorbés, on obtient 5,81 g de produit attendu que l'on chromatographie sur silice (éluant : acétate d'éthyle à 2 % de triéthylamine) on obtient 0,975 g de produit attendu isomère A et 1,80 g d'isomère B.

Obtention du maléate :

901 mg du produit brut (isomère A) sont dissous dans 6 $cm^3$ d'acétate d'éthyle, on ajoute 251 mg d'acide maléïque et chauffe à 60 °C. Refroidit à 20 °C 1 heure, essore, lave à l'acétate d'éthyle et à l'éther. On obtient 1,02 g de produit recherché. F = 140-141 °C.
$[alpha]_D$ +63 °5 ± 1 °5 (c = 1 % méthanol).

| Analyse pour $C_{23}H_{26}Cl_2N_2O,C_4H_4O_4$ | | | | |
|---|---|---|---|---|
| | C % | H % | N % | Cl % |
| % calculés | 60,79 | 5,27 | 5,25 | 13,29 |
| % trouvés | 61,1 | 5,6 | 5,3 | 13,2 |

RMN $CDCl_3$ 300 MHz (base)

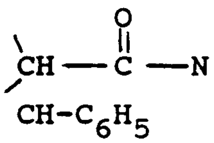

3,68 (dd, J = 7 et 9)
6,22 (dd, S = 12 et 4,5)

H aromatique { 7,20 (d,1) 1H ; 7,25 à 7,40 (m) 7H

1,62 à 3,37 les 16 autres protons.

2ème méthode :

On dissout à 20 ° C 3,01 g de produit préparé à l'exemple 13 dans 30 $cm^3$ d'éthanol, ajoute 0,6 g de borohydrure de sodium et agite pendant 6 heures. On ajoute 2 $cm^3$ d'eau, en maintenant la température à 20-25 ° C, ajoute 1 $cm^3$ d'acide acétique (pH = 6) élimine les solvants sous pression réduite, reprend le résidu dans une solution aqueuse de carbonate de sodium à 20 % puis extrait à l'acétate d'éthyle. On sèche et concentre à sec sous pression réduite. On obtient 3,1 g de produit attendu que l'on utilise tel quel.

Un essai de chromatographie sur silice de 615 mg de produit a permis d'obtenir 380 mg d'isomère A et 229 mg d'isomère B.

**EXEMPLE 15 : Fumarate de (1S)-3-(3,4-dichlorophényl) 1-[1-phényl 2-(1-pyrrolidinyl) éthyl] 2-piperidinone (isomère B).**

On dissout 1,75 g du produit obtenu à l'exemple 14 (isomère B) dans 8 $cm^3$ d'isopropanol et ajoute 490 mg d'acide fumarique, on chauffe jusqu'à dissolution, refroidit à 20 ° C, essore, lave à l'isopropanol à 5 ° C puis à l'éther. On obtient 1,594 g de produit que l'on recristallise dans 6 $cm^3$ d'éthanol 100.

On obtient 1,197 g de produit recherché. F = 186-188 ° C.
$[alpha]_D$ +152 ° ± 3 ° (c = 1 % méthanol).

RMN $CDCl_3$ 300 MHz

7,04 (dd) 7,27 (d) aromatiques

3,74 m
6,11 (dd, J = 10 et 6)

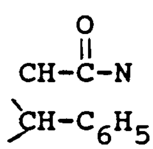

7,25 à 7,45 (m) 6H aromatiques
1,79 à 3,33 16 autres protons.

**EXEMPLE 16 : (S)-3-((4-trifluorométhyl) acétyl) (1-phényl 2-pyrrolidinyl) éthyl) amino) propanoate d'éthyle**

On opère comme au stade B de l'exemple 1 en utilisant 5,31 g d'acide 4-trifluorométhyl phénylacétique, 4,22 g de carbonyldiimidazole et 7,27 g de dichlorhydrate du (S)-3-[[1-phényl 2-(1-pyrrolidinyl) éthyl] amino] propanoate d'éthyle préparé comme au stade A de l'exemple 11 en maintenant le milieu réactionnel sous agitation pendant 20 heures à température ambiante.

Après chromatographie sur silice (éluant : acétate d'éthyle) on obtient 9,8 g de produit attendu.
$[alpha]_D$ +89,5 ° ± 2 ° (c = 1 % méthanol).

**EXEMPLE 17 : (1S)-3-(4-trifluorométhyl) 1-[1-phényl 2-(1-pyrrolidinyl) éthyl] 2,4-pipéridinedione**

On opère comme à l'exemple 2 à partir de 9,42 g du produit obtenu à l'exemple 16 en utilisant 1,15 g d'hydrure de sodium. On obtient 9,35 g de produit attendu utilisé tel quel pour l'exemple suivant.

**EXEMPLE 18 : (S)-3-(4-trifluorométhyl) 5,6-dihydro 1-[1-phényl 2-(1-pyrrolidinyl) éthyl] 2(1H)-pyridinone et son fumarate**

STADE A : (S)-[3-(4-trifluorométhyl) 2-oxo 1-[1-phényl 2-(1-pyrrolidinyl) éthyl) 1,2,5,6-tétrahydro 4-pyridyl] hydrazide de l'acide 4-méthyl benzène sulfonique.

On opère comme au stade A de l'exemple 13 à partir de 9,35 g du produit obtenu à l'exemple 17 et 4,86 g de p.toluène sulfonydrazide et obtient 15,16 g de produit attendu que l'on utilise tel que pour le stade suivant.

STADE B : (S)-3-(4-trifluorométhyl) 5,6-dihydro 1-[1-phényl 2-(1-pyrrolidinyl) éthyl] 2(1H)-pyridinone et son fumarate

On opère comme au stade B de l'exemple 3 à partir de 15,16 g de produit obtenu au stade A, 2,1 g de sodium et 85 $cm^3$ d'éthylène glycol. On obtient après chromatographie sur silice (éluant : acétate d'éthyle) 4,17 g de produit attendu sous forme de base.

En opérant comme à l'exemple 13 à partir de 201 mg de base et 60 mg d'acide fumarique, on obtient 116 mg de fumarate attendu. F = 198°-200°C.
$[alpha]_D$ +93,5° ± 3° (c = 0,5 % diméthylformamide).

| Analyse pour $C_{24}H_{25}F_3N_2O,C_4H_4O_4$ | | | | |
|---|---|---|---|---|
| | C % | H % | N % | F % |
| % calculés | 63,39 | 5,51 | 5,28 | 10,08 |
| % trouvés | 63,1 | 5,4 | 5,4 | 10,7 |

**EXEMPLE 19 : (S)-1-[1-phényl 2-(1-pyrrolidinyl) éthyl] 3-(4-trifluorométhyl) phényl) 2-pipéridinone (isomère A et isomère B) et fumarate de l'isomère A**

On agite pendant 5 heures à température ambiante 3,91 g de la base obtenue à l'exemple 18 dans 40 $cm^3$ d'éthanol en présence de 0,79 g de borohydrure de sodium.

On neutralise par addition de 8 $cm^3$ d'un mélange acide acétique-eau (1-1). On distille le solvant sous pression réduite, reprend le résidu avec 50 $cm^3$ d'acétate d'éthyle, 30 $cm^3$ de carbonate de sodium à 20 % et 20 $cm^3$ d'eau.

On agite le milieu réactionnel, décante, extrait à l'acétate d'éthyle, sèche, élimine les solvants sous pression réduite et obtient 4,15 g de produit brut que l'on chromatographie sur silice (éluant : acétate d'éthyle à 2 % de triéthylamine). On obtient 2,07 g d'isomère A. F = 126°-128°C et 1,2 g d'isomère B.

Préparation du fumarate de l'isomère A

On dissout 1,59 g de l'isomère A dans 5 $cm^3$ d'éthanol, ajoute 443 mg d'acide fumarique, filtre la solution et concentre à sec sous pression réduite. On amorce la cristallisation, dilue avec 10 $cm^3$ de 1,2-diméthoxyéthane, essore et rince à l'éther. On sèche sous pression réduite à 80°C et recueille 998 mg de produit brut.

Après recristallisation de 953 mg de produit dans l'acétate d'éthyle, on obtient 528 mg de produit attendu.
F = 176-178°C.
$[alpha]_D$ +73° ± 2,5° (c = 0,5 % $H_2O$).

| Analyse pour $C_{24}H_{27}ON_2F_3,C_6H_6O_4$ | | | | |
|---|---|---|---|---|
| | C % | H % | N % | F % |
| % calculés | 61,00 | 5,63 | 4,74 | 9,65 |
| % trouvés | 60,8 | 5,6 | 4,7 | 9,9 |

**EXEMPLE 20 : fumarate de (S)-1-[1-phényl 2-(1-pyrrolidinyl) éthyl] 3-(4-trifluorométhyl) phényl) 2-pipéridinone (isomère A et isomère B) et fumarate de l'isomère B**

On dissout, 1,16 g d'isomère B sous forme de base obtenue à l'exemple 19, dans 2 $cm^3$ d'éthanol, ajoute 333 mg d'acide fumarique et chauffe au reflux jusqu'à complète dissolution. On refroidit à température ambiante, essore les cristaux formés, les rince dans un mélange éthanol-éther puis éther. On sèche à 80 °C, sous pression réduite, et obtient 989 mg de produit attendu. F = 168-170 °C.
$[alpha]_D$ +150 ° ± 3,5 ° (c = 0,5 % $H_2O$).

| Analyse pour $C_{24}H_{27}F_3N_2O,1,5C_4H_2O_4$ | | | | |
|---|---|---|---|---|
| | C % | H % | N % | F % |
| % calculés | 63,15 | 5,87 | 5,26 | 10,70 |
| % trouvés | 62,9 | 5,8 | 5,3 | 10,6 |

**EXEMPLE 21 : (S)-3-(((3,4-dichlorophényl) acétyl) 1-(2-méthyl 1-((1-pyrrolidinyl) méthyl) propyl) amino) propanoate d'éthyle.**

STADE A : (S)-3-((2-méthyl 1-(1-pyrrolidinyl) méthyl) propyl) amino) propanoate d'éthyle.

On opère comme au stade A de l'exemple 1, en utilisant 18,24 g de 2-méthyl 1-((1-pyrrolidinyl) méthyl) propylamine et 15,1 $cm^3$ d'acrylate d'éthyle. On obtient 29 g de produit attendu.
$[alpha]_D$ +43,5 ° ± 1,5 ° (c = 1 % méthanol).

STADE B : (S)-3-(((3,4-dichlorophényl) acétyl) 1-(2-méthyl 1-((1-pyrrolidinyl) méthyl) propyl) amino) propanoate d'éthyle.

On opère comme au stade B de l'exemple 1 au départ de 28,7 g d'acide 3,4-dichlorophénylacétique, 27,52 g de produit obtenu au stade A et 22,7 g de carbonyldiimidazole. On obtient 57,2 g de produit brut que l'on purifie par chromatographie sur silice (éluant : acétate d'éthyle-méthanol (95-5)) et on obtient 39,2 g de produit attendu.
$[alpha]_D$ +26,5 ° ± 1,5 ° (c = 0,8 % méthanol).

Préparation de la 2-méthyl 1-((1-pyrrolidinyl) méthyl) propylamine utilisée au départ de l'exemple 21.

STADE A : 1-[3-méthyl (S)-2-[[(phénylméthoxy) carbonyl] amino] butanoyl] pyrrolidine

On mélange à température ambiante 32,6 g de 3-méthyl (S)-2-[[(phénylméthoxy) carbonyl] amino] butanoïque, 20 g d'hydroxybenzotriazole hydraté dans 326 $cm^3$ de chlorure de méthylène, refroidit à 0 °C et ajoute en 20 minutes une solution glacée comprenant 30 g de dicyclohexylcarbodiimide dans 128 $cm^3$ de chlorure de méthylène. On agite 1 heure à O °/+5 °C, ajoute en 10 minutes 15 $cm^3$ de pyrrolidine, et agite 20 heures en laissant revenir à température ambiante. On filtre, rince au chlorure de méthylène puis à l'éther et concentre à sec sous pression réduite.

Après chromatographie sur silice (éluant : acétate d'éthyle à 2 % de triéthylamine, on obtient 42,88 g de produit attendu utilisé tel quel pour le stade suivant.
$[alpha]_D$ -6 ° ± 1 ° (c = 1 % méthanol).

STADE B : 1-[(S)-2-amino 3-méthyl butanoyl] pyrrolidine

On hydrogène sous 1880 mbars pendant 3 heures, un mélange comprenant 42,88 g de produit obtenu au stade A dans 429 $cm^3$ d'éthanol et 17 $cm^3$ d'acide chlorhydrique en présence de 4,22 g de charbon actif à 10 % de palladium. On filtre, concentre le filtrat sous pression réduite. On amorce la cristallisation, reprend le résidu avec 50 $cm^3$ d'acétate d'éthyle, essore les cristaux de produit attendu sous forme de chlorhydrate, les dissout dans 25 $cm^3$ d'eau et ajoute 25 $cm^3$ de lessive de soude. On extrait au chlorure de méthylène, sèche et élimine le solvant sous pression réduite. On obtient 19,79 g de produit attendu, utilisé tel quel pour le stade suivant.

[alpha]$_D$ +35,5° ± 2,5° (c = 0,3 % méthanol).

STADE C : 2-méthyl 1-((1-pyrrolidinyl) méthyl) propylamine

On introduit 8,2 g d'hydrure d'aluminium lithium dans 400 cm$^3$ de tétrahydrofuranne refroidi à -10°C, puis ajoute en 20 minutes une solution comprenant 19,79 g de produit obtenu au stade B dans 100 cm$^3$ de tétrahydrofuranne, refroidie à -5°/-8°C. On agite 2 heures et demie en maintenant à 0°/+5°C, et ajoute 30 cm$^3$ d'une solution aqueuse de carbonate de sodium à 20 % puis agite 20 heures à température ambiante.

On filtre, concentre à sec le filtrat sous pression réduite et recueille 17,37 g de produit attendu, utilisé tel quel pour la suite de la synthèse.
[alpha]$_D$ +45,5° ± 1,5° (c = 1 % méthanol).

**EXEMPLE 22 : (S)-3-(3,4-dichlorophényl) 1-(2-méthyl 1-((1-pyrrolidinyl) méthyl) propyl) 2,4-pipéridinedione**

On opère comme à l'exemple 2 à partir de 16,71 g du produit obtenu à l'exemple 21 en utilisant 2,76 g d'hydrure de sodium. On obtient 13,37 g de produit attendu utilisé tel quel pour l'exemple suivant.
[alpha]$_D$ +23° ± 1,5° (c = 1 % méthanol).

**EXEMPLE 23 : (S)-3-(3,4-dichlorophényl) 5,6-dihydro 1-(2-méthyl 1-((1-pyrrolidinyl) méthyl) propyl) 2(1H) -pipéridinone**

STADE A : (S)-2-[3-(3,4-dichlorophényl) 2-oxo 1-(2-méthyl 1-((1-pyrrolidinyl) méthyl) propyl) 1,2,5,6-tétrahydro 4-pyridyl] hydrazide de l'acide 4-méthyl benzènesulfonique.

On opère comme au stade A de l'exemple 13 à partir de 11,85 g du produit obtenu à l'exemple 23 et 7,70 g de p.toluènesulfonidrazide et obtient 18,09 g de produit attendu que l'on utilise tel que pour le stade suivant.

STADE B : (S)-3-(3,4-dichlorophényl) 5,6-dihydro 1-(2-méthyl 1-((1-pyrrolidinyl) méthyl) propyl) 2(1H)-pipéridinone

On opère comme au stade B de l'exemple 3 à partir de 18,09 g de produit obtnu au stade A, 3,15 g de sodium et 130 cm$^3$ d'éthylène glycol. On obtient après chromatographie sur silice (éluant : acétate d'éthyle à 2 % de triéthylamine, 11,24 g de produit attendu sous forme de base). En opérant comme à l'exemple 13 à partir de 11,24 g de base et 3,57 g d'acide fumarique, on obtient 17 g de fumarate. F = 166-168°C.

Retour à la base.

On reprend les 17 g de produit obtenu ci-dessus dans 100 cm$^3$ d'acétate d'éthyle et 20 cm$^3$ d'eau et ajoute lentement 2,5 g de carbonate de sodium. On agite, décante, extrait à l'acétate d'éthyle, lave à l'eau salée, sèche et élimine les solvants sous pression réduite. On obtient 5,54 g de produit attendu sous forme de base.
[alpha]$_D$ +7,5° ± 1° (c = 1 % méthanol).

**EXEMPLE 24 : (S)-3-(3,4-dichlorophényl) 1-(2-méthyl 1-((1-pyrrolidinyl) méthyl) propyl) 2-pipéridinone isomère A, isomère B et maléate de l'isomère A**

On opère comme à l'exemple 19 à partir de 3,25 g de la base obtenue à l'exemple 23 et 0,77 g de borohydrure de sodium et obtient 3,17 g de produit brut. Après la chromatographie, on obtient 1,44 g d'isomère A et 1,37 g d'isomère B.

Préparation du maléate de l'isomère A :

On dissout 1,55 g d'isomère A obtenu comme précédemment dans 5 cm$^3$ d'éthanol, ajoute 515 mg d'acide maléique en solution dans 5 cm$^3$ d'éthanol, amorce la cristallisation, essore, rince à l'éthanol puis à l'éther et obtient 1,85 g de produit attendu.

$[alpha]_D$ +81° ± 2° (c = 0,5 % diméthylformamide).

| Analyse | | | | |
|---|---|---|---|---|
| | C % | H % | N % | Cl % |
| % calculés<br>% trouvés | 57,72<br>58,0 | 6,46<br>6,5 | 5,61<br>5,6 | 14,20<br>14,1 |

**EXEMPLE 25 : Maléate de (S)-3-(3,4-dichlorophényl) 1-(2-méthyl 1-((1-pyrrolidinyl) méthyl) propyl) 2-pipéridinone isomère B**

On dissout à chaud, 1,46 g de l'isomère B, obtenu à l'exemple 24 dans une solution comprenant 486 mg d'acide maléique dans 5 $cm^3$ d'acétate d'éthyle.

On amorce la cristallisation, essore les cristaux formés, les rince à l'acétate d'éthyle puis à l'éther et les sèche sous pression réduite à 80°C. On obtient 1,77 g de maléate attendu.
$[alpha]_D$ -60,5° ± 2,5° (c = 0,5 % diméthylformamide).

| Analyse | | | | |
|---|---|---|---|---|
| | C % | H % | N % | Cl % |
| % calculés<br>% trouvés | 57,72<br>57,7 | 6,46<br>6,4 | 5,61<br>5,4 | 14,20<br>13,8 |

**EXEMPLE 26 : (S)-3-phényl acétyl (1-phényl 2-(1-pyrrolidinyl) éthyl amino) propanoate d'éthyle**

On opère comme au stade B de l'exemple 1 en utilisant 3,54 g d'acide phényl acétique, 4,22 g de carbonyldiimidazole et 7,27 g de dichlorhydrate du (S)-3-((1-phényl 2-(1-pyrrolidinyl) éthyl) amino) propanoate d'éthyle préparé comme au stade A de l'exemple 11 en maintenant le milieu réactionnel sous agitation pendant 20 heures à température ambiante.

Après chromatographie sur silice (éluant : acétate d'éthyle) on obtient 8,15 g de produit attendu.
$[alpha]_D$ +96,5° ± 2° (c = 1 % méthanol).

**EXEMPLE 27 : (S)-3-phényl 1-(1-phényl 2-(1-pyrrolidinyl) éthyl 2,4-pipéridine dione**

On opère comme à l'exemple 2 à partir de 7,955 g de produit obtenu à l'exemple 26 et 1,12 g d'hydrure de sodium.

On obtient 6,70 g de produit attendu utilisé tel quel pour l'exemple suivant.

**EXEMPLE 28 : (S)-3-phényl 5,6-dihydro 1-(1-phényl 2-(1-pyrrolidinyl) éthyl) 2(1H)-piridinone**

STADE A : (S)-3-phényl 2-oxo 1-(1-phényl 2-(1-pyrrolidinyl) éthyl) 1,2,5,6-tétrahydro 4-pyridyl) hydrazide de l'acide 4 méthyl benzènesulfonique.

On opère comme au stade A de l'exemple 13 à partir de 6,70 g du produit obtenu à l'exemple 27 et 4,50 g de p.toluène sulfonydrazide et obtient 10,40 g de produit attendu que l'on utilise tel que pour le stade suivant.

STADE B : (S)-3-phényl 5,6-dihydro 1-(1-phényl 2-(1-pyrrolidinyl) éthyl) 2(1H)-piridinone

On opère comme au stade B de l'exemple 3 à partir de 10,40 g de produit obtenu au stade A, 2,0 g de sodium et 80 $cm^3$ d'éthylène glycol. On obtient après cristallisation dans l'éther isopropylique 2,75 g de produit attendu sous forme de base.
$[alpha]_D$ +177° ± 3° (c = 1 % méthanol).

**EXEMPLE 29 : (S)-3-phényl 1-(1-phényl 2-(1-pyrrolidinyl) éthyl) 2-pipéridinone (isomère A, isomère B et fumarate de l'isomère A)**

On opère comme à l'exemple 19 à partir de 2,66 g de produit obtenu à l'exemple 28 et 0,62 g de borohydrure de sodium. On obtient 2,7 g de produit brut. Après la chromatographie, on récupère 1,59 g d'isomère A et 1,05 g d'isomère B.

Préparation du fumarate de l'isomère A

On opère comme à l'exemple 19 à partir de 1,55 g de l'isomère A préparé ci-dessus et 568 mg d'acide fumarique. On obtient 1,92 g de fumarate attendu. F = 191°-193°C. $[alpha]_D$ +97,5° ± 2° (c = 1 % diméthylformamide).

| Analyse | | | |
|---|---|---|---|
| | C % | H % | N % |
| % calculés<br>% trouvés | 69,81<br>69,7 | 6,94<br>7,0 | 6,03<br>6,0 |

**EXEMPLE 30 : Fumarate de (S)-3-phényl 1-(1-phényl 2-(1-pyrrolidinyl) éthyl) 2-pipéridinone de l'isomère B**

On opère comme à l'exemple 20 à partir de 9,81 mg de l'isomère B préparé à l'exemple 29 et 325 mg d'acide fumarique. On obtient 1,07 g de fumarate attendu. F = 200°-202°C.
$[alpha]_D$ +116° ± 2° (c = 1 % diméthylformamide).

| Analyse | | | |
|---|---|---|---|
| | C % | H % | N % |
| % calculés<br>% trouvés | 69,81<br>69,5 | 6,94<br>7,0 | 6,03<br>6,0 |

**EXEMPLE 31 : (S)-3-((benzo(b)thien-4-yl) acétyl) 1-(1-phényl 2-(1-pyrrolidinyl) éthyl) amino) propanoate d'éthyle**

On opère comme au stade B de l'exemple 1 en utilisant 5 g d'acide 4-thianaphtène acétique, 4,22 g de carbonyldiimidazole et 7,27 g de dichlorhydrate du (S)-3-[[1-phényl 2-(1-pyrrolidinyl) éthyl] amino] propanoate d'éthyle préparé comme au stade A de l'exemple 11 en maintenant le milieu réactionnel sous agitation pendant 20 heures à température ambiante.

Après chromatographie sur silice (éluant : acétate d'éthyle) on obtient 9,66 g de produit attendu. $[alpha]_D$ +79° ± 2° (c = 1 % méthanol).

**EXEMPLE 32 : (S)-3-(benzo(b)thien-4-yl) 1-(1-phényl 2-(1-pyrrolidinyl) éthyl) 2,4-pipéridinedione**

On opère comme à l'exemple 2 à partir de 9,47 g du produit obtenu à l'exemple 31 en utilisant 1,15 g d'hydrure de sodium. On obtient 10,45 g de produit attendu utilisé tel quel pour l'exemple suivant.

**EXEMPLE 33 : (S)-3-(benzo(b)thien-4-yl) 5,6-dihydro 1-(1-phényl 2-(1-pyrrolidinyl) éthyl) 2(1H)-pyridinone et son fumarate**

STADE A : (S)-2-[3-(benzo(b)thien-4-yl) 2-oxo 1-[1-phényl 2-(1-pyrrolidinyl) éthyl] 1,2,5,6-tétrahydro 4-pyridyl] hydrazide de l'acide 4-méthyl benzènesulfonique.

On opère comme au stade A de l'exemple 13 à partir de 10,45 g du produit obtenu à l'exemple 32 et 4,86 g de p.toluène sulfonydrazide et obtient 15,03 g de produit attendu que l'on utilise tel que pour le stade suivant.

STADE B : (S)-3-(benzo(b)thien-4-yl) 5,6-dihydro 1-(1-phényl 2-(1-pyrrolidinyl) éthyl) 2(1H)-pyridinone et son fumarate.

On opère comme au stade B de l'exemple 3 à partir de 15,03 g de produit obtenu au stade A, 2,16 g de sodium et 85 $cm^3$ d'éthylène glycol. On obtient après chromatographie sur silice (éluant : acétate d'éthyle) 8,29 g de produit attendu sous forme de base. En opérant comme à l'exemple 13 à partir de 8,26 g de base et 2,5 g d'acide fumarique, on obtient 6,82 g de fumarate attendu. F = 194°-195°C.

Retour à la base :

On reprend 5,66 g du fumarate ci-dessus dans 20 $cm^3$ d'eau et ajoute 30 $cm^3$ d'une solution aqueuse à 20 % de carbonate de sodium et 100 $cm^3$ d'acétate d'éthyle. On agite, décante, extrait à l'acétate d'éthyle, sèche et concentre à sec sous pression réduite. On récupère 4,93 g de produit attendu sous forme de base, utilisé tel quel pour l'exemple suivant.

**EXEMPLE 34 : (S)-3-(benzo(b)thien-4-yl) 1-(1-phényl 2-(1-pyrrolidinyl) éthyl) 2-pipéridinone (isomère A, isomère B et fumarate de l'isomère A)**

On opère comme à l'exemple 19 à partir de 4,93 g de produit obtenu à l'exemple 33 et 3 fois 1 g de borohydrure de sodium. On obtient 5,23 g de produit brut. Après la chromatographie, on récupère 2,64 g d'isomère A et 2,07 g d'isomère B.

Préparation du fumarate de l'isomère A :

On opère comme à l'exemple 19, à partir de 2,55 g de l'isomère A préparé ci-dessus et 823 mg d'acide fumarique. On obtient 2,79 g de fumarate attendu. F = 234°-238°C.
$[alpha]_D$ +110° ± 2° (c = 1 % diméthylformamide).

| Analyse | | | | |
|---|---|---|---|---|
| | C % | H % | N % | S % |
| % calculés<br>% trouvés | 66,90<br>67,0 | 6,19<br>6,2 | 5,38<br>5,3 | 6,16<br>6,2 |

**EXEMPLE 35 : Fumarate de (S)-3-(benzo(b)thien-4-yl) 1-(1-phényl 2-(1-pyrrolidinyl) éthyl) 2-pipéridinone (isomère B)**

On opère comme à l'exemple 20 à partir de 1,98 g de l'isomère B préparé à l'exemple 34 et 628 mg d'acide fumarique. On obtient 1,38 g de fumarate attendu. F = 195°-197°C. $[alpha]_D$ +110° ± 2° (c = 1 % diméthylformamide).

| Analyse | | | | |
|---|---|---|---|---|
| | C % | H % | N % | S % |
| % calculés<br>% trouvés | 66,90<br>67,0 | 6,19<br>6,2 | 5,38<br>5,3 | 6,16<br>6,2 |

**EXEMPLE 36 : (S)-3-(3,4-dichlorophényl) 3-méthyl 1-(1-phényl 2-(1-pyrrolidinyl) éthyl) 2-pipéridinone (isomère A, isomère B et maléate de l'isomère A)**

On refroidit à -20°C, 2,67 g de (S)-3-(3,4-dichlorophényl) 1-(1-phényl 2-(1-pyrrolidinyl) éthyl) 2-pipéridinone brut obtenu à l'exemple 14 dans 40 $cm^3$ de tétrahydrofuranne et ajoute 1,16 g de terbutylate de potassium puis agite 1 heure la solution à -8° ± 2°C. On refroidit à -15°C, ajoute 0,6 $cm^3$ d'iodure de méthyle puis agite 2 heures et demie à -7°/-10°C. On laisse revenir la température à 0°C, ajoute 50 $cm^3$ d'eau glacée, extrait à l'acétate d'éthyle, sèche et concentre à sec sous pression réduite. On obtient 2,83 g de produit brut sous forme de base que l'on chromatographie sur silice (éluant : acétate d'éthyle n-hexane (9-1) puis acétate d'éthyle à 1 % de triéthylamine). On obtient 0,996 g d'isomère A et 1,307 g d'isomère B.

Maléate de l'isomère A :

On dissout en tiédissant 947 mg d'isomère A ci-dessus et 304 mg d'acide maléique dans 4 $cm^3$ d'acétate d'éthyle. On filtre, ajoute au filtrat 3 $cm^3$ d'éther éthylique, amorce la cristallisation, essore les cristaux, les rince à l'acétate d'éthyle et à l'éther, les sèche à 70°C sous pression réduite et recueille 823 mg du maléate attendu. F = 136°-138°C.
$[alpha]_D$ +60,5° ± 1,5° (c = 1 % méthanol).

| Analyse | | | | |
|---|---|---|---|---|
| | C % | H % | N % | Cl % |
| % calculés<br>% trouvés | 61,43<br>61,6 | 5,89<br>6,0 | 5,12<br>5,1 | 12,95<br>12,8 |

**EXEMPLE 37 : Fumarate de (S)-3-(3,4-dichlorophényl) 3-méthyl 1-(1-phényl 2-(1-pyrrolidinyl) éthyl) 2-pipéridinone (isomère B)**

On dissout en tiédissant 1,24 g d'isomère B obtenu à l'exemple 36 dans 5 $cm^3$ d'éthanol, ajoute 370 mg d'acide fumarique et chauffe au reflux jusqu'à totale dissolution. On refroidit, amorce la cristallisation, essore les cristaux, les rince à l'éthanol puis à l'éther, les sèche à 70°C sous pression réduite et recueille 1,25 g de fumarate attendu. F = 175°-176°C.
$[alpha]_D$ +125° ± 2° (c = 1 % méthanol).

| Analyse | | | | |
|---|---|---|---|---|
| | C % | H % | N % | Cl % |
| % calculés<br>% trouvés | 61,43<br>61,5 | 5,89<br>5,8 | 5,12<br>5,0 | 12,95<br>12,8 |

**EXEMPLE 38 : (1S)-3-(3,4-dichlorophényl) 3-éthyl 1-(1-phényl 2-(1-pyrrolidinyl) éthyl) 2-pipéridinone (isomère B, isomère A et méthane sulfonate de l'isomère B)**

On opère comme à l'exemple 36 au départ de 2 g du produit obtenu à l'exemple 14, 0,87 g de terbutylate de potassium et 0,6 $cm^3$ d'iodure d'éthyle. On obtient 2,1 g de produit brut sous forme de base, que l'on chromatographie sur silice (éluant : acétate d'éthyl-chlorure de méthylène (85-15)).

On obtient 1,05 g d'isomère A et 0,87 g d'isomère B.

On empâte 0,87 g d'isomère B dans 4 $cm^3$ d'un mélange éther isopropylique-n-hexane (1-1), essore le produit cristallisé, sèche et obtient 0,567 g de produit attendu isomère B. F = 113-114 °C.

Préparation du méthane sulfonate de l'isomère B :

On dissout à température ambiante 542 mg d'isomère B dans 2 $cm^3$ d'éthanol et 0,7 $cm^3$ d'une solution éthanolique d'acide méthane sulfonique 2M, amorce la cristallisation, dilue avec 1 $cm^3$ d'éther éthylique, essore et sèche les cristaux sous pression réduite) 70 °C. On obtient 268 mg de méthane sulfonate attendu. F = 207-209 °C.
$[alpha]_D$ +107 ° ± 2 ° (c = 1 % méthanol).

| Analyse | | | | | |
|---|---|---|---|---|---|
| | C % | H % | N % | S % | Cl % |
| % calculés | 57,67 | 6,33 | 5,17 | 5,92 | 13,09 |
| % trouvés | 57,7 | 6,4 | 5,1 | 5,9 | 13,4 |

**EXEMPLE 39 : Fumarate de (1S)-3-(3,4-dichlorophényl) 3-éthyl 1-(1-phényl 2-(1-pyrrolidinyl) éthyl) 2-pipéridinone (isomère A)**

On empâte 1,05 g d'isomère A obtenu à l'exemple 38 dans 4 $cm^3$ d'éther isopropylique à température ambiante, essore les cristaux, les rince avec du n-hexane, et sèche sous pression réduite. On recueille 599 mg de produit cristallisé sous forme de base. F = 123 °-124 °C. On opère comme à l'exemple 37, à partir de 587 mg de base et 168 mg d'acide fumarique. On obtient 559 mg de fumarate attendu. F = 148-150 °C.
$[alpha]_D$ +46,5 ° ± 1,5 ° (c = 1 % méthanol).

| Analyse | | | | |
|---|---|---|---|---|
| | C % | H % | N % | Cl % |
| % calculés | 62,02 | 6,10 | 4,99 | 12,63 |
| % trouvés | 61,9 | 6,1 | 4,8 | 12,9 |

**EXEMPLE 40 : (1S)-3-(3,4-dichlorophényl) 3-(phénylméthyl) 1-(1-phényl 2-(1-pyrrolidinyl) éthyl) 2-pipéridinone (isomère B, isomère A et fumarate de l'isomère B)**

On opère comme à l'exemple 36 au départ de 2,76 g de produit obtenu à l'exemple 14, 0,98 g de terbutylate de potassium et 1,2 $cm^3$ de bromure de benzyle. On obtient 3,22 g de produit attendu sous forme de base. Après chromatographie sur silice (éluant : acétate d'éthyle-chlorure de méthylène (75-15)), on obtient 668 mg d'isomère A et 533 mg d'isomère B.

Préparation du fumarate de l'isomère B :

On opère comme à l'exemple 37 à partir de 455 mg d'isomère B dissous dans 5 $cm^3$ d'isopropanol et 110 mg d'acide fumarique. On obtient 350 mg de fumarate attendu.
F = 195-197 °C.
$[alpha]_D$ +57,5 ° ± 1,5 ° (c = 1 % diméthylformamide)

| Analyse | | | | |
|---|---|---|---|---|
| | C % | H % | N % | Cl % |
| % calculés<br>% trouvés | 65,49<br>65,3 | 5,82<br>5,9 | 4,49<br>4,5 | 11,37<br>11,3 |

**EXEMPLE 41 : Chlorhydrate de (1S)-3-(3,4-dichlorophényl) 3-(phénylméthyl) 1-(1-phényl 2-(1-pyrrolidinyl) éthyl) 2-pipéridinone (isomère A)**

On dissout 462 mg de base isomère A obtenue à l'exemple 40, dans 4,5 $cm^3$ d'éthanol à température ambiante et ajoute 0,5 $cm^3$ d'une solution éthanolique de chlorure d'hydrogène 6,6 N. On amorce la cristallisation, essore et sèche les cristaux à 80°C sous pression réduite et recueille 340 mg de chlorhydrate attendu.
F = 206-208°C.
$[alpha]_D$ +41° ± 1,5° (c = 1 % diméthylformamide)

| Analyse | | | | |
|---|---|---|---|---|
| | C % | H % | N % | Cl % |
| % calculés<br>% trouvés | 66,24<br>66,0 | 6,11<br>6,2 | 5,15<br>5,1 | 19,55<br>19,2 |

**EXEMPLE 42 : (1S)-3-(3,4-dichlorophényl) 1-(1-phényl 2-(1-pyrrolidinyl) éthyl) 3-(2-propényl) 2-pipéridinone (isomère A, isomère B et méthanesulfonate de l'isomère A)**

On opère comme à l'exemple 36 au départ de 2,01 g du produit obtenu à l'exemple 14, 0,9 g de terbutylate de potassium et 0,65 $cm^3$ de bromure d'allyle. On obtient 2,28 g de produit brut attendu sous forme de base. Après chromatographie sur silice (éluant : acétate d'éthyle-chlorure de méthylène-n-hexane (3-4-3)) on obtient 868 mg d'isomère A et 940 mg d'isomère B.

Préparation du méthanesulfonate :

On opère comme à l'exemple 38 à partir de 806 mg d'isomère A et 1 $cm^3$ d'une solution éthanolique d'acide méthane sulfonique (≃ 2M) et obtient 628 mg de méthanesulfonate attendu. F = 171-172°C.
$[alpha]_D$ +41° ± 1,5° (c = 1 % diméthylformamide)

| Analyse | | | | | |
|---|---|---|---|---|---|
| | C % | H % | N % | S % | Cl % |
| % calculés<br>% trouvés | 58,58<br>58,7 | 6,19<br>6,3 | 5,06<br>5,2 | 5,79<br>5,7 | 12,81<br>12,8 |

**EXEMPLE 43 : Méthanesulfonate de (1S)-3-(3,4-dichlorophényl) 1-(1-phényl 2-(1-pyrrolidinyl) éthyl) 3-(2-propényl) 2-pipéridinone (isomère B)**

On opère comme à l'exemple 38 à partir de 913 mg d'isomère B, obtenu à l'exemple 42 et 1,2 $cm^3$ de solution éthanolique d'acide méthane sulfonique (2M) et obtient 780 mg de méthanesulfonate attendu. F = 239-241°C.
$[alpha]_D$ +92° ± 2° (c = 1 % diméthylformamide)

| Analyse | | | | | |
|---|---|---|---|---|---|
| | C % | H % | N % | S % | Cl % |
| % calculés<br>% trouvés | 58,58<br>58,9 | 6,19<br>6,3 | 5,06<br>5,1 | 5,79<br>5,8 | 12,81<br>12,6 |

Exemples de composition pharmaceutique.

**EXEMPLE 44 :**

On a préparé des comprimés répondant à la formule suivante :

| | |
|---|---|
| - produit de l'exemple 5<br>- excipient q.s.p. | 200 mg<br>800 mg |
| (détail de l'excipient : lactose, talc, amidon, stéarate de magnésium). | |

**EXEMPLE 45 :**

On a préparé un soluté injectable (voie intra-musculaire) répondant à la formule suivante :

| | |
|---|---|
| - produit de l'exemple 5<br>- solvant stérile q.s.p. | 50 mg<br>5 $cm^3$. |

**ETUDE PHARMACOLOGIOUE**

1) Liaison au récepteur opiacé K in vitro.

On utilise des culots membranaires conservés à -30°C (éventuellement pendant environ 30 jours) et préparés à partir de cervelles de cobayes.

Ces culots sont remis en suspension dans le tampon Tris pH 7,7. On répartit les fractions de 2 $cm^3$ dans des tubes à hémolyse et ajoute de la $9^3$H-éthylkétocyclazocine 1 nM et le produit à étudier. Le produit est d'abord testé à 5 x $10^{-6}$ M (en triple). Lorsque le produit testé déplace de plus de 50 % la radioactivité liée spécifiquement au récepteur, il est testé à nouveau selon une gamme de 7 doses afin de déterminer la dose qui inhibe de 50 % la radioactivité liée spécifiquement au récepteur. On détermine ainsi la concentration inhibitrice 50 %.

La liaison non spécifique est déterminée par addition de produit connu sous le nom U-50488 H (Lahti et al. 1982, Life Sci. 31, 2257) à $10^{-5}$ M (en triple). On incube à 25°C pendant 40 minutes, remet au bain-marie à 0°C, 5 minutes, filtre sous vide, rince au tampon Tris pH 7,7 et compte la radioactivité en présence de scintillant Trition.

Le résultat est exprimé directement en concentration inhibitrice 50 % ($CI_{50}$), c'est-à-dire en concentration de produit étudié, exprimée en nM, nécessaire pour déplacer 50 % de la radioactivité spécifique fixée sur le récepteur étudié.

| Produit de l'exemple | $CI_{50}$ en nM |
|---|---|
| 8 | 9 |
| 7 | 0,2 |
| 4 | 22 |

2) Action antiarythmique chez le rat.

On trachéotomise des rats mâles pesant 300-350 g anesthésiés par voie intrapéritonéale à l'aide de 1,20 g/kg d'uréthane et les soumet à une respiration artificielle (40-50 insufflations de 3 ml/minute).

On implante des aiguilles en sous-cutané de manière à enregistrer l'électrocardiogramme des rats sur le signal de dérivation DII.

On administre les produits à tester par voie intraveineuse. Cinq minutes après l'administration du produit, on perfuse par la veine jugulaire l'aconitine à 10 microgrammes/mn et on note le temps d'apparition des troubles du rythme cardiaque.

Les résultats sont exprimés en pourcentage d'allongement du temps d'apparition des troubles du rythme cardiaque par rapport aux témoins et en fonction de la dose du produit testé.

Les résultats figurant sur le tableau ci-après montrent que certains des produits de la présente demande sont doués de bonnes propriétés antiarythmiques.

| Produit de l'exemple | Dose mg/kg | Pourcentage d'allongement du temps |
|---|---|---|
| 4 | 5<br>2,5 | 48 %<br>23 % |
| 3 | 5<br>2,5 | 55 %<br>52 % |
| 5 | 5<br>2,5 | 33 %<br>26 % |

**Revendications**
**Revendications pour les Etats contractants suivants : AT, BE, CH, DE, DK, FR, GB, IT, LI, LU, NL, SE**

**1.** Composés de formule (I) :

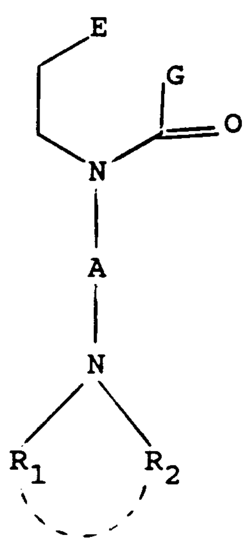

(I)

dans laquelle E et G sont tels que :

- soit E et G forment ensemble un groupement

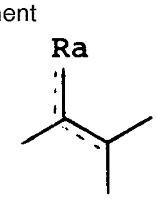

lequel représente l'un des groupements suivants :

a) 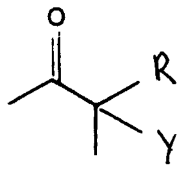 ou, le cas échéant, son tautomère 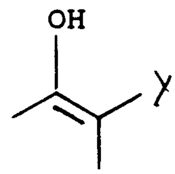 ,

dans lequel R représente un atome d'hydrogène, un radical alkyle linéaire ou ramifié renfermant de 1 à 6 atomes de carbone, un radical alkényle ou alkynyle linéaire ou ramifié renfermant de 2 à 6 atomes de carbone éventuellement substitué par un ou plusieurs substituants choisis soit parmi les radicaux aryle renfermant de 6 à 14 atomes de carbone, aryle substitué par un atome d'halogène, un radical hydroxy, trifluorométhyle, alkoxy renfermant de 1 à 6 atomes de carbone, nitro, amino et cyano, soit parmi les radicaux carboxy libre, estérifié ou salifié, acyle renfermant de 1 à 7 atomes de carbone, alcoxy renfermant de 1 à 6 atomes de carbone, hydroxy, amino, alkylamino, dialkylamino, acyloxy renfermant de 1 à 7 atomes de carbone, cyano, carbamoyle et les atomes d'halogènes

-- Y représente un radical aryle choisi parmi les radicaux aryles carbocycliques renfermant de 6 à 14 atomes de carbone, les radicaux aryles hétéromonocycliques renfermant 5 à 7 chaînons et les radicaux aryles hétérocycliques condensés, tous ces radicaux étant éventuellement substitués,

b) 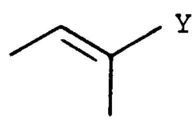 ou

c) 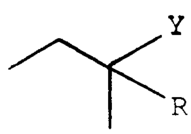

Y et R étant définis comme ci-dessus ;

- soit E représente un radical $-CO_2R_3$, dans lequel $R_3$ représente un atome d'hydrogène ou un radical alkyle linéaire ou ramifié (1 à 4 carbones) et G représente un groupement

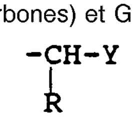

dans lequel Y et R sont définis comme ci-dessus ;

-- A représente :

- soit un radical carbocyclique, éventuellement substitué,
- soit le groupement

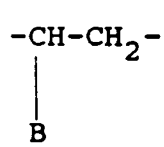

où B représente :

- un atome d'hydrogène,

- un radical aryle ou arylalkyle renfermant de 6 à 14 atomes de carbone, ces radicaux étant éventuellement substitués,
- un radical cycloalkyle renfermant de 3 à 7 atomes de carbone éventuellement substitué,
- un radical alkyle, alkényle, alkynyle ou alkyloxy, ces radicaux étant linéaires ou ramifiés, renfermant au plus 7 atomes de carbone et étant éventuellement substitués,

-- $R_1$ et $R_2$, identiques ou différents, représentent un atome d'hydrogène ou un radical alkyle, alkényle ou alkynyle, ces radicaux étant linéaires ou ramifiés, renfermant au plus 7 atomes de carbone et étant éventuellement substitués, ou bien $R_1$ et $R_2$ forment ensemble avec l'atome d'azote auquel ils sont liés un hétérocycle saturé ou insaturé à 5, 6 ou 7 chaînons comportant éventuellement un autre hétéroatome tel que l'oxygène, l'azote ou le soufre, cet hétérocycle étant éventuellement substitué,

lesdits produits de formule (I) étant sous toutes les formes isomères possibles racémiques, énantiomères et diastéréoisomères, ainsi que les sels d'addition avec les acides minéraux et organiques ou avec les basesdesdits produits de formule (I).

**2.** Les composés selon la revendication 1 répondant à la formule ($I_A$) :

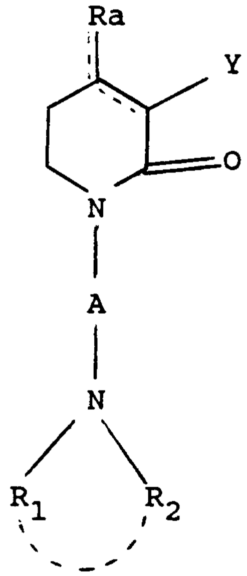

($I_A$)

dans laquelle le groupement :

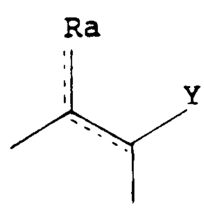

,

A, $R_1$ et $R_2$ sont définis comme à la revendication 1.

**3.** Les composés selon la revendication 2 dans laquelle le groupement

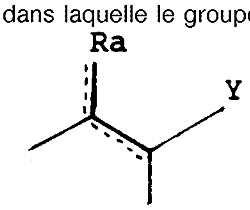

représente

a) 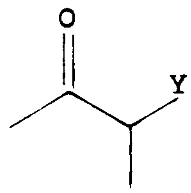 ou le cas échéant son tautomère 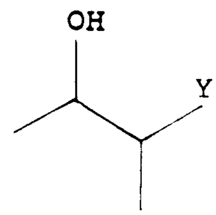

b) 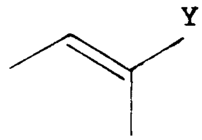

c) 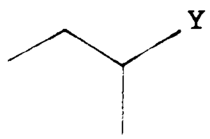

Y étant défini comme à la revendication 1.

**4.** Les composés selon la revendication 1 répondant à la formule ($I_B$) :

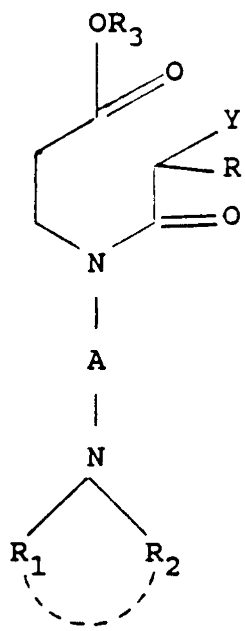

($I_B$)

dans laquelle $R_1$, $R_2$, $R_3$, A, R et Y sont définis comme à la revendication 1.

**5.** Composés de formule (I) tels que définis à l'une quelconque des revendications 1 à 4, caractérisés en ce que le ou les substituants, identiques ou différents, que peuvent porter les radicaux aryles, arylalkyles et hétérocycliques ou ceux que peuvent former $R_1$ et $R_2$ avec l'atome d'azote auquel ils sont liés, sont choisis dans le groupe formé par :

- les atomes d'halogène,
- les radicaux alkyle, alkényle, alkynyle ou alkyloxy, ces radicaux étant linéaires ou ramifiés renfermant au plus 7 atomes de carbone et étant éventuellement substitués,
- les radicaux hydroxy, trifluorométhyle, trifluorométhylthio, trifluorométhoxy, cyano, nitro, sulfamoyle, amino, monoalkyl-et dialkylamino,
- les radicaux formyle, acyle renfermant de 2 à 6 atomes de carbone et benzoyle,
- les radicaux carboxy libres, estérifiés par un radical alkyle linéaire ou ramifié renfermant au plus 5 atomes de carbone et salifiés,

- le radical carbamoyle éventuellement substitué par un ou plusieurs radicaux, identiques ou différents, choisis dans le groupe formé par les radicaux alkyle, alcoxy ou hydroxyalkyle et le radical carbamoylalkyle, ces radicaux alkyle et alcoxy renfermant de 1 à 4 atomes de carbone,
- et le radical phényle ou benzyle lui-même substitué éventuellement par un ou plusieurs radicaux, identiques ou différents, choisis dans le groupe formé par les atomes d'halogène, les radicaux hydroxy, cyano, nitro, les radicaux alkyle, alkényle, alkynyle et alcoxy linéaires ou ramifiés renfermant au plus 5 atomes de carbone,

lesdits produits de formule (I) étant sous toutes les formes isomères possibles racémiques, énantiomères et diastéréoisomères, ainsi que les sels d'addition avec les acides minéraux et organiques ou avec les bases desdits produits de formule (I).

**6.** Produits de formule (I) tels que définis à l'une quelconque des revendications 1 à 5 caractérisés en ce que le ou les substituants, identiques ou différents, que peuvent porter les radicaux alkyle, alkényle, alkynyle, cycloalkyle, carbocyclique et alcoxy sont choisis dans le groupe formé par :
- les atomes d'halogène,
- les radicaux alkyle, alkényle, alkynyle ou alcoxy renfermant au plus 5 atomes de carbone,
- les radicaux formyle, acyle renfermant de 2 à 6 atomes de carbone et benzoyle,
- les radicaux carboxy libres, estérifiés par un radical alkyle linéaire ou ramifié renfermant au plus 5 atomes de carbone et salifiés,
- les radicaux hydroxy, cyano, amino, monoalkyl- et dialkylamino,
- les radicaux aryle et arylalkyle renfermant de 6 à 14 atomes de carbone, ces radicaux étant éventuellement substitués par un ou plusieurs radicaux, identiques ou différents, choisis dans le groupe formé par les atomes d'halogène, les radicaux hydroxy, cyano, nitro, les radicaux alkyle, alkényle, alkynyle et alcoxy linéaires ou ramifiés renfermant au plus 5 atomes de carbone,

lesdits produits de formule (I) étant sous toutes les formes isomères possibles racémiques, énantiomères et diastéréoisomères, ainsi que les sels d'addition avec les acides minéraux et organiques ou avec les bases desdits produits de formule (I).

**7.** Composés de formule (I) telle que définie à l'une quelconque des revendications 1 à 6, caractérisés en ce que R, lorsqu'il représente un radical alkyle, alkényle ou alkynyle, substitué par un aryle ou un aryle substitué, est un tel radical substitué par un phényle ou un phényle substitué.

**8.** Composés de formule (I) tels que définis à l'une quelconque des revendications 1 à 7, caractérisés en ce que Y représente un radical phényle, naphtyle, thionaphtyle, benzofuryle, benzopyrrolyle ou indényle éventuellement substitué par un ou plusieurs radicaux choisis dans le groupe formé par :
- les atomes d'halogène,
- les radicaux alkyle, alkényle, alkynyle ou alcoxy linéaires ou ramifiés renfermant au plus 7 atomes de carbone et étant éventuellement substitués par un ou plusieurs radicaux choisis dans le groupe formé par le radical hydroxy et les radicaux alkyle et alcoxy linéaires ou ramifiés renfermant au plus 5 atomes de carbone,
- les radicaux acyle renfermant de 2 à 6 atomes de carbone,
- les radicaux hydroxy, trifluorométhyle, cyano, nitro, amino, les radicaux monoalkyl- et dialkylamino renfermant de 1 à 5 atomes de carbone,
- et le radical phényle lui-même substitué éventuellement par un ou plusieurs radicaux, identiques ou différents, choisis dans le groupe formé par les atomes d'halogène, les radicaux hydroxy, cyano, nitro, les radicaux alkyle, alkényle, alkynyle et alcoxy linéaires ou ramifiés renfermant au plus 5 atomes de carbone,

lesdits produits de formule (I) étant sous toutes les formes isomères possibles racémiques, énantiomères et diastéréoisomères, ainsi que les sels d'addition avec les acides minéraux et organiques ou avec les bases desdits produits de formule (I).

**9.** Produits de formule (I) tels que définis à l'une quelconque des revendications 1 à 8, caractérisés en ce que A représente :
- soit un radical cycloalkyle de formule

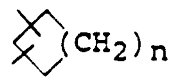

où n est un entier pouvant prendre les valeurs 0 à 4, éventuellement substitué par un radical alkyle ou alcoxy linéaire ou ramifié renfermant au plus 5 atomes de carbone,

- soit le groupement

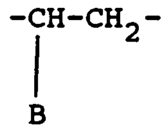

dans lequel B représente un radical phényle éventuellement substitué par un ou plusieurs radicaux, identiques ou différents, choisis dans le groupe formé par les atomes d'halogène, les radicaux hydroxy, cyano, nitro, les radicaux alkyle, alkényle, alkynyle et alcoxy linéaires ou ramifiés renfermant au plus 5 atomes de carbone,

lesdits produits de formule (I) étant sous toutes les formes isomères possibles racémiques, énantiomères et diastéréoisomères, ainsi que les sels d'addition avec les acides minéraux et organiques ou avec les bases desdits produits de formule (I).

**10.** Produits de formule (I) tels que définis à l'une quelconque des revendications 1 à 9 caractérisés en ce que l'hétérocycle que peuvent former $R_1$ et $R_2$, ensemble avec l'atome d'azote auquel ils sont liés, comporte un second atome d'azote et que celui-ci est éventuellement substitué par un radical alkyle ou alcoxy linéaire ou ramifié renfermant de 1 à 5 atomes de carbone, lesdits produits de formule (I) étant sous toutes les formes isomères possibles racémiques, énantiomères et diastéréoisomères, ainsi que les sels d'addition avec les acides minéraux et organiques ou avec les bases desdits produits de formule (I).

**11.** La (1S)-3-(3,4-dichlorophényl)-1-[1-phényl-2-(1-pyrrolidinyl) éthyl]-2-pipéridinone (isomère B)
la (1S)-3-(3,4-dichlorophényl)-1-(1-phényl-2-(1-pyrrolidinyl) éthyl)-2-pipéridinone (isomère A)
la [trans,(±)]-3-(3,4-dichlorophényl)-1-(2-(1-pyrrolidinyl) cyclohexyl)-2-pipéridinone (isomère A)
la (1S)-3-(3,4-dichlorophényl)-1-(2-méthyl-1-((1-pyrrolidinyl) méthyl) propyl)-2-pipéridinone (isomère A)
la (1S)-3-(benzo(b)thien-4-yl)-1-(1-phényl-2-(1-pyrrolidinyl) éthyl)-2-pipéridinone (isomère A)
la (1S)-3-(3,4-dichlorophényl)-3-méthyl-1-(1-phényl-2-(1-pyrrolidinyl) éthyl)-2-pipéridinone (isomère A)
la (1S)-3-(3,4-dichlorophényl)-3-éthyl-1-(1-phényl-2-(1-pyrrolidinyl) éthyl)-2-pipéridinone (isomère A)
la (1S)-3-(3,4-dichlorophényl)-1-(1-phényl-2-(1-pyrrolidinyl) éthyl)-3-(2-propényl)-2-pipéridinone (isomère A),
et le cas échéant leurs sels.

**12.** Procédé de préparation de produits de formule (I) telle que définie à la revendication 1 caractérisé en ce que l'on fait réagir la diamine de formule (II) :

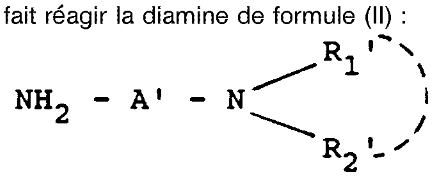

(II)

dans laquelle A′ et

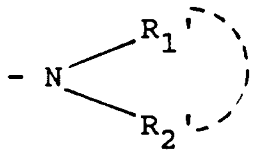

ont les significations indiquées à la revendication 1 respectivement pour A et

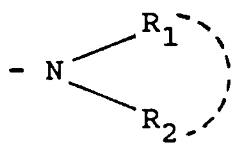

ou celles dans lesquelles les fonctions réactives que peuvent porter A et

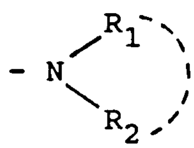

sont protégées, avec l'ester acrylique de formule (III) :

$CH_2 = CH - COOR_3$ (III)

dans laquelle $R_3$ représente un radical alkyle linéaire ou ramifié ayant au plus 6 atomes de carbone, pour obtenir le produit condensé de formule (IV) :

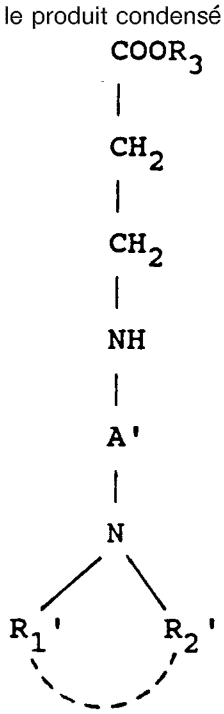

(IV)

dans laquelle A',

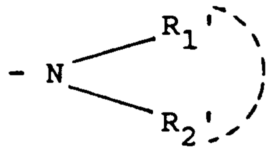

et $R_3$ ont les significations indiquées ci-dessus, que l'on fait réagir avec le composé de formule (V) :

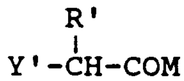

(V)

dans laquelle R' représente un atome d'hydrogène, les valeurs de R indiquées ci-dessus ou celles dans lesquelles les fonctions réactives sont protégées, M représente un radical hydroxyle ou un atome d'halogène et Y' représente les valeurs de Y indiquées ci-dessus, ou celles dans lesquelles les fonctions réactives sont protégées, pour obtenir le produit de formule (VI) :

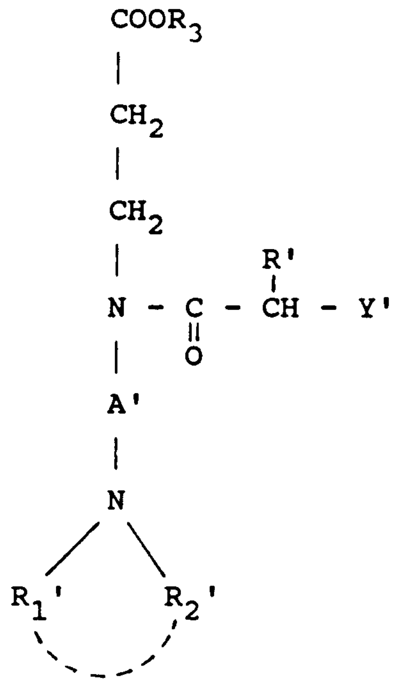

(VI)

dans laquelle A',

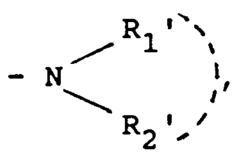

R', $R_3$ et Y' ont les significations indiquées ci-dessus, correspondant dans le cas où R' représente un atome d'hydrogène ou les valeurs de R et A', $NR'_1R'_2$ et Y' ont respectivement les valeurs de A, $NR_1R_2$ et Y, à un produit de formule ($I_B$) telle que définie ci-dessus, produit de formule (VI) dans lequel le cas échéant, l'on élimine le ou les groupements protecteurs, pour obtenir un même produit de formule ($I_B$) ou que l'on soumet éventuellement à une réaction de cyclisation pour obtenir le produit de formule (VII) :

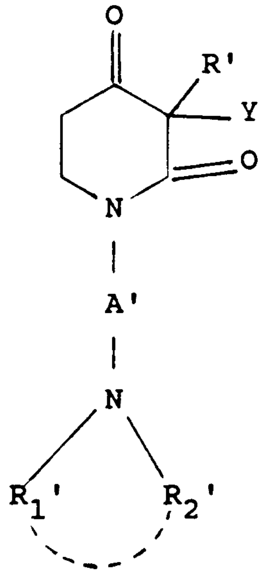

(VII)

dans laquelle A',

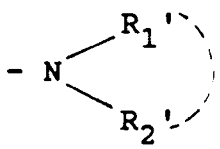

,

R' et Y' ont les significations indiquées ci-dessus, correspondant dans le cas où R' représente un atome d'hydrogène ou les valeurs de R et A', $NR'_1R'_2$ et Y' ont respectivement les valeurs de A, $NR_1R_2$ et Y, à un produit de formule ($I_A$) telle que définie ci-dessus appelé ($I_{A1}$), produit de formule (VII) dans lequel le cas échéant, l'on élimine le ou les groupements protecteurs, pour obtenir un même produit de formule ($I_{A1}$),puis, le cas échéant :

- soit, quand R' ou R représente un atome d'hydrogène, soumet ledit produit de formule (VII) ou ($I_{A1}$) :

  -- soit à une réaction de réduction de la fonction oxo en position gamma de l'atome d'azote pour obtenir le produit de formule (VIII) :

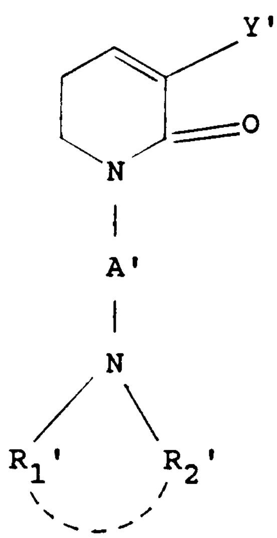

(VIII)

dans laquelle A',

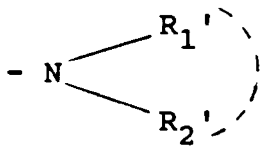

et Y' ont les significations indiquées ci-dessus, correspondant dans le cas où Y', A' et $NR'_1R'_2$ ont respectivement les valeurs de Y, A et $NR_1R_2$, à un produit de formule ($I_A$) telle que définie ci-dessus, appelé ($I_{A2}$), produit de formule (VIII) dans lequel l'on élimine, le cas échéant, les groupements protecteurs, pour obtenir un même produit de formule ($I_{A2}$),

-- soit à une réaction de réduction par hydrogénation catalytique de la même fonction oxo pour obtenir le produit de formule (IX) :

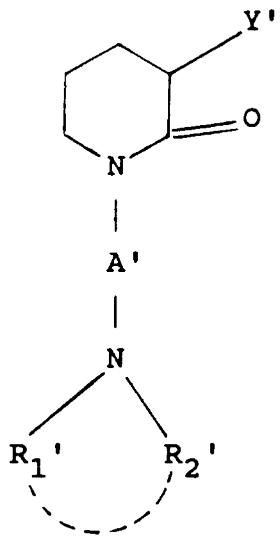

(IX)

dans laquelle A',

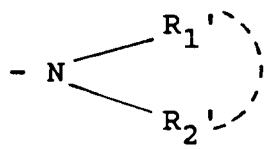

et Y' ont les significations indiquées ci-dessus, correspondant dans le cas où Y', A' et $NR'_1R'_2$ ont respectivement les valeurs de Y, A et $NR_1R_2$, à un produit de formule ($I_A$) telle que définie ci-dessus, appelé ($I_{A3}$), produit de formule (IX) dans lequel l'on élimine, le cas échéant, les groupements protecteurs, pour obtenir un même produit de formule ($I_{A3}$),produit de formule (IX) ou ($I_{A3}$) que l'on traite éventuellement au moyen d'une base forte pour former l'anion correspondant, que l'on soumet à l'action d'un réactif capable de greffer un radical R', R' ayant les valeurs indiquées ci-dessus à l'exception d'un atome d'hydrogène,

pour obtenir un produit de formule (X) :

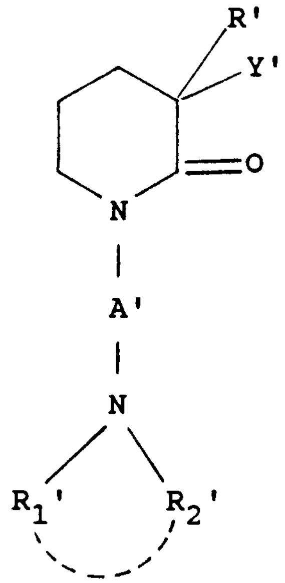

(X)

dans laquelle R′, Y′, A′ et

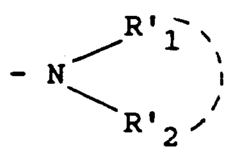

ont les valeurs indiquées ci-dessus, correspondant dans le cas où R′, Y′ A′ et $NR'_1R'_2$ ont respectivement les valeurs de R (à l'exception d'hydrogène), Y, A et $NR_1R_2$, à un produit de formule ($I_A$) telle que définie ci-dessus, appelé ($I_{A4}$), produit de formule (X) dans lequel l'on élimine, le cas échéant, les groupements protecteurs, pour obtenir un même produit de formule ($I_{A4}$) ;

- soit quand R′ ou R est différent d'un atome d'hydrogène, soumet ledit produit de formule (VII) ou ($I_{A1}$) à l'action d'un agent de réduction de la fonction oxo, pour obtenir le produit de formule (X) telle que définie précédemment qui ou bien correspond à un produit de formule ($I_{A4}$) ou bien est transformé en ce produit de formule ($I_{A4}$), et traite, si désiré, les produits de formule (I) par un acide minéral ou organique ou le cas échéant par une base, pour obtenir le sel correspondant, lesdits produits de formule (I) étant sous toutes les formes isomères possibles racémiques, énantiomères et diastéréoisomères.

**13.** A titre de médicaments, les produits répondant à la formule (I) telle que définie à la revendication 1, lesdits produits de formule (I) étant sous toutes les formes isomères possibles racémiques, énantiomères et diastéréoisomères, ainsi que les sels d'addition avec les acides minéraux et organiques ou avec les bases pharmaceutiquement acceptables desdits produits de formule (I).

**14.** A titre de médicaments, les produits de formule ($I_A$) telle que définie à la revendication 2, ainsi que les sels d'addition avec les acides minéraux et organiques ou avec les bases pharmaceutiquement acceptables desdits produits de formule ($I_A$).

**15.** A titre de médicaments, les produits de formule ($I_B$) telle que définie à la revendication 3, ainsi que les sels d'addition avec les acides minéraux et organiques ou avec les bases pharmaceutiquement acceptables desdits produits de formule ($I_B$).

**16.** A titre de médicaments les produits définis à la revendication 11.

**17.** Les compositions pharmaceutiques contenant à titre de principe actif, l'un au moins des médicaments tels que définis à l'une quelconque des revendications 13 à 16.

**18.** A titre de produits industriels, les composés de formules (IV), (VI), (VII), (VIII), (IX) et (X).

**Revendications pour l'Etat contractant suivant : ES**

**1.** Procédé de préparation des composés de formule (I) :

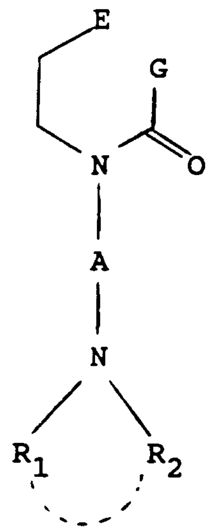

(I)

dans laquelle E et G sont tels que :

- soit E et G forment ensemble un groupement

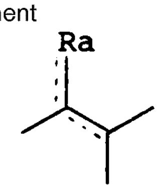

lequel représente l'un des groupements suivants :

a) 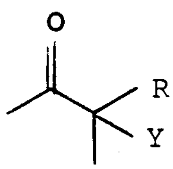 ou, le cas échéant, son tautomère 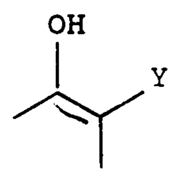 ,

dans lequel R représente un atome d'hydrogène, un radical alkyle linéaire ou ramifié renfermant de 1 à 6 atomes de carbone, un radical alkényle ou alkynyle linéaire ou ramifié renfermant de 2 à 6 atomes de carbone éventuellement substitué par un ou plusieurs substituants choisis soit parmi les radicaux aryle renfermant de 6 à 14 atomes de carbone, aryle substitué par un atome d'halogène, un radical hydroxy, trifluorométhyle, alkoxy renfermant de 1 à 6 atomes de carbone, nitro, amino et cyano, soit parmi les radicaux carboxy libre, estérifié ou salifié, acyle renfermant de 1 à 7 atomes de carbone, alcoxy renfermant de 1 à 6 atomes de carbone, hydroxy, amino, alkylamino, dialkylamino, acyloxy renfermant de 1 à 7 atomes de carbone, cyano, carbamoyle et les atomes d'halogènes

-- Y représente un radical aryle choisi parmi les radicaux aryles carbocycliques renfermant de 6 à 14 atomes de carbone, les radicaux aryles hétéromonocycliques renfermant 5 à 7 chaînons et les radicaux aryles hétérocycliques condensés, tous ces radicaux étant éventuellement substitués,

b) 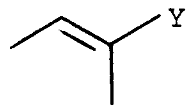 ou

c) 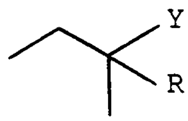

Y et R étant définis comme ci-dessus ;

- soit E représente un radical $-CO_2R_3$, dans lequel $R_3$ représente un atome d'hydrogène ou un radical alkyle linéaire ou ramifié (1 à 4 carbones) et G représente un groupement

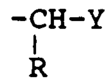

dans lequel Y et R sont définis comme ci-dessus ;

-- A représente :

- soit un radical carbocyclique, éventuellement substitué,
- soit le groupement

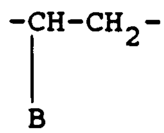

où B représente :

- un atome d'hydrogène,
- un radical aryle ou arylalkyle renfermant de 6 à 14 atomes de carbone, ces radicaux étant éventuellement substitués,
- un radical cycloalkyle renfermant de 3 à 7 atomes de carbone éventuellement substitué,
- un radical alkyle, alkényle, alkynyle ou alkyloxy, ces radicaux étant linéaires ou ramifiés, renfermant au plus 7 atomes de carbone et étant éventuellement substitués,

-- $R_1$ et $R_2$, identiques ou différents, représentent un atome d'hydrogène ou un radical alkyle, alkényle ou alkynyle, ces radicaux étant linéaires ou ramifiés, renfermant au plus 7 atomes de carbone et étant éventuellement substitués, ou bien $R_1$ et $R_2$ forment ensemble avec l'atome d'azote auquel ils sont liés un hétérocycle saturé ou insaturé à 5, 6 ou 7 chaînons comportant éventuellement un autre hétéroatome tel que l'oxygène, l'azote ou le soufre, cet hétérocycle étant éventuellement substitué,

lesdits produits de formule (I) étant sous toutes les formes isomères possibles racémiques, énantiomères et diastéréoisomères, ainsi que des sels d'addition avec les acides minéraux et organiques ou avec les basesdesdits produits de formule (I), caractérisé en ce que l'on fait réagir la diamine de formule (II) :

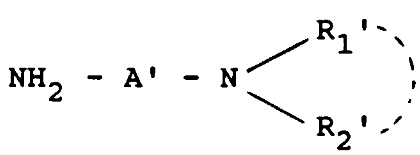 (II)

dans laquelle A′ et

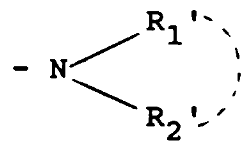

ont les significations indiquées plus haut respectivement pour A et

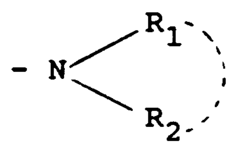

ou celles dans lesquelles les fonctions réactives que peuvent porter A et

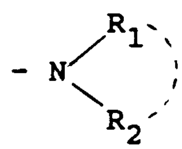

sont protégées, avec l'ester acrylique de formule (III) :

$CH_2 = CH - COOR_3$ (III)

dans laquelle $R_3$ représente un radical alkyle linéaire ou ramifié ayant au plus 6 atomes de carbone, pour obtenir le produit condensé de formule (IV) :

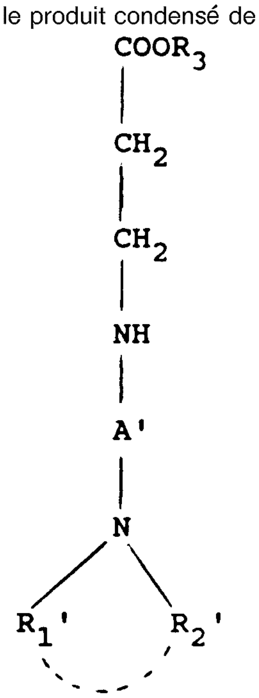

(IV)

dans laquelle A',

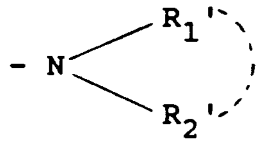

et $R_3$ ont les significations indiquées ci-dessus, que l'on fait réagir avec le composé de formule (V) :

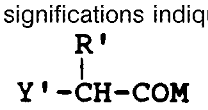

(V)

dans laquelle R′ représente un atome d'hydrogène, les valeurs de R indiquées ci-dessus ou celles dans lesquelles les fonctions réactives sont protégées, M représente un radical hydroxyle ou un atome d'halogène et Y′ représente les valeurs de Y indiquées ci-dessus, ou celles dans lesquelles les fonctions réactives sont protégées, pour obtenir le produit de formule (VI) :

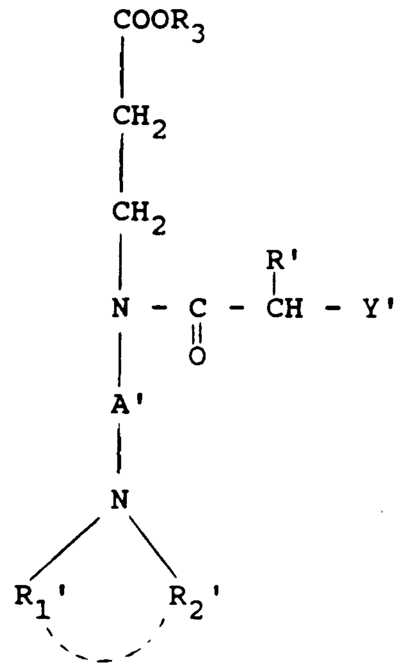

(VI)

dans laquelle A′,

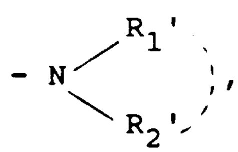

R′, $R_3$ et Y′ ont les significations indiquées ci-dessus, correspondant dans le cas où R′ représente un atome d'hydrogène ou les valeurs de R et A′, $NR'_1R'_2$ et Y′ ont respectivement les valeurs de A, $NR_1R_2$ et Y, à un produit de formule ($I_B$) telle que définie ci-dessus, produit de formule (VI) dans lequel le cas échéant, l'on élimine le ou les groupements protecteurs, pour obtenir un même produit de formule ($I_B$) ou que l'on soumet éventuellement à une réaction de cyclisation pour obtenir le produit de formule (VII) :

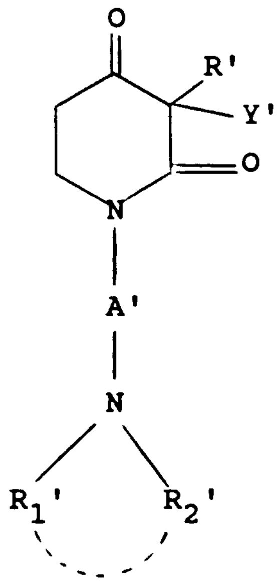

(VII)

dans laquelle A',

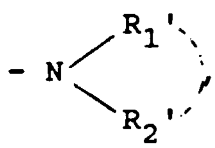

R' et Y' ont les significations indiquées ci-dessus, correspondant dans le cas où R' représente un atome d'hydrogène ou les valeurs de R et A', $NR'_1R'_2$ et Y' ont respectivement les valeurs de A, $NR_1R_2$ et Y, à un produit de formule ($I_A$) telle que définie ci-dessus appelé ($I_{A1}$), produit de formule (VII) dans lequel le cas échéant, l'on élimine le ou les groupements protecteurs, pour obtenir un même produit de formule ($I_{A1}$),puis, le cas échéant :

- soit, quand R' ou R représente un atome d'hydrogène, soumet ledit produit de formule (VII) ou ($I_{A1}$) :

  -- soit à une réaction de réduction de la fonction oxo en position gamma de l'atome d'azote pour obtenir le produit de formule (VIII) :

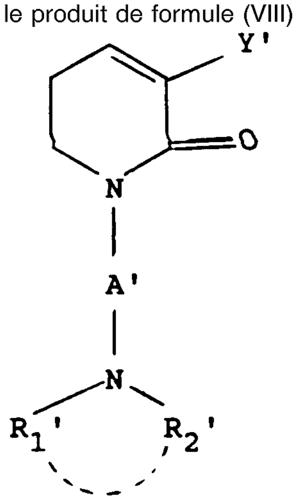

(VIII)

dans laquelle A',

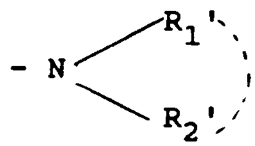

et Y′ ont les significations indiquées ci-dessus, correspondant dans le cas où Y′, A′ et $NR'_1R'_2$ ont respectivement les valeurs de Y, A et $NR_1R_2$, à un produit de formule ($I_A$) telle que définie ci-dessus, appelé ($I_{A2}$), produit de formule (VIII) dans lequel l'on élimine, le cas échéant, les groupements protecteurs, pour obtenir un même produit de formule ($I_{A2}$),
-- soit à une réaction de réduction par hydrogénation catalytique de la même fonction oxo pour obtenir le produit de formule (IX) :

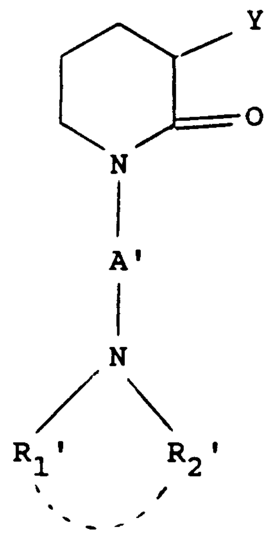

(IX)

dans laquelle A′,

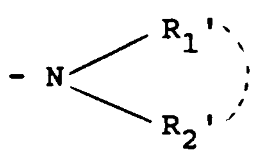

et Y′ ont les significations indiquées ci-dessus, correspondant dans le cas où Y′, A′ et $NR'_1R'_2$ ont respectivement les valeurs de Y, A et $NR_1R_2$, à un produit de formule ($I_A$) telle que définie ci-dessus, appelé ($I_{A3}$), produit de formule (IX) dans lequel l'on élimine, le cas échéant, les groupements protecteurs, pour obtenir un même produit de formule ($I_{A3}$),produit de formule (IX) ou ($I_{A3}$) que l'on traite éventuellement au moyen d'une base forte pour former l'anion correspondant, que l'on soumet à l'action d'un réactif capable de greffer un radical R′, R′ ayant les valeurs indiquées ci-dessus à l'exception d'un atome d'hydrogène,
pour obtenir un produit de formule (X) :

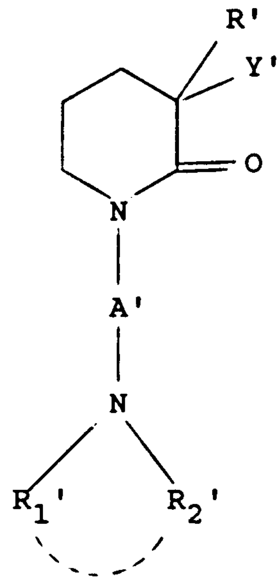

(X)

dans laquelle R′, Y′, A′ et

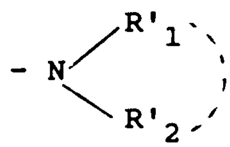

ont les valeurs indiquées ci-dessus, correspondant dans le cas où R′, Y′ A′ et $NR'_1R'_2$ ont respectivement les valeurs de R (à l'exception d'hydrogène), Y, A et $NR_1R_2$, à un produit de formule ($I_A$) telle que définie ci-dessus, appelé ($I_{A4}$), produit de formule (X) dans lequel l'on élimine, le cas échéant, les groupements protecteurs, pour obtenir un même produit de formule ($I_{A4}$) ;

- soit quand R′ ou R est différent d'un atome d'hydrogène, soumet ledit produit de formule (VII) ou ($I_{A1}$) à l'action d'un agent de réduction de la fonction oxo, pour obtenir le produit de formule (X) telle que définie précédemment qui ou bien correspond à un produit de formule ($I_{A4}$) ou bien est transformé en ce produit de formule ($I_{A4}$), et traite, si désiré, les produits de formule (I) par un acide minéral ou organique ou le cas échéant par une base, pour obtenir le sel correspondant, lesdits produits de formule (I) étant sous toutes les formes isomères possibles racémiques, énantiomères et diastéréoisomères.

**2.** Procédé selon la revendication 1, pour la préparation des produits répondant à la formule ($I_A$) :

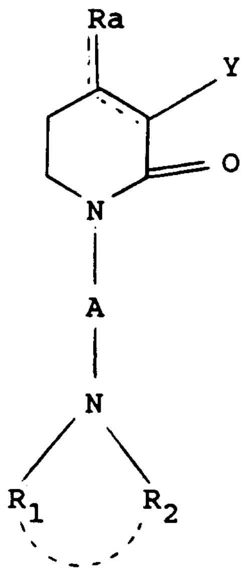

$(I_A)$

dans laquelle le groupement :

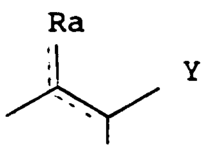

,

A, $R_1$ et $R_2$ sont définis comme à la revendication 1, caractérisé en ce que l'on fait réagir la diamine de formule (II) :

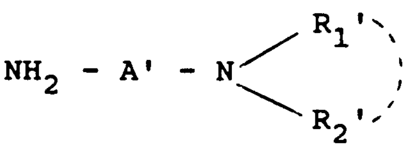

(II)

dans laquelle A′ et

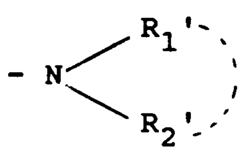

ont les significations indiquées plus haut respectivement pour A et

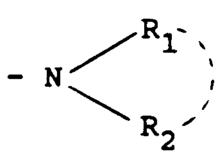

ou celles dans lesquelles les fonctions réactives que peuvent porter A et

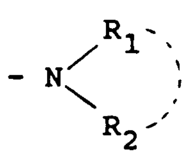

sont protégées, avec l'ester acrylique de formule (III) :

$CH_2 = CH - COOR_3$ (III)

dans laquelle $R_3$ représente un radical alkyle linéaire ou ramifié ayant au plus 6 atomes de carbone, pour obtenir le produit condensé de formule (IV) :

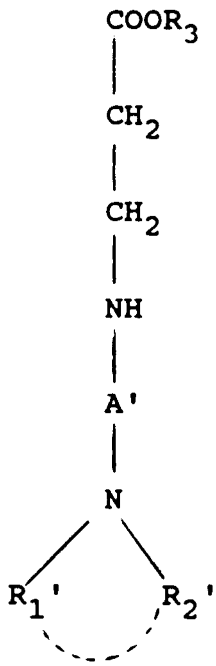

(IV)

dans laquelle A′,

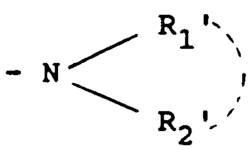

et $R_3$ ont les significations indiquées ci-dessus, que l'on fait réagir avec le composé de formule (V) :

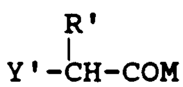

(V)

dans laquelle R′ représente un atome d'hydrogène, les valeurs de R indiquées ci-dessus ou celles dans lesquelles les fonctions réactives sont protégées, M représente un radical hydroxyle ou un atome d'halogène et Y′ représente les valeurs de Y indiquées ci-dessus, ou celles dans lesquelles les fonctions réactives sont protégées, pour obtenir le produit de formule (VI) :

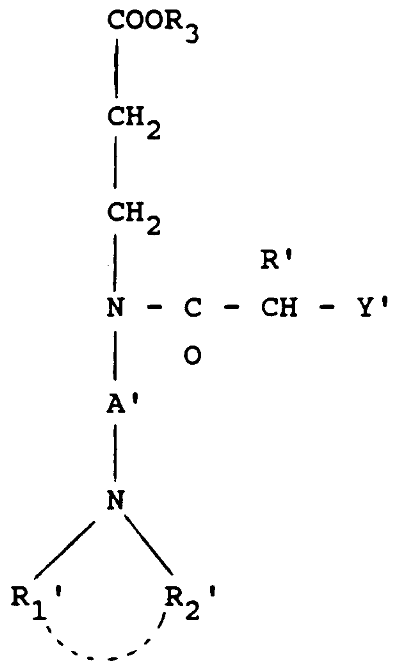

(VI)

dans laquelle A',

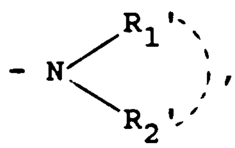

R', $R_3$ et Y' ont les significations indiquées ci-dessus, que l'on soumet à une réaction de cyclisation pour obtenir le produit de formule (VII) :

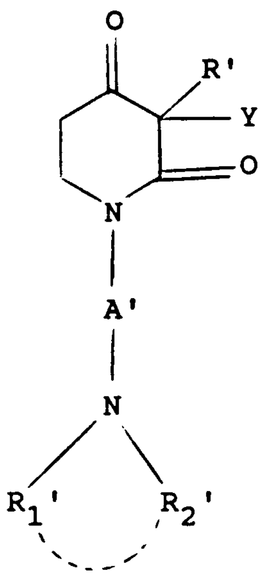

(VII)

dans laquelle A',

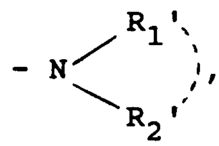

R′ et Y′ ont les significations indiquées ci-dessus, correspondant dans le cas où R′ représente un atome d'hydrogène ou les valeurs de R et A′, $NR'_1R'_2$ et Y′ ont respectivement les valeurs de A, $NR_1R_2$ et Y, à un produit de formule ($I_A$) telle que définie ci-dessus appelé ($I_{A1}$), produit de formule (VII) dans lequel le cas échéant, l'on élimine le ou les groupements protecteurs, pour obtenir un même produit de formule ($I_{A1}$),puis, le cas échéant :

- soit, quand R′ ou R représente un atome d'hydrogène, soumet ledit produit de formule (VII) ou ($I_{A1}$) :

  -- soit à une réaction de réduction de la fonction oxo en position gamma de l'atome d'azote pour obtenir le produit de formule (VIII) :

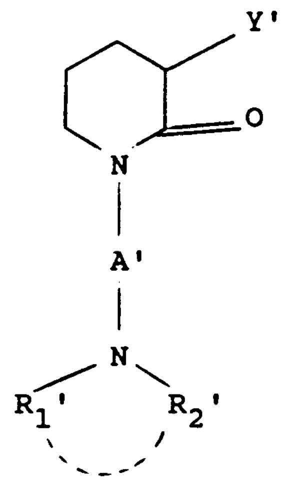

(VIII)

dans laquelle A′,

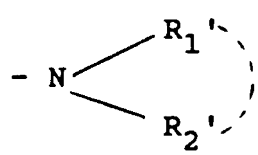

et Y′ ont les significations indiquées ci-dessus, correspondant dans le cas où Y′, A′ et $NR'_1R'_2$ ont respectivement les valeurs de Y, A et $NR_1R_2$, à un produit de formule ($I_A$) telle que définie ci-dessus, appelé ($I_{A2}$), produit de formule (VIII) dans lequel l'on élimine, le cas échéant, les groupements protecteurs, pour obtenir un même produit de formule ($I_{A2}$),

-- soit à une réaction de réduction par hydrogénation catalytique de la même fonction oxo pour obtenir le produit de formule (IX) :

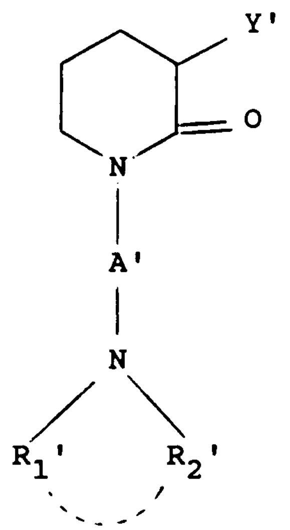

(IX)

dans laquelle A′,

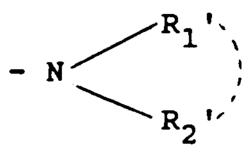

et Y′ ont les significations indiquées ci-dessus, correspondant dans le cas où Y′, A′ et $NR'_1R'_2$ ont respectivement les valeurs de Y, A et $NR_1R_2$, à un produit de formule ($I_A$) telle que définie ci-dessus, appelé ($I_{A3}$), produit de formule (IX) dans lequel l'on élimine, le cas échéant, les groupements protecteurs, pour obtenir un même produit de formule ($I_{A3}$),produit de formule (IX) ou ($I_{A3}$) que l'on traite éventuellement au moyen d'une base forte pour former l'anion correspondant, que l'on soumet à l'action d'un réactif capable de greffer un radical R′, R′ ayant les valeurs indiquées ci-dessus à l'exception d'un atome d'hydrogène,
pour obtenir un produit de formule (X) :

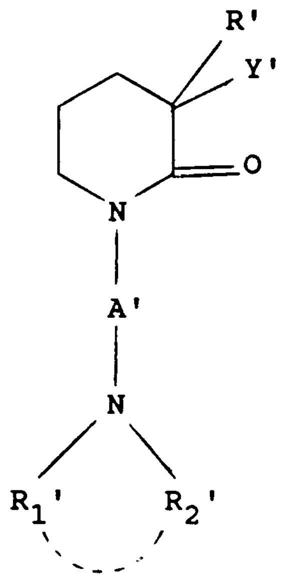

(X)

dans laquelle R′, Y′, A′ et

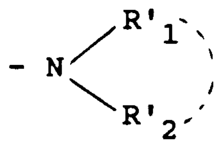

ont les valeurs indiquées ci-dessus, correspondant dans le cas où R', Y' A' et $NR'_1R'_2$ ont respectivement les valeurs de R (à l'exception d'hydrogène), Y, A et $NR_1R_2$, à un produit de formule ($I_A$) telle que définie ci-dessus, appelé ($I_{A4}$), produit de formule (X) dans lequel l'on élimine, le cas échéant, les groupements protecteurs, pour obtenir un même produit de formule ($I_{A4}$) ;

- soit quand R' ou R est différent d'un atome d'hydrogène, soumet ledit produit de formule (VII) ou ($I_{A1}$) à l'action d'un agent de réduction de la fonction oxo, pour obtenir le produit de formule (X) telle que définie précédemment qui ou bien correspond à un produit de formule ($I_{A4}$) ou bien est transformé en ce produit de formule ($I_{A4}$), et traite, si désiré, les produits de formule (I) par un acide minéral ou organique ou le cas échéant par une base, pour obtenir le sel correspondant, lesdits produits de formule ($I_A$) étant sous toutes les formes isomères possibles racémiques, énantiomères et diastéréoisomères.

**3.** Procédé selon la revendication 2, pour la préparation des produits de formule ($I_A$) dans laquelle le groupement

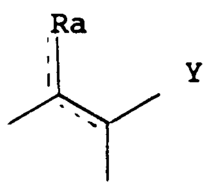

représente

a)

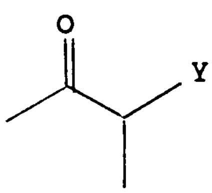

ou le cas échéant son tautomère

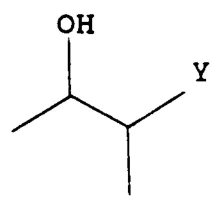

b)

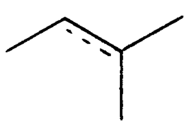

c)

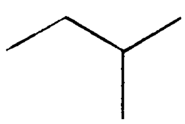

Y étant défini comme à la revendication 1, caractérisé en ce que l'on utilise un composé de formule (V) dans laquelle R' représente un atome d'hydrogène.

**4.** Procédé selon la revendication 1, pour la préparation des produits répondant à la formule ($I_B$) :

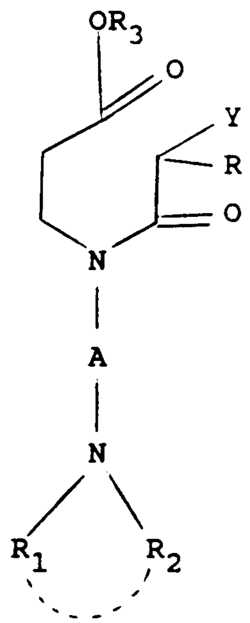

$(I_B)$

dans laquelle $R_1$, $R_2$, $R_3$, A, R et Y sont définis comme à la revendication 1, caractérisé en ce que l'on fait réagir la diamine de formule (II) :

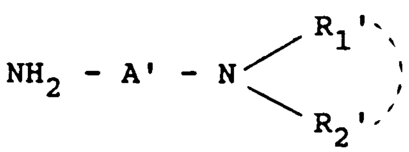

(II)

dans laquelle A′ et

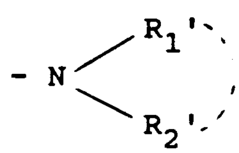

ont les significations indiquées plus haut respectivement pour A et

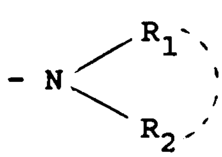

ou celles dans lesquelles les fonctions réactives que peuvent porter A et

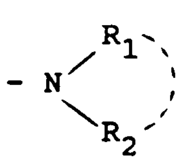

sont protégées, avec l'ester acrylique de formule (III) :

$CH_2$ = CH - $COOR_3$ (III)

dans laquelle $R_3$ représente un radical alkyle linéaire ou ramifié ayant au plus 6 atomes de carbone, pour obtenir le produit condensé de formule (IV) :

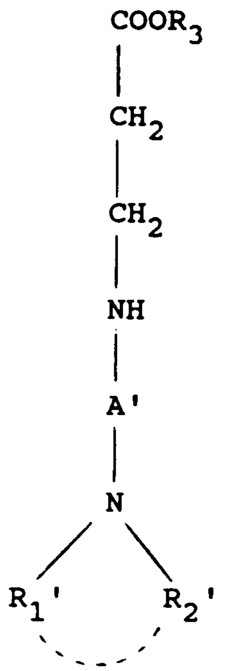

(IV)

dans laquelle A',

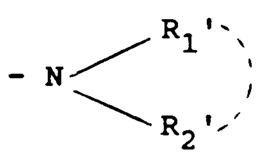

et $R_3$ ont les significations indiquées ci-dessus, que l'on fait réagir avec le composé de formule (V) :

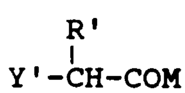

(V)

dans laquelle R' représente un atome d'hydrogène, les valeurs de R indiquées ci-dessus ou celles dans lesquelles les fonctions réactives sont protégées, M représente un radical hydroxyle ou un atome d'halogène et Y' représente les valeurs de Y indiquées ci-dessus, ou celles dans lesquelles les fonctions réactives sont protégées, pour obtenir le produit de formule (VI) :

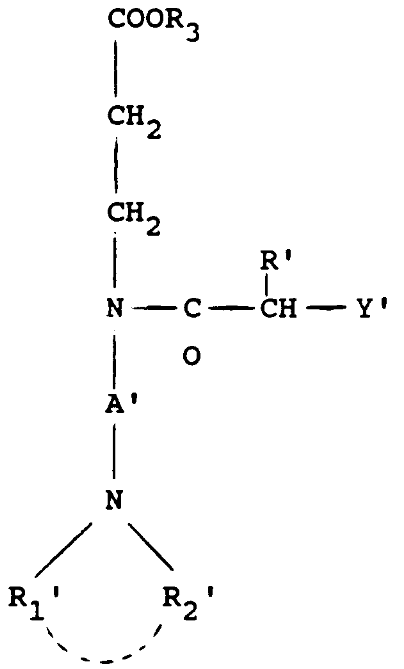

(VI)

dans laquelle A',

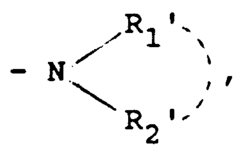

R', $R_3$ et Y' ont les significations indiquées ci-dessus, correspondant dans le cas où R' représente un atome d'hydrogène ou les valeurs de R et A', $NR'_1R'_2$ et Y' ont respectivement les valeurs de A, $NR_1R_2$ et Y, à un produit de formule ($I_B$) telle que définie ci-dessus, produit de formule (VI) dans lequel le cas échéant, l'on élimine le ou les groupements protecteurs, pour obtenir un même produit de formule ($I_B$) et traite, si désiré, les produits de formule (I) par un acide minéral ou organique ou le cas échéant par une base, pour obtenir le sel correspondant, lesdits produits de formule ($I_B$) étant sous toutes les formes isomères possibles racémiques, énantiomères et diastéréoisomères.

**5.** Procédé selon la revendication 4, pour la préparation des produits de formule ($I_B$) dans laquelle R représente un atome d'hydrogène, caractérisé en ce que l'on utilise un composé de formule (V) dans laquelle R' représente un atome d'hydrogène.

**6.** Procédé selon l'une quelconque des revendications 1 à 5, caractérisé en ce que l'on utilise au départ des composés dans lesquels le ou les substituants, identiques ou différents, que peuvent porter les radicaux aryles, arylalkyles et hétérocycliques ou ceux que peuvent former $R_1$ et $R_2$ avec l'atome d'azote auquel ils sont liés, sont choisis dans le groupe formé par :

- les atomes d'halogène,
- les radicaux alkyle, alkényle, alkynyle ou alkyloxy, ces radicaux étant linéaires ou ramifiés renfermant au plus 7 atomes de carbone et étant éventuellement substitués,
- les radicaux hydroxy, trifluorométhyle, trifluorométhylthio, trifluorométhoxy, cyano, nitro, sulfamoyle, amino, monoalkyl-et dialkylamino,
- les radicaux formyle, acyle renfermant de 2 à 6 atomes de carbone et benzoyle,
- les radicaux carboxy libres, estérifiés par un radical alkyle linéaire ou ramifié renfermant au plus 5 atomes de carbone et salifiés,
- le radical carbamoyle éventuellement substitué par un ou plusieurs radicaux, identiques ou différents, choisis dans le groupe formé par les radicaux alkyle, alcoxy ou hydroxyalkyle et le radical carbamoylalkyle, ces radicaux alkyle et alcoxy renfermant de 1 à 4 atomes de carbone,

- et le radical phényle ou benzyle lui-même substitué éventuellement par un ou plusieurs radicaux, identiques ou différents, choisis dans le groupe formé par les atomes d'halogène, les radicaux hydroxy, cyano, nitro, les radicaux alkyle, alkényle, alkynyle et alcoxy linéaires ou ramifiés renfermant au plus 5 atomes de carbone.

**7.** Procédé selon l'une quelconque des revendications 1 à 5, caractérisé en ce que l'on utilise au départ des composés dans lesquels le ou les substituants, identiques ou différents, que peuvent porter les radicaux alkyle, alkényle, alkynyle, cycloalkyle, carbocyclique et alcoxy sont choisis dans le groupe formé par :
- les atomes d'halogène,
- les radicaux alkyle, alkényle, alkynyle ou alcoxy renfermant au plus 5 atomes de carbone,
- les radicaux formyle, acyle renfermant de 2 à 6 atomes de carbone et benzoyle,
- les radicaux carboxy libres, estérifiés par un radical alkyle linéaire ou ramifié renfermant au plus 5 atomes de carbone et salifiés,
- les radicaux hydroxy, cyano, amino, monoalkyl- et dialkylamino,
- les radicaux aryle et arylalkyle renfermant de 6 à 14 atomes de carbone, ces radicaux étant éventuellement substitués par un ou plusieurs radicaux, identiques ou différents, choisis dans le groupe formé par les atomes d'halogène, les radicaux hydroxy, cyano, nitro, les radicaux alkyle, alkényle, alkynyle et alcoxy linéaires ou ramifiés renfermant au plus 5 atomes de carbone.

**8.** Procédé selon l'une quelconque des revendications 1, 2, 4 ou 6, caractérisé en ce que l'on utilise au départ un composé de formule (V) dans laquelle R′ représente un radical alkyle, alkényle ou alkynyle substitué par un phényle ou un phényl substitué, les éventuelles fonctions réactives étant, le cas échéant, protégées.

**9.** Procédé selon l'une quelconque des revendications 1 à 8, caractérisé en ce que l'on utilise au départ un composé de formule (V) dans laquelle Y′ représente un radical phényle, naphtyle, thionaphtyle, benzofuryle, benzopyrrolyle ou indényle éventuellement substitué par un ou plusieurs radicaux choisis dans le groupe formé par :
- les atomes d'halogène,
- les radicaux alkyle, alkényle, alkynyle ou alcoxy linéaires ou ramifiés renfermant au plus 7 atomes de carbone et étant éventuellement substitués par un ou plusieurs radicaux choisis dans le groupe formé par le radical hydroxy et les radicaux alkyle et alcoxy linéaires ou ramifiés renfermant au plus 5 atomes de carbone,
- les radicaux acyle renfermant de 2 à 6 atomes de carbone,
- les radicaux hydroxy, trifluorométhyle, cyano, nitro, amino, les radicaux monoalkyl- et dialkylamino renfermant de 1 à 5 atomes de carbone,
- et le radical phényle lui-même substitué éventuellement par un ou plusieurs radicaux, identiques ou différents, choisis dans le groupe formé par les atomes d'halogène, les radicaux hydroxy, cyano, nitro, les radicaux alkyle, alkényle, alkynyle et alcoxy linéaires ou ramifiés renfermant au plus 5 atomes de carbone, les éventuelles fonctions réactives étant, le cas échéant, protégées.

**10.** Procédé selon l'une quelconque des revendications 1 à 9, caractérisé en ce que l'on utilise au départ un composé de formule (II) dans laquelle A′ représente :
- soit un radical cycloalkyle de formule

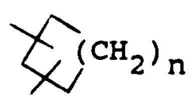

  où n est un entier pouvant prendre les valeurs 0 à 4, éventuellement substitué par un radical alkyle ou alcoxy linéaire ou ramifié renfermant au plus 5 atomes de carbone,
- soit le groupement

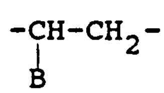

dans lequel B représente un radical phényle éventuellement substitué par un ou plusieurs radicaux, identiques ou différents, choisis dans le groupe formé par les atomes d'halogène, les radicaux hydroxy, cyano, nitro, les radicaux alkyle, alkényle, alkynyle et alcoxy linéaires ou ramifiés renfermant au plus 5 atomes de carbone, les éventuelles fonctions réactives étant, le cas échéant, protégées.

**11.** Procédé selon l'une quelconque des revendications 1 à 10, caractérisé en ce que l'on utilise au départ un composé de formule (II) dans laquelle $R'_1$ et $R'_2$ forment avec l'atome d'azote auquel ils sont liés, un hétérocycle comportant un second atome d'azote, celui-ci étant éventuellement substitué par un radical alkyle ou alcoxy linéaire ou ramifié renfermant de 1 à 5 atomes de carbone.

**12.** Procédé selon la revendication 1, caractérisé en ce que les produits de départ sont choisis de manière telle que l'on prépare l'un quelconque des produits de formule (I) dont les noms suivent :
la (1S)-3-(3,4-dichlorophényl)-1-[1-phényl-2-(1-pyrrolidinyl) éthyl]-2-pipéridinone (isomère B)
la (1S)-3-(3,4-dichlorophényl)-1-(1-phényl-2-(1-pyrrolidinyl) éthyl)-2-pipéridinone (isomère A)
la [trans,(±)]-3-(3,4-dichlorophényl)-1-(2-(1-pyrrolidinyl) cyclohexyl)-2-pipéridinone (isomère A)
la (1S)-3-(3,4-dichlorophényl)-1-(2-méthyl-1-((1-pyrrolidinyl) méthyl) propyl)-2-pipéridinone (isomère A)
la (1S)-3-(benzo(b)thien-4-yl)-1-(1-phényl-2-(1-pyrrolidinyl) éthyl)-2-pipéridinone (isomère A)
la (1S)-3-(3,4-dichlorophényl)-3-méthyl-1-(1-phényl-2-(1-pyrrolidinyl) éthyl)-2-pipéridinone (isomère A)
la (1S)-3-(3,4-dichlorophényl)-3-éthyl-1-(1-phényl-2-(1-pyrrolidinyl) éthyl)-2-pipéridinone (isomère A)
la (1S)-3-(3,4-dichlorophényl)-1-(1-phényl-2-(1-pyrrolidinyl) éthyl)-3-(2-propényl)-2-pipéridinone (isomère A),
et le cas échéant leurs sels.

**Revendications pour l'Etat contractant suivant : GR**

**1.** Procédé de préparation des composés de formule (I) :

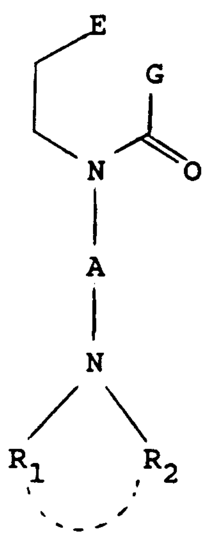

(I)

dans laquelle E et G sont tels que :

- soit E et G forment ensemble un groupement

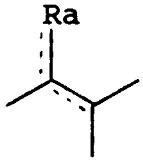

lequel représente l'un des groupements suivants :

a) 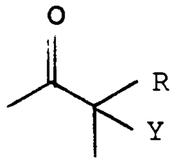 ou, le cas échéant, son tautomère 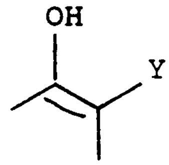 ,

dans lequel R représente un atome d'hydrogène, un radical alkyle linéaire ou ramifié renfermant de 1 à 6 atomes de carbone, un radical alkényle ou alkynyle linéaire ou ramifié renfermant de 2 à 6 atomes de carbone éventuellement substitué par un ou plusieurs substituants choisis soit parmi les radicaux aryle renfermant de 6 à 14 atomes de carbone, aryle substitué par un atome d'halogène, un radical hydroxy, trifluorométhyle, alkoxy renfermant de 1 à 6 atomes de carbone, nitro, amino et cyano, soit parmi les radicaux carboxy libre, estérifié ou salifié, acyle renfermant de 1 à 7 atomes de carbone, alcoxy renfermant de 1 à 6 atomes de carbone, hydroxy, amino, alkylamino, dialkylamino, acyloxy renfermant de 1 à 7 atomes de carbone, cyano, carbamoyle et les atomes d'halogènes
-- Y représente un radical aryle choisi parmi les radicaux aryles carbocycliques renfermant de 6 à 14 atomes de carbone, les radicaux aryles hétéromonocycliques renfermant 5 à 7 chaînons et les radicaux aryles hétérocycliques condensés, tous ces radicaux étant éventuellement substitués,

b) 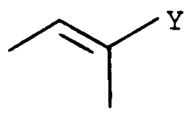 ou

c) 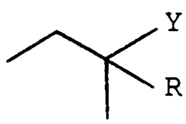

Y et R étant définis comme ci-dessus ;

- soit E représente un radical $-CO_2R_3$, dans lequel $R_3$ représente un atome d'hydrogène ou un radical alkyle linéaire ou ramifié (1 à 4 carbones) et G représente un groupement

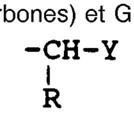

dans lequel Y et R sont définis comme ci-dessus ;
-- A représente :
  - soit un radical carbocyclique, éventuellement substitué,
  - soit le groupement

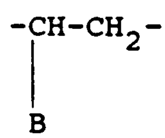

où B représente :
- un atome d'hydrogène,
- un radical aryle ou arylalkyle renfermant de 6 à 14 atomes de carbone, ces radicaux étant éventuellement substitués,
- un radical cycloalkyle renfermant de 3 à 7 atomes de carbone éventuellement substitué,

- un radical alkyle, alkényle, alkynyle ou alkyloxy, ces radicaux étant linéaires ou ramifiés, renfermant au plus 7 atomes de carbone et étant éventuellement substitués,

-- $R_1$ et $R_2$, identiques ou différents, représentent un atome d'hydrogène ou un radical alkyle, alkényle ou alkynyle, ces radicaux étant linéaires ou ramifiés, renfermant au plus 7 atomes de carbone et étant éventuellement substitués, ou bien $R_1$ et $R_2$ forment ensemble avec l'atome d'azote auquel ils sont liés un hétérocycle saturé ou insaturé à 5, 6 ou 7 chaînons comportant éventuellement un autre hétéroatome tel que l'oxygène, l'azote ou le soufre, cet hétérocycle étant éventuellement substitué,

lesdits produits de formule (I) étant sous toutes les formes isomères possibles racémiques, énantiomères et diastéréoisomères, ainsi que des sels d'addition avec les acides minéraux et organiques ou avec les basesdesdits produits de formule (I), caractérisé en ce que l'on fait réagir la diamine de formule (II) :

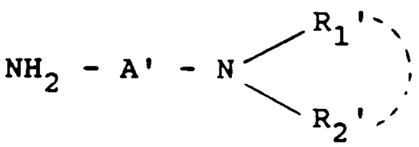

(II)

dans laquelle A′ et

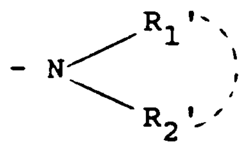

ont les significations indiquées plus haut respectivement pour A et

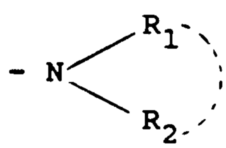

ou celles dans lesquelles les fonctions réactives que peuvent porter A et

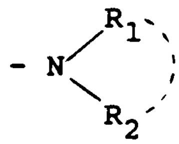

sont protégées, avec l'ester acrylique de formule (III) :

$CH_2 = CH - COOR_3$ (III)

dans laquelle $R_3$ représente un radical alkyle linéaire ou ramifié ayant au plus 6 atomes de carbone, pour obtenir le produit condensé de formule (IV) :

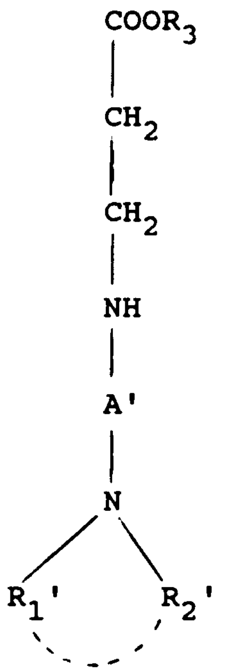

(IV)

dans laquelle A′,

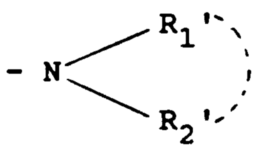

et $R_3$ ont les significations indiquées ci-dessus, que l'on fait réagir avec le composé de formule (V) :

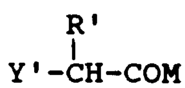

(V)

dans laquelle R′ représente un atome d'hydrogène, les valeurs de R indiquées ci-dessus ou celles dans lesquelles les fonctions réactives sont protégées, M représente un radical hydroxyle ou un atome d'halogène et Y′ représente les valeurs de Y indiquées ci-dessus, ou celles dans lesquelles les fonctions réactives sont protégées, pour obtenir le produit de formule (VI) :

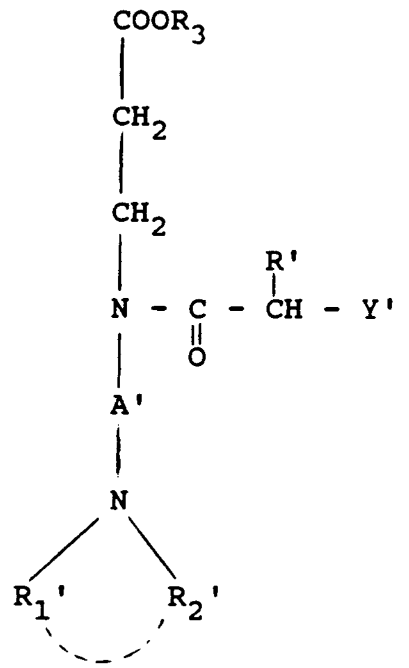

(VI)

dans laquelle A',

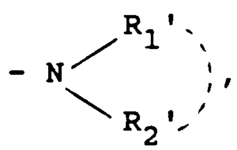

R', $R_3$ et Y' ont les significations indiquées ci-dessus, correspondant dans le cas où R' représente un atome d'hydrogène ou les valeurs de R et A', $NR'_1R'_2$ et Y' ont respectivement les valeurs de A, $NR_1R_2$ et Y, à un produit de formule ($I_B$) telle que définie ci-dessus, produit de formule (VI) dans lequel le cas échéant, l'on élimine le ou les groupements protecteurs, pour obtenir un même produit de formule ($I_B$) ou que l'on soumet éventuellement à une réaction de cyclisation pour obtenir le produit de formule (VII) :

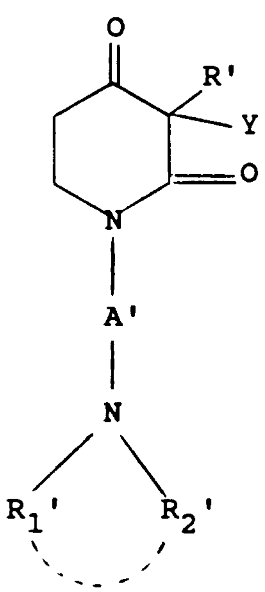

(VII)

dans laquelle A',

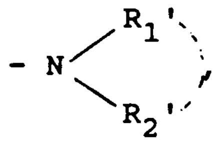

R′ et Y′ ont les significations indiquées ci-dessus, correspondant dans le cas où R′ représente un atome d'hydrogène ou les valeurs de R et A′, $NR'_1R'_2$ et Y′ ont respectivement les valeurs de A, $NR_1R_2$ et Y, à un produit de formule ($I_A$) telle que définie ci-dessus appelé ($I_{A1}$), produit de formule (VII) dans lequel le cas échéant, l'on élimine le ou les groupements protecteurs, pour obtenir un même produit de formule ($I_{A1}$),puis, le cas échéant :

- soit, quand R′ ou R représente un atome d'hydrogène, soumet ledit produit de formule (VII) ou ($I_{A1}$) :

-- soit à une réaction de réduction de la fonction oxo en position gamma de l'atome d'azote pour obtenir le produit de formule (VIII) :

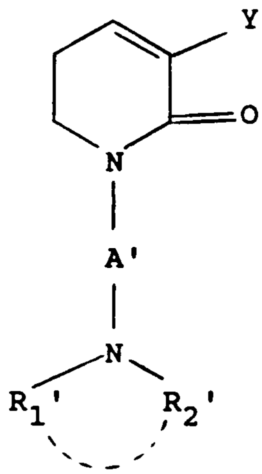

(VIII)

dans laquelle A′,

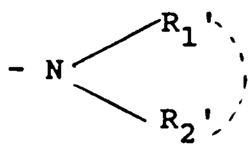

et Y′ ont les significations indiquées ci-dessus, correspondant dans le cas où Y′, A′ et $NR'_1R'_2$ ont respectivement les valeurs de Y, A et $NR_1R_2$, à un produit de formule ($I_A$) telle que définie ci-dessus, appelé ($I_{A2}$), produit de formule (VIII) dans lequel l'on élimine, le cas échéant, les groupements protecteurs, pour obtenir un même produit de formule ($I_{A2}$),

-- soit à une réaction de réduction par hydrogénation catalytique de la même fonction oxo pour obtenir le produit de formule (IX) :

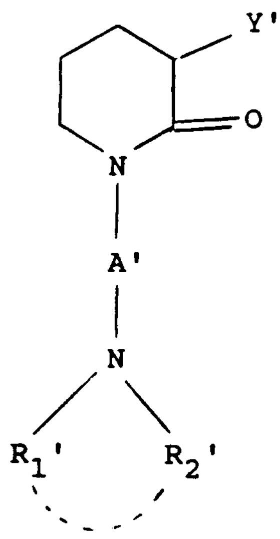

(IX)

dans laquelle A′,

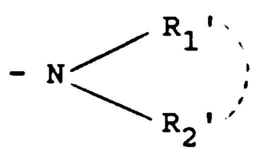

et Y′ ont les significations indiquées ci-dessus, correspondant dans le cas où Y′, A′ et $NR'_1R'_2$ ont respectivement les valeurs de Y, A et $NR_1R_2$, à un produit de formule ($I_A$) telle que définie ci-dessus, appelé ($I_{A3}$), produit de formule (IX) dans lequel l'on élimine, le cas échéant, les groupements protecteurs, pour obtenir un même produit de formule ($I_{A3}$),produit de formule (IX) ou ($I_{A3}$) que l'on traite éventuellement au moyen d'une base forte pour former l'anion correspondant, que l'on soumet à l'action d'un réactif capable de greffer un radical R′, R′ ayant les valeurs indiquées ci-dessus à l'exception d'un atome d'hydrogène,
pour obtenir un produit de formule (X) :

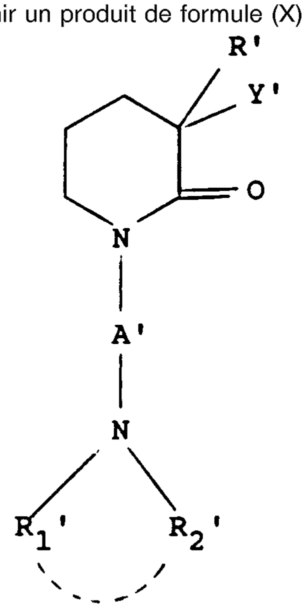

(X)

dans laquelle R′, Y′, A′ et

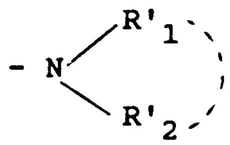

ont les valeurs indiquées ci-dessus, correspondant dans le cas où R′, Y′ A′ et $NR'_1R'_2$ ont respectivement les valeurs de R (à l'exception d'hydrogène), Y, A et $NR_1R_2$, à un produit de formule ($I_A$) telle que définie ci-dessus, appelé ($I_{A4}$), produit de formule (X) dans lequel l'on élimine, le cas échéant, les groupements protecteurs, pour obtenir un même produit de formule ($I_{A4}$) ;

- soit quand R′ ou R est différent d'un atome d'hydrogène, soumet ledit produit de formule (VII) ou ($I_{A1}$) à l'action d'un agent de réduction de la fonction oxo, pour obtenir le produit de formule (X) telle que définie précédemment qui ou bien correspond à un produit de formule ($I_{A4}$) ou bien est transformé en ce produit de formule ($I_{A4}$), et traite, si désiré,

les produits de formule (I) par un acide minéral ou organique ou le cas échéant par une base, pour obtenir le sel correspondant, lesdits produits de formule (I) étant sous toutes les formes isomères possibles racémiques, énantiomères et diastéréoisomères.

2. Procédé selon la revendication 1, pour la préparation des produits répondant à la formule ($I_A$) :

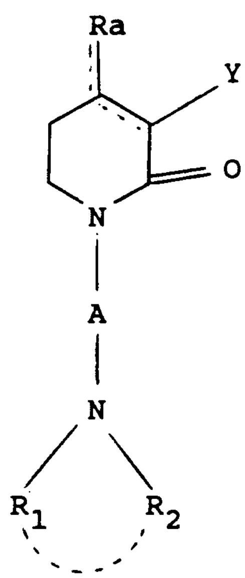

($I_A$)

dans laquelle le groupement :

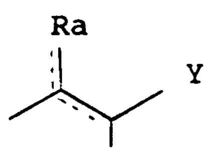

,

A, $R_1$ et $R_2$ sont définis comme à la revendication 1, caractérisé en ce que l'on fait réagir la diamine de formule (II) :

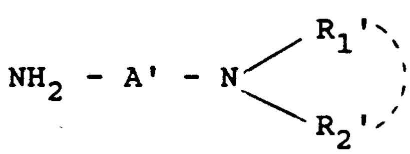

(II)

dans laquelle A′ et

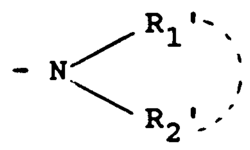

ont les significations indiquées plus haut respectivement pour A et

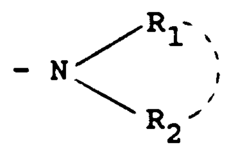

ou celles dans lesquelles les fonctions réactives que peuvent porter A et

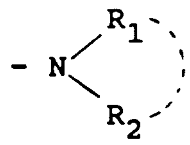

sont protégées, avec l'ester acrylique de formule (III) :

$CH_2 = CH - COOR_3$ (III)

dans laquelle $R_3$ représente un radical alkyle linéaire ou ramifié ayant au plus 6 atomes de carbone, pour obtenir le produit condensé de formule (IV) :

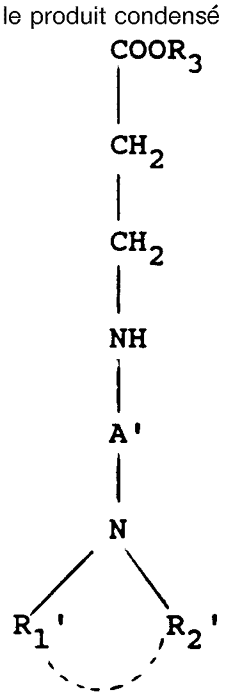

(IV)

dans laquelle A',

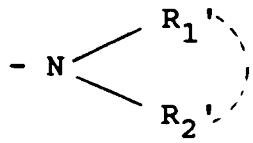

et $R_3$ ont les significations indiquées ci-dessus, que l'on fait réagir avec le composé de formule (V) :

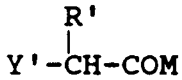

(V)

dans laquelle R′ représente un atome d'hydrogène, les valeurs de R indiquées ci-dessus ou celles dans lesquelles les fonctions réactives sont protégées, M représente un radical hydroxyle ou un atome d'halogène et Y′ représente les valeurs de Y indiquées ci-dessus, ou celles dans lesquelles les fonctions réactives sont protégées, pour obtenir le produit de formule (VI) :

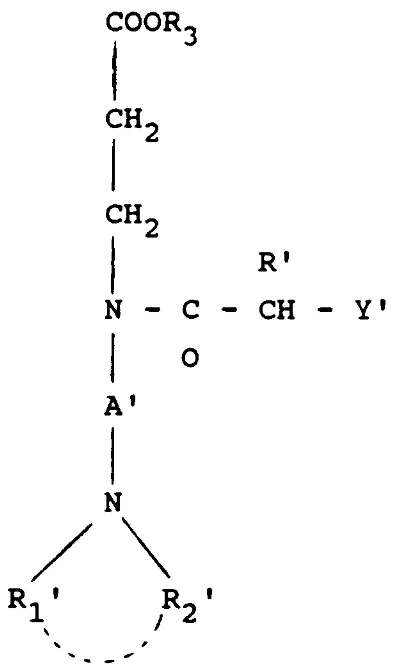

(VI)

dans laquelle A′,

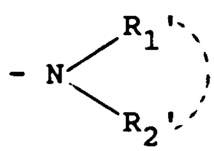,

R′, $R_3$ et Y′ ont les significations indiquées ci-dessus, que l'on soumet à une réaction de cyclisation pour obtenir le produit de formule (VII) :

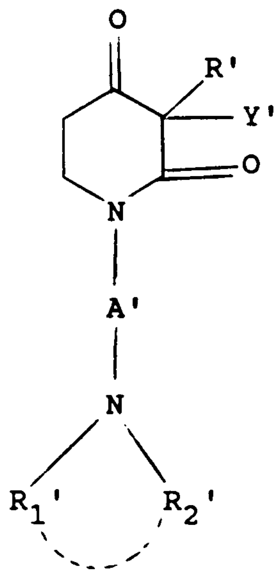

(VII)

dans laquelle A',

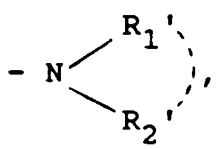

R' et Y' ont les significations indiquées ci-dessus, correspondant dans le cas où R' représente un atome d'hydrogène ou les valeurs de R et A', $NR'_1R'_2$ et Y' ont respectivement les valeurs de A, $NR_1R_2$ et Y, à un produit de formule ($I_A$) telle que définie ci-dessus appelé ($I_{A1}$), produit de formule (VII) dans lequel le cas échéant, l'on élimine le ou les groupements protecteurs, pour obtenir un même produit de formule ($I_{A1}$),puis, le cas échéant :

- soit, quand R' ou R représente un atome d'hydrogène, soumet ledit produit de formule (VII) ou ($I_{A1}$) :
  -- soit à une réaction de réduction de la fonction oxo en position gamma de l'atome d'azote pour obtenir le produit de formule (VIII) :

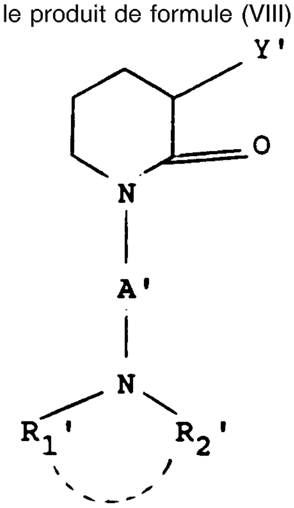

(VIII)

dans laquelle A',

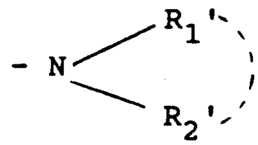

et Y′ ont les significations indiquées ci-dessus, correspondant dans le cas où Y′, A′ et $NR'_1R'_2$ ont respectivement les valeurs de Y, A et $NR_1R_2$, à un produit de formule ($I_A$) telle que définie ci-dessus, appelé ($I_{A2}$), produit de formule (VIII) dans lequel l'on élimine, le cas échéant, les groupements protecteurs, pour obtenir un même produit de formule ($I_{A2}$),
-- soit à une réaction de réduction par hydrogénation catalytique de la même fonction oxo pour obtenir le produit de formule (IX) :

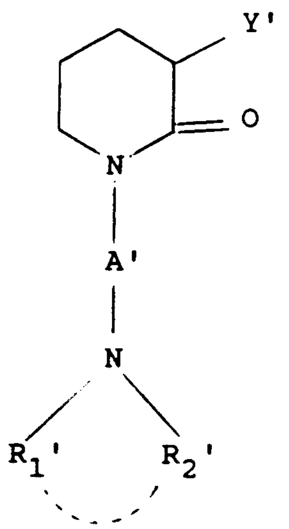

(IX)

dans laquelle A′,

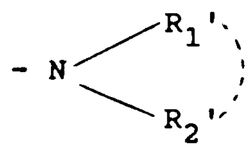

et Y′ ont les significations indiquées ci-dessus, correspondant dans le cas où Y′, A′ et $NR'_1R'_2$ ont respectivement les valeurs de Y, A et $NR_1R_2$, à un produit de formule ($I_A$) telle que définie ci-dessus, appelé ($I_{A3}$), produit de formule (IX) dans lequel l'on élimine, le cas échéant, les groupements protecteurs, pour obtenir un même produit de formule ($I_{A3}$),produit de formule (IX) ou ($I_{A3}$) que l'on traite éventuellement au moyen d'une base forte pour former l'anion correspondant, que l'on soumet à l'action d'un réactif capable de greffer un radical R′, R′ ayant les valeurs indiquées ci-dessus à l'exception d'un atome d'hydrogène,
pour obtenir un produit de formule (X) :

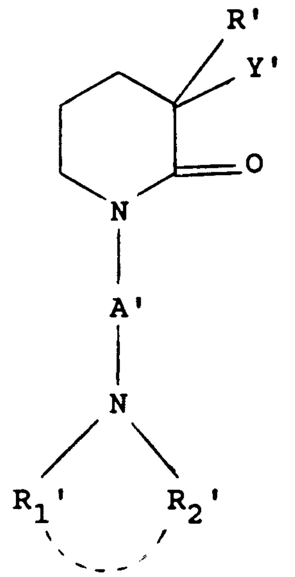

(X)

dans laquelle R′, Y′, A′ et

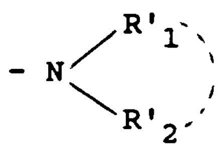

ont les valeurs indiquées ci-dessus, correspondant dans le cas où R′, Y′ A′ et $NR'_1R'_2$ ont respectivement les valeurs de R (à l'exception d'hydrogène), Y, A et $NR_1R_2$, à un produit de formule ($I_A$) telle que définie ci-dessus, appelé ($I_{A4}$), produit de formule (X) dans lequel l'on élimine, le cas échéant, les groupements protecteurs, pour obtenir un même produit de formule ($I_{A4}$) ;

- soit quand R′ ou R est différent d'un atome d'hydrogène, soumet ledit produit de formule (VII) ou ($I_{A1}$) à l'action d'un agent de réduction de la fonction oxo, pour obtenir le produit de formule (X) telle que définie précédemment qui ou bien correspond à un produit de formule ($I_{A4}$) ou bien est transformé en ce produit de formule ($I_{A4}$), et traite, si désiré, les produits de formule (I) par un acide minéral ou organique ou le cas échéant par une base, pour obtenir le sel correspondant, lesdits produits de formule ($I_A$) étant sous toutes les formes isomères possibles racémiques, énantiomères et diastéréoisomères.

**3.** Procédé selon la revendication 2, pour la préparation des produits de formule ($I_A$) dans laquelle le groupement

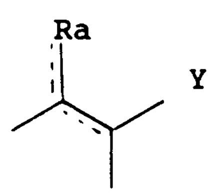

représente

a) 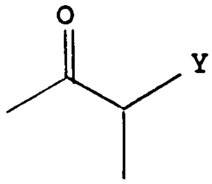 ou le cas échéant son tautomère 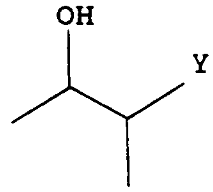

b) 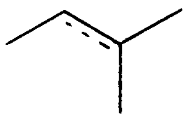

c) 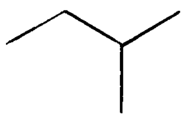

Y étant défini comme à la revendication 1, caractérisé en ce que l'on utilise un composé de formule (V) dans laquelle R′ représente un atome d'hydrogène.

4. Procédé selon la revendication 1, pour la préparation des produits répondant à la formule ($I_B$) :

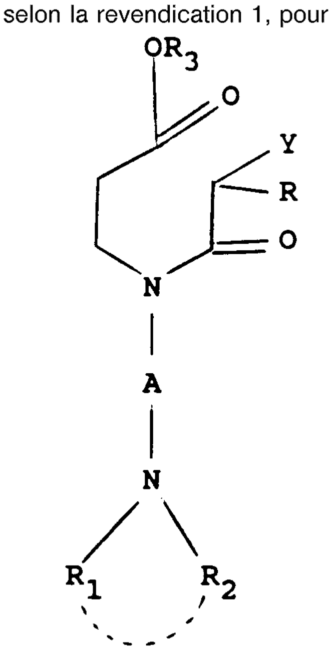

($I_B$)

dans laquelle $R_1$, $R_2$, $R_3$, A, R et Y sont définis comme à la revendication 1, caractérisé en ce que l'on fait réagir la diamine de formule (II) :

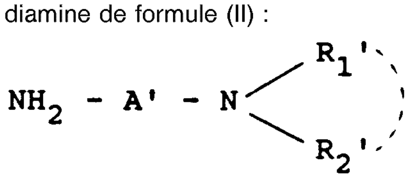

(II)

dans laquelle A′ et

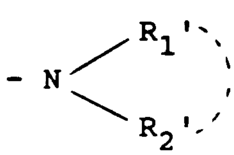

ont les significations indiquées plus haut respectivement pour A et

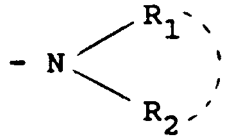

ou celles dans lesquelles les fonctions réactives que peuvent porter A et

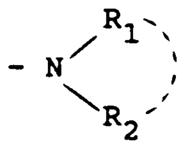

sont protégées, avec l'ester acrylique de formule (III) :

$CH_2 = CH - COOR_3$ (III)

dans laquelle $R_3$ représente un radical alkyle linéaire ou ramifié ayant au plus 6 atomes de carbone, pour obtenir le produit condensé de formule (IV) :

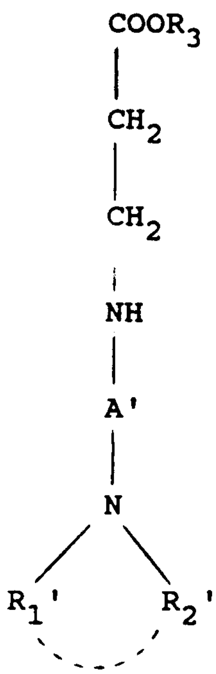

(IV)

dans laquelle A',

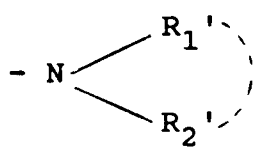

et $R_3$ ont les significations indiquées ci-dessus, que l'on fait réagir avec le composé de formule (V) :

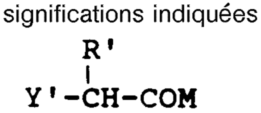

(V)

dans laquelle R′ représente un atome d'hydrogène, les valeurs de R indiquées ci-dessus ou celles dans lesquelles les fonctions réactives sont protégées, M représente un radical hydroxyle ou un atome d'halogène et Y′ représente les valeurs de Y indiquées ci-dessus, ou celles dans lesquelles les fonctions réactives sont protégées, pour obtenir le produit de formule (VI) :

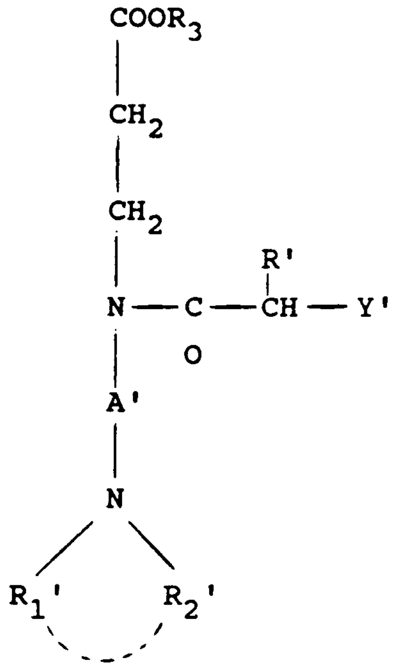

(VI)

dans laquelle A′,

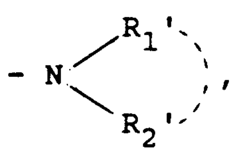

R′, $R_3$ et Y′ ont les significations indiquées ci-dessus, correspondant dans le cas où R′ représente un atome d'hydrogène ou les valeurs de R et A′, $NR'_1R'_2$ et Y′ ont respectivement les valeurs de A, $NR_1R_2$ et Y, à un produit de formule ($I_B$) telle que définie ci-dessus, produit de formule (VI) dans lequel le cas échéant, l'on élimine le ou les groupements protecteurs, pour obtenir un même produit de formule ($I_B$) et traite, si désiré, les produits de formule (I) par un acide minéral ou organique ou le cas échéant par une base, pour obtenir le sel correspondant, lesdits produits de formule ($I_B$) étant sous toutes les formes isomères possibles racémiques, énantiomères et diastéréoisomères.

**5.** Procédé selon la revendication 4, pour la préparation des produits de formule ($I_B$) dans laquelle R représente un atome d'hydrogène, caractérisé en ce que l'on utilise un composé de formule (V) dans laquelle R′ représente un atome d'hydrogène.

**6.** Procédé selon l'une quelconque des revendications 1 à 5, caractérisé en ce que l'on utilise au départ des composés dans lesquels le ou les substituants, identiques ou différents, que peuvent porter les radicaux aryles, arylalkyles et hétérocycliques ou ceux que peuvent former $R_1$ et $R_2$ avec l'atome d'azote auquel ils sont liés, sont choisis dans le groupe formé par :

- les atomes d'halogène,
- les radicaux alkyle, alkényle, alkynyle ou alkyloxy, ces radicaux étant linéaires ou ramifiés renfermant au plus 7 atomes de carbone et étant éventuellement substitués,
- les radicaux hydroxy, trifluorométhyle, trifluorométhylthio, trifluorométhoxy, cyano, nitro, sulfamoyle, amino, monoalkyl-et dialkylamino,
- les radicaux formyle, acyle renfermant de 2 à 6 atomes de carbone et benzoyle,
- les radicaux carboxy libres, estérifiés par un radical alkyle linéaire ou ramifié renfermant au plus 5 atomes de carbone et salifiés,

- le radical carbamoyle éventuellement substitué par un ou plusieurs radicaux, identiques ou différents, choisis dans le groupe formé par les radicaux alkyle, alcoxy ou hydroxyalkyle et le radical carbamoylalkyle, ces radicaux alkyle et alcoxy renfermant de 1 à 4 atomes de carbone,
- et le radical phényle ou benzyle lui-même substitué éventuellement par un ou plusieurs radicaux, identiques ou différents, choisis dans le groupe formé par les atomes d'halogène, les radicaux hydroxy, cyano, nitro, les radicaux alkyle, alkényle, alkynyle et alcoxy linéaires ou ramifiés renfermant au plus 5 atomes de carbone.

**7.** Procédé selon l'une quelconque des revendications 1 à 5, caractérisé en ce que l'on utilise au départ des composés dans lesquels le ou les substituants, identiques ou différents, que peuvent porter les radicaux alkyle, alkényle, alkynyle, cycloalkyle, carbocyclique et alcoxy sont choisis dans le groupe formé par :
- les atomes d'halogène,
- les radicaux alkyle, alkényle, alkynyle ou alcoxy renfermant au plus 5 atomes de carbone,
- les radicaux formyle, acyle renfermant de 2 à 6 atomes de carbone et benzoyle,
- les radicaux carboxy libres, estérifiés par un radical alkyle linéaire ou ramifié renfermant au plus 5 atomes de carbone et salifiés,
- les radicaux hydroxy, cyano, amino, monoalkyl- et dialkylamino,
- les radicaux aryle et arylalkyle renfermant de 6 à 14 atomes de carbone, ces radicaux étant éventuellement substitués par un ou plusieurs radicaux, identiques ou différents, choisis dans le groupe formé par les atomes d'halogène, les radicaux hydroxy, cyano, nitro, les radicaux alkyle, alkényle, alkynyle et alcoxy linéaires ou ramifiés renfermant au plus 5 atomes de carbone.

**8.** Procédé selon l'une quelconque des revendications 1, 2, 4 ou 6, caractérisé en ce que l'on utilise au départ un composé de formule (V) dans laquelle R′ représente un radical alkyle, alkényle ou alkynyle substitué par un phényle ou un phényl substitué, les éventuelles fonctions réactives étant, le cas échéant, protégées.

**9.** Procédé selon l'une quelconque des revendications 1 à 8, caractérisé en ce que l'on utilise au départ un composé de formule (V) dans laquelle Y′ représente un radical phényle, naphtyle, thionaphtyle, benzofuryle, benzopyrrolyle ou indényle éventuellement substitué par un ou plusieurs radicaux choisis dans le groupe formé par :
- les atomes d'halogène,
- les radicaux alkyle, alkényle, alkynyle ou alcoxy linéaires ou ramifiés renfermant au plus 7 atomes de carbone et étant éventuellement substitués par un ou plusieurs radicaux choisis dans le groupe formé par le radical hydroxy et les radicaux alkyle et alcoxy linéaires ou ramifiés renfermant au plus 5 atomes de carbone,
- les radicaux acyle renfermant de 2 à 6 atomes de carbone,
- les radicaux hydroxy, trifluorométhyle, cyano, nitro, amino, les radicaux monoalkyl- et dialkylamino renfermant de 1 à 5 atomes de carbone,
- et le radical phényle lui-même substitué éventuellement par un ou plusieurs radicaux, identiques ou différents, choisis dans le groupe formé par les atomes d'halogène, les radicaux hydroxy, cyano, nitro, les radicaux alkyle, alkényle, alkynyle et alcoxy linéaires ou ramifiés renfermant au plus 5 atomes de carbone, les éventuelles fonctions réactives étant, le cas échéant, protégées.

**10.** Procédé selon l'une quelconque des revendications 1 à 9, caractérisé en ce que l'on utilise au départ un composé de formule (II) dans laquelle A′ représente :
- soit un radical cycloalkyle de formule

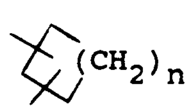

où n est un entier pouvant prendre les valeurs 0 à 4, éventuellement substitué par un radical alkyle ou alcoxy linéaire ou ramifié renfermant au plus 5 atomes de carbone,
- soit le groupement

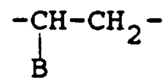

dans lequel B représente un radical phényle éventuellement substitué par un ou plusieurs radicaux, identiques ou différents, choisis dans le groupe formé par les atomes d'halogène, les radicaux hydroxy, cyano, nitro, les radicaux alkyle, alkényle, alkynyle et alcoxy linéaires ou ramifiés renfermant au plus 5 atomes de carbone, les éventuelles fonctions réactives étant, le cas échéant, protégées.

**11.** Procédé selon l'une quelconque des revendications 1 à 10, caractérisé en ce que l'on utilise au départ un composé de formule (II) dans laquelle $R'_1$ et $R'_2$ forment avec l'atome d'azote auquel ils sont liés, un hétérocycle comportant un second atome d'azote, celui-ci étant éventuellement substitué par un radical alkyle ou alcoxy linéaire ou ramifié renfermant de 1 à 5 atomes de carbone.

**12.** Procédé selon la revendication 1, caractérisé en ce que les produits de départ sont choisis de manière telle que l'on prépare l'un quelconque des produits de formule (I) dont les noms suivent :
la (1S)-3-(3,4-dichlorophényl)-1-[1-phényl-2-(1-pyrrolidinyl) éthyl]-2-pipéridinone (isomère B)
la (1S)-3-(3,4-dichlorophényl)-1-(1-phényl-2-(1-pyrrolidinyl) éthyl)-2-pipéridinone (isomère A)
la [trans,(±)]-3-(3,4-dichlorophényl)-1-(2-(1-pyrrolidinyl) cyclohexyl)-2-pipéridinone (isomère A)
la (1S)-3-(3,4-dichlorophényl)-1-(2-méthyl-1-((1-pyrrolidinyl) méthyl) propyl)-2-pipéridinone (isomère A)
la (1S)-3-(benzo(b)thien-4-yl)-1-(1-phényl-2-(1-pyrrolidinyl) éthyl)-2-pipéridinone (isomère A)
la (1S)-3-(3,4-dichlorophényl)-3-méthyl-1-(1-phényl-2-(1-pyrrolidinyl) éthyl)-2-pipéridinone (isomère A)
la (1S)-3-(3,4-dichlorophényl)-3-éthyl-1-(1-phényl-2-(1-pyrrolidinyl) éthyl)-2-pipéridinone (isomère A)
la (1S)-3-(3,4-dichlorophényl)-1-(1-phényl-2-(1-pyrrolidinyl) éthyl)-3-(2-propényl)-2-pipéridinone (isomère A),
et le cas échéant leurs sels.

**13.** Procédé de préparation de compositions pharmaceutiques, caractérisé en ce que l'on met, à titre de principe actif, l'un au moins des produits de formule (I) telle que définie à la revendication 1, ou l'un au moins de leurs sels pharmaceutiquement acceptables, sous une forme destinée à cet usage.

**14.** Procédé de préparation de compositions pharmaceutiques, caractérisé en ce que l'on met, à titre de principe actif, l'un au moins des produits de formule ($I_A$) telle que définie à la revendication 3, ou l'un au moins de leurs sels pharmaceutiquement acceptables, sous une forme destinée à cet usage.

**15.** Procédé de préparation de compositions pharmaceutiques, caractérisé en ce que l'on met, à titre de principe actif, l'un au moins des produits de formule (I) telle que définie à la revendication 12, ou l'un au moins de leurs sels pharmaceutiquement acceptables, sous une forme destinée à cet usage.

**16.** A titre de produits industriels, les composés de formules (IV), (VI), (VII), (VIII), (IX) et (X).

## Claims

### Claims for the following Contracting States : AT, BE, CH, DE, DK, FR, GB, IT, LI, LU, NL, SE

**1.** Compounds of formula (I):

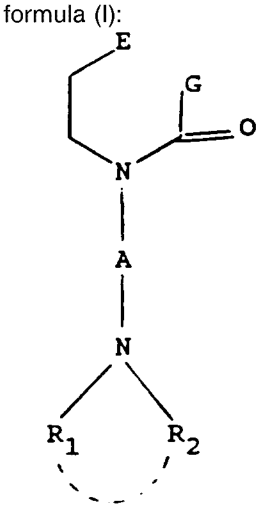

(I)

in which E and G are such that:

- either E and G together form a

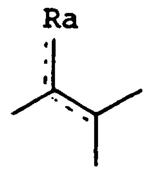

group which represents one of the following groups:

a) 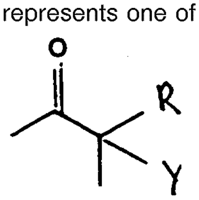

or, if appropriate, its tautomer,

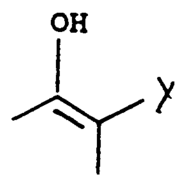

in which R represents a hydrogen atom, a linear or branched alkyl radical containing 1 to 6 carbon atoms, a linear or branched alkenyl or alkynyl radical containing 2 to 6 carbon atoms optionally substituted by one or more substituents chosen either from aryl radicals containing 6 to 14 carbon atoms, aryl radicals substituted by a halogen atom, a hydroxy, trifluoromethyl radical, an alkoxy radical containing 1 to 6 carbon atoms, a nitro, amino and cyano radical, or from free, esterified or salified carboxy radicals, acyl radicals containing 1 to 7 carbon atoms, alkoxy radicals containing 1 to 6 carbon atoms, hydroxy, amino, alkylamino, dialkylamino radicals, acyloxy radicals containing 1 to 7 carbon atoms, cyano, carbamoyl radicals and halogen atoms

-- Y represents an aryl radical chosen from carbocyclic aryl radicals containing 6 to 14 carbon atoms, heteromonocyclic aryl radicals containing 5 to 7 members and condensed heterocyclic aryl radicals, all these radicals being optionally substituted,

b) 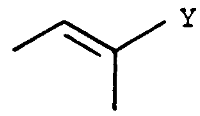 or

c) 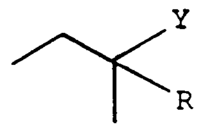

Y and R being as defined above;

- or E represents a $-CO_2R_3$ radical, in which $R_3$ represents a hydrogen atom or a linear or branched alkyl radical (1 to 4 carbons) and G represents a

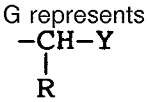

group in which Y and R are as defined above;

-- A represents:

- either a carbocyclic radical, optionally substituted,
- or the

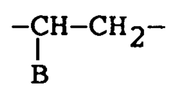

group where B represents:

- a hydrogen atom,
- an aryl or arylalkyl radical containing 6 to 14 carbon atoms, these radicals being optionally substituted,
- an optionally substituted cycloalkyl radical containing 3 to 7 carbon atoms,
- an alkyl, alkenyl, alkynyl or alkyloxy radical, these radicals being linear or branched, containing at most 7 carbon atoms and being optionally substituted,

-- $R_1$ and $R_2$, identical or different, represent a hydrogen atom or an alkyl, alkenyl or alkynyl radical, these radicals being linear or branched, containing at most 7 carbon atoms and being optionally substituted,

or $R_1$ and $R_2$ form together with the nitrogen atom to which they are linked a saturated or unsaturated heterocycle with 5, 6 or 7 members optionally containing another heteroatom such as oxygen, nitrogen or sulphur, this heterocycle being optionally substituted,

said products of formula (I) being in all possible racemic, enantiomeric and diastereoisomeric isomer forms, as well as the addition salts with mineral and organic acids or bases of said products of formula (I).

**2.** The compounds according to claim 1 corresponding to formula ($I_A$):

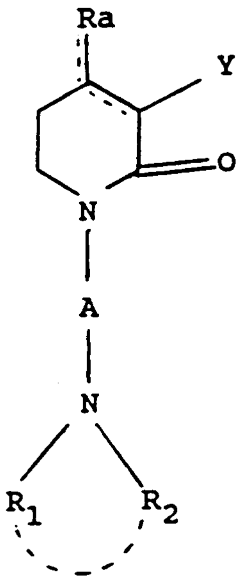

$(I_A)$

in which the group:

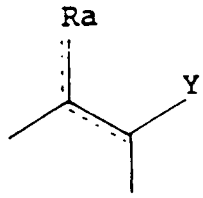

,

A, $R_1$ and $R_2$ are as defined in claim 1.

**3.** The compounds according to claim 2 in which the group

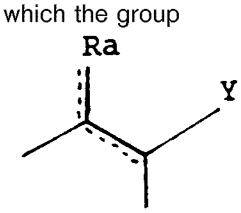

represents

a) 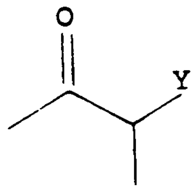 or if appropriate its tautomer 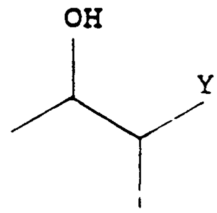

b) 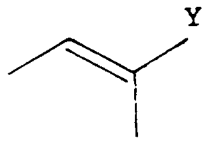

c) 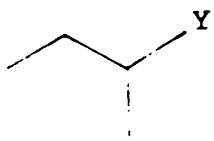

Y being as defined in claim 1.

**4.** The compounds according to claim 1 corresponding to formula ($I_B$):

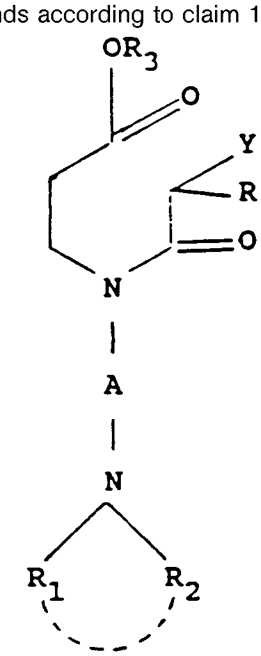

($I_B$)

in which $R_1$ $R_2$, $R_3$, A, R and Y are as defined in claim 1.

**5.** Compounds of formula (I) as defined in any one of claims 1 to 4, characterized in that the substituent or substituents, identical or different, which can be carried by the aryl, arylalkyl and heterocyclic radicals or those which can be formed by $R_1$ and $R_2$ with the nitrogen atom to which they are linked, are chosen from the group formed by:

- halogen atoms,
- alkyl, alkenyl, alkynyl or alkyloxy radicals, these radicals being linear or branched containing at most 7 carbon atoms and being optionally substituted,
- hydroxy, trifluoromethyl, trifluoromethylthio, trifluoromethoxy, cyano, nitro, sulphamoyl, amino, monoalkyl-and dialkylamino radicals,
- formyl radicals, acyl radicals containing 2 to 6 carbon atoms and benzoyl radicals,

- free carboxy radicals, carboxy radicals esterified by a linear or branched alkyl radical containing at most 5 carbon atoms and salified carboxy radicals,
- the carbamoyl radical optionally substituted by one or more identical or different radicals, chosen from the group formed by alkyl, alkoxy or hydroxyalkyl radicals and the carbamoylalkyl radical, these alkyl and alkoxy radicals containing 1 to 4 carbon atoms,
- and the phenyl or benzyl radical itself optionally substituted by one or more identical or different radicals, chosen from the group formed by halogen atoms and the following radicals: hydroxy, cyano, nitro, linear or branched alkyl, alkenyl, alkynyl and alkoxy containing at most 5 carbon atoms,

said products of formula (I) being in all possible racemic, enantiomeric and diastereoisomeric isomer forms, as well as the addition salts with mineral and organic acids or with bases of said products of formula (I).

**6.** Products of formula (I) as defined in any one of claims 1 to 5 characterized in that the substituent or substituents, identical or different, which can be carried by the alkyl, alkenyl, alkynyl, cycloalkyl, carbocyclic and alkoxy radicals are chosen from the group formed by:
- halogen atoms,
- alkyl, alkenyl, alkynyl or alkoxy radicals containing at most 5 carbon atoms,
- formyl radicals, acyl radicals containing 2 to 6 carbon atoms and benzoyl radicals,
- free carboxy radicals, carboxy radicals esterified by a linear or branched alkyl radical containing at most 5 carbon atoms and salified carboxy radicals,
- hydroxy, cyano, amino, monoalkyl- and dialkylamino radicals,
- aryl and arylalkyl radicals containing 6 to 14 carbon atoms, these radicals being optionally substituted by one or more identical or different radicals, chosen from the group formed by halogen atoms, hydroxy, cyano, nitro radicals, linear or branched alkyl, alkenyl, alkynyl and alkoxy radicals containing at most 5 carbon atoms,

said products of formula (I) being in all possible racemic, enantiomeric and diastereoisomeric isomer forms, as well as the addition salts with mineral and organic acids or with bases of said products of formula (I).

**7.** Compounds of formula (I) as defined in any one of claims 1 to 6, characterized in that R, when it represents an alkyl, alkenyl or alkynyl radical, substituted by an aryl or a substituted aryl, is such a radical substituted by a phenyl or a substituted phenyl.

**8.** Compounds of formula (I) as defined in any one of claims 1 to 7, characterized in that Y represents a phenyl, naphthyl, thionaphthyl, benzofuryl, benzopyrrolyl or indenyl radical optionally substituted by one or more radicals chosen from the group formed by:
- halogen atoms,
- linear or branched alkyl, alkenyl, alkynyl or alkoxy radicals containing at most 7 carbon atoms and being optionally substituted by one or more radicals chosen from the group formed by the hydroxy radical and linear or branched alkyl and alkoxy radicals containing at most 5 carbon atoms,
- acyl radicals containing 2 to 6 carbon atoms,
- hydroxy, trifluoromethyl, cyano, nitro, amino radicals, monoalkyl- and dialkylamino radicals containing 1 to 5 carbon atoms,
- and the phenyl radical itself optionally substituted by one or more radicals, identical or different, chosen from the group formed by halogen atoms, hydroxy, cyano, nitro radicals, linear or branched alkyl, alkenyl, alkynyl and alkoxy radicals containing at most 5 carbon atoms,

said products of formula (I) being in all possible racemic, enantiomeric and diastereoisomeric isomer forms, as well as the addition salts with mineral and organic acids or with bases of said products of formula (I).

**9.** Products of formula (I) as defined in any one of claims 1 to 8, characterized in that A represents:
- either a cycloalkyl radical of formula

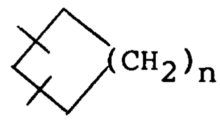

where n is an integer which can take the values 0 to 4, optionally substituted by a linear or branched alkyl or alkoxy radical containing at most 5 carbon atoms,

- or the

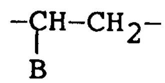

group in which B represents a phenyl radical optionally substituted by one or more identical or different radicals, chosen from the group formed by halogen atoms, hydroxy, cyano, nitro radicals, linear or branched alkyl, alkenyl, alkynyl and alkoxy radicals containing at most 5 carbon atoms,

said products of formula (I) being in all possible racemic, enantiomeric and diastereoisomeric isomer forms, as well as the addition salts with mineral and organic acids or with bases of said products of formula (I).

**10.** Products of formula (I) as defined in any one of claims 1 to 9 characterized in that the heterocycle which can be formed by $R_1$ and R2, together with the nitrogen atom to which they are linked, contains a second nitrogen atom and that this can be optionally substituted by a linear or branched alkyl or alkoxy radical containing 1 to 5 carbon atoms, said products of formula (I) being in all possible racemic, enantiomeric and diastereoisomeric isomer forms, as well as the addition salts with mineral and organic acids or with bases of said products of formula (I).

**11.** (1S)-3-(3,4-dichlorophenyl)-1-[1-phenyl-2-(1-pyrrolidinyl) ethyl]-2-piperidinone (isomer B)
(1S)-3-(3,4-dichlorophenyl)-1-(1-phenyl-2-(1-pyrrolidinyl) ethyl)-2-piperidinone (isomer A)
[trans,(±)]-3-(3,4-dichlorophenyl)-1-(2-(1-pyrrolidinyl) cyclohexyl)-2-piperidinone (isomer A)
(1S)-3-(3,4-dichlorophenyl)-1-(2-methyl-1-((1-pyrrolidinyl) methyl) propyl)-2-piperidinone (isomer A)
(1S)-3-(benzo(b)thien-4-yl)-1-(1-phenyl-2-(1-pyrrolidinyl) ethyl)-2-piperidinone (isomer A)
(1S)-3-(3,4-dichlorophenyl)-3-methyl-1-(1-phenyl-2-(1-pyrrolidinyl) ethyl)-2-piperidinone (isomer A)
(1S)-3-(3,4-dichlorophenyl)-3-ethyl-1-(1-phenyl-2-(1-pyrrolidinyl) ethyl)-2-piperidinone (isomer A)
(1S)-3-(3,4-dichlorophenyl)-1-(1-phenyl-2-(1-pyrrolidinyl) ethyl)-3-(2-propenyl)-2-piperidinone (isomer A)
and if appropriate their salts.

**12.** Preparation process for the products of formula (I) as defined in claim 1 characterized in that the diamine of formula (II):

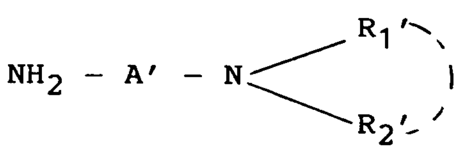

(II)

in which A' and

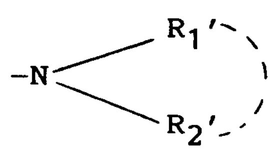

have the meanings indicated in claim 1 for A and

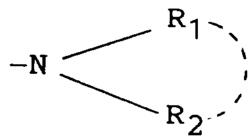

respectively
or those in which the reactive functions which can be carried by A and

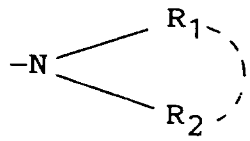

are protected, is reacted with the acrylic ester of formula (III):

$CH_2 = CH - COOR_3$ (III)

in which $R_3$ represents a linear or branched alkyl radical having at most 6 carbon atoms, in order to obtain the condensed product of formula (IV):

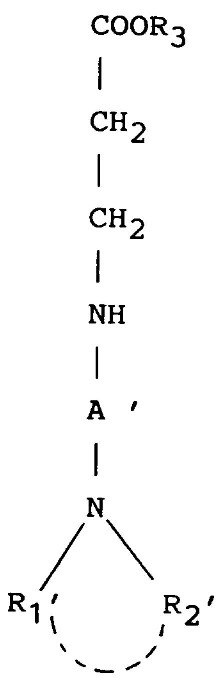

(IV)

in which A',

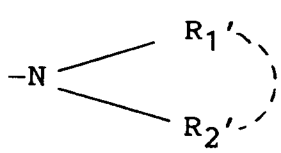

and $R_3$ have the meanings indicated above, which is reacted with the compound of formula (V):

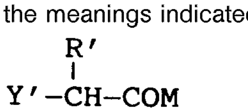

(V)

in which R' represents a hydrogen atom, the values of R indicated above or those in which the reactive functions are protected, M represents a hydroxyl radical or a halogen atom and Y' represents the values of Y indicated above, or those in which the reactive functions are protected, in order to obtain the product of formula (VI):

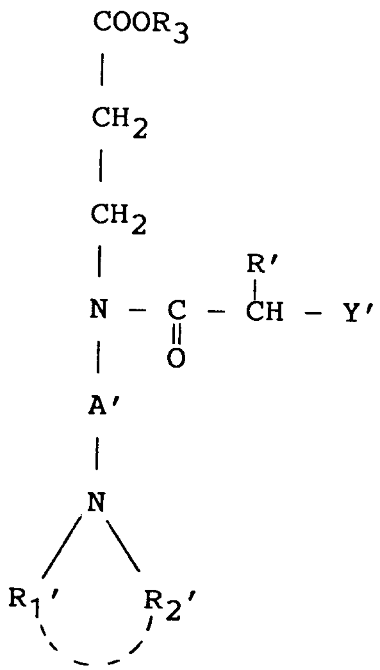

(VI)

in which A',

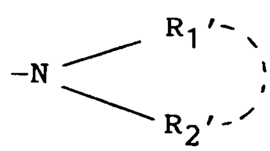

R', $R_3$ and Y' have meanings indicated above, corresponding in the case where R' represents a hydrogen atom or the values of R and A', $NR'_1R'_2$ and Y' have the values of A, $NR_1R_2$ and Y respectively, to a product of formula ($I_B$) as defined above, in which product of formula (VI) if appropriate, the protective group or groups are eliminated, in order to obtain a same product of formula ($I_B$) or which is optionally subjected to a cyclization reaction in order to obtain the product of formula (VII):

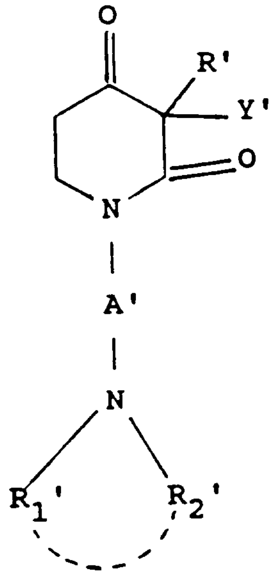

(VII)

in which A',

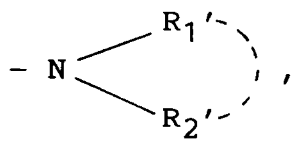

R' and Y' have the meanings indicated above, corresponding in the case where R' represents a hydrogen atom or the values of R and A', $NR'_1R'_2$ and Y' have the values of A, $NR_1R_2$ and Y respectively, to a product of formula ($I_A$) as defined above called ($I_{A1}$), in which product of formula (VII) if appropriate, the protective group or groups are eliminated, in order to obtain a same product of formula ($I_{A1}$), then, if appropriate:

- either, when R' or R represents a hydrogen atom, said product of formula (VII) or ($I_{A1}$) is subjected:

  -- either to a reduction reaction of the oxo function in gamma position of the nitrogen atom in order to obtain the product of formula (VIII):

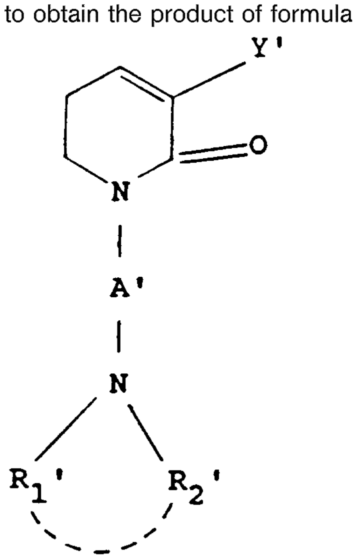

(VIII)

in which A',

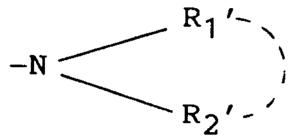

and Y' have meanings indicated above, corresponding in the case where Y', A' and $NR'_1R'_2$ have the values of Y, A and $NR_1R_2$ respectively, to a product of formula ($I_A$) as defined above, called ($I_{A2}$), in which product of formula (VIII), if appropriate, the protective groups are eliminated, in order to obtain a same product of formula ($I_{A2}$),

-- or to a reduction reaction by catalytic hydrogenation of the same oxo function in order to obtain the product of formula (IX):

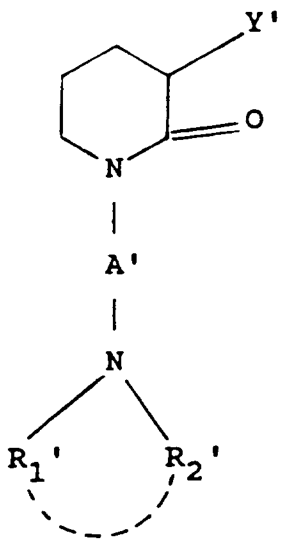

(IX)

in which A',

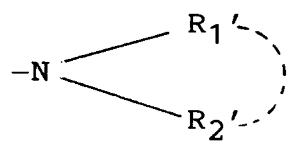

and Y' have the meanings indicated above, corresponding in the case where Y', A' and $NR'_1R'_2$ have the values of Y, A and $NR_1R_2$ respectively, to a product of formula (IA) as defined above, called ($I_{A3}$), in which product of formula (IX), if appropriate, the protective groups are eliminated, in order to obtain a same product of formula ($I_{A3}$), which product of formula (IX) or ($I_{A3}$) is optionally treated with a strong base to form the corresponding anion, which is subjected to the action of a reagent capable of grafting an R' radical, R' having the values indicated above with the exception of a hydrogen atom,

in order to obtain a product of formula (X):

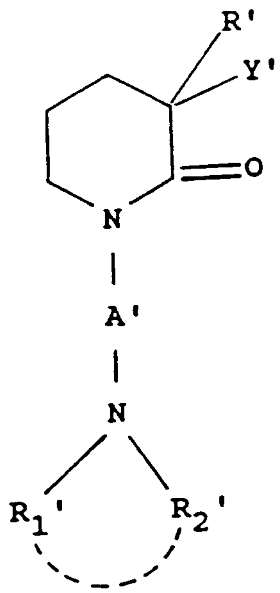

(X)

in which R', Y', A' and

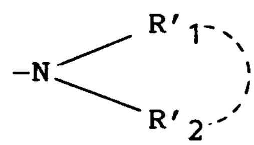

have the values indicated above, corresponding in the case where R', Y', A' and $NR'_1R'_2$ have the values of R (with the exception of hydrogen), Y, A and $NR_1R_2$ respectively, to a product of formula ($I_A$) as defined above, called ($I_{A4}$), in which product of formula (X), if appropriate, the protective groups are eliminated, in order to obtain a same product of formula ($I_{A4}$);

- or when R' or R is different from a hydrogen atom, said product of formula (VII) or ($I_{A1}$) is subjected to the action of a reducing agent of the oxo function, in order to obtain the product of formula (X) as defined previously which either corresponds to a product of formula ($I_{A4}$) or is converted into this product of formula ($I_{A4}$), and if desired, the products of formula (I) are treated with a mineral or organic acid or if appropriate with a base, in order to obtain the corresponding salt, said products of formula (I) being in all possible racemic, enantiomeric and diastereoisomeric isomer forms.

**13.** As medicaments, the products corresponding to formula (I) as defined in claim 1, said products of formula (I) being in all possible racemic, enantiomeric and diastereoisomeric isomer forms, as well as the addition salts with pharmaceutically acceptable mineral and organic acids or bases of said products of formula (I).

**14.** As medicaments, the products of formula ($I_A$) as defined in claim 2, as well as the addition salts with pharmaceutically acceptable mineral and organic acids or bases of said products of formula ($I_A$).

**15.** As medicaments, the products of formula ($I_B$) as defined in claim 3, as well as the addition salts with pharmaceutically mineral and organic acids or bases of said products of formula ($I_B$).

**16.** As medicaments the products defined in claim 11.

**17.** The pharmaceutical composition containing at least one of the medicaments as defined in any one of claims 13 to 16 as active ingredient.

**18.** As new industrial products, the compounds of formulae (IV), (VI), (VII), (VIII), (IX) and (X).

**Claims for the following Contracting State : ES**

**1.** Preparation process for the compounds of formula (I):

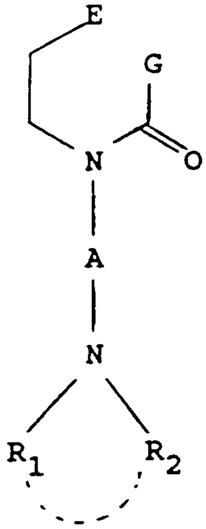

(I)

in which E and G are such that:

- <u>either</u> E and G together form a

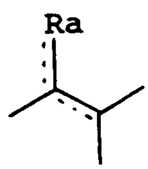

group which represents one of the following groups:

a) 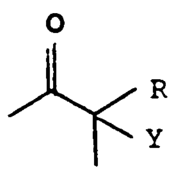 or, if appropriate, its tautomer, 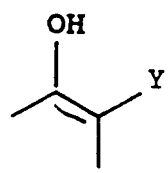

in which R represents a hydrogen atom, a linear or branched alkyl radical containing 1 to 6 carbon atoms, a linear or branched alkenyl or alkynyl radical containing 2 to 6 carbon atoms optionally substituted by one or more substituents chosen either from aryl radicals containing 6 to 14 carbon atoms, aryl radicals substituted by a halogen atom, a hydroxy, trifluoromethyl radical, an alkoxy radical containing 1 to 6 carbon atoms, a nitro, amino and cyano radical, or from free, esterified or salified carboxy radicals, acyl radicals containing 1 to 7 carbon atoms, alkoxy radicals containing 1 to 6 carbon atoms, hydroxy, amino, alkylamino, dialkylamino radicals, acyloxy radicals containing 1 to 7 carbon atoms, cyano, carbamoyl radicals and halogen atoms

-- Y represents an aryl radical chosen from the carbocyclic aryl radicals containing 6 to 14 carbon atoms, the heteromonocyclic aryl radicals containing 5 to 7 members and the condensed heterocyclic aryl radicals, all these radicals being optionally substituted,

b) 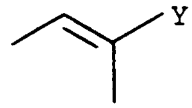 or

c) 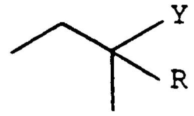

Y and R being as defined above;

- or E represents a $-CO_2R_3$ radical, in which $R_3$ represents a hydrogen atom or a linear or branched alkyl radical (1 to 4 carbons) and G represents a

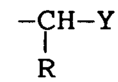

group in which Y and R are as defined above;

-- A represents:
  - either a carbocyclic radical, optionally substituted,
  - or the

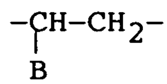

group
where B represents:
    - a hydrogen atom,
    - an aryl or arylalkyl radical containing 6 to 14 carbon atoms, these radicals being optionally substituted,
    - an optionally substituted cycloalkyl radical containing 3 to 7 carbon atoms,
    - an alkyl, alkenyl, alkynyl or alkyloxy radical, these radicals being linear or branched, containing at most 7 carbon atoms and being optionally substituted,

-- $R_1$ and $R_2$, identical or different, represent a hydrogen atom or an alkyl, alkenyl or alkynyl radical, these radicals being linear or branched, containing at most 7 carbon atoms and being optionally substituted,
or $R_1$ and $R_2$ form together with the nitrogen atom to which they are linked a saturated or unsaturated heterocycle with 5, 6 or 7 members optionally containing another heteroatom such as oxygen, nitrogen or sulphur, this heterocycle being optionally substituted,

said products of formula (I) being in all possible racemic, enantiomeric and diastereoisomeric isomer forms, as well as the addition salts with mineral and organic acids or bases of said products of formula (I), characterized in that the diamine of formula (II):

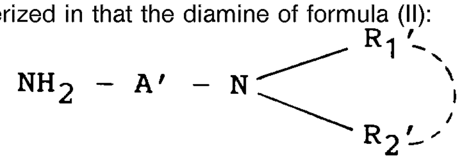 (II)

in which A' and

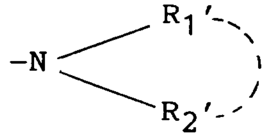

have the meanings indicated above for A and

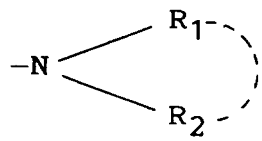

respectively
or those in which the reactive functions which can be carried by A and

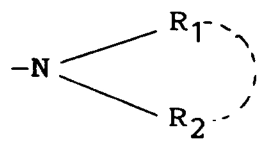

are protected, is reacted with the acrylic ester of formula (III):

$CH_2 = CH - COOR_3$ (III)

in which $R_3$ represents a linear or branched alkyl radical having at most 6 carbon atoms, in order to obtain the condensed product of formula (IV):

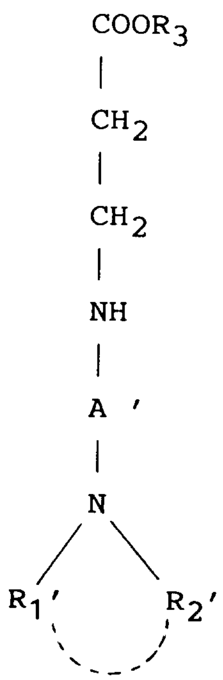

(IV)

in which A',

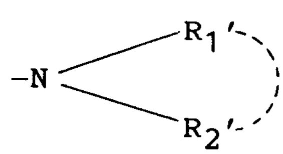

and $R_3$ have the meanings indicated above, which is reacted with the compound of formula (V):

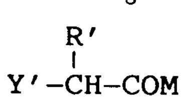

(V)

in which R' represents a hydrogen atom, the values of R indicated above or those in which the reactive functions are protected, M represents a hydroxyl radical or a halogen atom and Y' represents the values of Y indicated above, or those in which the reactive functions are protected, in order to obtain the product of formula (VI):

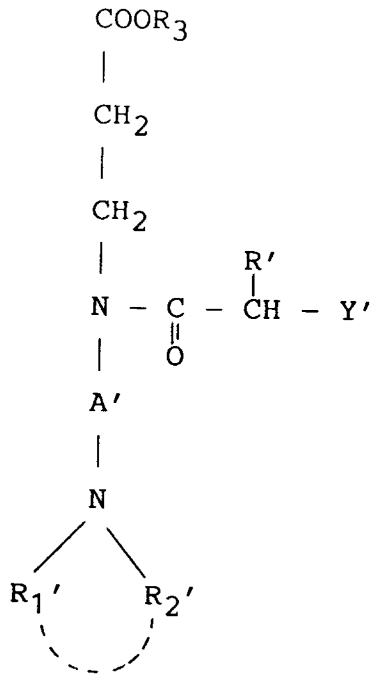

(VI)

in which A',

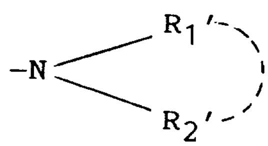

,

R', $R_3$ and Y' have the meanings indicated above, corresponding in the case where R' represents a hydrogen atom or the values of R and A', $NR'_1R'_2$ and Y' have the values of A, $NR_1R_2$ and Y respectively, to a product of formula ($I_B$) as defined above, in which product of formula (VI) if appropriate, the protective group or groups are eliminated, in order to obtain a same product of formula ($I_B$) or which is optionally subjected to a cyclization reaction in order to obtain the product of formula (VII):

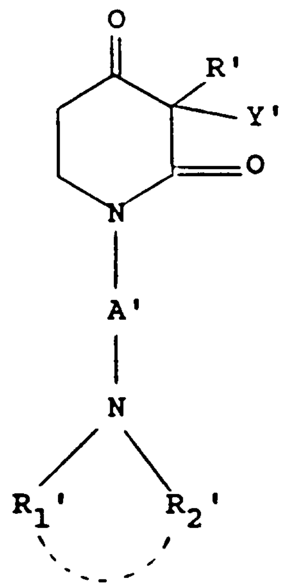

(VII)

in which A',

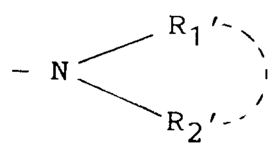

,

R' and Y' have the meanings indicated above, corresponding in the case where R' represents a hydrogen atom or the values of R and A', $NR'_1R'_2$ and Y' have the values of A, $NR_1R_2$ and Y respectively, to a product of formula ($I_A$) as defined above called ($I_{A1}$), in which product of formula (VII) if appropriate, the protective group or groups are eliminated, in order to obtain a same product of formula ($I_{A1}$), then, if appropriate:

- either, when R' or R represents a hydrogen atom, said product of formula (VII) or ($I_{A1}$) is subjected:

  -- either to a reduction reaction of the oxo function in gamma position of the nitrogen atom in order to obtain the product of formula (VIII):

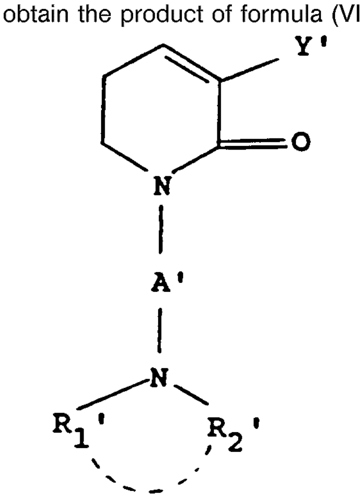

(VIII)

in which A',

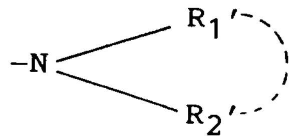

and Y' have the meanings indicated above, corresponding in the case where Y', A' and $NR'_1R'_2$ have the values of Y, A and $NR_1R_2$ respectively, to a product of formula ($I_A$) as defined above, called ($I_{A2}$), in which product of formula (VIII), if appropriate, the protective groups are eliminated, in order to obtain a same product of formula ($I_{A2}$),

-- or to a reduction reaction by catalytic hydrogenation of the same oxo function in order to obtain the product of formula (IX):

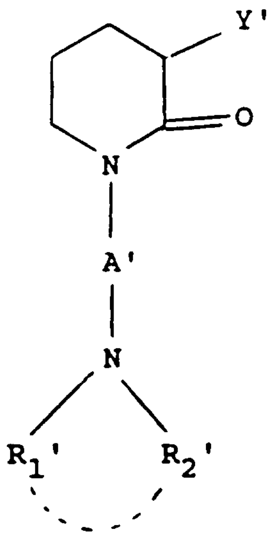

(IX)

in which A',

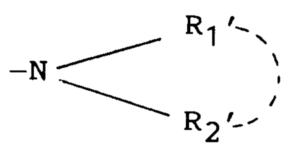

and Y' have the meanings indicated above, corresponding in the case where Y', A' and $NR'_1R'_2$ have the values of Y, A and $NR_1R_2$ respectively, to a product of formula ($I_A$) as defined above, called ($I_{A3}$), in which product of formula (IX), if appropriate, the protective groups are eliminated, in order to obtain a same product of formula ($I_{A3}$), which product of formula (IX) or ($I_{A3}$) is optionally treated with a strong base to form the corresponding anion, which is subjected to the action of a reagent capable of grafting an R' radical, R' having the values indicated above with the exception of a hydrogen atom,

in order to obtain a product of formula (X):

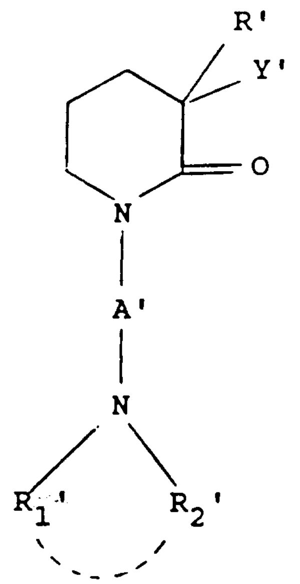

(X)

in which R', Y', A' and

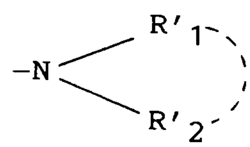

have the values indicated above, corresponding in the case where R', Y', A' and $NR'_1R'_2$ have the values of R (with the exception of hydrogen), Y, A and $NR_1R_2$ respectively, to a product of formula ($I_A$) as defined above, called ($I_{A4}$), in which product of formula (X), if appropriate, the protective groups are eliminated, in order to obtain a same product of formula ($I_{A4}$);

- or when R' or R is different from a hydrogen atom, the said product of formula (VII) or ($I_{A1}$) is subjected to the action of a reducing agent of the oxo function, in order to obtain the product of formula (X) as defined previously which either corresponds to a product of formula ($I_{A4}$) or is converted into this product of formula ($I_{A4}$), and if desired, the products of formula (I) are treated with a mineral or organic acid or if appropriate with a base, in order to obtain the corresponding salt, said products of formula (I) being in all possible racemic, enantiomeric and diastereoisomeric isomer forms.

**2.** Process according to claim 1 for the preparation of products corresponding to formula ($I_A$):

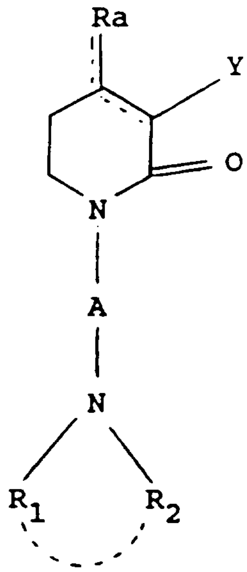

($I_A$)

in which the group:

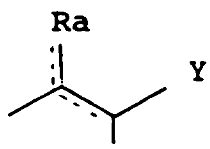

,

A, $R_1$ and $R_2$ are as defined in claim 1, characterized in that the diamine of formula (II):

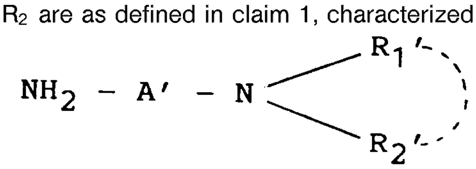

(II)

in which A' and

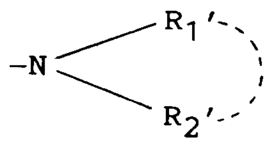

have the meanings indicated above for A and

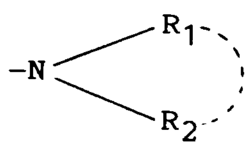

respectively or those in which the reactive functions which can be carried by A and

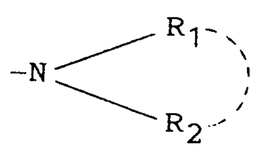

are protected, is reacted with the acrylic ester of formula (III):

$CH_2 = CH - COOR_3$ (III)

in which $R_3$ represents a linear or branched alkyl radical having at most 6 carbon atoms, in order to obtain the condensed product of formula (IV):

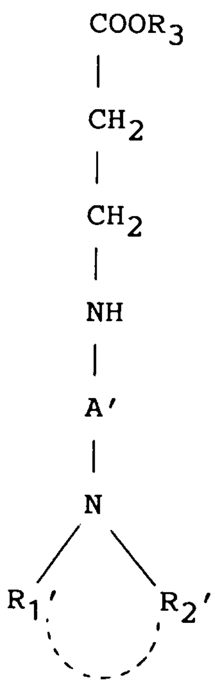

(IV)

in which A',

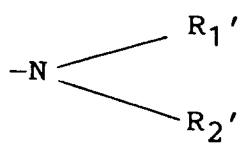

and $R_3$ have the meanings indicated above, which is reacted with the compound of formula (V):

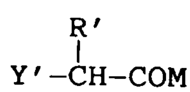

(V)

in which R' represents a hydrogen atom, the values of R indicated above or those in which the reactive functions are protected, M represents a hydroxyl radical or a halogen atom and Y' represents the values of Y indicated above, or those in which the reactive functions are protected, in order to obtain the product of formula (VI):

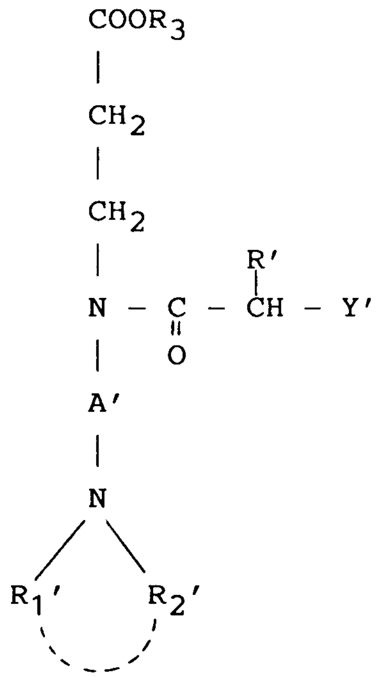

(VI)

in which A',

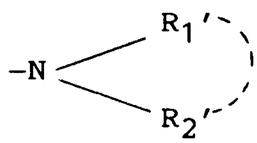

,

R', $R_3$ and Y' have the meanings indicated above, which is subjected to a cyclization reaction in order to obtain the product of formula (VII):

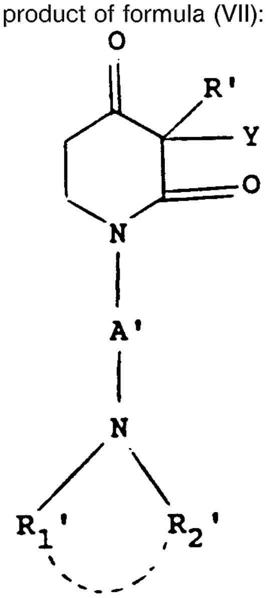

(VII)

in which A',

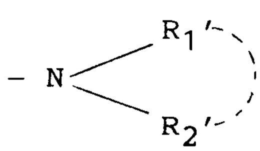

,

R' and Y' have the meanings indicated above, corresponding in the case where R' represents a hydrogen atom or the values of R and A', $NR'_1R'_2$ and Y' have the values of A, $NR_1R_2$ and Y respectively, to a product of formula ($I_A$) as defined above called ($I_{A1}$), in which product of formula (VII) if appropriate, the protective group or groups are eliminated, in order to obtain a same product of formula ($I_{A1}$), then, if appropriate:

- either, when R' or R represents a hydrogen atom, said product of formula (VII) or ($I_{A1}$) is subjected:

  -- either to a reduction reaction of the oxo function in gamma position of the nitrogen atom in order to obtain the product of formula (VIII):

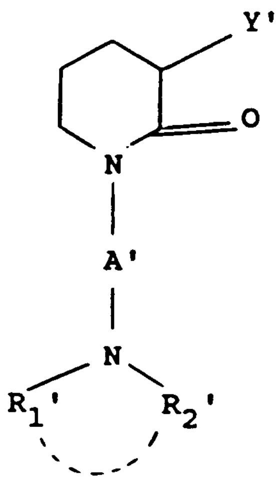

(VIII)

in which A',

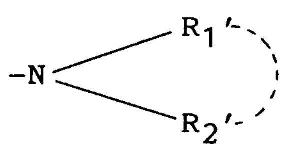

and Y' have the meanings indicated above, corresponding in the case where Y', A' and $NR'_1R'_2$ have the values of Y, A and $NR_1R_2$ respectively, to a product of formula ($I_A$) as defined above, called ($I_{A2}$), in which product of formula (VIII), if appropriate, the protective groups are eliminated, in order to obtain a same product of formula ($I_{A2}$),

-- or to a reduction reaction by catalytic hydrogenation of the same oxo function in order to obtain the product of formula (IX):

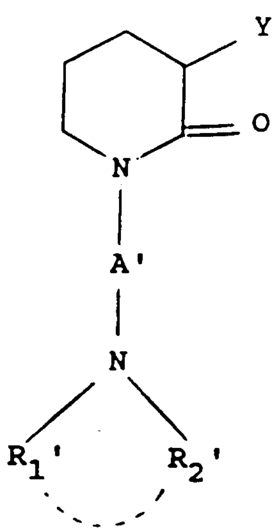

(IX)

in which A',

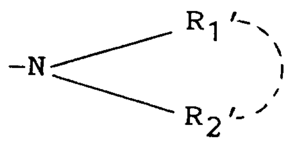

and Y' have the meanings indicated above, corresponding in the case where Y', A' and $NR'_1R'_2$ have the values of Y, A and $NR_1R_2$ respectively, to a product of formula (IA) as defined above, called ($I_{A3}$), in which product of formula (IX), if appropriate, the protective groups are eliminated, in order to obtain a same product of formula ($I_{A3}$), which product of formula (IX) or ($I_{A3}$) is optionally treated with a strong base to form the corresponding anion, which is subjected to the action of a reagent capable of grafting an R' radical, R' having the values indicated above with the exception of a hydrogen atom,
in order to obtain a product of formula (X):

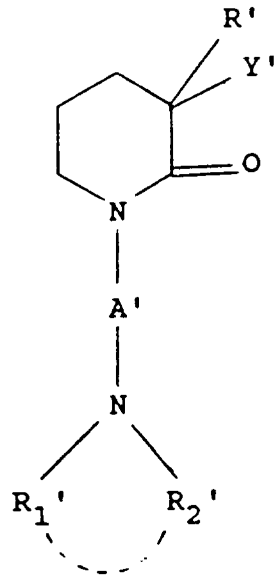

(X)

in which R', Y', A' and

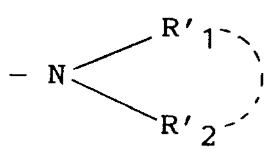

have the values indicated above, corresponding in the case where R', Y', A' and $NR'_1R'_2$ have the values of R (with the exception of hydrogen), Y, A and $NR_1R_2$ respectively, to a product of formula ($I_A$) as defined above, called ($I_{A4}$), in which product of formula (X), if appropriate, the protective groups are eliminated, in order to obtain a same product of formula ($I_{A4}$);

- or when R' or R is different from a hydrogen atom, said product of formula (VII) or ($I_{A1}$),is subjected to the action of a reducing agent of the oxo function, in order to obtain the product of formula (X) as defined previously which either corresponds to a product of formula ($I_{A4}$) or is converted into this product of formula ($I_{A4}$), and if desired, the products of formula (I) are treated with a mineral or organic acid or if appropriate with a base, in order to obtain the corresponding salt, said products of formula ($I_A$) being in all possible racemic, enantiomeric and diastereoisomeric isomer forms.

**3.** Process according to claim 2 for the preparation of products of formula ($I_A$) in which the group

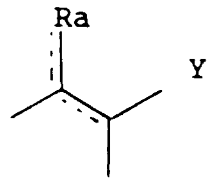

represents

a) 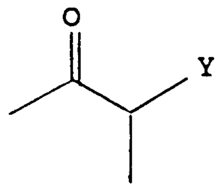 or if appropriate its tautomer 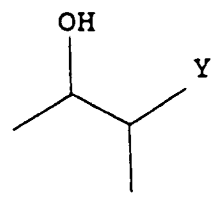

b) 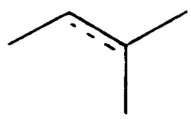

c) 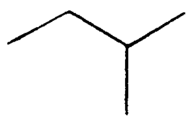

Y being as defined in claim 1, characterized in that a compound of formula (V) is used in which R' represents a hydrogen atom.

**4.** Process according to claim 1 for the preparation of products corresponding to formula ($I_B$):

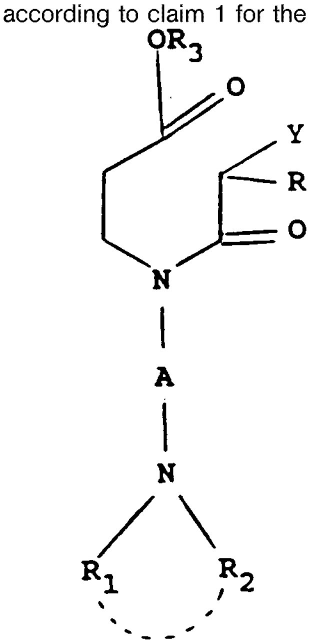

($I_B$)

in which $R_1$ $R_2$, $R_3$, A, R and Y are as defined in claim 1 characterized in that the diamine of formula (II):

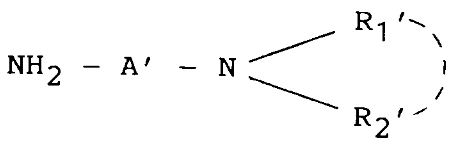

(II)

in which A' and

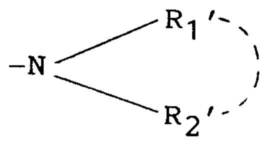

have meanings indicated above for A and

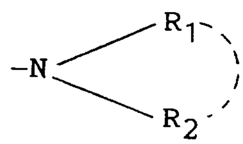

respectively
or those in which the reactive functions which can be carried by A and

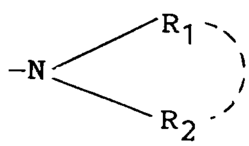

are protected, is reacted with the acrylic ester of formula (III):

$CH_2 = CH - COOR_3$ (III)

in which $R_3$ represents a linear or branched alkyl radical having at most 6 carbon atoms, in order to obtain the condensed product of formula (IV):

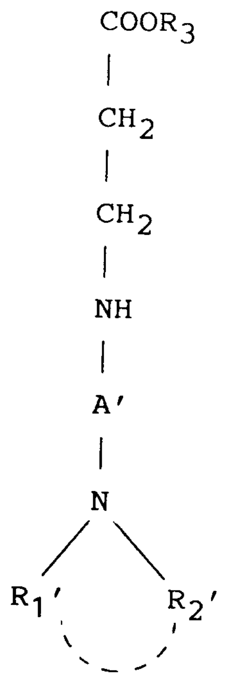

(IV)

in which A',

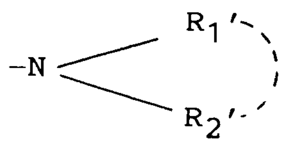

and $R_3$ have the meanings indicated above, which is reacted with the compound of formula (V):

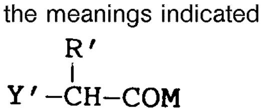

(V)

in which R' represents a hydrogen atom, the values of R indicated above or those in which the reactive functions are protected, M represents a hydroxyl radical or a halogen atom and Y' represents the values of Y indicated above, or those in which the reactive functions are protected, in order to obtain the product of formula (VI):

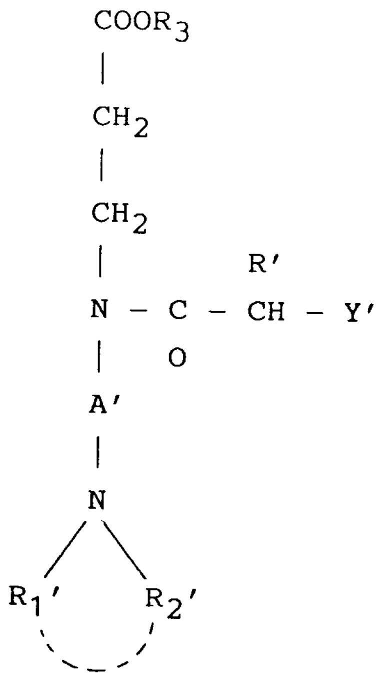

(VI)

in which A',

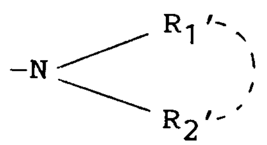

,

R', $R_3$ and Y' have the meanings indicated above, corresponding in the case where R' represents a hydrogen atom or the values of R and A', $NR'_1R'_2$ and Y' have the values of A, $NR_1R_2$ and Y respectively, to a product of formula ($I_B$) as defined above, in which product of formula (VI) if appropriate, the protective group or groups are eliminated, in order to obtain a same product of formula ($I_B$) and, if desired, the products of formula (I) are treated with a mineral or organic acid or, if appropriate, with a base, in order to obtain the corresponding salt, said products of formula ($I_B$) being in all possible racemic, enantiomeric and diastereoisomeric isomer forms.

**5.** Process according to claim 4 for the preparation of products of formula ($I_B$) in which R represents a hydrogen atom, characterized in that a compound of formula (V) is used in which R' represents a hydrogen atom.

**6.** Process according to any one of claims 1 to 5, characterized in that compounds are used at the start in which the substituent or substituents, identical or different, which can be carried by the aryl, arylalkyl and heterocyclic radicals or those which can be formed by $R_1$ and $R_2$ with the nitrogen atom to which they are linked, are chosen from the group formed by:

- halogen atoms,
- alkyl, alkenyl, alkynyl or alkyloxy radicals, these radicals being linear or branched containing at most 7 carbon atoms and being optionally substituted,
- hydroxy, trifluoromethyl, trifluoromethylthio, trifluoromethoxy, cyano, nitro, sulphamoyl, amino, monoalkyl-and dialkylamino radicals,
- formyl radicals, acyl radicals containing 2 to 6 carbon atoms and benzoyl radicals,
- free carboxy radicals, carboxy radicals esterified by a linear or branched alkyl radical containing at most 5 carbon atoms and salified carboxy radicals,
- the carbamoyl radical optionally substituted by one or more identical or different radicals, chosen from the group formed by alkyl, alkoxy or hydroxyalkyl radicals and the carbamoylalkyl radical, these alkyl and alkoxy radicals containing 1 to 4 carbon atoms,

- and the phenyl or benzyl radical itself optionally substituted by one or more identical or different radicals, chosen from the group formed by halogen atoms and the following radicals: hydroxy, cyano, nitro, linear or branched alkyl, alkenyl, alkynyl and alkoxy containing at most 5 carbon atoms.

**7.** Process according to any one of claims 1 to 5, characterized in that compounds are used at the start in which the substituent or substituents, identical or different, which can be carried by the alkyl, alkenyl, alkynyl, cycloalkyl, carbocyclic and alkoxy radicals are chosen from the group formed by:
- halogen atoms,
- alkyl, alkenyl, alkynyl or alkoxy radicals containing at most 5 carbon atoms,
- formyl radicals, acyl radicals containing 2 to 6 carbon atoms and benzoyl radicals,
- free carboxy radicals, carboxy radicals esterified by a linear or branched alkyl radical containing at most 5 carbon atoms and salified carboxy radicals,
- hydroxy, cyano, amino, monoalkyl- and dialkylamino radicals,
- aryl and arylalkyl radicals containing 6 to 14 carbon atoms, these radicals being optionally substituted by one or more identical or different radicals, chosen from the group formed by halogen atoms, hydroxy, cyano, nitro radicals, linear or branched alkyl, alkenyl, alkynyl and alkoxy radicals containing at most 5 carbon atoms.

**8.** Process according to any one of claims 1, 2, 4 or 6, characterized in that a compound of formula (V) is used at the start in which R' represents an alkyl, alkenyl or alkynyl radical substituted by a phenyl or a substituted phenyl, the optional reactive functions being, if appropriate, protected.

**9.** Process according to any one of claims 1 to 8, characterized in that a compound of formula (V) is used at the start in which Y' represents a phenyl, naphthyl, thionaphthyl, benzofuryl, benzopyrrolyl or indenyl radical optionally substituted by one or more radicals chosen from the group formed by:
- halogen atoms,
- linear or branched alkyl, alkenyl, alkynyl or alkoxy radicals containing at most 7 carbon atoms and being optionally substituted by one or more radicals chosen from the group formed by the hydroxy radical and linear or branched alkyl and alkoxy radicals containing at most 5 carbon atoms,
- acyl radicals containing 2 to 6 carbon atoms,
- hydroxy, trifluoromethyl, cyano, nitro, amino radicals, monoalkyl- and dialkylamino radicals containing 1 to 5 carbon atoms,
- and the phenyl radical itself optionally substituted by one or more radicals, identical or different, chosen from the group formed by halogen atoms, hydroxy, cyano, nitro radicals, linear or branched alkyl, alkenyl, alkynyl and alkoxy radicals containing at most 5 carbon atoms, the optional reactive functions being, if appropriate, protected.

**10.** Process according to any one of claims 1 to 9, characterized in that a compound of formula (II) is used at the start in which A' represents:
- either a cycloalkyl radical of formula

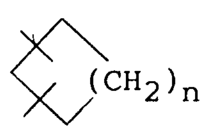

where n is an integer which can take the values 0 to 4, optionally substituted by a linear or branched alkyl or alkoxy radical containing at most 5 carbon atoms,
- or the

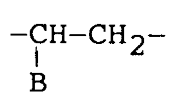

group in which B represents a phenyl radical optionally substituted by one or more identical or different radicals, chosen from the group formed by halogen atoms, hydroxy, cyano, nitro

radicals, linear or branched alkyl, alkenyl, alkynyl and alkoxy radicals containing at most 5 carbon atoms, the optional reactive functions being, if appropriate, protected.

**11.** Process according to any one of claims 1 to 10, characterized in that a compound of formula (II) is used at the start in which $R'_1$ and $R'_2$ form together with the nitrogen atom to which they are linked a heterocycle containing a second nitrogen atom, this being optionally substituted by a linear or branched alkyl or alkoxy radical containing 1 to 5 carbon atoms.

**12.** Process according to claim 1, characterized in that the starting products are chosen in such a way that any one of the products of formula (I) are prepared the names of which follow:
(1S)-3-(3,4-dichlorophenyl)-1-[1-phenyl-2-(1-pyrrolidinyl) ethyl]-2-piperidinone (isomer B)
(1S)-3-(3,4-dichlorophenyl)-1-(1-phenyl-2-(1-pyrrolidinyl) ethyl)-2-piperidinone (isomer A)
[trans,(±)]-3-(3,4-dichlorophenyl)-1-(2-(1-pyrrolidinyl) cyclohexyl)-2-piperidinone (isomer A)
(1S)-3-(3,4-dichlorophenyl)-1-(2-methyl 1-((1-pyrrolidinyl) methyl) propyl)-2-piperidinone (isomer A)
(1S)-3-(benzo(b)thien-4-yl)-1-(1-phenyl-2-(1-pyrrolidinyl) ethyl)-2-piperidinone (isomer A)
(1S)-3-(3,4-dichlorophenyl)-3-methyl-1-(1-phenyl-2-(1-pyrrolidinyl) ethyl)-2-piperidinone (isomer A)
(1S)-3-(3,4-dichlorophenyl)-3-ethyl-1-(1-phenyl-2-(1-pyrrolidinyl) ethyl)-2-piperidinone (isomer A)
(1S)-3-(3,4-dichlorophenyl)-1-(1-phenyl-2-(1-pyrrolidinyl) ethyl)-3-(2-propenyl)-2-piperidinone (isomer A)
and if appropriate their salts.

**Claims for the following Contracting State : GR**

**1.** Preparation process for the compounds of formula (I):

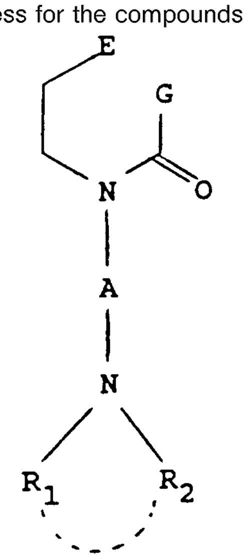

(I)

in which E and G are such that:
- either E and G together form a

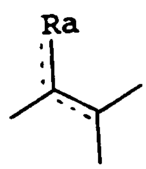

group which represents one of the following groups:

a) 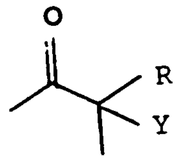 or, if appropriate, its tautomer, 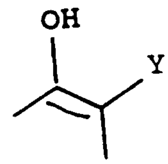

in which R represents a hydrogen atom, a linear or branched alkyl radical containing 1 to 6 carbon atoms, a linear or branched alkenyl or alkynyl radical containing 2 to 6 carbon atoms optionally substituted by one or more substituents chosen either from the aryl radicals containing 6 to 14 carbon atoms, aryl radicals substituted by a halogen atom, a hydroxy, trifluoromethyl radical, an alkoxy radical containing 1 to 6 carbon atoms, a nitro, amino and cyano radical, or from free, esterified or salified carboxy radicals, acyl radicals containing 1 to 7 carbon atoms, alkoxy radicals containing 1 to 6 carbon atoms, hydroxy, amino, alkylamino, dialkylamino radicals, acyloxy radicals containing 1 to 7 carbon atoms, cyano, carbamoyl radicals and halogen atoms
-- Y represents an aryl radical chosen from the carbocyclic aryl radicals containing 6 to 14 carbon atoms, the heteromonocyclic aryl radicals containing 5 to 7 members and the condensed heterocyclic aryl radicals, all these radicals being optionally substituted,

b) 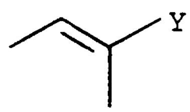 or

c) 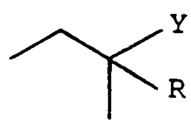

Y and R being as defined above;

- or E represents a $-CO_2R_3$ radical, in which $R_3$ represents a hydrogen atom or a linear or branched alkyl radical (1 to 4 carbons) and G represents a

$$-\underset{\substack{|\\ R}}{CH}-Y$$

group in which Y and R are as defined above;

-- A represents:

- either a carbocyclic radical, optionally substituted,
- or the

$$-\underset{\substack{|\\ B}}{CH}-CH_2-$$

group where B represents:

- a hydrogen atom,
- an aryl or arylalkyl radical containing 6 to 14 carbon atoms, these radicals being optionally substituted,
- an optionally substituted cycloalkyl radical containing 3 to 7 carbon atoms,
- an alkyl, alkenyl, alkynyl or alkyloxy radical, these radicals being linear or branched, containing at most 7 carbon atoms and being optionally substituted,

-- $R_1$ and $R_2$, identical or different, represent a hydrogen atom or an alkyl, alkenyl or alkynyl radical, these radicals being linear or branched, containing at most 7 carbon atoms and being optionally substituted,

or $R_1$ and $R_2$ form together with the nitrogen atom to which they are linked a saturated or unsaturated heterocycle with 5, 6 or 7 members optionally containing another heteroatom such as oxygen, nitrogen or sulphur, this heterocycle being optionally substituted,

said products of formula (I) being in all possible racemic, enantiomeric and diastereoisomeric isomer forms, as well as the addition salts with mineral and organic acids or bases of said products of formula (I), characterized in that the diamine of formula (II):

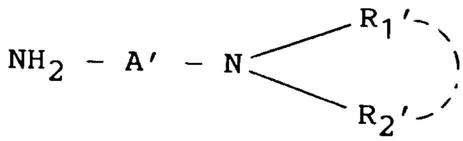

(II)

in which A' and

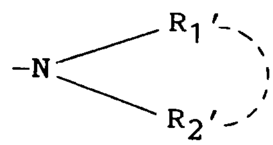

have the meanings indicated above for
A and

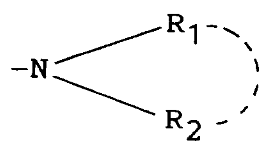

respectively
or those in which the reactive functions which can be carried by
A and

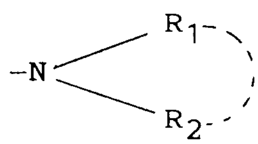

are protected, is reacted with the acrylic ester of formula (III):

$CH_2 = CH - COOR_3$ (III)

in which $R_3$ represents a linear or branched alkyl radical having at most 6 carbon atoms, in order to obtain the condensed product of formula (IV):

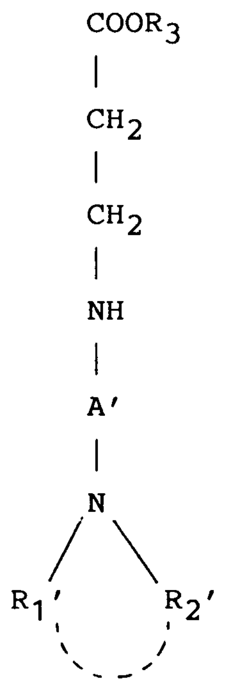

(IV)

in which A',

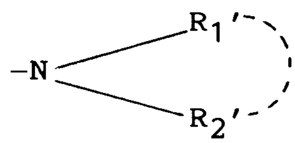

and $R_3$ have the meanings indicated above, which is reacted with the compound of formula (V):

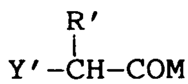

(V)

in which R' represents a hydrogen atom, the values of R indicated above or those in which the reactive functions are protected, M represents a hydroxyl radical or a halogen atom and Y' represents the values of Y indicated above, or those in which the reactive functions are protected, in order to obtain the product of formula (VI):

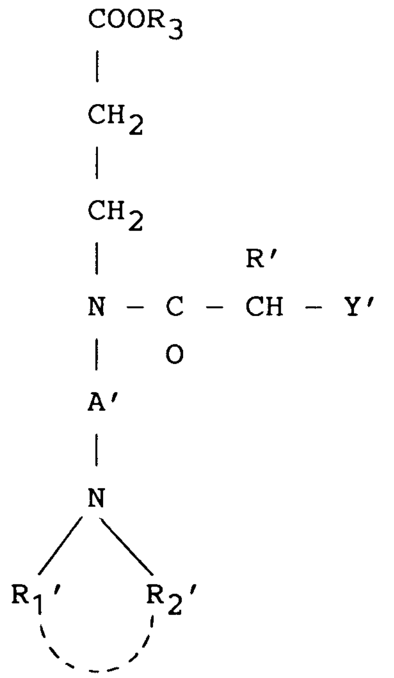

(VI)

in which A',

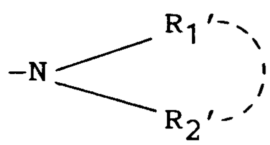

,

R', $R_3$ and Y' have the meanings indicated above, corresponding in the case where R' represents a hydrogen atom or the values of R and A', $NR'_1R'_2$ and Y' have the values of A, $NR_1R_2$ and Y respectively, to a product of formula ($I_B$) as defined above, in which product of formula (VI) if appropriate, the protective group or groups are eliminated, in order to obtain a same product of formula ($I_B$) or which is optionally subjected to a cyclization reaction in order to obtain the product of formula (VII):

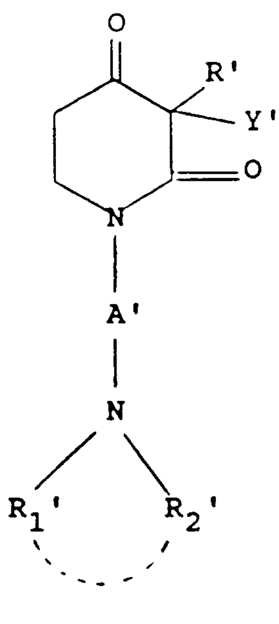

(VII)

in which A',

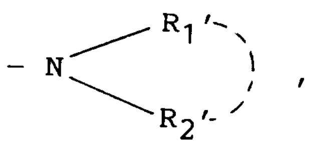

R' and Y' have the meanings indicated above, corresponding in the case where R' represents a hydrogen atom or the values of R and A', $NR'_1R'_2$ and Y' have the values of A, $NR_1R_2$ and Y respectively, to a product of formula ($I_A$) as defined above called ($I_{A1}$), in which product of formula (VII) if appropriate, the protective group or groups are eliminated, in order to obtain a same product of formula ($I_{A1}$), then, if appropriate:

- either, when R' or R represents a hydrogen atom, said product of formula (VII) or ($I_{A1}$) is subjected:

  -- either to a reduction reaction of the oxo function in gamma position of the nitrogen atom in order to obtain the product of formula (VIII):

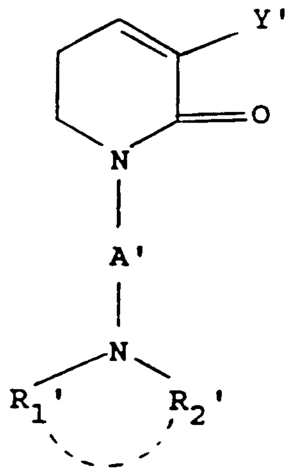

(VIII)

in which A',

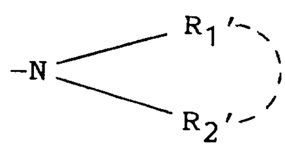

and Y' have the meanings indicated above, corresponding in the case where Y', A' and $NR'_1R'_2$ have the values of Y, A and $NR_1R_2$ respectively, to a product of formula ($I_A$) as defined above, called ($I_{A2}$), in which product of formula (VIII), if appropriate, the protective groups are eliminated, in order to obtain a same product of formula ($I_{A2}$),

-- or to a reduction reaction by catalytic hydrogenation of the same oxo function in order to obtain the product of formula (IX):

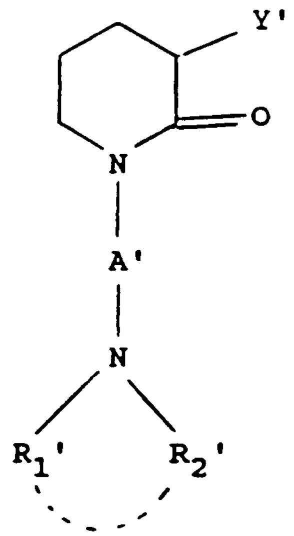

(IX)

in which A',

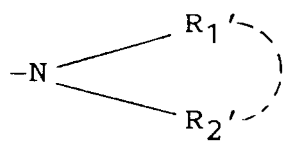

and Y' have the meanings indicated above, corresponding in the case where Y', A' and $NR'_1R'_2$ have the values of Y, A and $NR_1R_2$ respectively, to a product of formula ($I_A$) as defined above, called ($I_{A3}$), in which product of formula (IX), if appropriate, the protective groups are eliminated, in order to obtain a same product of formula ($I_{A3}$), which product of formula (IX) or ($I_{A3}$) is optionally treated with a strong base to form the corresponding anion, which is subjected to the action of a reagent capable of grafting an R' radical, R' having the values indicated above with the exception of a hydrogen atom,
in order to obtain a product of formula (X):

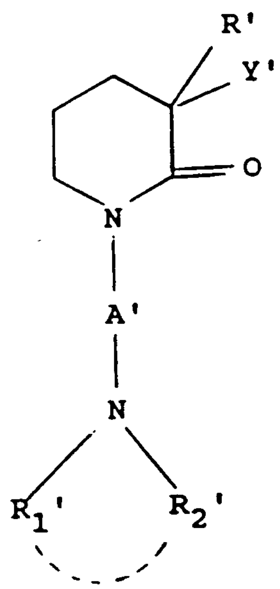

(X)

in which R', Y', A' and

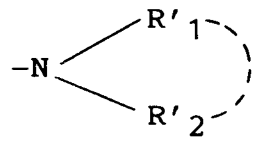

have the values indicated above, corresponding in the case where R', Y', A' and $NR'_1R'_2$ have the values of R (with the exception of hydrogen), Y, A and $NR_1R_2$ respectively, to a product of formula ($I_A$) as defined above, called ($I_{A4}$), in which product of formula (X), if appropriate, the protective groups are eliminated, in order to obtain a same product of formula ($I_{A4}$);

- or when R' or R is different from a hydrogen atom, the said product of formula (VII) or ($I_{A1}$) is subjected to the action of a reducing agent of the oxo function, in order to obtain the product of formula (X) as defined previously which either corresponds to a product of formula ($I_{A4}$) or is converted into this product of formula ($I_{A4}$) and if desired, the products of formula (I) are treated with a mineral or organic acid or if appropriate with a base, in order to obtain the corresponding salt, said products of formula (I) being in all possible racemic, enantiomeric and diastereoisomeric isomer forms.

**2.** Process according to claim 1 for the preparation of products corresponding to formula ($I_A$):

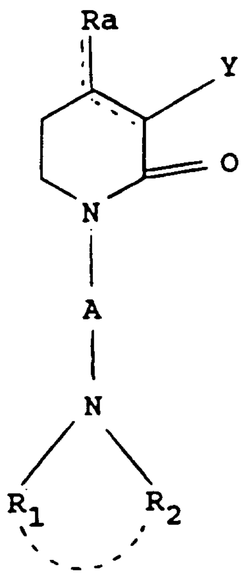

($I_A$)

in which the group:

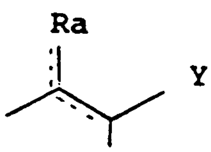

,

A, $R_1$ and $R_2$ are as defined in claim 1, characterized in that the diamine of formula (II):

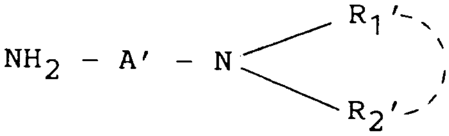

(II)

in which A' and

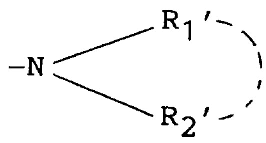

have the meanings indicated above for
A and

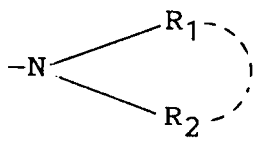

respectively
or those in which the reactive functions which can be carried by
A and

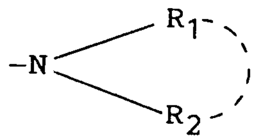

are protected, is reacted with the acrylic ester of formula (III):

$CH_2 = CH - COOR_3$ (III)

in which $R_3$ represents a linear or branched alkyl radical having at most 6 carbon atoms, in order to obtain the condensed product of formula (IV):

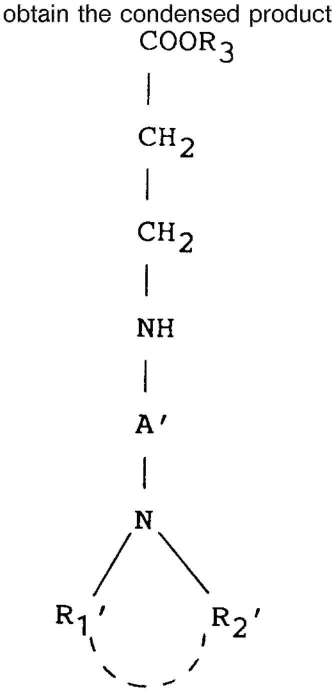

(IV)

in which A',

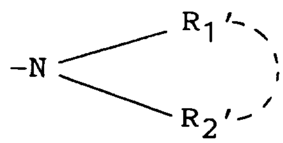

and $R_3$ have the meanings indicated above, which is reacted with the compound of formula (V):

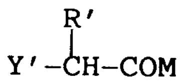

(V)

in which R' represents a hydrogen atom, the values of R indicated above or those in which the reactive functions are protected, M represents a hydroxyl radical or a halogen atom and Y' represents the values of Y indicated above, or those in which the reactive functions are protected, in order to obtain the product of formula (VI):

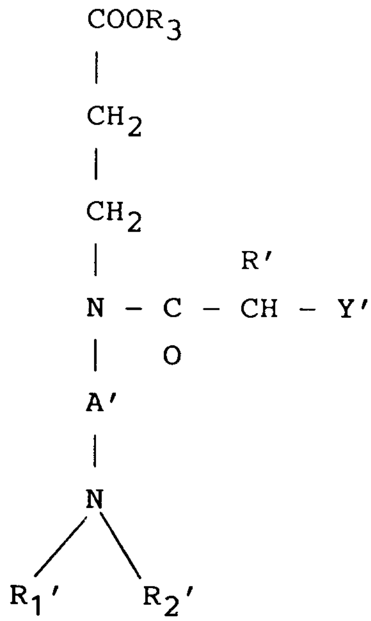

(VI)

in which A',

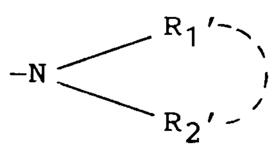

,

R', $R_3$ and Y' have the meanings indicated above, which is subjected to a cyclization reaction in order to obtain the product of formula (VII):

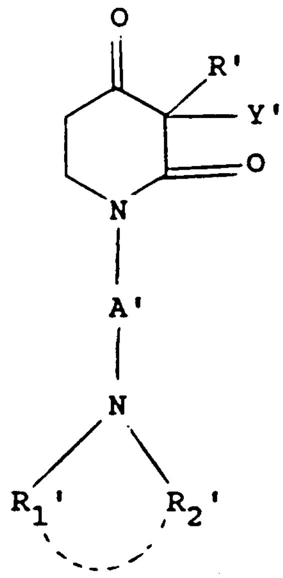

(VII)

in which A',

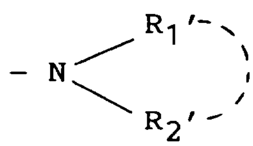

,

R' and Y' have the meanings indicated above, corresponding in the case where R' represents a hydrogen atom or the values of R and A', $NR'_1R'_2$ and Y' have the values of A, $NR_1R_2$ and Y respectively, to a product of formula ($I_A$) as defined above called ($I_{A1}$), in which product of formula (VII) if appropriate, the protective group or groups are eliminated, in order to obtain a same product of formula ($I_{A1}$), then, if appropriate:

- either, when R' or R represents a hydrogen atom, said product of formula (VII) or ($I_{A1}$) is subjected:

  -- either to a reduction reaction of the oxo function in gamma position of the nitrogen atom in order to obtain the product of formula (VIII):

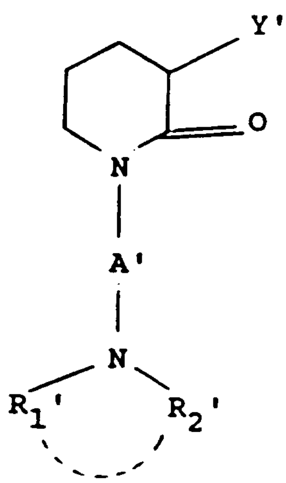

(VIII)

in which A',

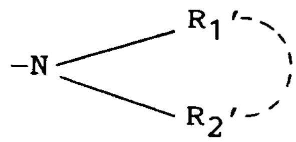

and Y' have the meanings indicated above, corresponding in the case where Y', A' and $NR'_1R'_2$ have the values of Y, A and $NR_1R_2$ respectively, to a product of formula ($I_A$) as defined above, called ($I_{A2}$), in which product of formula (VIII), if appropriate, the protective groups are eliminated, in order to obtain a same product of formula ($I_{A2}$),

-- or to a reduction reaction by catalytic hydrogenation of the same oxo function in order to obtain the product of formula (IX):

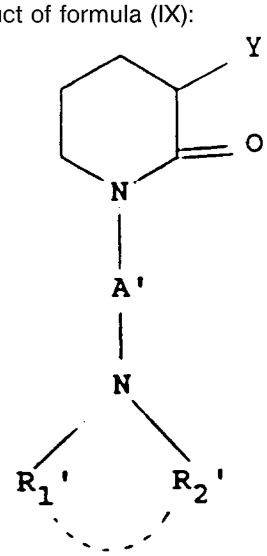

(IX)

in which A',

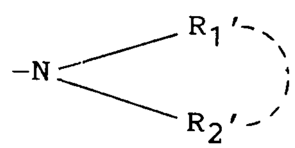

and Y' have the meanings indicated above, corresponding in the case where Y', A' and $NR'_1R'_2$ have the values of Y, A and $NR_1R_2$ respectively, to a product of formula ($I_A$) as defined above, called ($I_{A3}$), in which product of formula (IX), if appropriate, the protective groups are eliminated, in order to obtain a same product of formula ($I_{A3}$), which product of formula (IX) or ($I_{A3}$) is optionally treated with a strong base to form the corresponding anion, which is subjected to the action of a reagent capable of grafting an R' radical, R' having the values indicated above with the exception of a hydrogen atom,

in order to obtain a product of formula (X):

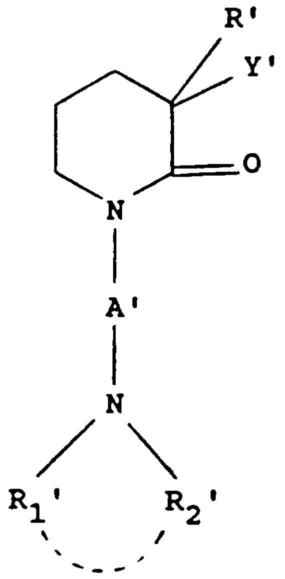

(X)

in which R', Y', A' and

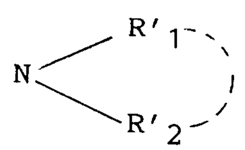

-have the values indicated above, corresponding in the case where R', Y', A' and $NR'_1R'_2$ have the values of R (with the exception of hydrogen), Y, A and $NR_1R_2$ respectively, to a product of formula ($I_A$) as defined above, called ($I_{A4}$), in which product of formula (X), if appropriate, the protective groups are eliminated, in order to obtain a same product of formula ($I_{A4}$);

- or when R' or R is different from a hydrogen atom, said product of formula (VII) or ($I_{A1}$) is subjected to the action of a reducing agent of the oxo function, in order to obtain the product of formula (X) as defined previously which either corresponds to a product of formula ($I_{A4}$) or is converted into this product of formula ($I_{A4}$), and if desired, the products of formula (I) are treated with a mineral or organic acid or if appropriate with a base, in order to obtain the corresponding salt, said products of formula ($I_A$) being in all possible racemic, enantiomeric and diastereoisomeric isomer forms.

**3.** Process according to claim 2 for the preparation of products of formula ($I_A$) in which the group

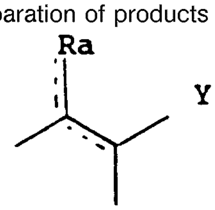

represents

a) 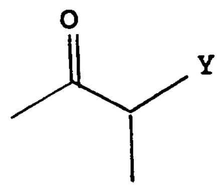 or if appropriate its tautomer 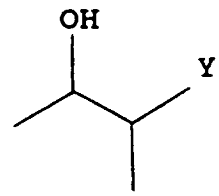

b) 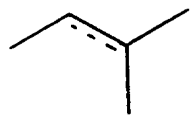

c) 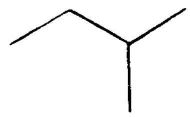

Y being as defined in claim 1, characterized in that a compound of formula (V) is used in which R' represents a hydrogen atom.

**4.** Process according to claim 1 for the preparation of products corresponding to formula ($I_B$):

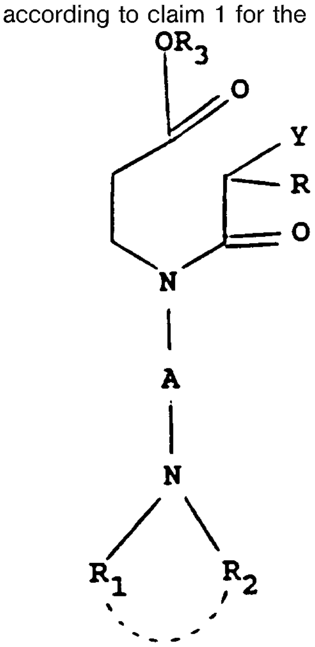

($I_B$)

in which $R_1$ $R_2$, $R_3$, A, R and Y are as defined in claim 1 characterized in that the diamine of formula (II):

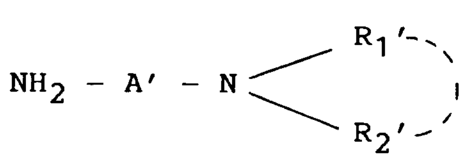

(II)

in which A' and

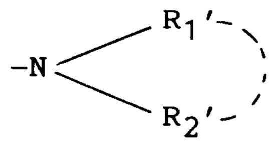

have the meanings indicated above for
A and

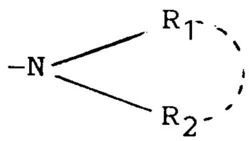

respectively
or those in which the reactive functions which can be carried by
A and

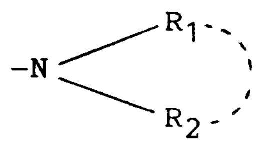

are protected, is reacted with the acrylic ester of formula (III):

$CH_2 = CH - COOR_3$ (III)

in which $R_3$ represents a linear or branched alkyl radical having at most 6 carbon atoms, in order to obtain the condensed product of formula (IV):

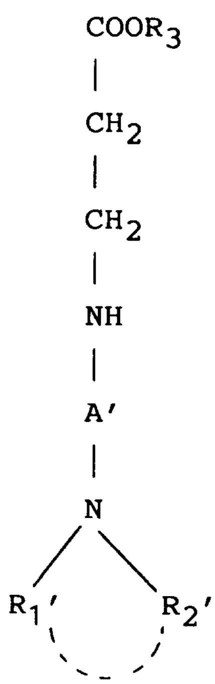

(IV)

in which A',

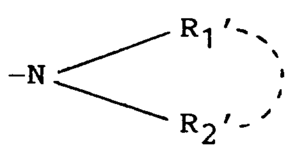

and $R_3$ have the meanings indicated above, which is reacted with the compound of formula (V):

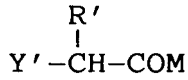

(V)

in which R' represents a hydrogen atom, the values of R indicated above or those in which the reactive functions are protected, M represents a hydroxyl radical or a halogen atom and Y' represents the values of Y indicated above, or those in which the reactive functions are protected, in order to obtain the product of formula (VI):

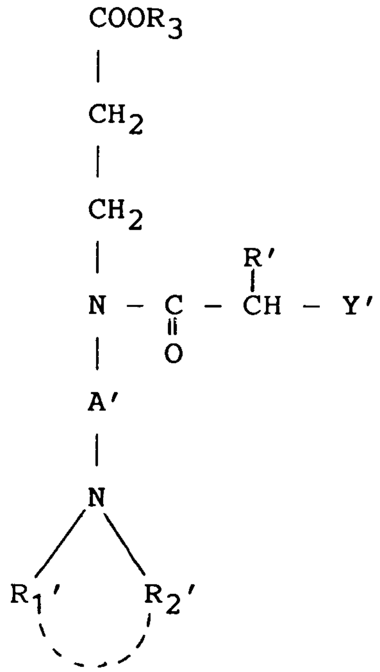

(VI)

in which A',

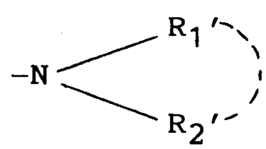

,

R', $R_3$ and Y' have the meanings indicated above, corresponding in the case where R' represents a hydrogen atom or the values of R and A', $NR'_1R'_2$ and Y' have the values of A, $NR_1R_2$ and Y respectively, to a product of formula ($I_B$) as defined above, in which product of formula (VI) if appropriate, the protective group or groups are eliminated, in order to obtain a same product of formula ($I_B$) and, if desired, the products of formula (I) are treated with a mineral or organic acid or, if appropriate, with a base, in order to obtain the corresponding salt, said products of formula ($I_B$) being in all possible racemic, enantiomeric and diastereoisomeric isomer forms.

**5.** Process according to claim 4 for the preparation of products of formula ($I_B$) in which R represents a hydrogen atom, characterized in that a compound of formula (V) is used in which R' represents a hydrogen atom.

**6.** Process according to any one of claims 1 to 5, characterized in that compounds are used at the start in which the substituent or substituents, identical or different, which can be carried by the aryl, arylalkyl and heterocyclic radicals or those which can be formed by $R_1$ and $R_2$ with the nitrogen atom to which they are linked, are chosen from the group formed by:

- halogen atoms,

- alkyl, alkenyl, alkynyl or alkyloxy radicals, these radicals being linear or branched containing at most 7 carbon atoms and being optionally substituted,
- hydroxy, trifluoromethyl, trifluoromethylthio, trifluoromethoxy, cyano, nitro, sulphamoyl, amino, monoalkyl-and dialkylamino radicals,
- formyl radicals, acyl radicals containing of 2 to 6 carbon atoms and benzoyl radicals,
- free carboxy radicals, carboxy radicals esterified by a linear or branched alkyl radical containing at most 5 carbon atoms and salified carboxy radicals,
- the carbamoyl radical optionally substituted by one or more identical or different radicals, chosen from the group formed by alkyl, alkoxy or hydroxyalkyl radicals and the carbamoylalkyl radical, these alkyl and alkoxy radicals containing 1 to 4 carbon atoms,
- and the phenyl or benzyl radical itself optionally substituted by one or more identical or different radicals, chosen from the group formed by halogen atoms and the following radicals: hydroxy, cyano, nitro, linear or branched alkyl, alkenyl, alkynyl and alkoxy containing at most 5 carbon atoms.

**7.** Process according to any one of claims 1 to 5, characterized in that compounds are used at the start in which the substituent or substituents, identical or different, which can be carried by the alkyl, alkenyl, alkynyl, cycloalkyl, carbocyclic and alkoxy radicals are chosen from the group formed by:
- halogen atoms,
- alkyl, alkenyl, alkynyl or alkoxy radicals containing at most 5 carbon atoms,
- formyl radicals, acyl radicals containing 2 to 6 carbon atoms and benzoyl radicals,
- free carboxy radicals, carboxy radicals esterified by a linear or branched alkyl radical containing at most 5 carbon atoms and salified carboxy radicals,
- hydroxy, cyano, amino, monoalkyl- and dialkylamino radicals,
- aryl and arylalkyl radicals containing 6 to 14 carbon atoms, these radicals being optionally substituted by one or more identical or different radicals, chosen from the group formed by halogen atoms, hydroxy, cyano, nitro radicals, linear or branched alkyl, alkenyl, alkynyl and alkoxy radicals containing at most 5 carbon atoms.

**8.** Process according to any one of claims 1, 2, 4 or 6, characterized in that a compound of formula (V) is used at the start in which R' represents an alkyl, alkenyl or alkynyl radical, substituted by a phenyl or a substituted phenyl, the optional reactive functions being, if appropriate, protected.

**9.** Process according to any one of claims 1 to 8, characterized in that a compound of formula (V) is used at the start in which Y' represents a phenyl, naphthyl, thionaphthyl, benzofuryl, benzopyrrolyl or indenyl radical optionally substituted by one or more radicals chosen from the group formed by:
- halogen atoms,
- linear or branched alkyl, alkenyl, alkynyl or alkoxy radicals containing at most 7 carbon atoms and being optionally substituted by one or more radicals chosen from the group formed by the hydroxy radical and linear or branched alkyl and alkoxy radicals containing at most 5 carbon atoms,
- acyl radicals containing 2 to 6 carbon atoms,
- hydroxy, trifluoromethyl, cyano, nitro, amino radicals, monoalkyl- and dialkylamino radicals containing 1 to 5 carbon atoms,
- and the phenyl radical itself optionally substituted by one or more radicals, identical or different, chosen from the group formed by halogen atoms, hydroxy, cyano, nitro radicals, linear or branched alkyl, alkenyl, alkynyl and alkoxy radicals containing at most 5 carbon atoms the optional reactive functions being, if appropriate, protected.

**10.** Process according to any one of claims 1 to 9, characterized in that a compound of formula (II) is used at the start in which A' represents:
- either a cycloalkyl radical of formula

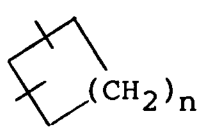

where n is an integer which can take the values 0 to 4, optionally substituted by a linear or branched alkyl or alkoxy radical containing at most 5 carbon atoms,

- or the

$$-\underset{B}{\underset{|}{C}}H-CH_2-$$

group in which B represents a phenyl radical optionally substituted by one or more identical or different radicals, chosen from the group formed by halogen atoms, hydroxy, cyano, nitro radicals, linear or branched alkyl, alkenyl, alkynyl and alkoxy radicals containing at most 5 carbon atoms, the optional reactive functions being, if appropriate, protected.

**11.** Process according to any one of claims 1 to 10, characterized in that a compound of formula (II) is used at the start in which $R'_1$ and $R'_2$ form together with the nitrogen atom to which they are linked a heterocycle containing a second nitrogen atom, this being optionally substituted by a linear or branched alkyl or alkoxy radical containing 1 to 5 carbon atoms.

**12.** Process according to claim 1, characterized in that the starting products are chosen in such a way that any one of the products of formula (I) are prepared the names of which follow:
(1S)-3-(3,4-dichlorophenyl)-1-[1-phenyl-2-(1-pyrrolidinyl) ethyl]-2-piperidinone (isomer B)
(1S)-3-(3,4-dichlorophenyl)-1-(1-phenyl-2-(1-pyrrolidinyl) ethyl)-2-piperidinone (isomer A)
[trans,(±)]-3-(3,4-dichlorophenyl)-1-(2-methyl-(1-pyrrolidinyl) cyclohexyl)-2-piperidinone (isomer A)
(1S)-3-(3,4-dichlorophenyl)-1-(2-1-((1-pyrrolidinyl) methyl) propyl)-2-piperidinone (isomer A)
(1S)-3-(benzo(b)thien-4-yl)-1-(1-phenyl-2-(1-pyrrolidinyl) ethyl)-2-piperidinone (isomer A)
(1S)-3-(3,4-dichlorophenyl)-3-methyl-1-(1-phenyl-2-(1-pyrrolidinyl) ethyl)-2-piperidinone (isomer A)
(1S)-3-(3,4-dichlorophenyl)-3-ethyl-1-(1-phenyl-2-(1-pyrrolidinyl) ethyl)-2-piperidinone (isomer A)
(1S)-3-(3,4-dichlorophenyl)-1-(1-phenyl-2-(1-pyrrolidinyl) ethyl)-3-(2-propenyl)-2-piperidinone (isomer A)
and if appropriate their salts.

**13.** Preparation process for pharmaceutical compositions, characterized in that at least one of the products of formula (I) as defined in claim 1, or at least one of their pharmaceutically acceptable salts, is used as active ingredient in a form intended for this use.

**14.** Preparation process for pharmaceutical compositions, characterized in that at least one of the products of formula ($I_A$) as defined in claim 3, or at least one of their pharmaceutically acceptable salts, is used as active ingredient in a form intended for this use.

**15.** Preparation process for pharmaceutical compositions, characterized in that at least one of the products of formula (I) as defined in claim 12, or at least one of their pharmaceutically acceptable salts, is used as active ingredient in a form intended for this use.

**16.** As industrial products, the compounds of formulae (IV), (VI), (VII), (VIII), (IX) and (X).

**Patentansprüche**

**Patentansprüche für folgende Vertragsstaaten : AT, BE, CH, DE, DK, FR, GB, IT, LI, LU, NL, SE**

1. Verbindungen der Formel (I)

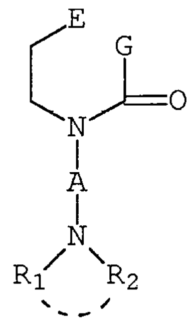

(I)

in der E und G bedeuten:

- entweder bilden E und G zusammen eine Gruppe

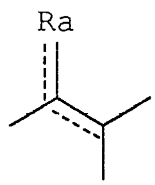

die eine der folgenden Gruppen darstellt:

a) 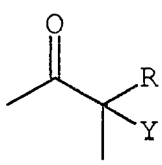 oder gegebenenfalls ihr Tautomer 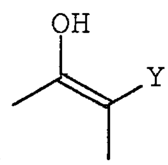

worin R darstellt: ein Wasserstoffatom, einen linearen oder verzweigten Alkylrest mit 1 bis 6 Kohlenstoffatomen, einen linearen oder verzweigten Alkenyl- oder Alkinylrest mit 2 bis 6 Kohlenstoffatomen, gegebenenfalls substituiert durch einen oder mehrere Substituenten, entweder ausgewählt unter den Arylresten mit 6 bis 14 Kohlenstoffatomen, den Arylresten, substituiert durch ein Halogenatom, einen Hydroxyrest, einen Trifluormethylrest, einen Alkoxyrest mit 1 bis 6 Kohlenstoffatomen, einen der Reste Nitro, Amino und Cyano, oder den freien, veresterten oder in Salz überführten Carboxyresten, den Acylresten mit 1 bis 7 Kohlenstoffatomen, den Alkoxyresten mit 1 bis 6 Kohlenstoffatomen, den Resten Hydroxy, Amino, Alkylamino, Dialkylamino, Acyloxy mit 1 bis 7 Kohlenstoffatomen, Cyano, Carbamoyl und den Halogenatomen,

-- Y darstellt: einen Arylrest, ausgewählt unter den carbocyclischen Arylresten mit 6 bis 14 Kohlenstoffatomen, den heteromonocyclischen Arylresten mit 5 bis 7 Kettengliedern und den kondensierten heterocyclischen Arylresten, wobei alle diese Reste gegebenenfalls substituiert sind,

b) 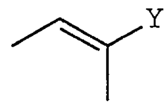 oder

c) 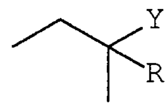

worin Y und R wie vorstehend definiert sind;

- <u>oder</u> E stellt einen Rest $-CO_2R_3$ dar, worin $R_3$ ein Wasserstoffatom oder einen linearen oder verzweigten Alkylrest mit 1 bis 4 Kohlenstoffatomen bedeutet, und G stellt eine Gruppe

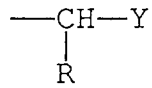

dar, worin Y und R wie vorstehend definiert sind;

-- A bedeutet:

- entweder einen gegebenenfalls substituierten carbocyclischen Rest, oder
- die Gruppe

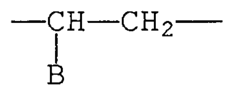

worin B darstellt:

- ein Wasserstoffatom, einen Aryl- oder Arylalkylrest mit 6 bis 14 Kohlenstoffatomen, wobei diese Reste gegebenenfalls substituiert sind,
- einen gegebenenfalls substituierten Cycloalkylrest mit 3 bis 7 Kohlenstoffatomen,
- einen linearen oder verzweigten Alkyl-, Alkenyl-, Alkinyl- oder Alkyloxyrest mit höchstens 7 Kohlenstoffatomen, gegebenenfalls substituiert,

-- $R_1$ und $R_2$, gleich oder verschieden, ein Wasserstoffatom oder einen linearen oder verzweigten Alkyl-, Alkenyl- oder Alkinylrest mit höchstens 7 Kohlenstoffatomen bedeuten, gegebenenfalls substituiert,

oder $R_1$ und $R_2$ zusammen mit dem Stickstoffatom, an das sie gebunden sind, einen gesättigten oder ungesättigten Heterocyclus mit 5, 6 oder 7 Ringgliedern bilden, der gegebenenfalls noch ein anderes Heteroatom umfaßt, wie Sauerstoff, Stickstoff oder Schwefel, und gegebenenfalls substituiert ist,

die genannten Produkte der Formel (I) in allen möglichen isomeren, racemischen, enantiomeren und diastereoisomeren Formen sowie die Additionssalze der Produkte der Formel (I) mit Mineralsäuren und organischen Säuren oder mit Basen.

**2.** Verbindungen nach Anspruch 1 der Formel ($I_A$)

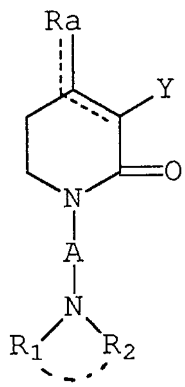

$(I_A)$

in der die Gruppe

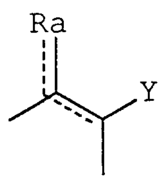

sowie A, $R_1$ und $R_2$ wie in Anspruch 1 definiert sind.

**3.** Verbindungen nach Anspruch 2, in der die Gruppe

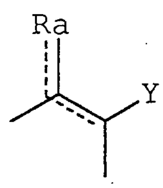

darstellt:

a) 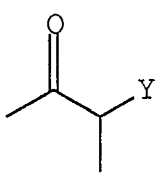 oder gegebenenfalls ihr Tautomer 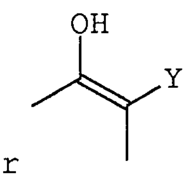

b) 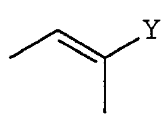 oder

c) 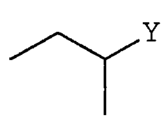

worin Y wie in Anspruch 1 definiert ist.

**4.** Verbindungen nach Anspruch 1 der Formel ($I_B$)

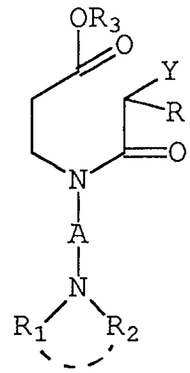

($I_B$)

worin $R_1$, $R_2$, $R_3$, A, R und Y wie in Anspruch 1 definiert sind.

**5.** Verbindungen der Formel (I) wie in irgendeinem der Ansprüche 1 bis 4 definiert, dadurch gekennzeichnet, daß der oder die gleichen oder verschiedenen Substituenten, die von den Arylresten, Arylalkylresten und heterocyclischen Resten oder von den von $R_1$ und $R_2$ zusammen mit dem Stickstoffatom, an das sie gebunden sind, gebildeten Resten getragen werden können, aus der Gruppe gewählt werden, die gebildet wird von:

- den Halogenatomen,
- den linearen oder verzweigten Alkyl-, Alkenyl-, Alkinyl- oder Alkyloxyresten mit höchstens 7 Kohlenstoffatomen, gegebenenfalls substituiert,
- den Resten Hydroxy, Trifluormethyl, Trifluormethylthio, Trifluormethoxy, Cyano, Nitro, Sulfamoyl, Amino, Monoalkyl- und Dialkylanino,
- den Resten Formyl, Acyl mit 2 bis 6 Kohlenstoffatomen und Benzoyl,
- den freien, durch einen linearen oder verzweigten Alkylrest mit höchstens 5 Kohlenstoffatomen veresterten und in Salz überführten Carboxyresten,
- dem Carbamoylrest, gegebenenfalls substituiert durch einen oder mehrere gleiche oder verschieden Reste, gewählt aus der Gruppe, die gebildet wird von den Resten Alkyl, Alkoxy oder Hydroxyalkyl und dem Carbamoylalkylrest, wobei diese Alkyl- und Alkoxyreste 1 bis 4 Kohlenstoffatome umfassen,
- und dem Phenyl- oder Benzylrest, gegebenenfalls selbst substituiert durch einen oder mehrere gleiche oder verschiedene Reste, gewählt aus der Gruppe, die gebildet wird von den Halogenatomen, den Resten Hydroxy, Cyano, Nitro, den linearen oder verzweigten Resten Alkyl, Alkenyl, Alkinyl oder Alkoxy mit höchstens 5 Kohlenstoffatomen,

die genannten Produkte der Formel (I) in allen möglichen isomeren, racemischen, enantiomeren und diastereoisomeren Formen sowie die Additionssalze der Produkte der Formel (I) mit Mineralsäuren und organischen Säuren oder mit Basen.

**6.** Produkte der Formel (I) wie in irgendeinem der Ansprüche 1 bis 5 definiert, dadurch gekennzeichnet, daß der oder die gleichen oder verschiedenen Substituenten, die von den Alkylresten, Alkenylresten, Alkinylresten, Cycloalkylresten, Alkoxyresten und carbocyclischen Resten getragen werden können, aus der Gruppe gewählt werden, die gebildet wird von:

- den Halogenatomen,
- den Alkyl-, Alkenyl-, Alkinyl- oder Alkoxyresten mit höchstens 5 Kohlenstoffatomen,
- den Resten Formyl, Acyl mit 2 bis 6 Kohlenstoffatomen und Benzoyl,
- den freien, durch einen linearen oder verzweigten Alkylrest mit höchstens 5 Kohlenstoffatomen veresterten und in Salz überführten Carboxyresten,
- den Resten Hydroxy, Cyano, Amino, Monoalkyl- und Dialkylamino,
- den Arylresten und Arylalkylresten mit 6 bis 14 Kohlenstoffatomen, wobei diese Reste gegebenenfalls durch einen oder mehrere gleiche oder verschiedene Reste substituiert sind, gewählt aus der Gruppe, die gebildet wird von den Halogenatomen, den Resten Hydroxy, Cyano, Nitro, den linearen oder verzweigten Resten Alkyl, Alkenyl, Alkinyl und Alkoxy mit höchstens 5 Kohlenstoffatomen,

die genannten Produkte der Formel (I) in allen möglichen isomeren, racemischen, enantiomeren und

diastereoisomeren Formen sowie die Additionssalze der Produkte der Formel (I) mit Mineralsäuren und organischen Säuren oder mit Basen.

**7.** Verbindungen der Formel (I) wie in irgendeinem der Ansprüche 1 bis 6 definiert, dadurch gekennzeichnet, daß R, wenn es einen Alkyl-, Alkenyl- oder Alkinylrest darstellt, substituiert durch ein Aryl oder substituiertes Aryl, ein solcher Rest ist, der durch ein Phenyl oder substituiertes Phenyl substituiert ist.

**8.** Verbindungen der Formel (I) wie in irgendeinem der Ansprüche 1 bis 7 definiert, dadurch gekennzeichnet, daß Y einen der Reste Phenyl, Naphthyl, Thionaphthyl, Benzofuryl, Benzopyrrolyl oder Indenyl darstellt, gegebenenfalls substituiert durch einen oder mehrere Reste, gewählt aus der Gruppe, die gebildet wird von:
- den Halogenatomen,
- den linearen oder verzweigten Alkyl-, Alkenyl-, Alkinyl- oder Alkoxyresten mit höchstens 7 Kohlenstoffatomen und gegebenenfalls substituiert durch einen oder mehrere Reste, gewählt aus der Gruppe, die gebildet wird von dem Hydroxyrest und den linearen oder verzweigten Alkyl- und Alkoxyresten mit höchstens 5 Kohlenstoffatomen,
- den Acylresten mit 2 bis 6 Kohlenstoffatomen,
- den Resten Hydroxy, Trifluormethyl, Cyano, Nitro, Amino, Monoalkyl- und Dialkylamino mit 1 bis 5 Kohlenstoffatomen,
- und dem Phenylrest, gegebenenfalls selbst substituiert durch einen oder mehrere gleiche oder verschiedene Reste, gewählt aus der Gruppe, die gebildet wird von den Halogenatomen, den Resten Hydroxy, Cyano, Nitro, den linearen oder verzweigten Resten Alkyl, Alkenyl, Alkinyl oder Alkoxy mit höchstens 5 Kohlenstoffatomen,

die genannten Produkte der Formel (I) in allen möglichen isomeren, racemischen, enantiomeren und diastereoisomeren Formen sowie die Additionssalze der Produkte der Formel (I) mit Mineralsäuren und organischen Säuren oder mit Basen.

**9.** Produkte der Formel (I) wie in irgendeinem der Ansprüche 1 bis 8 definiert, dadurch gekennzeichnet, daß A darstellt:
- entweder einen Cycloalkylrest der Formel

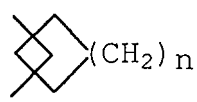

worin n die ganzzahligen Werte von 0 bis 4 annehmen kann, gegebenenfalls substituiert durch einen linearen oder verzweigten Alkyl- oder Alkoxyrest mit höchstens 5 Kohlenstoffatomen,
- oder die Gruppe

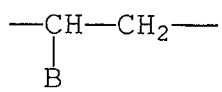

worin B einen Phenylrest darstellt, gegebenenfalls substituiert durch einen oder mehrere gleiche oder verschiedene Reste, gewählt aus der Gruppe, die gebildet wird von:
- den Halogenatomen,
- den Resten Hydroxy, Cyano, Nitro, den linearen oder verzweigten Resten Alkyl, Alkenyl, Alkinyl und Alkoxy mit höchstens 5 Kohlenstoffatomen,

die genannten Produkte der Formel (I) in allen möglichen isomeren, racemischen, enantiomeren und diastereoisomeren Formen sowie die Additionssalze der Produkte der Formel (I) mit Mineralsäuren und organischen Säuren oder mit Basen.

**10.** Produkte der Formel (I) wie in irgendeinem der Ansprüche 1 bis 9 definiert, dadurch gekennzeichnet, daß der Heterocyclus, der von $R_1$ und $R_2$ zusammen mit dem Stickstoffatom, an das sie gebunden sind, gebildet werden kann, ein zweites Stickstoffatom umfaßt, und daß dieses gegebenenfalls durch einen linearen oder verzweigten Alkyl- oder Alkoxyrest mit 1 bis 5 Kohlenstoffatomen substituiert ist,
die genannten Produkte der Formel (I) in allen möglichen isomeren, racemischen, enantiomeren und diastereoisomeren Formen sowie die Additionssalze der Produkte der Formel (I) mit Mineralsäuren und

organischen Säuren oder mit Basen.

**11.** (1S)-3-(3,4-Dichlorphenyl)-1-[1-phenyl-2-(1-pyrrolidinyl)-ethyl]-2-piperidinon (Isomer B), (1S)-3-(3,4-Dichlorphenyl)-1-[1-phenyl-2-(1-pyrrolidinyl)-ethyl]-2-piperidinon (Isomer A), [trans,(±)]-3-(3,4-Dichlorphenyl)-1-[2-(1-pyrrolidinyl)-cyclohexyl]-2-piperidinon (Isomer A), (1S)-3-(3,4-Dichlorphenyl)-1-{2-methyl-1-[(1-pyrrolidinyl)-methyl]-propyl}-2-piperidinon (Isomer A), (1S)-3-(Benzo(b)thien-4-yl)-1-[1-phenyl-2-(1-pyrrolidinyl)-ethyl]-2-piperidinon (Isomer A), (1S)-3-(3,4-Dichlorphenyl)-3-methyl-1-[1-phenyl-2-(1-pyrrolidinyl)-ethyl]-2-piperidinon (Isomer A), (1S)-3-(3,4-Dichlorphenyl)-3-ethyl-1-[1-phenyl-2-(1-pyrrolidinyl)-ethyl]-2-piperidinon (Isomer A), (1S)-3-(3,4-Dichlorphenyl)-1-[1-phenyl-2-(1-pyrrolidinyl)-ethyl]-3-(2-propenyl)-2-piperidinon (Isomer A), und gegebenenfalls ihre Salze.

**12.** Verfahren zur Herstellung der Produkte der Formel (I) wie in Anspruch 1 definiert, dadurch gekennzeichnet, daß man das Diamin der Formel (II)

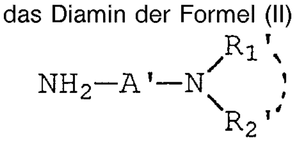

(II)

worin A' und

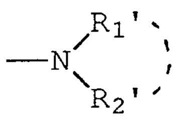

die jeweils in Anspruch 1 für A und

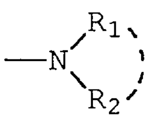

angegebenen Bedeutungen besitzen oder diejenigen, in denen die reaktiven Funktionen, die A und

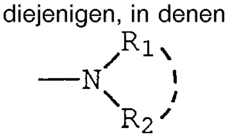

tragen können, geschützt sind, mit dem Acrylester der Formel (III)

$CH_2 = CH - COOR_3$ (III)

zur Reaktion bringt, worin $R_3$ einen linearen oder verzweigten Alkylrest mit höchstens 6 Kohlenstoffatomen darstellt, um das kondensierte Produkt der Formel (IV)

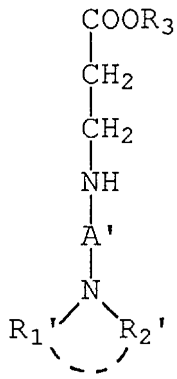

(IV)

zu erhalten, in der A',

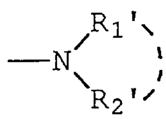

und $R_3$ die vorstehend angegebenen Bedeutungen besitzen, das man mit der Verbindung der Formel (V)

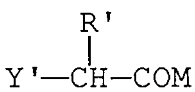

(V)

zur Reaktion bringt, in der R' ein Wasserstoffatom oder die oben angegebenen Werte von R oder diejenigen darstellt, in denen die reaktiven Funktionen geschützt sind, M einen Hydroxylrest oder ein Halogenatom bedeutet und Y' die oben angegebenen Werte von Y oder diejenigen darstellt, in denen die reaktiven Funktionen geschützt sind, um das Produkt der Formel (VI)

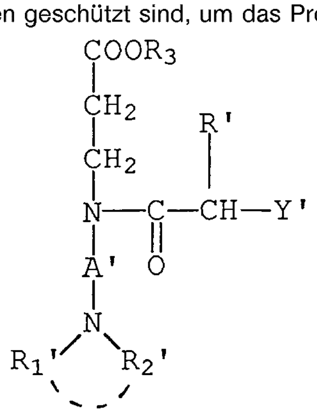

(VI)

zu erhalten, in der A',

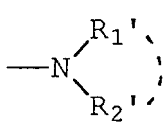

R', $R_3$ und Y' die vorstehend angegebenen Bedeutungen besitzen, die in dem Fall, wo R' ein Wasserstoffatom darstellt oder die Werte von R und A', $NR_1'R_2'$ und Y' jeweils die Werte von A, $NR_1R_2$ und Y haben, einem wie vorstehend definierten Produkt der Formel ($I_B$) entsprechen, wobei man gegebenenfalls bei dem Produkt der Formel (VI) die Schutzgruppe(n) entfernt, um ein gleiches Produkt der Formel ($I_B$) zu erhalten, oder dieses gegebenenfalls einer Cyclisierungsreaktion unterzieht, um das

Produkt der Formel (VII)

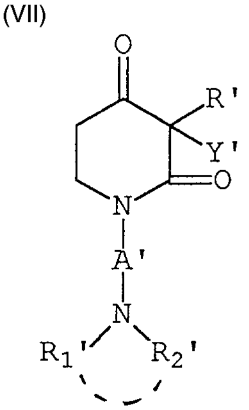

(VII)

zu erhalten, in der A',

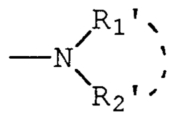

R' und Y' die vorstehend angegebenen Bedeutungen besitzen, die in dem Fall, wo R' ein Wasserstoffatom darstellt oder die Werte von R und A', $NR_1'R_2'$ und Y' jeweils die Werte von A, $NR_1R_2$ und Y haben, einem wie vorstehend definierten Produkt der Formel ($I_A$) entsprechen, ($I_{A1}$) genannt, wobei man gegebenenfalls bei dem Produkt der Formel (VII) die Schutzgruppe(n) entfernt, um ein gleiches Produkt der Formel ($I_{A1}$) zu erhalten, und man anschließend gegebenenfalls

- entweder, wenn R' oder R ein Wasserstoffatom darstellen, das genannte Produkt der Formel (VII) oder ($I_{A1}$);

  -- entweder einer Reduktions-Reaktion der Oxo-Funktion in gamma-Position zum Stickstoffatom unterzieht, um das Produkt der Formel (VIII)

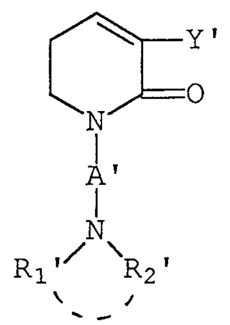

(VIII)

zu erhalten, in der A',

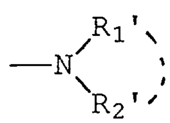

und Y' die vorstehend angegebenen Bedeutungen besitzen, die in dem Fall, wo Y', A' und $NR_1'R_2'$ jeweils die Werte von Y, A und $NR_1R_2$ haben, einem wie vorstehend definierten Produkt der Formel ($I_A$) entsprechen, ($I_{A2}$) genannt, wobei man gegebenenfalls bei dem Produkt der Formel (VIII) die Schutzgruppen entfernt, um ein gleiches Produkt der Formel ($I_{A2}$) zu erhalten,

  -- oder einer Reduktions-Reaktion durch katalytische Hydrierung der gleichen Oxo-Funktion, um das Produkt der Formel (IX)

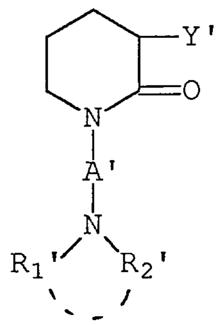

(IX)

zu erhalten, in der A',

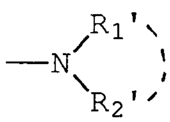

und Y' die vorstehend angegebenen Bedeutungen besitzen, die in dem Fall, wo Y', A' und $NR_1'R_2'$ jeweils die Werte von Y, A und $NR_1R_2$ haben, einem wie vorstehend definierten Produkt der Formel ($I_A$) entsprechen, ($I_{A3}$) genannt, wobei man gegebenenfalls bei dem Produkt der Formel (IX) die Schutzgruppen entfernt, um ein gleiches Produkt der Formel ($I_{A3}$) zu erhalten, und man das Produkt der Formel (IX) oder ($I_{A3}$) gegebenenfalls mit einer starken Base behandelt, um das entsprechende Anion zu bilden, das man der Einwirkung eines Reaktanden unterzieht, der fähig ist, einen Rest R' einzubringen, wobei R' die vorstehend angegebenen Werte mit Ausnahme eines Wasserstoffatomes besitzt, um ein Produkt der Formel (X)

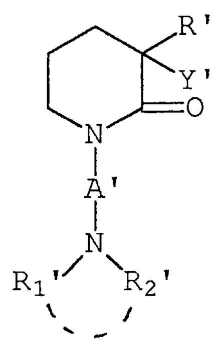

(X)

zu erhalten, in der R', Y', A' und

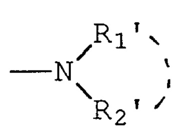

die vorstehend angegebenen Bedeutungen besitzen, die in dem Fall, wo R', Y', A' und $NR_1'R_2'$ jeweils die Werte von R (mit Ausnahme von Wasserstoff), Y, A und $NR_1R_2$ haben, einem wie vorstehend definierten Produkt der Formel ($I_A$) entsprechen, ($I_{A4}$) genannt, wobei man gegebenenfalls bei dem Produkt der Formel (X) die Schutzgruppen entfernt, um ein gleiches Produkt der Formel ($I_{A4}$) zu erhalten;

- oder, wenn R' oder R von einem Wasserstoffatom verschieden sind, das genannte Produkt der Formel (VII) oder ($I_{A1}$) der Einwirkung eines Reduktionsmittels für die Oxo-Funktion unterzieht, um das wie vorstehend definierte Produkt der Formel (X) zu erhalten, das entweder einem Produkt der Formel ($I_{A4}$) entspricht oder in dieses Produkt der Formel ($I_{A4}$) umgewandelt wird, und wenn gewünscht, die Produkte der Formel (I) mit einer Mineralsäure oder organischen Säure oder gegebenenfalls mit einer Base behandelt, um das entsprechende Salz zu erhalten, wobei die

genannten Produkte der Formel (I) in allen möglichen isomeren, racemischen, enantiomeren und diastereoisomeren Formen vorliegen können.

**13.** Als Medikamente die Produkte der Formel (I) wie in Anspruch 1 definiert, die genannten Produkte der Formel (I) in allen möglichen isomeren, racemischen, enantiomeren und diastereoisomeren Formen, sowie die Additionssalze der genannten Produkte der Formel (I) mit pharmazeutisch akzeptablen Mineralsäuren und organischen Säuren oder mit Basen.

**14.** Als Medikamente die Produkte der Formel ($I_A$) wie in Anspruch 2 definiert, sowie die Additionssalze der genannten Produkte der Formel ($I_A$) mit pharmazeutisch akzeptablen Mineralsäuren und organischen Säuren oder mit Basen.

**15.** Als Medikamente die Produkte der Formel ($I_B$) wie in Anspruch 3 definiert, sowie die Additionssalze der genannten Produkte der Formel ($I_B$) mit pharmazeutisch akzeptablen Mineralsäuren und organischen Säuren oder mit Basen.

**16.** Als Medikamente die in Anspruch 11 definierten Produkte.

**17.** Pharmazeutische Zusammensetzungen, die als Wirkstoff mindestens eines der in irgendeinem der Ansprüche 13 bis 16 definierten Medikamente enthalten.

**18.** Als industrielle Produkte die Verbindungen der Formeln (IV), (VI), (VII), (VIII), (IX) und (X).

**Patentansprüche für folgenden Vertragsstaat : ES**

**1.** Verfahren zur Herstellung von Verbindungen der Formel (I)

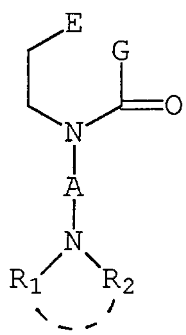

(I)

in der E und G bedeuten:

- <u>entweder</u> bilden E und G zusammen eine Gruppe

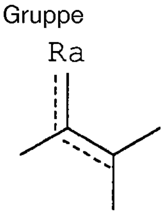

die eine der folgenden Gruppen darstellt:

a) 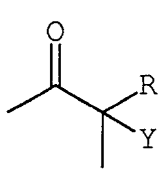 oder gegebenenfalls ihr Tautomer 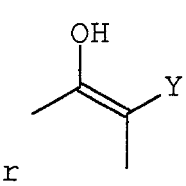

worin R darstellt: ein Wasserstoffatom, einen linearen oder verzweigten Alkylrest mit 1 bis 6 Kohlenstoffatomen, einen linearen oder verzweigten Alkenyl- oder Alkinylrest mit 2 bis 6 Kohlenstoffatomen, gegebenenfalls substituiert durch einen oder mehrere Substituenten, entweder ausgewählt unter den Arylresten mit 6 bis 14 Kohlenstoffatomen, den Arylresten, substituiert durch ein Halogenatom, einen Hydroxyrest, einen Trifluormethylrest, einen Alkoxyrest mit 1 bis 6 Kohlenstoffatomen, einen der Reste Nitro, Amino und Cyano, oder den freien, veresterten oder in Salz überführten Carboxyresten, den Acylresten mit 1 bis 7 Kohlenstoffatomen, den Alkoxyresten mit 1 bis 6 Kohlenstoffatomen, den Resten Hydroxy, Amino, Alkylamino, Dialkylamino, Acyloxy mit 1 bis 7 Kohlenstoffatomen, Cyano, Carbamoyl und den Halogenatomen,

-- Y darstellt: einen Arylrest, ausgewählt unter den carbocyclischen Arylresten mit 6 bis 14 Kohlenstoffatomen, den heteromonocyclischen Arylresten mit 5 bis 7 Kettengliedern und den kondensierten heterocyclischen Arylresten, wobei alle diese Reste gegebenenfalls substituiert sind,

b) 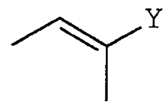 oder

c) 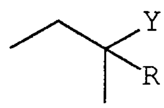

worin Y und R wie vorstehend definiert sind;

- oder E stellt einen Rest $-CO_2R_3$ dar, worin $R_3$ ein Wasserstoffatom oder einen linearen oder verzweigten Alkylrest mit 1 bis 4 Kohlenstoffatomen bedeutet, und G stellt eine Gruppe

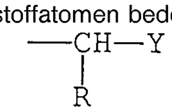

dar, worin Y und R wie vorstehend definiert sind;

-- A bedeutet:
- entweder einen gegebenenfalls substituierten carbocyclischen Rest, oder
- die Gruppe

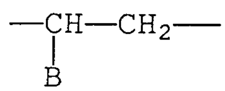

worin B darstellt:
- ein Wasserstoffatom,
- einen Aryl- oder Arylalkylrest mit 6 bis 14 Kohlenstoffatomen, wobei diese Reste gegebenenfalls substituiert sind,
- einen gegebenenfalls substituierten Cycloalkylrest mit 3 bis 7 Kohlenstoffatomen,
- einen linearen oder verzweigten Alkyl-, Alkenyl-, Alkinyl- oder Alkyloxyrest mit höchstens 7 Kohlenstoffatomen, gegebenenfalls substituiert,

-- $R_1$ und $R_2$, gleich oder verschieden, ein Wasserstoffatom oder einen linearen oder verzweigten Alkyl-, Alkenyl- oder Alkinylrest mit höchstens 7 Kohlenstoffatomen bedeuten, gegebenenfalls substituiert,

oder $R_1$ und $R_2$ zusammen mit dem Stickstoffatom, an das sie gebunden sind, einen gesättigten oder ungesättigten Heterocyclus mit 5, 6 oder 7 Ringgliedern bilden, der gegebenenfalls noch ein anderes Heteroatom umfassen kann, wie Sauerstoff, Stickstoff oder Schwefel, und gegebenenfalls substituiert ist,

die genannten Produkte der Formel (I) in allen möglichen isomeren, racemischen, enantiomeren und diastereoisomeren Formen sowie die Additionssalze der Produkte der Formel (I) mit Mineralsäuren und organischen Säuren oder mit Basen, dadurch gekennzeichnet, daß man das Diamin der Formel (II)

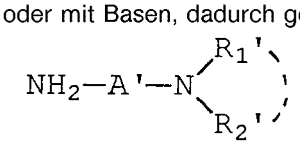 (II)

worin A' und

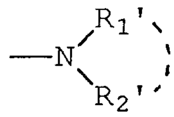

die jeweils weiter oben für A und

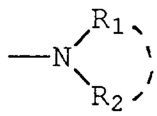

angegebenen Bedeutungen besitzen oder diejenigen, in denen die reaktiven Funktionen, die A und

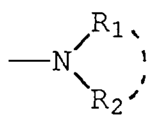

tragen können, geschützt sind,
mit dem Acrylester der Formel (III)

$CH_2 = CH - COOR_3$ (III)

zur Reaktion bringt, worin $R_3$ einen linearen oder verzweigten Alkylrest mit höchstens 6 Kohlenstoffatomen darstellt, um das kondensierte Produkt der Formel (IV)

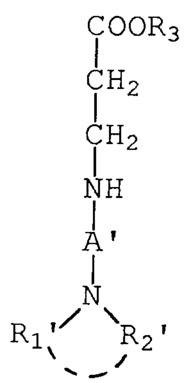

(IV)

zu erhalten, in der A',

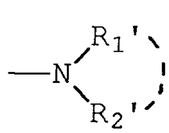

und $R_3$ die vorstehend angegebenen Bedeutungen besitzen, das man mit der Verbindung der Formel (V)

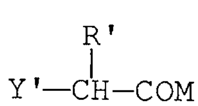

(V)

zur Reaktion bringt, in der R' ein Wasserstoffatom oder die oben angegebenen Werte von R oder diejenigen darstellt, in denen die reaktiven Funktionen geschützt sind, M einen Hydroxylrest oder ein Halogenatom bedeutet und Y' die oben angegebenen Werte von Y oder diejenigen darstellt, in denen

die reaktiven Funktionen geschützt sind, um das Produkt der Formel (VI)

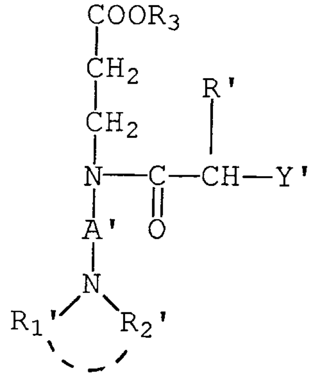

(VI)

zu erhalten, in der A',

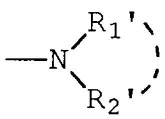

R', $R_3$ und Y' die vorstehend angegebenen Bedeutungen besitzen, die in den Fall, wo R' ein Wasserstoffatom darstellt oder die Werte von R und A', $NR_1'R_2'$ und Y' jeweils die Werte von A, $NR_1R_2$ und Y haben, einem wie vorstehend definierten Produkt der Formel ($I_B$) entsprechen, wobei man gegebenenfalls bei dem Produkt der Formel (VI) die Schutzgruppe(n) entfernt, um ein gleiches Produkt der Formel ($I_B$) zu erhalten, oder dieses gegebenenfalls einer Cyclisierungsreaktion unterzieht, um das Produkt der Formel (VII)

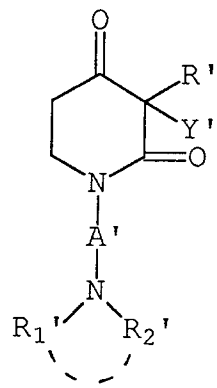

(VII)

zu erhalten, in der A',

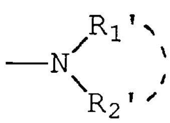

R' und Y' die vorstehend angegebenen Bedeutungen besitzen, die in dem Fall, wo R' ein Wasserstoffatom darstellt oder die Werte von R und A', $NR_1'R_2'$ und Y' jeweils die Werte von A, $NR_1R_2$ und Y haben, einem wie vorstehend definierten Produkt der Formel ($I_A$) entsprechen, ($I_{A1}$) genannt, wobei man gegebenenfalls bei dem Produkt der Formel (VII) die Schutzgruppe(n) entfernt, um ein gleiches Produkt der Formel ($I_{A1}$) zu erhalten, und man anschließend gegebenenfalls

- entweder, wenn R' oder R ein Wasserstoffatom darstellen, das genannte Produkt der Formel (VII) oder ($I_{A1}$);

  -- entweder einer Reduktions-Reaktion der Oxo-Funktion in gamma-Position zum Stickstoffatom unterzieht, um das Produkt der Formel (VIII)

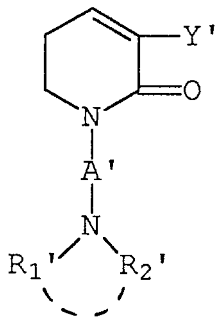

(VIII)

zu erhalten, in der A',

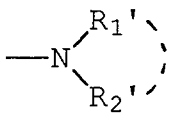

und Y' die vorstehend angegebenen Bedeutungen besitzen, die in dem Fall, wo Y', A' und $NR_1'R_2'$ jeweils die Werte von Y, A und $NR_1R_2$ haben, einem wie vorstehend definierten Produkt der Formel ($I_A$) entsprechen, ($I_{A2}$) genannt, wobei man gegebenenfalls bei dem Produkt der Formel (VIII) die Schutzgruppen entfernt, um ein gleiches Produkt der Formel ($I_{A2}$) zu erhalten,
-- oder einer Reduktions-Reaktion durch katalytische Hydrierung der gleichen Oxo-Funktion, um das Produkt der Formel (IX)

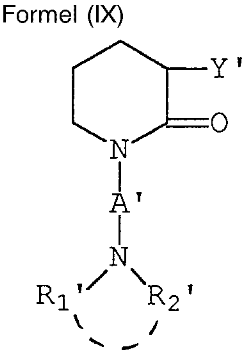

(IX)

zu erhalten, in der A',

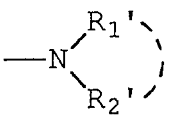

und Y' die vorstehend angegebenen Bedeutungen besitzen, die in dem Fall, wo Y', A' und $NR_1'R_2'$ jeweils die Werte von Y, A und $NR_1R_2$ haben, einem wie vorstehend definierten Produkt der Formel ($I_A$) entsprechen, ($I_{A3}$) genannt, wobei man gegebenenfalls bei dem Produkt der Formel (IX) die Schutzgruppen entfernt, um ein gleiches Produkt der Formel ($I_{A3}$) zu erhalten, und man das Produkt der Formel (IX) oder ($I_{A3}$) gegebenenfalls mit einer starken Base behandelt, um das entsprechende Anion zu bilden, das man der Einwirkung eines Reaktanden unterzieht, der fähig ist, einen Rest R' einzubringen, wobei R' die vorstehend angegebenen Werte mit Ausnahme eines Wasserstoffatomes besitzt, um ein Produkt der Formel (X)

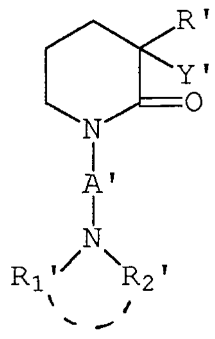

(X)

zu erhalten, in der R', Y', A' und

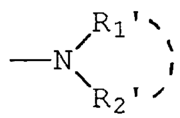

die vorstehend angegebenen Bedeutungen besitzen, die in dem Fall, wo R', Y', A' und $NR_1'R_2'$ jeweils die Werte von R (mit Ausnahme von Wasserstoff), Y, A und $NR_1R_2$ haben, einem wie vorstehend definierten Produkt der Formel ($I_A$) entsprechen, ($I_{A4}$) genannt, wobei man gegebenenfalls bei dem Produkt der Formel (X) die Schutzgruppen entfernt, um ein gleiches Produkt der Formel ($I_{A4}$) zu erhalten;

- oder, wenn R' oder R von einem Wasserstoffatom verschieden sind, das genannte Produkt der Formel (VII) oder ($I_{A1}$) der Einwirkung eines Reduktionsmittels für die Oxo-Funktion unterzieht, um das wie vorstehend definierte Produkt der Formel (X) zu erhalten, das entweder einem Produkt der Formel ($I_{A4}$) entspricht oder in dieses Produkt der Formel ($I_{A4}$) umgewandelt wird, und wenn gewünscht, die Produkte der Formel (I) mit einer Mineralsäure oder organischen Säure oder gegebenenfalls mit einer Base behandelt, um das entsprechende Salz zu erhalten, wobei die genannten Produkte der Formel (I) in allen möglichen isomeren, racemischen, enantiomeren und diastereoisomeren Formen vorliegen können.

**2.** Verfahren nach Anspruch 1 zur Herstellung der Produkte der Formel ($I_A$)

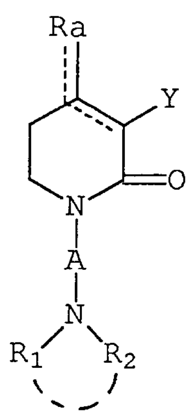

($I_A$)

in der die Gruppe

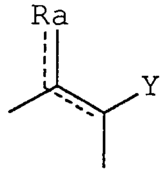

sowie A, $R_1$ und $R_2$ wie in Anspruch 1 definiert sind, dadurch gekennzeichnet, daß man das Diamin der Formel (II)

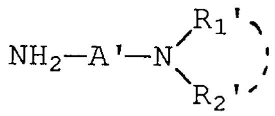

(II)

worin A' und

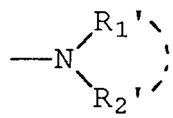

die jeweils weiter oben für A und

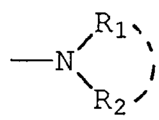

angegebenen Bedeutungen besitzen oder diejenigen, in denen die reaktiven Funktionen, die A und

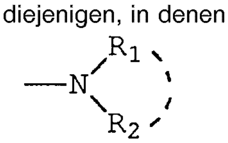

tragen können, geschützt sind,
mit dem Acrylester der Formel (III)

$CH_2$ = CH - $COOR_3$ (III)

zur Reaktion bringt, worin $R_3$ einen linearen oder verzweigten Alkylrest mit höchstens 6 Kohlenstoffatomen darstellt, um das kondensierte Produkt der Formel (IV)

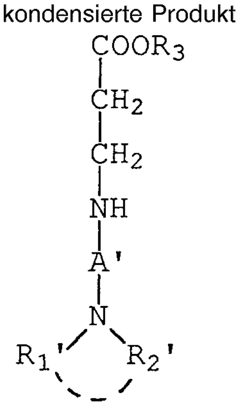

(IV)

zu erhalten, in der A',

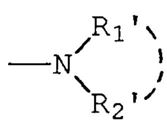

und $R_3$ die vorstehend angegebenen Bedeutungen besitzen, das man mit der Verbindung der Formel (V)

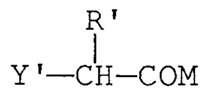

(V)

zur Reaktion bringt, in der R' ein Wasserstoffatom oder die oben angegebenen Werte von R oder diejenigen darstellt, in denen die reaktiven Funktionen geschützt sind, M einen Hydroxylrest oder ein Halogenatom bedeutet und Y' die oben angegebenen Werte von Y oder diejenigen darstellt, in denen die reaktiven Funktionen geschützt sind, um das Produkt der Formel (VI)

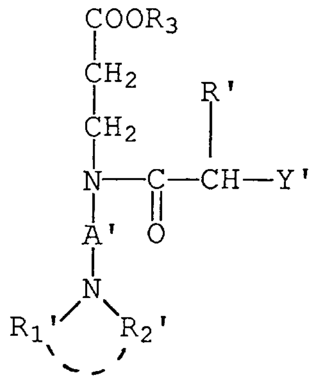

(VI)

zu erhalten, in der A',

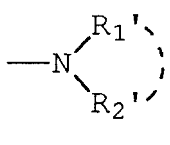

R', $R_3$ und Y' die vorstehend angegebenen Bedeutungen besitzen, das man einer Cyclisierungsreaktion unterzieht, um das Produkt der Formel (VII)

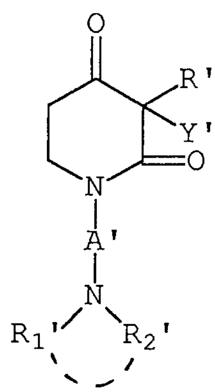

(VII)

zu erhalten, in der A',

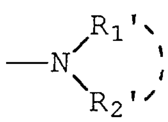

R' und Y' die vorstehend angegebenen Bedeutungen besitzen, die in dem Fall, wo R' ein Wasserstoffatom darstellt oder die Werte von R und A', $NR_1'R_2'$ und Y' jeweils die Werte von A, $NR_1R_2$ und Y haben, einem wie vorstehend definierten Produkt der Formel ($I_A$) entsprechen, ($I_{A1}$) genannt, wobei man gegebenenfalls bei dem Produkt der Formel (VII) die Schutzgruppe(n) entfernt, um ein gleiches Produkt der Formel ($I_{A1}$) zu erhalten, und man anschließend gegebenenfalls

- entweder, wenn R' oder R ein Wasserstoffatom darstellen, das genannte Produkt der Formel (VII) oder ($I_{A1}$);
  -- entweder einer Reduktions-Reaktion der Oxo-Funktion in gamma-Position zum Stickstoffatom unterzieht, um das Produkt der Formel (VIII)

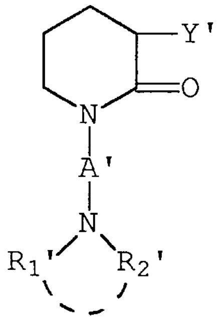

(VIII)

zu erhalten, in der A',

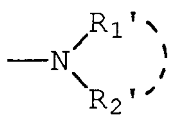

und Y' die vorstehend angegebenen Bedeutungen besitzen, die in dem Fall, wo Y', A' und $NR_1'R_2'$ jeweils die Werte von Y, A und $NR_1R_2$ haben, einem wie vorstehend definierten Produkt der Formel ($I_A$) entsprechen, ($I_{A2}$) genannt, wobei man gegebenenfalls bei dem Produkt der Formel (VIII) die Schutzgruppen entfernt, um ein gleiches Produkt der Formel ($I_{A2}$) zu erhalten,
-- oder einer Reduktions-Reaktion durch katalytische Hydrierung der gleichen Oxo-Funktion, um das Produkt der Formel (IX)

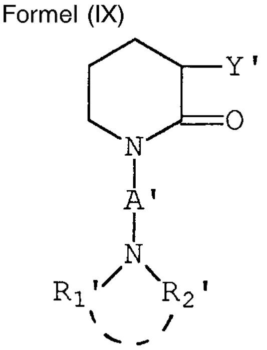

(IX)

zu erhalten, in der A',

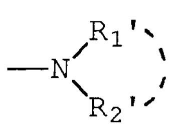

und Y' die vorstehend angegebenen Bedeutungen besitzen, die in dem Fall, wo Y', A' und $NR_1'R_2'$ jeweils die Werte von Y, A und $NR_1R_2$ haben, einem wie vorstehend definierten Produkt der Formel ($I_A$) entsprechen, ($I_{A3}$) genannt, wobei man gegebenenfalls bei dem Produkt der Formel (IX) die Schutzgruppen entfernt, um ein gleiches Produkt der Formel ($I_{A3}$) zu erhalten, und man das Produkt der Formel (IX) oder ($I_{A3}$) gegebenenfalls mit einer starken Base behandelt, um das entsprechende Anion zu bilden, das man der Einwirkung eines Reaktanden unterzieht, der fähig ist, einen Rest R' einzubringen, wobei R' die vorstehend angegebenen Werte mit Ausnahme eines Wasserstoffatomes besitzt, um ein Produkt der Formel (X)

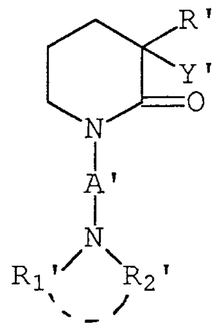

(X)

zu erhalten, in der R', Y', A' und

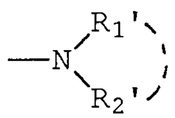

die vorstehend angegebenen Bedeutungen besitzen, die in dem Fall, wo R', Y', A' und $NR_1'R_2'$ jeweils die Werte von R (mit Ausnahme von Wasserstoff), Y, A und $NR_1R_2$ haben, einem wie vorstehend definierten Produkt der Formel ($I_A$) entsprechen, ($I_{A4}$) genannt, wobei man gegebenenfalls bei dem Produkt der Formel (X) die Schutzgruppen entfernt, um ein gleiches Produkt der Formel ($I_{A4}$) zu erhalten;

- oder, wenn R' oder R von einem Wasserstoffatom verschieden sind, das genannte Produkt der Formel (VII) oder ($I_{A1}$) der Einwirkung eines Reduktionsnittels für die Oxo-Funktion unterzieht, um das wie vorstehend definierte Produkt der Formel (X) zu erhalten, das entweder einem Produkt der Formel ($I_{A4}$) entspricht oder in dieses Produkt der Formel ($I_{A4}$) umgewandelt wird, und wenn gewünscht, die Produkte der Formel (I) mit einer Mineralsäure oder organischen Säure oder gegebenenfalls mit einer Base behandelt, um das entsprechende Salz zu erhalten, wobei die genannten Produkte der Formel (I) in allen möglichen isomeren, racemischen, enantiomeren und diastereoisomeren Formen vorliegen können.

**3.** Verfahren nach Anspruch 2 zur Herstellung der Produkte der Formel ($I_A$), in der die Gruppe

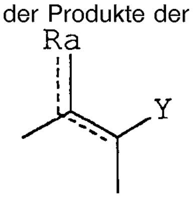

darstellt:

a) 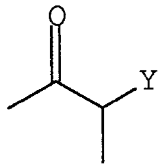 oder gegebenenfalls ihr Tautomer 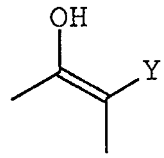

b) 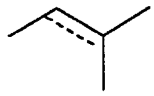 oder

c) 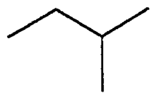

worin Y wie in Anspruch 1 definiert ist, dadurch gekennzeichnet, daß man eine Verbindung der Formel (V) verwendet, in der R' ein Wasserstoffatom darstellt.

**4.** Verfahren nach Anspruch 1 zur Herstellung der Produkte der Formel ($I_B$)

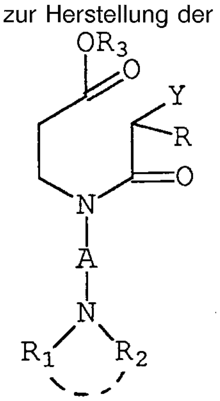 ($I_B$)

worin $R_1$, $R_2$, $R_3$, A, R und Y wie in Anspruch 1 definiert sind, dadurch gekennzeichnet, daß man das Diamin der Formel (II)

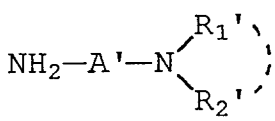 (II)

worin A' und

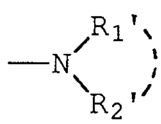

die jeweils weiter oben für A und

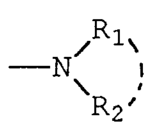

angegebenen Bedeutungen besitzen oder diejenigen, in denen die reaktiven Funktionen, die A und

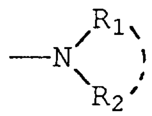

tragen können, geschützt sind,
mit dem Acrylester der Formel (III)

$CH_2 = CH - COOR_3$ (III)

zur Reaktion bringt, worin $R_3$ einen linearen oder verzweigten Alkylrest mit höchstens 6 Kohlenstoffatomen darstellt, um das kondensierte Produkt der Formel (IV)

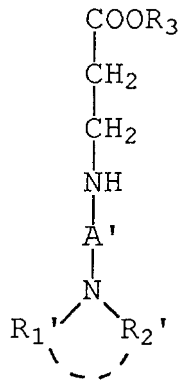

(IV)

zu erhalten, in der A',

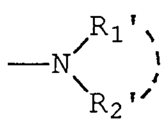

und $R_3$ die vorstehend angegebenen Bedeutungen besitzen, das man mit der Verbindung der Formel (V)

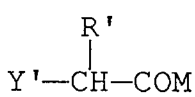

(V)

zur Reaktion bringt, in der R' ein Wasserstoffatom oder die oben angegebenen Werte von R oder diejenigen darstellt, in denen die reaktiven Funktionen geschützt sind, M einen Hydroxylrest oder ein Halogenatom bedeutet und Y' die oben angegebenen Werte von Y oder diejenigen darstellt, in denen die reaktiven Funktionen geschützt sind, um das Produkt der Formel (VI)

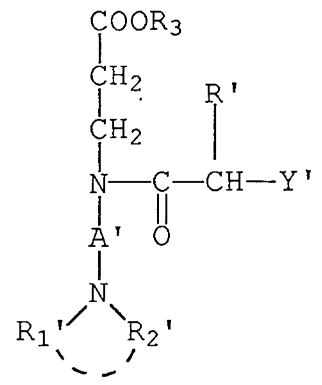

(VI)

zu erhalten, in der A',

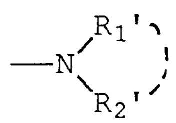

R', $R_3$ und Y' die vorstehend angegebenen Bedeutungen besitzen, die in dem Fall, wo R' ein Wasserstoffatom darstellt oder die Werte von R und A', $NR_1'R_2'$ und Y' jeweils die Werte von A, $NR_1R_2$ und Y haben, einem wie vorstehend definierten Produkt der Formel ($I_B$) entsprechen, wobei man gegebenenfalls bei dem Produkt der Formel (VI) die Schutzgruppe(n) entfernt, um ein gleiches Produkt der Formel ($I_B$) zu erhalten, und wenn gewünscht, die Produkte der Formel (I) mit einer Mineralsäure oder organischen Säure oder gegebenenfalls mit einer Base behandelt, um das entsprechende Salz zu erhalten, wobei die genannten Produkte der Formel ($I_B$) in allen möglichen isomeren, racemischen, enantiomeren und diastereoisomeren Formen vorliegen können.

**5.** Verfahren nach Anspruch 4 zur Herstellung der Produkte der Formel ($I_B$), in der R ein Wasserstoffatom darstellt, dadurch gekennzeichnet, daß man eine Verbindung der Formel (V) verwendet, in der R' ein Wasserstoffatom bedeutet.

**6.** Verfahren nach irgendeinem der Ansprüche 1 bis 5, dadurch gekennzeichnet, daß man von Verbindungen ausgeht, in denen der oder die gleichen oder verschiedenen Substituenten, die von den Arylresten, Arylalkylresten und heterocyclischen Resten oder von den von $R_1$ und $R_2$ zusammen mit dem Stickstoffatom, an das sie gebunden sind, gebildeten Resten getragen werden können, aus der Gruppe gewählt werden, die gebildet wird von:
- den Halogenatomen,
- den linearen oder verzweigten Alkyl-, Alkenyl-, Alkinyl- oder Alkyloxyresten mit höchstens 7 Kohlenstoffatomen, gegebenenfalls substituiert,
- den Resten Hydroxy, Trifluormethyl, Trifluormethylthio, Trifluormethoxy, Cyano, Nitro, Sulfamoyl, Amino, Monoalkyl- und Dialkylamino,
- den Resten Formyl, Acyl mit 2 bis 6 Kohlenstoffatomen und Benzoyl,
- den freien, durch einen linearen oder verzweigten Alkylrest mit höchstens 5 Kohlenstoffatomen veresterten und in Salz überführten Carboxyresten,
- dem Carbamoylrest, gegebenenfalls substituiert durch einen oder mehrere gleiche oder verschiedene Reste, gewählt aus der Gruppe, die gebildet wird von den Resten Alkyl, Alkoxy oder Hydroxyalkyl und dem Carbamoylalkylrest, wobei diese Alkyl- und Alkoxyreste 1 bis 4 Kohlenstoffatome umfassen,
- und dem Phenyl- oder Benzylrest, gegebenenfalls selbst substituiert durch einen oder mehrere gleiche oder verschiedene Reste, gewählt aus der Gruppe, die gebildet wird von den Halogenatomen, den Resten Hydroxy, Cyano, Nitro, den linearen oder verzweigten Resten Alkyl, Alkenyl, Alkinyl oder Alkoxy mit höchstens 5 Kohlenstoffatomen.

**7.** Verfahren nach irgendeinem der Ansprüche 1 bis 5, dadurch gekennzeichnet, daß man von Verbindungen ausgeht, in denen der oder die gleichen oder verschiedenen Substituenten, die von den Alkylresten, Alkenylresten, Alkinylresten, Cycloalkylresten, Alkoxyresten und carbocyclischen Resten getragen werden können, aus der Gruppe gewählt werden, die gebildet wird von:

- den Halogenatomen,
- den Alkyl-, Alkenyl-, Alkinyl- oder Alkoxyresten mit höchstens 5 Kohlenstoffatomen,
- den Resten Formyl, Acyl mit 2 bis 6 Kohlenstoffatomen und Benzoyl,
- den freien, durch einen linearen oder verzweigten Alkylrest mit höchstens 5 Kohlenstoffatomen veresterten und in Salz überführten Carboxyresten,
- den Resten Hydroxy, Cyano, Amino, Monoalkyl- und Dialkylamino,
- den Arylresten und Arylalkylresten mit 6 bis 14 Kohlenstoffatomen, wobei diese Reste gegebenenfalls durch einen oder mehrere gleiche oder verschiedene Reste substituiert sind, gewählt aus der Gruppe, die gebildet wird von den Halogenatomen, den Resten Hydroxy, Cyano, Nitro, den linearen oder verzweigten Resten Alkyl, Alkenyl, Alkinyl und Alkoxy mit höchstens 5 Kohlenstoffatomen.

**8.** Verfahren nach irgendeinem der Ansprüche 1, 2, 4 oder 6, dadurch gekennzeichnet, daß man von einer Verbindung der Formel (V) ausgeht, in der R' einen Alkyl-, Alkenyl- oder Alkinylrest darstellt, substituiert durch ein Phenyl oder ein substituiertes Phenyl, wobei die eventuellen reaktiven Funktionen gegebenenfalls geschützt sind.

**9.** Verfahren nach irgendeinem der Ansprüche 1 bis 8, dadurch gekennzeichnet, daß man von einer Verbindung der Formel (V) ausgeht, in der Y' einen der Reste Phenyl, Naphthyl, Thionaphthyl, Benzofuryl, Benzopyrrolyl oder Indenyl darstellt, gegebenenfalls substituiert durch einen oder mehrere Reste, gewählt aus der Gruppe, die gebildet wird von:

- den Halogenatomen,
- den linearen oder verzweigten Alkyl-, Alkenyl-, Alkinyl- oder Alkoxyresten mit höchstens 7 Kohlenstoffatomen und gegebenenfalls substituiert durch einen oder mehrere Reste, gewählt aus der Gruppe, die gebildet wird von dem Hydroxyrest und den linearen oder verzweigten Alkyl- und Alkoxyresten mit höchstens 5 Kohlenstoffatomen,
- den Acylresten mit 2 bis 6 Kohlenstoffatomen,
- den Resten Hydroxy, Trifluormethyl, Cyano, Nitro, Amino, Monoalkyl- und Dialkylamino mit 1 bis 5 Kohlenstoffatomen,
- und dem Phenylrest, gegebenenfalls selbst substituiert durch einen oder mehrere gleiche oder verschiedene Reste, gewählt aus der Gruppe, die gebildet wird von den Halogenatomen, den Resten Hydroxy, Cyano, Nitro, den linearen oder verzweigten Resten Alkyl, Alkenyl, Alkinyl und Alkoxy mit höchstens 5 Kohlenstoffatomen, wobei die eventuellen reaktiven Funktionen gegebenenfalls geschützt sind.

**10.** Verfahren nach irgendeinem der Ansprüche 1 bis 9, dadurch gekennzeichnet, daß man von einer Verbindung der Formel (II) ausgeht, in der A darstellt:

- entweder einen Cycloalkylrest der Formel

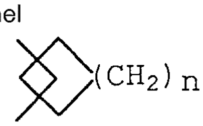

  worin n die ganzzahligen Werte von 0 bis 4 annehmen kann, gegebenenfalls substituiert durch einen linearen oder verzweigten Alkyl- oder Alkoxyrest mit höchstens 5 Kohlenstoffatomen,
- oder die Gruppe

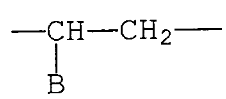

  worin B einen Phenylrest darstellt, gegebenenfalls substituiert durch einen oder mehrere gleiche oder verschiedene Reste, gewählt aus der Gruppe, die gebildet wird von:
- den Halogenatomen,
- den Resten Hydroxy, Cyano, Nitro, den linearen oder verzweigten Resten Alkyl, Alkenyl, Alkinyl und Alkoxy mit höchstens 5 Kohlenstoffatomen, wobei die eventuellen reaktiven Funktionen

gegebenenfalls geschützt sind.

**11.** Verfahren nach irgendeinem der Ansprüche 1 bis 10, dadurch gekennzeichnet, daß man von einer Verbindung der Formel (II) ausgeht, in der $R_1$' und $R_2$' zusammen mit dem Stickstoffatom, an das sie gebunden sind, einen Heterocyclus bilden, der ein zweites Stickstoffatom umfaßt, das selbst gegebenenfalls durch einen linearen oder verzweigten Alkyl- oder Alkoxyrest mit 1 bis 5 Kohlenstoffatomen substituiert ist.

**12.** Verfahren nach Anspruch 1, dadurch gekennzeichnet, daß die Ausgangsprodukte in der Weise gewählt werden, daß man irgendeines der Produkte der Formel (I) herstellt, deren Bezeichnungen folgen:
(1S)-3-(3,4-Dichlorphenyl)-1-[1-phenyl-2-(1-pyrrolidinyl)-ethyl]-2-piperidinon (Isomer B),
(1S)-3-(3,4-Dichlorphenyl)-1-[1-phenyl-2-(1-pyrrolidinyl)-ethyl]-2-piperidinon (Isomer A),
[trans,(±)]-3-(3,4-Dichlorphenyl)-1-[2-(1-pyrrolidinyl)-cyclohexyl]-2-piperidinon (Isomer A),
(1S)-3-(3,4-Dichlorphenyl)-1-{2-methyl-1-[(1-pyrrolidinyl)-methyl]-propyl}-2-piperidinon (Isomer A),
(1S)-3-(Benzo(b)thien-4-yl)-1-[1-phenyl-2-(1-pyrrolidinyl)-ethyl]-2-piperidinon (Isomer A),
(1S)-3-(3,4-Dichlorphenyl)-3-methyl-1-[1-phenyl-2-(1-pyrrolidinyl)-ethyl]-2-piperidinon (Isomer A),
(1S)-3-(3,4-Dichlorphenyl)-3-ethyl-1-[1-phenyl-2-(1-pyrrolidinyl)-ethyl]-2-piperidinon (Isomer A),
(1S)-3-(3,4-Dichlorphenyl)-1-[1-phenyl-2-(1-pyrrolidinyl)-ethyl]-3-(2-propenyl)-2-piperidinon (Isomer A),
und gegebenenfalls ihre Salze.

**Patentansprüche für folgenden Vertragsstaat : GR**

**1.** Verfahren zur Herstellung von Verbindungen der Formel (I)

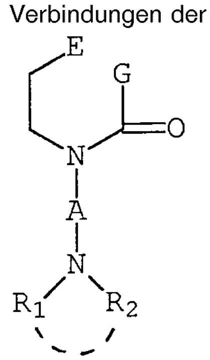

(I)

in der E und G bedeuten:

- entweder bilden E und G zusammen eine Gruppe

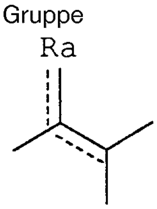

die eine der folgenden Gruppen darstellt:

a) 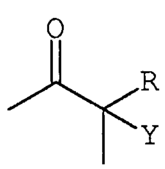 oder gegebenenfalls ihr Tautomer 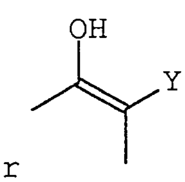

worin R darstellt: ein Wasserstoffatom, einen linearen oder verzweigten Alkylrest mit 1 bis 6 Kohlenstoffatomen, einen linearen oder verzweigten Alkenyl- oder Alkinylrest mit 2 bis 6 Kohlenstoffatomen, gegebenenfalls substituiert durch einen oder mehrere Substituenten, entweder ausgewählt unter den Arylresten mit 6 bis 14 Kohlenstoffatomen, den Arylresten, substituiert durch ein Halogenatom, einen Hydroxyrest, einen Trifluormethylrest, einen Alkoxyrest mit 1 bis 6

Kohlenstoffatomen, einen der Reste Nitro, Amino und Cyano, oder den freien, veresterten oder in Salz überführten Carboxyresten, den Acylresten mit 1 bis 7 Kohlenstoffatomen, den Alkoxyresten mit 1 bis 6 Kohlenstoffatomen, den Resten Hydroxy, Amino, Alkylamino, Dialkylamino, Acyloxy mit 1 bis 7 Kohlenstoffatomen, Cyano, Carbamoyl und den Halogenatomen,

-- Y darstellt: einen Arylrest, ausgewählt unter den carbocyclischen Arylresten mit 6 bis 14 Kohlenstoffatomen, den heteromonocyclischen Arylresten mit 5 bis 7 Kettengliedern und den kondensierten heterocyclischen Arylresten, wobei alle diese Reste gegebenenfalls substituiert sind,

b) 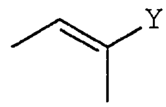 oder

c) 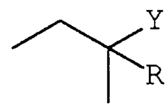

worin Y und R wie vorstehend definiert sind;

- <u>oder</u> E stellt einen Rest $-CO_2R_3$ dar, worin $R_3$ ein Wasserstoffatom oder einen linearen oder verzweigten Alkylrest mit 1 bis 4 Kohlenstoffatomen bedeutet, und G stellt eine Gruppe

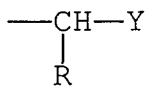

dar, worin Y und R wie vorstehend definiert sind;

-- A bedeutet:

- entweder einen gegebenenfalls substituierten carbocyclischen Rest, oder
- die Gruppe

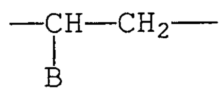

worin B darstellt:

- ein Wasserstoffatom,
- einen Aryl- oder Arylalkylrest mit 6 bis 14 Kohlenstoffatomen, wobei diese Reste gegebenenfalls substituiert sind,
- einen gegebenenfalls substituierten Cycloalkylrest mit 3 bis 7 Kohlenstoffatomen,
- einen linearen oder verzweigten Alkyl-, Alkenyl-, Alkinyl- oder Alkyloxyrest mit höchstens 7 Kohlenstoffatomen, gegebenenfalls substituiert,

-- $R_1$ und $R_2$, gleich oder verschieden, ein Wasserstoffatom oder einen linearen oder verzweigten Alkyl-, Alkenyl- oder Alkinylrest mit höchstens 7 Kohlenstoffatomen bedeuten, gegebenenfalls substituiert,

oder $R_1$ und $R_2$ zusammen mit dem Stickstoffatom, an das sie gebunden sind, einen gesättigten oder ungesättigten Heterocyclus mit 5, 6 oder 7 Ringgliedern bilden, der gegebenenfalls noch ein anderes Heteroatom umfaßt, wie Sauerstoff, Stickstoff oder Schwefel, und gegebenenfalls substituiert ist,

die genannten Produkte der Formel (I) in allen möglichen isomeren, racemischen, enantiomeren und diastereoisomeren Formen sowie die Additionssalze der Produkte der Formel (I) mit Mineralsäuren und organischen Säuren oder mit Basen, dadurch gekennzeichnet, daß man das Diamin der Formel (II)

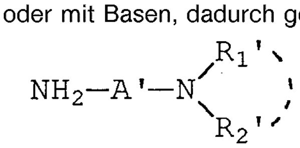 (II)

worin A' und

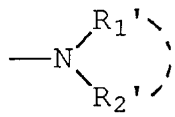

die jeweils weiter oben für A und

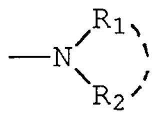

angegebenen Bedeutungen besitzen oder diejenigen, in denen die reaktiven Funktionen, die A und

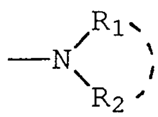

tragen können, geschützt sind,
mit dem Acrylester der Formel (III)

$CH_2 = CH - COOR_3$ (III)

zur Reaktion bringt, worin $R_3$ einen linearen oder verzweigten Alkylrest mit höchstens 6 Kohlenstoffatomen darstellt, um das kondensierte Produkt der Formel (IV)

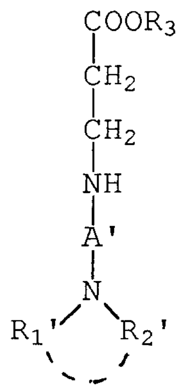

(IV)

zu erhalten, in der A',

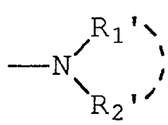

und $R_3$ die vorstehend angegebenen Bedeutungen besitzen, das man mit der Verbindung der Formel (V)

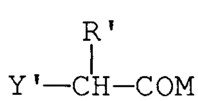

(V)

zur Reaktion bringt, in der R' ein Wasserstoffatom, die oben angegebenen Werte von R oder diejenigen darstellt, in denen die reaktiven Funktionen geschützt sind, M einen Hydroxylrest oder ein Halogenatom bedeutet und Y' die oben angegebenen Werte von Y oder diejenigen darstellt, in denen die reaktiven

Funktionen geschützt sind, um das Produkt der Formel (VI)

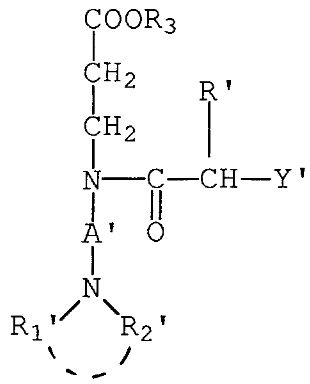

(VI)

zu erhalten, in der A',

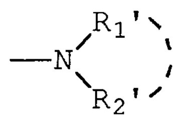

R', $R_3$ und Y' die vorstehend angegebenen Bedeutungen besitzen, die in dem Fall, wo R' ein Wasserstoffatom darstellt oder die Werte von R und A', $NR_1'R_2'$ und Y' jeweils die Werte von A, $NR_1R_2$ und Y haben, einem wie vorstehend definierten Produkt der Formel ($I_B$) entsprechen, wobei man gegebenenfalls bei dem Produkt der Formel (VI) die Schutzgruppe(n) entfernt, um ein gleiches Produkt der Formel ($I_B$) zu erhalten, oder dieses gegebenenfalls einer Cyclisierungsreaktion unterzieht, um das Produkt der Formel (VII)

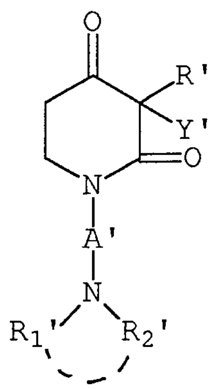

(VII)

zu erhalten, in der A',

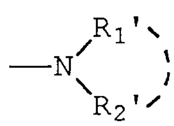

R' und Y' die vorstehend angegebenen Bedeutungen besitzen, die in dem Fall, wo R' ein Wasserstoffatom darstellt oder die Werte von R und A', $NR_1'R_2'$ und Y' jeweils die Werte von A, $NR_1R_2$ und Y haben, einem wie vorstehend definierten Produkt der Formel ($I_A$) entsprechen, ($I_{A1}$) genannt, wobei man gegebenenfalls bei dem Produkt der Formel (VII) die Schutzgruppe(n) entfernt, um ein gleiches Produkt der Formel ($I_{A1}$) zu erhalten, und man anschließend gegebenenfalls

- entweder, wenn R' oder R ein Wasserstoffatom darstellen, das genannte Produkt der Formel (VII) oder ($I_{A1}$);
  -- entweder einer Reduktions-Reaktion der Oxo-Funktion in gamma-Position zum Stickstoffatom unterzieht, um das Produkt der Formel (VIII)

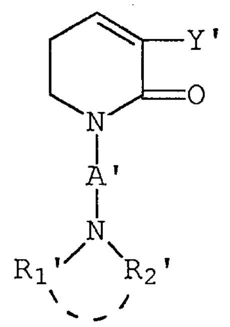

(VIII)

zu erhalten, in der A',

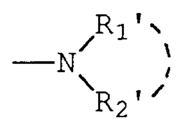

und Y' die vorstehend angegebenen Bedeutungen besitzen, die in dem Fall, wo Y', A' und $NR_1'R_2'$ jeweils die Werte von Y, A und $NR_1R_2$ haben, einem wie vorstehend definierten Produkt der Formel ($I_A$) entsprechen, ($I_{A2}$) genannt, wobei man gegebenenfalls bei dem Produkt der Formel (VIII) die Schutzgruppen entfernt, um ein gleiches Produkt der Formel ($I_{A2}$) zu erhalten,
-- oder einer Reduktions-Reaktion durch katalytische Hydrierung der gleichen Oxo-Funktion, um das Produkt der Formel (IX)

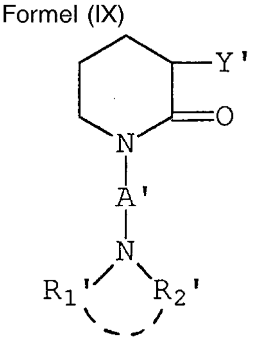

(IX)

zu erhalten, in der A',

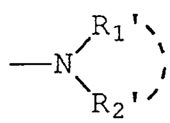

und Y' die vorstehend angegebenen Bedeutungen besitzen, die in dem Fall, wo Y', A' und $NR_1'R_2'$ jeweils die Werte von Y, A und $NR_1R_2$ haben, einem wie vorstehend definierten Produkt der Formel ($I_A$) entsprechen, ($I_{A3}$) genannt, wobei man gegebenenfalls bei dem Produkt der Formel (IX) die Schutzgruppen entfernt, um ein gleiches Produkt der Formel ($I_{A3}$) zu erhalten, und man das Produkt der Formel (IX) oder ($I_{A3}$) gegebenenfalls mit einer starken Base behandelt, um das entsprechende Anion zu bilden, das man der Einwirkung eines Reaktanden unterzieht, der fähig ist, einen Rest R' einzubringen, wobei R' die vorstehend angegebenen Werte mit Ausnahme eines Wasserstoffatomes besitzt, um ein Produkt der Formel (X)

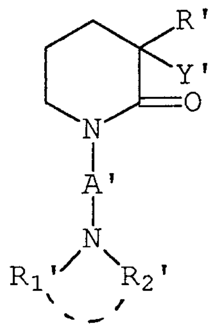

(X)

zu erhalten, in der R', Y', A' und

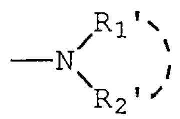

die vorstehend angegebenen Bedeutungen besitzen, die in dem Fall, wo R', Y', A' und $NR_1'R_2'$ jeweils die Werte von R (mit Ausnahme von Wasserstoff), Y, A und $NR_1R_2$ haben, einem wie vorstehend definierten Produkt der Formel ($I_A$) entsprechen, ($I_{A4}$) genannt, wobei man gegebenenfalls bei dem Produkt der Formel (X) die Schutzgruppen entfernt, um ein gleiches Produkt der Formel ($I_{A4}$) zu erhalten;

- oder, wenn R' oder R von einem Wasserstoffatom verschieden sind, das genannte Produkt der Formel (VII) oder ($I_{A1}$) der Einwirkung eines Reduktionsmittels für die Oxo-Funktion unterzieht, um das wie vorstehend definierte Produkt der Formel (X) zu erhalten, das entweder einem Produkt der Formel ($I_{A4}$) entspricht oder in dieses Produkt der Formel ($I_{A4}$) umgewandelt wird, und wenn gewünscht, die Produkte der Formel (I) mit einer Mineralsäure oder organischen Säure oder gegebenenfalls mit einer Base behandelt, um das entsprechende Salz zu erhalten, wobei die genannten Produkte der Formel (I) in allen möglichen isomeren, racemischen, enantiomeren und diastereoisomeren Formen vorliegen können.

**2.** Verfahren nach Anspruch 1 zur Herstellung der Produkte der Formel ($I_A$)

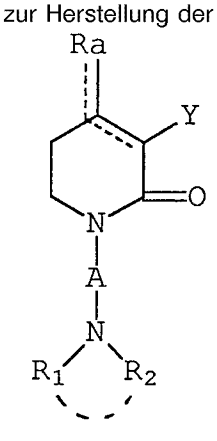

($I_A$)

in der die Gruppe

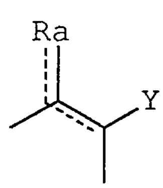

sowie A, $R_1$ und $R_2$ wie in Anspruch 1 definiert sind, dadurch gekennzeichnet, daß man das Diamin der Formel (II)

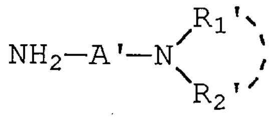

(II)

worin A' und

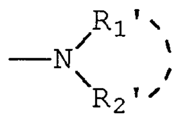

die jeweils weiter oben für A und

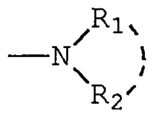

angegebenen Bedeutungen besitzen oder diejenigen, in denen die reaktiven Funktionen, die A und

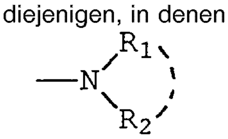

tragen können, geschützt sind,
mit dem Acrylester der Formel (III)

$CH_2 = CH - COOR_3$ (III)

zur Reaktion bringt, worin $R_3$ einen linearen oder verzweigten Alkylrest mit höchstens 6 Kohlenstoffatomen darstellt, um das kondensierte Produkt der Formel (IV)

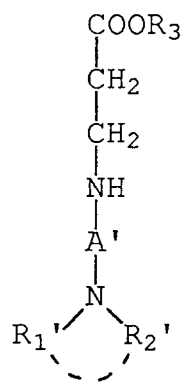

(IV)

zu erhalten, in der A',

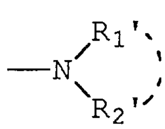

und $R_3$ die vorstehend angegebenen Bedeutungen besitzen, das man mit der Verbindung der Formel (V)

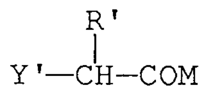

(V)

zur Reaktion bringt, in der R' ein Wasserstoffatom oder die oben angegebenen Werte von R oder diejenigen darstellt, in denen die reaktiven Funktionen geschützt sind, M einen Hydroxylrest oder ein Halogenatom bedeutet und Y' die oben angegebenen Werte von Y oder diejenigen darstellt, in denen die reaktiven Funktionen geschützt sind, um das Produkt der Formel (VI)

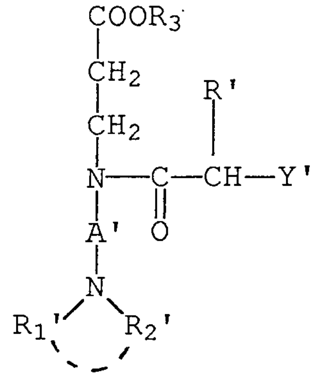

(VI)

zu erhalten, in der A',

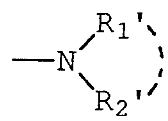

R', $R_3$ und Y' die vorstehend angegebenen Bedeutungen besitzen, das man einer Cyclisierungsreaktion unterzieht, um das Produkt der Formel (VII)

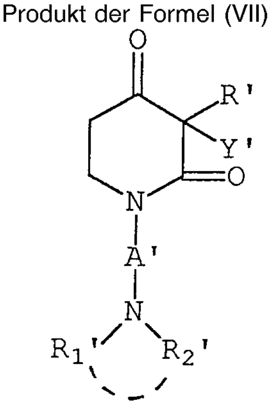

(VII)

zu erhalten, in der A',

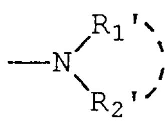

R' und Y' die vorstehend angegebenen Bedeutungen besitzen, die in dem Fall, wo R' ein Wasserstoffatom darstellt oder die Werte von R und A', $NR_1'R_2'$ und Y' jeweils die Werte von A, $NR_1R_2$ und Y haben, einem wie vorstehend definierten Produkt der Formel ($I_A$) entsprechen, ($I_{A1}$) genannt, wobei man gegebenenfalls bei dem Produkt der Formel (VII) die Schutzgruppe(n) entfernt, um ein gleiches Produkt der Formel ($I_{A1}$) zu erhalten, und man anschließend gegebenenfalls

- entweder, wenn R' oder R ein Wasserstoffatom darstellen, das genannte Produkt der Formel (VII) oder ($I_{A1}$);
  -- entweder einer Reduktions-Reaktion der Oxo-Funktion in gamma-Position zum Stickstoffatom unterzieht, um das Produkt der Formel (VIII)

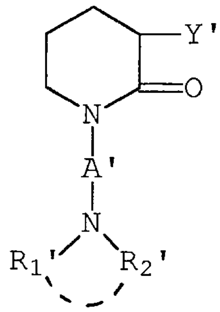

(VIII)

zu erhalten, in der A',

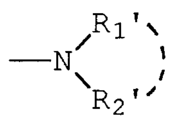

und Y' die vorstehend angegebenen Bedeutungen besitzen, die in dem Fall, wo Y', A' und $NR_1'R_2'$ jeweils die Werte von Y, A und $NR_1R_2$ haben, einem wie vorstehend definierten Produkt der Formel ($I_A$) entsprechen, ($I_{A2}$) genannt, wobei man gegebenenfalls bei dem Produkt der Formel (VIII) die Schutzgruppen entfernt, um ein gleiches Produkt der Formel ($I_{A2}$) zu erhalten,
-- oder einer Reduktions-Reaktion durch katalytische Hydrierung der gleichen Oxo-Funktion, um das Produkt der Formel (IX)

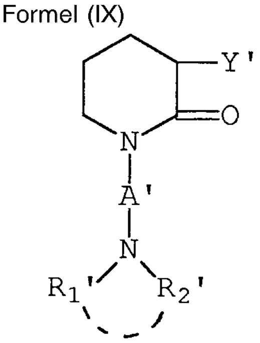

(IX)

zu erhalten, in der A',

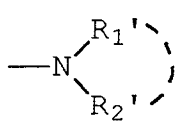

und Y' die vorstehend angegebenen Bedeutungen besitzen, die in dem Fall, wo Y', A' und $NR_1'R_2'$ jeweils die Werte von Y, A und $NR_1R_2$ haben, einem wie vorstehend definierten Produkt der Formel ($I_A$) entsprechen, ($I_{A3}$) genannt, wobei man gegebenenfalls bei dem Produkt der Formel (IX) die Schutzgruppen entfernt, um ein gleiches Produkt der Formel ($I_{A3}$) zu erhalten, und man das Produkt der Formel (IX) oder ($I_{A3}$) gegebenenfalls mit einer starken Base behandelt, um das entsprechende Anion zu bilden, das man der Einwirkung eines Reaktanden unterzieht, der fähig ist, einen Rest R' einzubringen, wobei R' die vorstehend angegebenen Werte mit Ausnahme eines Wasserstoffatomes besitzt, um ein Produkt der Formel (X)

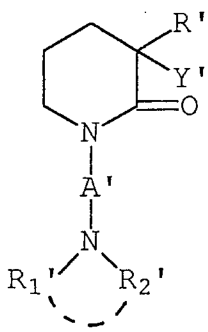

(X)

zu erhalten, in der R', Y', A' und

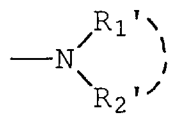

die vorstehend angegebenen Bedeutungen besitzen, die in dem Fall, wo R', Y', A' und $NR_1'R_2'$ jeweils die Werte von R (mit Ausnahme von Wasserstoff), Y, A und $NR_1R_2$ haben, einem wie vorstehend definierten Produkt der Formel ($I_A$) entsprechen, ($I_{A4}$) genannt, wobei man gegebenenfalls bei dem Produkt der Formel (X) die Schutzgruppen entfernt, um ein gleiches Produkt der Formel ($I_{A4}$) zu erhalten;

- oder, wenn R' oder R von einem Wasserstoffatom verschieden sind, das genannte Produkt der Formel (VII) oder ($I_{A1}$) der Einwirkung eines Reduktionsmittels für die Oxo-Funktion unterzieht, um das wie vorstehend definierte Produkt der Formel (X) zu erhalten, das entweder einem Produkt der Formel ($I_{A4}$) entspricht oder in dieses Produkt der Formel ($I_{A4}$) umgewandelt wird, und wenn gewünscht, die Produkte der Formel (I) mit einer Mineralsäure oder organischen Säure oder gegebenenfalls mit einer Base behandelt, um das entsprechende Salz zu erhalten, wobei die genannten Produkte der Formel (I) in allen möglichen isomeren, racemischen, enantiomeren und diastereoisomeren Formen vorliegen können.

**3.** Verfahren nach Anspruch 2 zur Herstellung der Produkte der Formel ($I_A$), in der die Gruppe

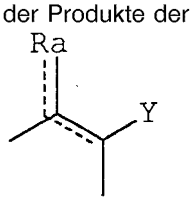

darstellt:

a) 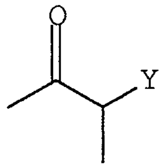 oder gegebenenfalls ihr Tautomer 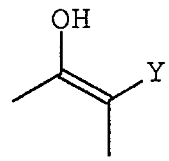

b) 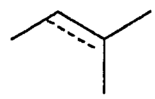 oder

c) 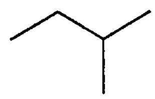

worin Y wie in Anspruch 1 definiert ist, dadurch gekennzeichnet, daß man eine Verbindung der Formel (V) verwendet, in der R' ein Wasserstoffatom darstellt.

**4.** Verfahren nach Anspruch 1 zur Herstellung der Produkte der Formel ($I_B$)

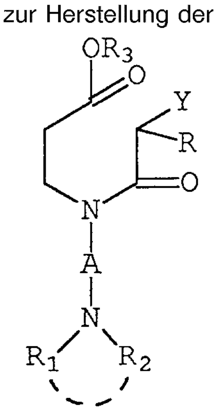

($I_B$)

worin $R_1$, $R_2$, $R_3$, A, R und Y wie in Anspruch 1 definiert sind, dadurch gekennzeichnet, daß man das Diamin der Formel (II)

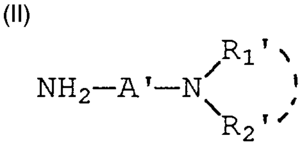

(II)

worin A' und

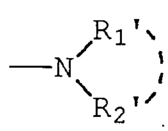

die jeweils weiter oben für A und

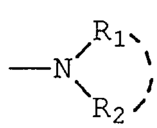

angegebenen Bedeutungen besitzen oder diejenigen, in denen die reaktiven Funktionen, die A und

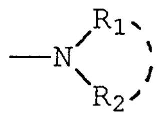

tragen können, geschützt sind,
mit dem Acrylester der Formel (III)

$CH_2 = CH - COOR_3$ (III)

zur Reaktion bringt, worin $R_3$ einen linearen oder verzweigten Alkylrest mit höchstens 6 Kohlenstoffatomen darstellt, um das kondensierte Produkt der Formel (IV)

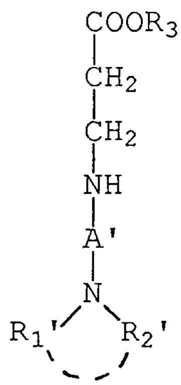

(IV)

zu erhalten, in der A',

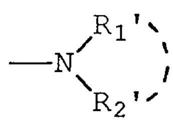

und $R_3$ die vorstehend angegebenen Bedeutungen besitzen, das man mit der Verbindung der Formel (V)

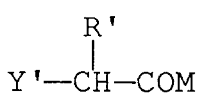

(V)

zur Reaktion bringt, in der R' ein Wasserstoffatom, die oben angegebenen Werte von R oder diejenigen darstellt, in denen die reaktiven Funktionen geschützt sind, M einen Hydroxylrest oder ein Halogenatom bedeutet und Y' die oben angegebenen Werte von Y oder diejenigen darstellt, in denen die reaktiven Funktionen geschützt sind, um das Produkt der Formel (VI)

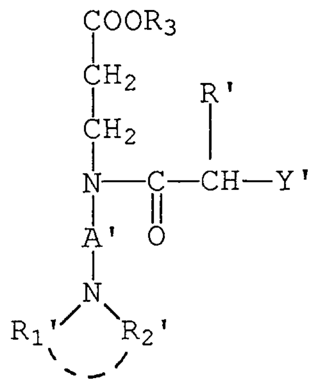

(VI)

zu erhalten, in der A',

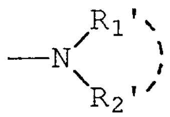

R', $R_3$ und Y' die vorstehend angegebenen Bedeutungen besitzen, die in dem Fall, wo R' ein Wasserstoffatom darstellt oder die Werte von R und A', $NR_1'R_2'$ und Y' jeweils die Werte von A, $NR_1R_2$ und Y haben, einem wie vorstehend definierten Produkt der Formel ($I_B$) entsprechen, wobei man gegebenenfalls bei dem Produkt der Formel (VI) die Schutzgruppe(n) entfernt, um ein gleiches Produkt der Formel ($I_B$) zu erhalten, und wenn gewünscht, die Produkte der Formel (I) mit einer Mineralsäure oder organischen Säure oder gegebenenfalls mit einer Base behandelt, um das entsprechende Salz zu erhalten, wobei die genannten Produkte der Formel ($I_B$) in allen möglichen isomeren, racemischen, enantiomeren und diastereoisomeren Formen vorliegen können.

5. Verfahren nach Anspruch 4 zur Herstellung der Produkte der Formel ($I_B$), in der R ein Wasserstoffatom darstellt, dadurch gekennzeichnet, daß man eine Verbindung der Formel (V) verwendet, in der R' ein Wasserstoffatom bedeutet.

6. Verfahren nach irgendeinem der Ansprüche 1 bis 5, dadurch gekennzeichnet, daß man von Verbindungen ausgeht, in denen der oder die gleichen oder verschiedenen Substituenten, die von den Arylresten, Arylalkylresten und heterocyclischen Resten oder von den von $R_1$ und $R_2$ zusammen mit dem Stickstoffatom, an das sie gebunden sind, gebildeten Resten getragen werden können, aus der Gruppe gewählt werden, die gebildet wird von:
   - den Halogenatomen,
   - den linearen oder verzweigten Alkyl-, Alkenyl-, Alkinyl- oder Alkyloxyresten mit höchstens 7 Kohlenstoffatomen, gegebenenfalls substituiert,
   - den Resten Hydroxy, Trifluormethyl, Trifluormethylthio, Trifluormethoxy, Cyano, Nitro, Sulfamoyl, Amino, Monoalkyl- und Dialkylamino,
   - den Resten Formyl, Acyl mit 2 bis 6 Kohlenstoffatomen und Benzoyl,
   - den freien, durch einen linearen oder verzweigten Alkylrest mit höchstens 5 Kohlenstoffatomen veresterten und in Salz überführten Carboxyresten,
   - dem Carbamoylrest, gegebenenfalls substituiert durch einen oder mehrere gleiche oder verschiedene Reste, gewählt aus der Gruppe, die gebildet wird von den Resten Alkyl, Alkoxy oder Hydroxyalkyl und dem Carbamoylalkylrest, wobei diese Alkyl- und Alkoxyreste 1 bis 4 Kohlenstoffatome umfassen,
   - und dem Phenyl- oder Benzylrest, gegebenenfalls selbst substituiert durch einen oder mehrere gleiche oder verschiedene Reste, gewählt aus der Gruppe, die gebildet wird von den Halogenatomen, den Resten Hydroxy, Cyano, Nitro, den linearen oder verzweigten Resten Alkyl, Alkenyl, Alkinyl oder Alkoxy mit höchstens 5 Kohlenstoffatomen.

7. Verfahren nach irgendeinem der Ansprüche 1 bis 5, dadurch gekennzeichnet, daß man von Verbindungen ausgeht, in denen der oder die gleichen oder verschiedenen Substituenten, die von den Alkylresten, Alkenylresten, Alkinylresten, Cycloalkylresten, Alkoxyresten und carbocyclischen Resten getragen werden können, aus der Gruppe gewählt werden, die gebildet wird von:
- den Halogenatomen,
- den Alkyl-, Alkenyl-, Alkinyl- oder Alkoxyresten mit höchstens 5 Kohlenstoffatomen,
- den Resten Formyl, Acyl mit 2 bis 6 Kohlenstoffatomen und Benzoyl,
- den freien, durch einen linearen oder verzweigten Alkylrest mit höchstens 5 Kohlenstoffatomen veresterten und in Salz überführten Carboxyresten,
- den Resten Hydroxy, Cyano, Amino, Monoalkyl- und Dialkylamino,
- den Arylresten und Arylalkylresten mit 6 bis 14 Kohlenstoffatomen, wobei diese Reste gegebenenfalls durch einen oder mehrere gleiche oder verschiedene Reste substituiert sind, gewählt aus der Gruppe, die gebildet wird von den Halogenatomen, den Resten Hydroxy, Cyano, Nitro, den linearen oder verzweigten Resten Alkyl, Alkenyl, Alkinyl und Alkoxy mit höchstens 5 Kohlenstoffatomen.

**8.** Verfahren nach irgendeinem der Ansprüche 1, 2, 4 oder 6, dadurch gekennzeichnet, daß man von einer Verbindung der Formel (V) ausgeht, in der R' einen Alkyl-, Alkenyl- oder Alkinylrest darstellt, substituiert durch ein Phenyl oder ein substituiertes Phenyl, wobei die eventuellen reaktiven Funktionen gegebenenfalls geschützt sind.

**9.** Verfahren nach irgendeinem der Ansprüche 1 bis 8, dadurch gekennzeichnet, daß man von einer Verbindung der Formel (V) ausgeht, in der Y' einen der Reste Phenyl, Naphthyl, Thionaphthyl, Benzofuryl, Benzopyrrolyl oder Indenyl darstellt, gegebenenfalls substituiert durch einen oder mehrere Reste, gewählt aus der Gruppe, die gebildet wird von:
- den Halogenatomen,
- den linearen oder verzweigten Alkyl-, Alkenyl-, Alkinyl- oder Alkoxyresten mit höchstens 7 Kohlenstoffatomen und gegebenenfalls substituiert durch einen oder mehrere Reste, gewählt aus der Gruppe, die gebildet wird von dem Hydroxyrest und den linearen oder verzweigten Alkyl- und Alkoxyresten mit höchstens 5 Kohlenstoffatomen,
- den Acylresten mit 2 bis 6 Kohlenstoffatomen,
- den Resten Hydroxy, Trifluormethyl, Cyano, Nitro, Amino, Monoalkyl- und Dialkylamino mit 1 bis 5 Kohlenstoffatomen,
- und dem Phenylrest, gegebenenfalls selbst substituiert durch einen oder mehrere gleiche oder verschiedene Reste, gewählt aus der Gruppe, die gebildet wird von den Halogenatomen, den Resten Hydroxy, Cyano, Nitro, den linearen oder verzweigten Resten Alkyl, Alkenyl, Alkinyl und Alkoxy mit höchstens 5 Kohlenstoffatomen, wobei die eventuellen reaktiven Funktionen gegebenenfalls geschützt sind.

**10.** Verfahren nach irgendeinem der Ansprüche 1 bis 9, dadurch gekennzeichnet, daß man von einer Verbindung der Formel (II) ausgeht, in der A darstellt:
- entweder einen Cycloalkylrest der Formel

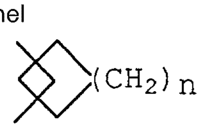

worin n die ganzzahligen Werte von 0 bis 4 annehmen kann, gegebenenfalls substituiert durch einen linearen oder verzweigten Alkyl- oder Alkoxyrest mit höchstens 5 Kohlenstoffatomen,
- oder die Gruppe

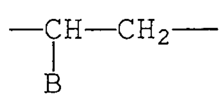

worin B einen Phenylrest darstellt, gegebenenfalls substituiert durch einen oder mehrere gleiche oder verschiedene Reste, gewählt aus der Gruppe, die gebildet wird von:
- den Halogenatomen,
- den Resten Hydroxy, Cyano, Nitro, den linearen oder verzweigten Resten Alkyl, Alkenyl, Alkinyl und Alkoxy mit höchstens 5 Kohlenstoffatomen, wobei die eventuellen reaktiven Funktionen

gegebenenfalls geschützt sind.

11. Verfahren nach irgendeinem der Ansprüche 1 bis 10, dadurch gekennzeichnet, daß man von einer Verbindung der Formel (II) ausgeht, in der $R_1$' und $R_2$' zusammen mit dem Stickstoffatom, an das sie gebunden sind, einen Heterocyclus bilden, der ein zweites Stickstoffatom umfaßt, das selbst gegebenenfalls durch einen linearen oder verzweigten Alkyl- oder Alkoxyrest mit 1 bis 5 Kohlenstoffatomen substituiert ist.

12. Verfahren nach Anspruch 1, dadurch gekennzeichnet, daß die Ausgangsprodukte in der Weise gewählt werden, daß man irgendeines der Produkte der Formel (I) herstellt, deren Bezeichnungen folgen:
(1S)-3-(3,4-Dichlorphenyl)-1-[1-phenyl-2-(1-pyrrolidinyl)-ethyl]-2-piperidinon (Isomer B),
(1S)-3-(3,4-Dichlorphenyl)-1-[1-phenyl-2-(1-pyrrolidinyl)-ethyl]-2-piperidinon (Isomer A),
[trans,(±)]-3-(3,4-Dichlorphenyl)-1-[2-(1-pyrrolidinyl)-cyclohexyl]-2-piperidinon (Isomer A),
(1S)-3-(3,4-Dichlorphenyl)-1-{2-methyl-1-[(1-pyrrolidinyl)-methyl]-propyl}-2-piperidinon (Isomer A),
(1S)-3-(Benzo(b)thien-4-yl)-1-[1-phenyl-2-(1-pyrrolidinyl)-ethyl]-2-piperidinon (Isomer A),
(1S)-3-(3,4-Dichlorphenyl)-3-methyl-1-[1-phenyl-2-(1-pyrrolidinyl)-ethyl]-2-piperidinon (Isomer A),
(1S)-3-(3,4-Dichlorphenyl)-3-ethyl-1-[1-phenyl-2-(1-pyrrolidinyl)-ethyl]-2-piperidinon (Isomer A),
(1S)-3-(3,4-Dichlorphenyl)-1-[1-phenyl-2-(1-pyrrolidinyl)-ethyl]-3-(2-propenyl)-2-piperidinon (Isomer A),
und gegebenenfalls ihre Salze.

13. Verfahren zur Herstellung von pharmazeutischen Zusammensetzungen, dadurch gekennzeichnet, daß man als Wirkstoff mindestens eines der Produkte der Formel (I) wie in Anspruch 1 definiert oder mindestens eines seiner pharmazeutisch akzeptablen Salze in eine für diesen Zweck vorgesehene Form bringt.

14. Verfahren zur Herstellung von pharmazeutischen Zusammensetzungen, dadurch gekennzeichnet, daß man als Wirkstoff mindestens eines der Produkte der Formel ($I_A$) wie in Anspruch 3 definiert oder mindestens eines seiner pharmazeutisch akzeptablen Salze in eine für diesen Zweck vorgesehene Form bringt.

15. Verfahren zur Herstellung von pharmazeutischen Zusammensetzungen, dadurch gekennzeichnet, daß man als Wirkstoff mindestens eines der Produkte der Formel (I) wie in Anspruch 12 definiert oder mindestens eines seiner pharmazeutisch akzeptablen Salze in eine für diesen Zweck vorgesehene Form bringt.

16. Als industrielle Produkte die Verbindungen der Formeln (IV), (VI), (VII) , (VIII), (IX) und (X).